# EUROPÄISCHE PATENTSCHRIFT

(11) **EP 3 638 675 B1**
(45) Veröffentlichungstag und Bekanntmachung des Hinweises auf die Patenterteilung: **17.04.2024**
(21) Anmeldenummer: 18728646.3
(22) Anmeldetag: 07.06.2018
(51) Int. Cl.: C07D 471/08, C07D 498/08, A61K 31/4995, A61K 31/5386, A61P 11/00, A61P 25/00

(54) **DIAZABICYCLISCH SUBSTITUIERTE IMIDAZOPYRIMIDINE UND IHRE VERWENDUNG ZUR BEHANDLUNG VON ATEMSTÖRUNGEN**
DIAZABICYCLIC SUBSTITUTED IMIDAZOPYRIMIDINES AND THEIR USE FOR THE TREATMENT OF BREATHING DISORDERS
IMIDAZOPYRIMIDINES À SUBSTITUTION DIAZABICYCLIQUE ET LEUR UTILISATION POUR TRAITER DES MALADIES DES VOIES RESPIRATOIRES

(30) Priorität: 14.06.2017 EP 17176046; 26.09.2017 EP 17193252
(43) Veröffentlichungstag der Anmeldung: 22.04.2020
(73) Patentinhaber: Bayer Aktiengesellschaft, 51373 Leverkusen (DE); Bayer Pharma Aktiengesellschaft, 13353 Berlin (DE)
(72) Erfinder: DELBECK, Martina, 42579 Heiligenhaus (DE); HAHN, Michael, 40764 Langenfeld (DE); MÜLLER, Thomas, 40764 Langenfeld (DE); LUSTIG, Klemens, 42113 Wuppertal (DE); COLLINS, Karl, 40215 Düsseldorf (DE); LINDNER, Niels, 42115 Wuppertal (DE); NICOLAI, Janine, 45239 Essen (DE); BECK-BROICHSITTER, Moritz, 64291 Darmstadt (DE); ALBUS, Udo, 61197 Florstadt (DE); GEHRING, Doris, 65779 Kelkheim (DE); ROSENSTEIN, Björn, 63628 Bad Soden-Salmünster (DE)
(74) Vertreter: BIP Patents
(86) Internationale Anmeldenummer: PCT/EP2018/064977
(87) Internationale Veröffentlichungsnummer: WO 2018/228907

(56) Entgegenhaltungen:
- WO-A1-2013/037914
- WO-A1-2014/187922
- WO-A1-2016/085783

## Beschreibung

Die vorliegende Anmeldung betrifft neue, diazabicyclisch substituierte Imidazo[1,2-a]pyrimidin-Derivate, Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von Atemstörungen, einschließlich schlafbedingter Atemstörungen wie obstruktiven und zentralen Schlafapnoen und Schnarchen.

Kaliumkanäle sind nahezu ubiquitär vorkommende Membranproteine, die an einer Vielzahl von unterschiedlichen physiologischen Prozessen beteiligt sind. Dazu gehört auch die Regulation des Membranpotentials und der elektrischen Erregbarkeit von Neuronen und Muskelzellen. Kaliumkanäle werden in drei größere Gruppen unterteilt, welche sich in der Zahl der Transmembrandomänen (2, 4 oder 6) unterscheiden. Die Gruppe von Kaliumkanälen, bei der zwei porenbildende Domänen von vier Transmembrandomänen flankiert werden, wird K2P-Kanäle genannt. Funktionell vermitteln die K2P-Kanäle weitgehend zeit- und spannungsunabhängig K⁺-Hintergrundströme und tragen entscheidend zur Aufrechterhaltung des Ruhemembranpotentials bei. Die Familie der K2P-Kanäle umfasst 15 Mitglieder, die in sechs Subfamilien untergliedert sind, basierend auf Ähnlichkeiten in Sequenz, Struktur und Funktion: TWIK, TREK, TASK, TALK, THIK und TRESK.

Von besonderem Interesse sind TASK-1 (KCNK3 oder K2P3.1) und TASK-3 (KCNK9 oder K2P9.1) der TASK *(TWIK-related acid-sensitive K⁺ channel*)-Subfamilie. Diese Kanäle sind funktionell dadurch gekennzeichnet, dass durch sie bei Erhaltung der spannungsunabhängigen Kinetik sogenannte "Leck"- oder "Hintergrund"-Ströme fließen, wobei sie auf eine Vielzahl von physiologischen und pathologischen Einflüssen mit einer Zu- oder Abnahme der Aktivität reagieren. Charakteristisch für TASK-Kanäle ist die sensitive Reaktion auf eine Änderung des extrazellulären pH-Wertes: Die Kanäle werden bei saurem pH-Wert inhibiert und bei alkalischem pH-Wert aktiviert.

TASK-1 wird überwiegend im Zentralnervensystem und im kardiovaskulären System exprimiert. Eine relevante Expression von TASK-1 konnte im Gehirn, in Spinalganglien, in Motoneuronen des *Nervus hypoglossus* und *Nervus trigeminus*, in Herz, *Glomus caroticum*, Pulmonalarterie, Aorta, Lunge, Pankreas, Placenta, Uterus, Niere, Nebenniere, Dünndarm und Magen sowie auf T-Lymphozyten nachgewiesen werden. TASK-3 wird hauptsächlich im Zentralnervensystem exprimiert. Eine relevante Expression von TASK-3 konnte im Gehirn, in Motoneuronen des *Nervus hypoglossus* und *Nervus trigeminus* und in neuroepithelialen Zellen des *Glomus caroticum* und der Lunge sowie auf T-Lymphozyten nachgewiesen werden. Eine geringere Expression ist in Herz, Magen, Hodengewebe und Nebenniere zu finden.

TASK-1- und TASK-3-Kanäle spielen eine Rolle bei der Regulation der Atmung. Beide Kanäle werden in den respiratorischen Neuronen des Atemzentrums im Hirnstamm exprimiert, unter anderem in Neuronen, die den Atemrhythmus generieren (ventrale respiratorische Gruppe mit dem prä-Bötzinger-Komplex), und im noradrenergen *Locus caeruleus* sowie in serotonergen Neuronen der Raphe-Kerne. Aufgrund der pH-Abhängigkeit übernehmen die TASK-Kanäle hier die Funktion eines Sensors, der extrazelluläre pH-Wert-Änderungen in entsprechende zelluläre Signale übersetzt [Bayliss et al., Pflügers Arch. 467, 917-929 (2015)]. Auch im *Glomus caroticum*, einem peripheren Chemorezeptor, der pH, O₂- und CO₂-Gehalt des Blutes misst und Signale an das Atemzentrum im Hirnstamm übermittelt, um die Atmung zu regulieren, werden TASK-1 und TASK-3 exprimiert. Es wurde gezeigt, dass TASK-1 knock-out-Mäuse eine verringerte ventilatorische Reaktion (Anstieg der Atemfrequenz und des Atemzugvolumens) auf Hypoxie und normoxische Hyperkapnie aufweisen [Trapp et al., J. Neurosci. 28, 8844-8850 (2008)]. Des Weiteren wurden TASK-1- und TASK-3-Kanäle in Motoneuronen des *Nervus hypoglossus*, dem XII. Hirnnerv, nachgewiesen, der eine wichtige Funktion für die Offenhaltung der oberen Atemwege hat [Berg et al., J. Neurosci. 24, 6693-6702 (2004)].

In einem Schlafapnoe-Modell am anästhesierten Schwein führte die intranasale Gabe eines Kaliumkanal-Blockers, der im nanomolaren Bereich den TASK-1-Kanal blockiert, zu einer Hemmung der Kollapsibilität der pharyngealen Atemwegsmuskulatur und zu einer Sensibilisierung des negativen Druckreflexes der oberen Atemwege. Man vermutet, dass die intranasale Gabe des Kaliumkanal-Blockers Mechanorezeptoren in den oberen Atemwegen depolarisiert und über Aktivierung des negativen Druckreflexes zu einer verstärkten Aktivität der Muskulatur der oberen Atemwege führt, wodurch eine Stabilisierung der oberen Atemwege erfolgt und ein Kollaps verhindert wird. Über eine solche Stabilisierung der oberen Atemwege kann die TASK-Kanal-Blockade für obstruktive Schlafapnoe und auch Schnarchen von großer Bedeutung sein [Wirth et al., Sleep 36, 699-708 (2013); Kiper et al., Pflügers Arch. 467, 1081-1090 (2015)].

Die obstruktive Schlafapnoe (OSA) ist eine schlafbezogene Atemstörung, die gekennzeichnet ist durch wiederholte Episoden der Obstruktion der oberen Atemwege. Bei der Einatmung wird die Durchgängigkeit der oberen Atemwege durch das Zusammenspiel zweier Gegenkräfte gewährleistet. Die dilatierenden Effekte der Muskulatur der oberen Atemwege wirken dem das Lumen verengenden negativen intraluminaren Druck entgegen. Die aktive Kontraktion des Zwerchfells und der anderen Atemhilfsmuskeln erzeugt einen Unterdruck in den Atemwegen und stellt so die treibende Kraft für die Atmung dar. Die Stabilität der oberen Atemwege wird maßgeblich von der Koordination und Kontraktionseigenschaft der dilatierenden Muskeln der oberen Atemwege bestimmt.

Der *Musculus genioglossus* spielt bei der Pathogenese der obstruktiven Schlafapnoe eine entscheidende Rolle. Die Aktivität des *Musculus genioglossus* nimmt mit abnehmendem Druck im Pharynx im Sinne eines dilatierenden Kompensationsmechanismus zu. Innerviert vom *Nervus hypoglossus* zieht er die Zunge nach vorne und unten und erweitert auf diese Weise den pharyngealen Luftweg [Verse et al., Somnologie 3, 14-20 (1999)]. Die Anspannung der dilatierenden Muskeln der oberen Atemwege wird unter anderem über Mechanorezeptoren/Dehnungsrezeptoren im Nasen-Rachen-Raum moduliert [Bouillette et al., J. Appl. Physiol. Respir. Environ. Exerc. Physiol. 46, 772-779 (1979)]. Durch Lokalanästhesie des oberen Luftwegs kann bei Patienten mit schwerer Schlafapnoe im Schlaf eine zusätzliche Reduktion der Aktivität des *Musculus genioglossus* beobachtet werden [Berry et al., Am. J. Respir. Crit. Care Med. 156, 127-132 (1997)]. Patienten mit obstruktiver Schlafapnoe haben eine hohe Mortalität und Morbidität infolge von Herz-Kreislauf-Erkrankungen wie z.B. Bluthochdruck, Herzinfarkt und Schlaganfall [Vrints et al., Acta Clin. Belg. 68, 169-178 (2013)].

Bei der zentralen Schlafapnoe kommt es infolge einer gestörten Hirnfunktion bzw. einer gestörten Atemregulation zu episodischen Hemmungen des Atemantriebs. Zentral bedingte Atemstörungen führen zu mechanischen Atemstillständen, d.h. es findet während dieser Episoden keine Atmungsaktivität statt, sämtliche Atemmuskeln einschließlich Zwerchfell stehen vorübergehend still. Bei der zentralen Schlafapnoe liegt keine Obstruktion der oberen Atemwege vor.

Beim primären Schnarchen kommt es ebenfalls nicht zu einer Obstruktion der oberen Atemwege. Durch die Verengung der oberen Atemwege erhöht sich allerdings die Strömungsgeschwindigkeit der ein- und ausgeatmeten Luft. Dies bewirkt im Verbund mit der erschlafften Muskulatur, dass die weichen Gewebe des Mund- und Rachenraumes im Luftstrom flattern. Dieses leichte Vibrieren erzeugt dann die typischen Schnarchgeräusche.

Das obstruktive Schnarchen (*upper airway resistance syndrome*, *heavy snoring*, Hypopnoe-Syndrom) wird durch eine wiederkehrende partielle Obstruktion der oberen Atemwege im Schlaf verursacht. Hierdurch kommt es zu einer Erhöhung des Atemwegswiderstands und somit zu einem Anstieg der Atemarbeit mit erheblichen intrathorakalen Druckschwankungen. Die negative intrathorakale Druckentwicklung während der Inspiration kann dabei Werte erreichen, wie sie infolge einer kompletten Atemwegsobstruktion bei der obstruktiven Schlafapnoe auftreten. Die pathophysiologischen Auswirkungen auf Herz, Kreislauf und Schlafqualität entsprechen denen bei der obstruktiven Schlafapnoe. Die Pathogenese ist wie bei der obstruktiven Schlafapnoe in einem gestörten Reflexmechanismus der Pharynx-dilatierenden Muskeln im Schlaf während der Inspiration anzunehmen. Das obstruktive Schnarchen stellt häufig die Vorstufe für die obstruktive Schlafapnoe dar [Hollandt et al., HNO 48, 628-634 (2000)].

TASK-Kanäle scheinen darüber hinaus auch beim Zelltod von Neuronen eine Rolle zu spielen. Im Tiermodell der Myelin-Oligodendrozyten-Glykoprotein (MOG)-induzierten autoimmunen Enzephalomyelitis, einem Tiermodell für Multiple Sklerose, zeigten TASK-1 knock-out-Mäuse eine verminderte neuronale Degeneration. Die Hemmung von TASK-Kanälen scheint über eine Verhinderung der neuronalen Apoptose neuroprotektiv zu wirken und kann daher für die Behandlung von neurodegenerativen Erkrankungen von Interesse sein [Bittner et al., Brain 132, 2501-2516 (2009)].

Weiterhin wurde beschrieben, dass T-Lymphozyten TASK-1- und TASK-3-Kanäle exprimieren und die Hemmung dieser Kanäle zu einer verminderten Zytokinproduktion und Proliferation nach Stimulation der T-Lymphozyten führt. Die selektive Hemmung von TASK-Kanälen auf T-Lymphozyten verbesserte den Krankheitsverlauf in einem Tiermodell der Multiplen Sklerose. Die Blockade von TASK-Kanälen kann daher auch für die Behandlung von Autoimmunerkrankungen von Bedeutung sein [Meuth et al., J. Biol. Chem. 283, 14559-14579 (2008)].

TASK-1 und TASK-3 werden auch im Herzen exprimiert [Rinné et al., J. Mol. Cell. Cardiol. 81, 71-80 (2015)]. Da TASK-1 besonders stark im Reizleitungssystem und im Vorhof exprimiert wird, könnte dieser Kanal bei der Auslösung von Reizleitungsstörungen oder supraventrikulären Arrhythmien eine Rolle spielen. Im Herzen scheint TASK-1 zu einem Hintergrund-Strom beizutragen, der seinerseits zur Aufrechterhaltung des Ruhepotentials, zur Aktionspotentialdauer und zur Repolarisation beiträgt [Kim et al., Am. J. Physiol. 277, H1669-1678 (1999)]. Es wurde an Herzmuskelzellen vom Menschen gezeigt, dass die Blockade des TASK-1-Ionenstroms zu einer Verlängerung des Aktionspotentials führt [Limberg et al., Cell. Physiol. Biochem. 28, 613-624 (2011)]. Des Weiteren wurde bei TASK-1 knock-out-Mäusen eine verlängerte QT-Zeit nachgewiesen [Decher et al., Cell. Physiol. Biochem. 28, 77-86 (2011)]. Die Hemmung von TASK-Kanälen könnte somit für die Behandlung von Herzrhythmusstörungen, insbesondere Vorhofflimmern, von Bedeutung sein.

TASK-Kanäle scheinen auch bei bestimmten Gefäßen bei der Regulation des Gefäßtonus eine Rolle zu spielen. Eine relevante Expression von TASK-1 konnte in der glatten Muskulatur von Pulmonal- und Mesenterialarterien festgestellt werden. In Untersuchungen an glatten Muskelzellen aus humanen Pulmonalarterien wurde gezeigt, dass TASK-1 eine Rolle bei der Regulation des pulmonalen Gefäßtonus spielt. TASK-1 könnte an der hypoxischen und Azidose-induzierten pulmonalen Vasokonstriktion beteiligt sein [Tang et al., Am. J. Respir. Cell. Mol. Biol. 41, 476-483 (2009)].

In Glomerulosazellen der Nebennierenrinde spielt TASK-1 eine Rolle für die Kaliumleitfähigkeit [Czirjak et al., Mol. Endocrinol. 14, 863-874 (2000)].

TASK-Kanäle haben möglicherweise auch bei Apoptose und Tumorgenese eine wichtige Bedeutung. In Brustkrebs-, Kolonkrebs- und Lungenkrebs-Biopsien sowie beim metastasierenden Prostatakrebs und in Melanomzellen wurde gefunden, dass TASK-3 stark überexprimiert ist [Mu et al., Cancer Cell 3, 297-302 (2003); Kim et al., APMIS 112, 588-594 (2004); Pocsai et al., Cell. Mol. Life Sci. 63, 2364-2376 (2006)]. Eine Punktmutation am TASK-3-Kanal, welche die Kanalfunktion abschaltet, hebt gleichzeitig die tumorbildende Wirkung (Proliferation, Tumorwachstum, Apoptose-Resistenz) auf [Mu et al., Cancer Cell 3, 297-302 (2003)]. Die Überexpression von TASK-3 und TASK-1 in einer murinen Fibroblasten-Zelllinie (C8-Zellen) hemmt intrazelluläre Apoptosewege [Liu et al., Brain Res. 1031, 164-173 (2005)]. Die Blockade von TASK-Kanälen kann daher auch für die Behandlung verschiedener Krebserkrankungen von Bedeutung sein.

Die Aufgabe der vorliegenden Erfindung liegt somit in der Bereitstellung neuer Substanzen, die als potente und selektive Blocker von TASK-1- und TASK-3-Kanälen agieren und sich als solche insbesondere zur Behandlung und/oder Prävention von Atemstörungen, einschließlich schlafbedingter Atemstörungen wie obstruktiven und zentralen Schlafapnoen und Schnarchen, sowie anderer Erkrankungen eignen.

In US 2002/0022624-A1 werden verschiedene Azaindol-Derivate, darunter auch Imidazo[1,2-a]-pyridine, als Substanz P-Antagonisten zur Behandlung von ZNS-Erkrankungen beschrieben. Aus WO 02/02557-A2 und WO 2009/143156-A2 sind 2-Phenylimidazo[1,2-a]pyridin-Derivate bekannt, die sich als Modulatoren von GABA_{A}-Rezeptoren gleichfalls zur Behandlung von ZNS-Erkrankungen eignen. In WO 2011/113606-A1 und WO 2012/143796-A2 werden bicyclische Imidazol-Derivate offenbart, die für die Behandlung von bakteriellen Infektionen bzw. inflammatorischen Erkrankungen geeignet sind. Aus EP 2 671 582-A1 sind weitere bicyclische Imidazol-Derivate und ihre therapeutischen Anwendungsmöglichkeiten als Inhibitoren von T-Typ-Calciumkanälen bekannt. In WO 2012/130322-A1 werden 2,6-Diaryl-3-(piperazinomethyl)imidazo[1,2-a]-pyridin-Derivate beschrieben, die sich aufgrund ihrer HIF-1 inhibierenden Aktivität insbesondere zur Behandlung von inflammatorischen und hyperproliferativen Erkrankungen eignen. In WO 2014/187922-A1 werden verschiedene 2-Phenyl-3-(heterocyclomethyl)imidazo[1,2-a]pyridin- und -imidazo[1,2-a]pyrazin-Derivate offenbart, die als Inhibitoren von Glucose-Transportern (GLUT) für die Behandlung von inflammatorischen, proliferativen, metabolischen, neurologischen und/ oder Autoimmun-Erkrankungen eingesetzt werden können. Unter anderem in WO 2015/144605-A1 und WO 2017/050732-A1 werden acylierte bicyclische Amin-Verbindungen beschrieben, die als Inhibitoren von Autotaxin und der Lysophosphatidsäure-Produktion für die Behandlung verschiedener Erkrankungen geeignet sind. In WO 2016/084866-A1, WO 2016/085783-A1 und WO 2016/088813-A1 werden acylierte diazabicyclische Verbindungen offenbart, die infolge ihrer antagonistischen Wirkung auf Orexin-Rezeptoren zur Behandlung von neurodegenerativen, neurologischen und psychiatrischen Erkrankungen, mentalen Störungen und Ess- und Schlafstörungen, insbesondere von Schlaflosigkeit, verwendet werden können. WO 2013/037914 A1 beschreibt 4,5,6,7-tetrahydro-IH-pyrazolo[4,3-c]pyridine als TASK-Inhibitoren zur Behandlung von schlafbedingten Atemstörungen.

Weiterhin ist die Verbindung Ethyl-4-[(2-phenylimidazo[1,2-a]pyrimidin-3-yl)methyl]piperazin-1-carboxylat [CAS Registry-Nr. 1783141-19-4] von *Chemical Abstracts* als "Chemical Library"-Substanz ohne Literaturreferenz indexiert; eine medizinisch-therapeutische Anwendung ist für diese Verbindung bislang nicht beschrieben.

Gegenstand der vorliegenden Erfindung sind Verbindungen der allgemeinen Formel (I) 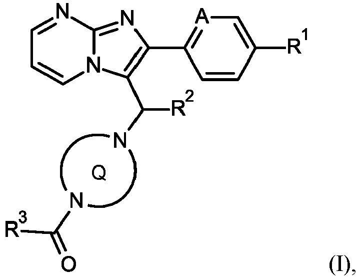 in welcher
der Ring Q für einen Diaza-Heterobicyclus der Formel 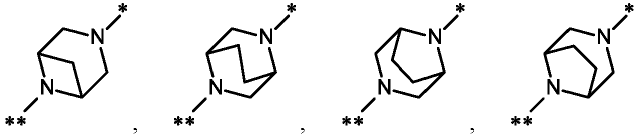 oder 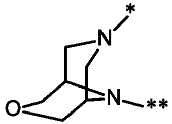 steht,
worin * die Verknüpfung zur angrenzenden CHR²-Gruppe und ** die Verknüpfung zur Carbonyl-Gruppe kennzeichnet,
- A: für CH oder N steht,
- R¹: für Halogen, Cyano, (C₁-C₄)-Alkyl, Cyclopropyl oder Cyclobutyl steht, wobei (C₁-C₄)-Alkyl bis zu dreifach mit Fluor substituiert sein kann und Cyclopropyl und Cyclobutyl bis zu zweifach mit Fluor substituiert sein können,
- R²: für Wasserstoff oder Methyl steht,
und
- R³: für (C₄-C₆)-Cycloalkyl, worin eine Ring-CH₂-Gruppe gegen -O- ausgetauscht sein kann, steht
oder
- R³: für eine Phenyl-Gruppe der Formel (a), eine Pyridyl-Gruppe der Formel (b) oder (c) oder eine Azol-Gruppe der Formel (d), (e) oder (f) 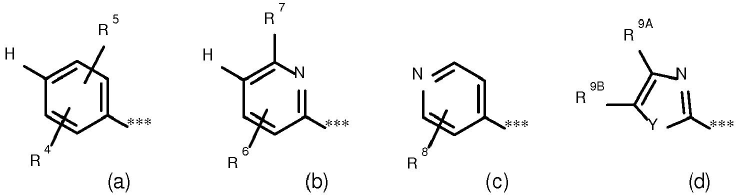 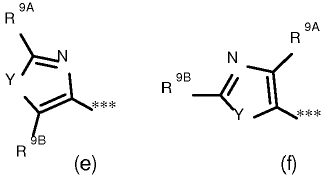 steht, worin *** die Verknüpfung zur angrenzenden Carbonyl-Gruppe markiert und
- R⁴: Wasserstoff, Fluor, Chlor, Brom oder Methyl bedeutet,
- R⁵: Wasserstoff, Fluor, Chlor, Brom, Cyano, (C₁-C₃)-Alkyl oder (C₁-C₃)-Alkoxy bedeutet, wobei (C₁-C₃)-Alkyl und (C₁-C₃)-Alkoxy jeweils bis zu dreifach mit Fluor substituiert sein können,
- R⁶: Wasserstoff, Fluor, Chlor, Brom oder Methyl bedeutet,
- R⁷: Wasserstoff, (C₁-C₃)-Alkoxy, Cyclobutyloxy, Oxetan-3-yloxy, Tetrahydrofuran-3-yloxy, Tetrahydro-2H-pyran-4-yloxy, Mono-(C₁-C₃)-alkylamino, Di-(C₁-C₃)-alkylamino oder (C₁-C₃)-Alkylsulfanyl bedeutet, wobei (C₁-C₃)-Alkoxy bis zu dreifach mit Fluor substituiert sein kann,
- R⁸: Wasserstoff, Fluor, Chlor, Brom, (C₁-C₃)-Alkyl oder (C₁-C₃)-Alkoxy bedeutet,
- R^{9A} und R^{9B}: gleich oder verschieden sind und unabhängig voneinander Wasserstoff, Fluor, Chlor, Brom, (C₁-C₃)-Alkyl, Cyclopropyl oder (C₁-C₃)-Alkoxy bedeuten, wobei (C₁-C₃)-Alkyl und (C₁-C₃)-Alkoxy jeweils bis zu dreifach mit Fluor substituiert sein können,
und
- Y: O oder S bedeutet,
oder

- R³: für eine Gruppe -OR¹⁰ oder -NR¹¹R¹² steht, worin

- R¹⁰: (C₁-C₆)-Alkyl, (C₄-C₆)-Cycloalkyl oder [(C₃-C₆)-Cycloalkyl]methyl bedeutet,
- R¹¹: Wasserstoff oder (C₁-C₃)-Alkyl bedeutet
und
- R¹²: (C₁-C₆)-Alkyl, (C₃-C₆)-Cycloalkyl, Phenyl oder Benzyl bedeutet, wobei (C₁-C₆)-Alkyl bis zu dreifach mit Fluor substituiert sein kann und wobei Phenyl sowie die Phenyl-Gruppe in Benzyl bis zu dreifach, gleich oder verschieden, mit einem Rest ausgewählt aus der Reihe Fluor, Chlor, Methyl, Ethyl, Trifluormethyl, Methoxy, Ethoxy, Trifluormethoxy und (Trifluormethyl)sulfanyl substituiert sein können,
oder
- R¹¹ und R¹²: miteinander verknüpft sind und gemeinsam mit dem Stickstoffatom, an das sie gebunden sind, einen Pyrrolidin-, Piperidin-, Morpholin- oder Thiomorpholin-Ring bilden,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln (I-A), (I-B), (I-C), (I-D) und (I-E) und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Benzolsulfonsäure, Toluolsulfonsäure, Naphthalindisulfonsäure, Ameisensäure, Essigsäure, Trifluoressigsäure, Propionsäure, Bernsteinsäure, Fumarsäure, Maleinsäure, Milchsäure, Weinsäure, Äpfelsäure, Zitronensäure, Gluconsäure, Benzoesäure und Embonsäure.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren angewandt, insbesondere die HPLC-Chromatographie an chiralen bzw. achiralen Trennphasen. Im Falle von chiralen Aminen als Zwischen- oder Endprodukten kann alternativ auch eine Trennung über diastereomere Salze mit Hilfe enantiomerenreiner Carbonsäuren erfolgen.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise zu einer Verlängerung der Halbwertszeit im Körper oder zu einer Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem hierbei entsprechende isotopische Modifikationen der jeweiligen Reagentien und/oder Ausgangsverbindungen eingesetzt werden.

Im Rahmen der vorliegenden Erfindung haben die Substituenten und Reste, soweit nicht anders spezifiziert, die folgende Bedeutung:
(C₁-C₆)-Alkyl steht im Rahmen der Erfindung für einen geradkettigen oder verzweigten Alkylrest mit 1 bis 6 Kohlenstoffatomen. Beispielhaft seien genannt: Methyl, Ethyl, *n*-Propyl, Isopropyl, *n*-Butyl, Isobutyl, *sec*.-Butyl, *tert*.-Butyl, *n*-Pentyl, 2-Pentyl, 3-Pentyl, Neopentyl, *n*-Hexyl, 2-Hexyl und 3-Hexyl.
(C₁-C₄)-Alkyl steht im Rahmen der Erfindung für einen geradkettigen oder verzweigten Alkylrest mit 1 bis 4 Kohlenstoffatomen. Beispielhaft seien genannt: Methyl, Ethyl, *n*-Propyl, Isopropyl, *n*-Butyl, Isobutyl, *sec.*-Butyl und *tert*.-Butyl.
(C₁-C₃)-Alkyl steht im Rahmen der Erfindung für einen geradkettigen oder verzweigten Alkylrest mit 1 bis 3 Kohlenstoffatomen. Beispielhaft seien genannt: Methyl, Ethyl, *n*-Propyl und Isopropyl.
(C₁-C₃)-Alkoxy steht im Rahmen der Erfindung für einen geradkettigen oder verzweigten Alkoxyrest mit 1 bis 3 Kohlenstoffatomen. Beispielhaft seien genannt: Methoxy, Ethoxy, *n*-Propoxy und Isopropoxy.
Mono-(C₁-C₃)-alkylamino steht im Rahmen der Erfindung für eine Amino-Gruppe mit einem geradkettigen oder verzweigten Alkylsubstituenten, der 1 bis 3 Kohlenstoffatome aufweist. Beispielhaft seien genannt: Methylamino, Ethylamino, *n*-Propylamino und Isopropylamino.
Di-(C₁-C₃)-alkylamino steht im Rahmen der Erfindung für eine Amino-Gruppe mit zwei gleichen oder verschiedenen geradkettigen oder verzweigten Alkylsubstituenten, die jeweils 1 bis 3 Kohlenstoffatome aufweisen. Beispielhaft seien genannt: *N,N*-Dimethylamino, *N,N*-Diethylamino, *N-*Ethyl-*N*-methylamino, *N*-Methyl-*N*-*n*-propylamino, *N*-Isopropyl-*N*-methylamino, *N,N*-Di-*n*-propylamino, *N*-Isopropyl-*N-n*-propylamino und *N,N*-Diisopropylamino.
(C₁-C₃)-Alkylsulfanyl [auch als (C₁-C₃)-Alkylthio bezeichnet] steht im Rahmen der Erfindung für einen geradkettigen oder verzweigten Alkylrest mit 1 bis 3 Kohlenstoffatomen, der über ein S-Atom mit dem Rest des Moleküls verknüpft ist. Beispielhaft seien genannt: Methylsulfanyl, Ethylsulfanyl, *n*-Propylsulfanyl und Isopropylsulfanyl.
(C₃-C₆)-Cycloalkyl steht im Rahmen der Erfindung für eine monocyclische, gesättigte Cycloalkylgruppe mit 3 bis 6 Ring-Kohlenstoffatomen. Beispielhaft seien genannt: Cyclopropyl, Cyclobutyl, Cyclopentyl und Cyclohexyl.
(C₄-C₆)-Cycloalkyl steht im Rahmen der Erfindung für eine monocyclische, gesättigte Cycloalkylgruppe mit 4 bis 6 Kohlenstoffatomen. Beispielhaft seien genannt: Cyclobutyl, Cyclopentyl und Cyclohexyl.
Halogen schließt im Rahmen der Erfindung Fluor, Chlor, Brom und Iod ein. Bevorzugt sind Fluor, Chlor oder Brom.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei gleichen oder verschiedenen Substituenten ist bevorzugt. Besonders bevorzugt ist die Substitution mit einem Substituenten.

Bevorzugt im Rahmen der vorliegenden Erfindung sind Verbindungen der Formel (I), in welcher der Ring Q für einen Diaza-Heterobicyclus der Formel 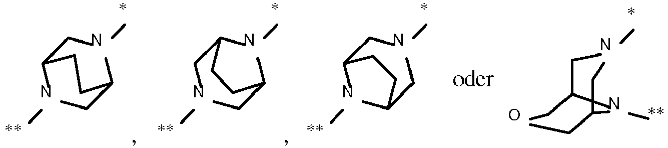 steht,
worin * die Verknüpfung zur angrenzenden CHR²-Gruppe und ** die Verknüpfung zur Carbonyl-Gruppe kennzeichnet,
- A: für CH steht,
- R¹: für Fluor, Chlor, Brom, Methyl, Isopropyl, tert.-Butyl, Cyclopropyl oder Cyclobutyl steht,
- R²: für Wasserstoff steht,
und
- R³: für Cyclobutyl, Cyclopentyl oder Cyclohexyl steht
oder
- R³: für eine Phenyl-Gruppe der Formel (a), eine Pyridyl-Gruppe der Formel (b) oder eine Azol-Gruppe der Formel (d), (e) oder (f) 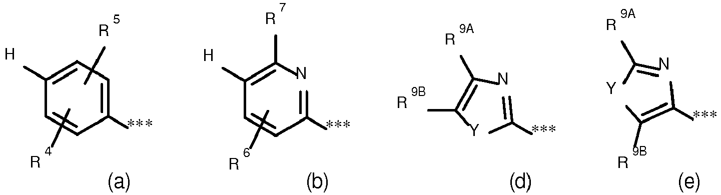 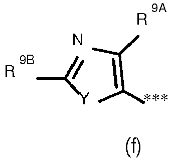 steht, worin *** die Verknüpfung zur angrenzenden Carbonyl-Gruppe markiert und
- R⁴: Wasserstoff, Fluor oder Chlor bedeutet,
- R⁵: Fluor, Chlor, Cyano, (C₁-C₃)-Alkyl, (C₁-C₃)-Alkoxy oder Trifluormethoxy bedeutet,
- R⁶: Wasserstoff, Fluor, Chlor, Brom oder Methyl bedeutet,
- R⁷: (C₁-C₃)-Alkoxy, Cyclobutyloxy oder (C₁-C₃)-Alkylsulfanyl bedeutet, wobei (C₁-C₃)-Alkoxy bis zu dreifach mit Fluor substituiert sein kann,
- R^{9A} und R^{9B}: gleich oder verschieden sind und unabhängig voneinander Wasserstoff, Chlor, Brom, (C₁-C₃)-Alkyl oder Cyclopropyl bedeuten, wobei (C₁-C₃)-Alkyl bis zu dreifach mit Fluor substituiert sein kann,
und
- Y: O oder S bedeutet,
sowie ihre Salze, Solvate und Solvate der Salze.

Eine besondere Ausführungsform der vorliegenden Erfindung betrifft Verbindungen der Formel (I), in welcher
der Ring Q für einen Diaza-Heterobicyclus der Formel 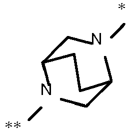 steht,
   worin * die Verknüpfung zur angrenzenden CHR²-Gruppe und ** die Verknüpfung zur Carbonyl-Gruppe kennzeichnet,
sowie ihre Salze, Solvate und Solvate der Salze.

Eine weitere besondere Ausführungsform der vorliegenden Erfindung betrifft Verbindungen der Formel (I), in welcher
der Ring Q für einen Diaza-Heterobicyclus der Formel 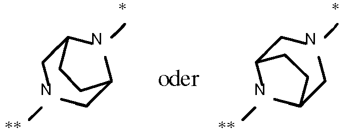 steht,
   worin * die Verknüpfung zur angrenzenden CHR²-Gruppe und ** die Verknüpfung zur Carbonyl-Gruppe kennzeichnet,
sowie ihre Salze, Solvate und Solvate der Salze.

Eine weitere besondere Ausführungsform der vorliegenden Erfindung betrifft Verbindungen der Formel (I), in welcher
der Ring Q für einen Diaza-Heterobicyclus der Formel 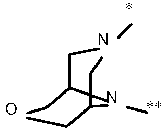 steht,
   worin * die Verknüpfung zur angrenzenden CHR²-Gruppe und ** die Verknüpfung zur Carbonyl-Gruppe kennzeichnet,
sowie ihre Salze, Solvate und Solvate der Salze.

Eine weitere besondere Ausführungsform der vorliegenden Erfindung betrifft Verbindungen der Formel (I), in welcher
- A: für CH steht,
sowie ihre Salze, Solvate und Solvate der Salze.

Eine weitere besondere Ausführungsform der vorliegenden Erfindung betrifft Verbindungen der Formel (I), in welcher
- R¹: für Chlor, Brom, Isopropyl oder Cyclopropyl steht,
sowie ihre Salze, Solvate und Solvate der Salze.

Eine weitere besondere Ausführungsform der vorliegenden Erfindung betrifft Verbindungen der Formel (I), in welcher
- R²: für Wasserstoff steht,
sowie ihre Salze, Solvate und Solvate der Salze.

Eine weitere besondere Ausführungsform der vorliegenden Erfindung betrifft Verbindungen der Formel (I), in welcher
- R³: für Cyclopentyl oder Cyclohexyl steht,
sowie ihre Salze, Solvate und Solvate der Salze.

Eine weitere besondere Ausführungsform der vorliegenden Erfindung betrifft Verbindungen der Formel (I), in welcher
- R³: für eine Phenyl-Gruppe der Formel (a) 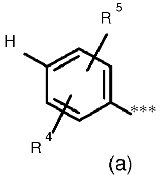 steht, worin *** die Verknüpfung zur angrenzenden Carbonyl-Gruppe markiert,
R⁴ Wasserstoff, Fluor oder Chlor bedeutet
und
R⁵ Fluor, Chlor, (C₁-C₃)-Alkyl oder (C₁-C₃)-Alkoxy bedeutet,
sowie ihre Salze, Solvate und Solvate der Salze.

Eine weitere besondere Ausführungsform der vorliegenden Erfindung betrifft Verbindungen der Formel (I), in welcher
- R³: für eine Pyridyl-Gruppe der Formel (b) 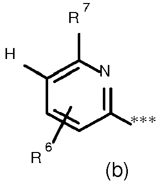 steht,
worin *** die Verknüpfung zur angrenzenden Carbonyl-Gruppe markiert,
R⁶ Wasserstoff, Fluor, Chlor, Brom oder Methyl bedeutet
und
R⁷ (C₁-C₃)-Alkoxy, Cyclobutyloxy oder (C₁-C₃)-Alkylsulfanyl bedeutet, wobei (C₁-C₃)-Alkoxy bis zu dreifach mit Fluor substituiert sein kann,
sowie ihre Salze, Solvate und Solvate der Salze.

Eine weitere besondere Ausführungsform der vorliegenden Erfindung betrifft Verbindungen der Formel (I), in welcher
- R³: für eine Azol-Gruppe der Formel (d), (e) oder (f) 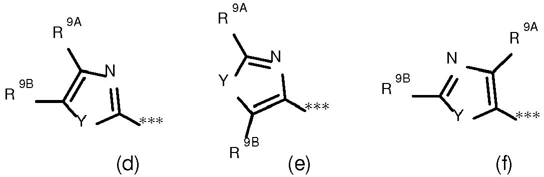 steht,
worin *** die Verknüpfung zur angrenzenden Carbonyl-Gruppe markiert,
R^{9A} und R^{9B} gleich oder verschieden sind und unabhängig voneinander Wasserstoff, Chlor, Brom, (C₁-C₃)-Alkyl oder Cyclopropyl bedeuten, wobei (C₁-C₃)-Alkyl bis zu dreifach mit Fluor substituiert sein kann,
und
Y O oder S bedeutet,
sowie ihre Salze, Solvate und Solvate der Salze.

Besonders bevorzugt im Rahmen der vorliegenden Erfindung sind Verbindungen der Formel (I), in welcher
der Ring Q für einen Diaza-Heterobicyclus der Formel 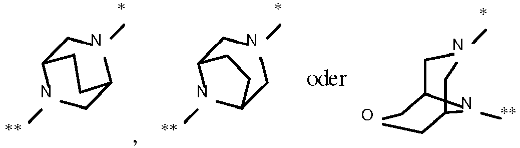 steht,
worin * die Verknüpfung zur angrenzenden CHR²-Gruppe und ** die Verknüpfung zur Carbonyl-Gruppe kennzeichnet,
- A: für CH steht,
- R¹: für Chlor, Brom, Isopropyl oder Cyclopropyl steht,
- R²: für Wasserstoff steht,
- und R³: für Cyclopentyl oder Cyclohexyl steht
- oder R³: für eine Phenyl-Gruppe der Formel (a), eine Pyridyl-Gruppe der Formel (b) oder eine Azol-Gruppe der Formel (d), (e) oder (f) 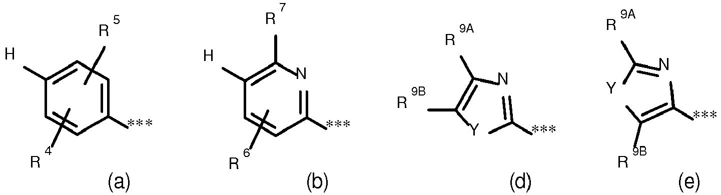 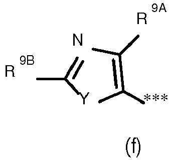 steht, worin *** die Verknüpfung zur angrenzenden Carbonyl-Gruppe markiert und
- R⁴: Wasserstoff, Fluor oder Chlor bedeutet,
- R⁵: Fluor, Chlor, Methyl, Isopropyl, Methoxy oder Ethoxy bedeutet,
- R⁶: Wasserstoff, Fluor, Chlor, Brom oder Methyl bedeutet,
- R⁷: Methoxy, Difluormethoxy, Trifluormethoxy, Isopropoxy, Cyclobutyloxy oder Methylsulfanyl bedeutet,
- R^{9A} und R^{9B}: gleich oder verschieden sind und unabhängig voneinander Wasserstoff, Methyl, Trifluormethyl, Ethyl, Isopropyl oder Cyclopropyl bedeuten
- und Y: O oder S bedeutet,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt. Besonders bevorzugt sind Kombinationen von zwei oder mehreren der oben genannten Vorzugsbereiche.

Weiterer Gegenstand der Erfindung ist ein Verfahren zur Herstellung von erfindungsgemäßen Verbindungen der Formel (I), in welcher der Rest R² für Wasserstoff steht, dadurch gekennzeichnet, dass man eine Verbindung der Formel (II) 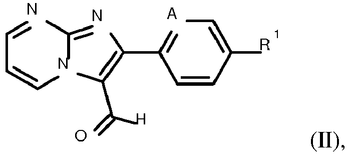
in welcher A und R¹ die oben angegebenen Bedeutungen haben,
in Gegenwart eines geeigneten Reduktionsmittels entweder

[A] mit einer Verbindung der Formel (III) 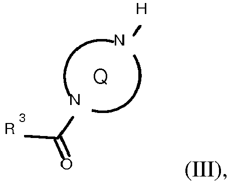
   in welcher R³ und der Ring Q die oben angegebenen Bedeutungen haben,
   zu einer Verbindung der Formel (I-A) 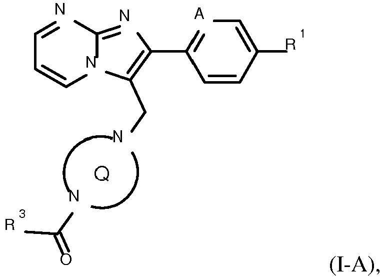
   in welcher A, R¹, R³ und der Ring Q die oben angegebenen Bedeutungen haben, umsetzt
      oder
[B] mit einem geschützten Diaza-Heterobicyclus der Formel (IV) 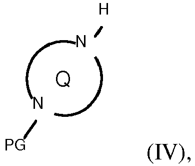
   in welcher der Ring Q die oben angegebene Bedeutung hat
      und
      - PG: für eine geeignete Amino-Schutzgruppe wie beispielsweise tert.-Butoxycarbonyl, Benzyloxycarbonyl oder (9H-Fluoren-9-ylmethoxy)carbonyl steht,
   zunächst zu einer Verbindung der Formel (V) 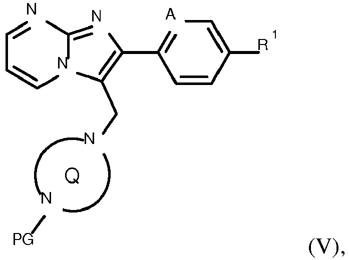
   in welcher A, PG, R¹ und der Ring Q die oben angegebenen Bedeutungen haben,
   umsetzt, anschließend die Schutzgruppe PG abspaltet und die resultierende Verbindung der Formel (VI) 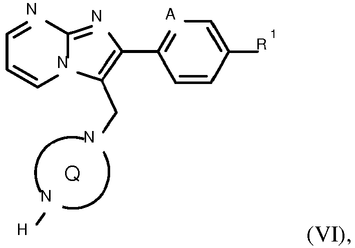
   in welcher A, R¹ und der Ring Q die oben angegebenen Bedeutungen haben,
   dann in Abhängigkeit von der spezifischen Bedeutung des Restes R³
   [B-1] mit einer Carbonsäure der Formel (VII) 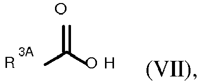 in welcher
      - R^{3A}: für (C₄-C₆)-Cycloalkyl, worin eine Ring-CH₂-Gruppe gegen -O- ausgetauscht sein kann, oder für eine Phenyl-Gruppe der Formel (a), eine Pyridyl-Gruppe der Formel (b) oder (c) oder eine Azol-Gruppe der Formel (d), (e) oder (f), wie oben beschrieben, steht,

      unter Aktivierung der Carbonsäure-Funktion in (VII) oder mit dem korrespondierenden Säurechlorid der Formel (VIII) 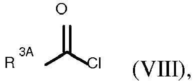
      in welcher R^{3A} die oben angegebene Bedeutung hat,
      zu einer Verbindung der Formel (I-B) 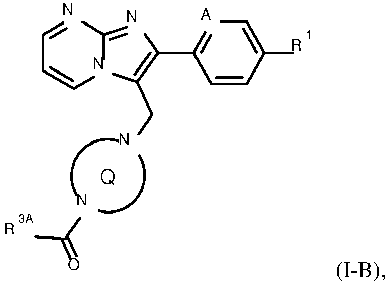
      in welcher A, R¹, R^{3A} und der Ring Q die oben angegebenen Bedeutungen haben,
      umsetzt
      oder
   [B-2] mit einem Chlorformiat beziehungsweise Carbamoylchlorid der Formel (IX) 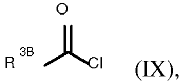 in welcher
      - R^{3B}: für die Gruppe -OR¹⁰ oder -NR^{11A}R¹² steht, worin
      R¹⁰ und R¹² die oben angegebenen Bedeutungen haben
      und
      R^{11A} die oben angegebene Bedeutung von R¹¹ hat, jedoch ungleich Wasserstoff ist,
      zu einer Verbindung der Formel (I-C) 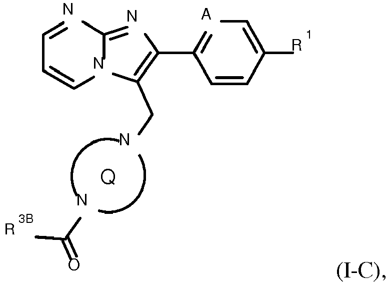 in welcher A, R¹, R^{3B} und der Ring Q die oben angegebenen Bedeutungen haben, umsetzt
      oder
   [B-3] mit einem Isocyanat der Formel (X) R¹²-N=C=O (X),
      in welcher R¹² die oben angegebene Bedeutung hat,
      zu einer Verbindung der Formel (I-D) 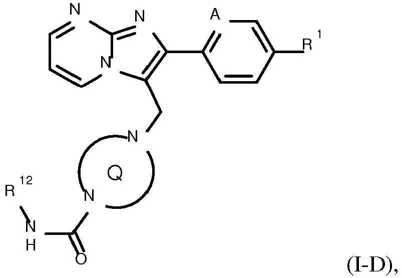
      in welcher A, R¹, R¹² und der Ring Q die oben angegebenen Bedeutungen haben,
      umsetzt
         und die so erhaltenen Verbindungen der Formeln (I-A), (I-B), (I-C) beziehungsweise (I-D) gegebenenfalls in ihre Enantiomere und/oder Diastereomere trennt und/oder gegebenenfalls mit den entsprechenden (*i*) Lösungsmitteln und/oder (*ii*) Säuren in ihre Solvate, Salze und/oder Solvate der Salze überführt.

Als Reduktionsmittel für die Verfahrensschritte [A] (II) + (III) -> (I-A) und [B] (II) + (IV) -> (V) [reduktive Aminierungen] eignen sich für solche Zwecke übliche Alkaliborhydride wie Natriumborhydrid, Natriumcyanoborhydrid oder Natriumtriacetoxyborhydrid; vorzugsweise wird Natriumtriacetoxyborhydrid eingesetzt. Der Zusatz einer Säure, wie insbesondere Essigsäure, und/oder eines wasserentziehenden Mittels, wie beispielsweise Molekularsieb oder Trimethyl- oder Triethylorthoformiat, kann bei diesen Reaktionen von Vorteil sein.

Als Lösungsmittel für diese Umsetzungen sind insbesondere Alkohole wie Methanol, Ethanol, *n*-Propanol oder Isopropanol, Ether wie Diisopropylether, Methyl-*tert*.-butylether, Tetrahydrofuran, 1,4-Dioxan oder 1,2-Dimethoxyethan, polar-aprotische Lösungsmittel wie Acetonitril oder *N,N*-Dimethylformamid (DMF) oder Gemische solcher Lösungsmittel geeignet; bevorzugt wird Tetrahydrofuran verwendet. Die Reaktionen erfolgen im Allgemeinen in einem Temperaturbereich von 0°C bis +50°C.

Als Schutzgruppe PG in Verbindung (IV) kann eine übliche Amino-Schutzgruppe wie beispielsweise *tert*.-Butoxycarbonyl (Boc), Benzyloxycarbonyl (Z) oder (9*H*-Fluoren-9-ylmethoxy)carbonyl (Fmoc) eingesetzt werden; bevorzugt wird *tert*.-Butoxycarbonyl (Boc) verwendet. Die Abspaltung der Schutzgruppe im Verfahrensschritt [B] (V) —> (VI) erfolgt nach bekannten Methoden. So wird die *tert*.-Butoxycarbonyl-Gruppe üblicherweise durch Behandlung mit einer starken Säure, wie Chlorwasserstoff, Bromwasserstoff oder Trifluoressigsäure, in einem inerten Lösungsmittel wie Diethylether, 1,4-Dioxan, Dichlormethan oder Essigsäure abgespalten. Im Falle von Benzyloxycarbonyl als Schutzgruppe wird diese bevorzugt durch Hydrogenolyse in Gegenwart eines geeigneten Palladium-Katalysators, wie beispielsweise Palladium auf Aktivkohle, entfernt. Die (9*H-*Fluoren-9-ylmethoxy)carbonyl-Gruppe wird im Allgemeinen mit Hilfe einer sekundären Aminbase wie Diethylamin oder Piperidin abgespalten [siehe z.B. T.W. Greene und P.G.M. Wuts, Protective Groups in Organic Synthesis, Wiley, New York, 1999; P.J. Kocienski, Protecting Groups, 3rd edition, Thieme, 2005].

Bestimmte Verbindungen der Formel (V), insbesondere solche, in denen PG für *tert*.-Butoxycarbonyl steht, weisen ebenfalls eine signifikante inhibitorische Aktivität gegenüber TASK-1 und/ oder TASK-3 auf und sind insoweit auch vom Bedeutungsumfang der vorliegenden Erfindung, d.h. den Verbindungen der Formel (I) umfasst.

Der Verfahrensschritt [B-1] (VI) + (VII) -> (I-B) [Amid-Bildung] wird nach bekannter Methodik mit Hilfe eines Kondensations- oder Aktivierungsmittels durchgeführt. Als solches Mittel eignen sich beispielsweise Carbodiimide wie *N,N*'-Diethyl-, *N,N*'-Dipropyl-, *N,N*'-Diisopropyl-, *N,N'-*Dicyclohexylcarbodiimid (DCC) oder N-(3-Dimethylaminopropyl)-*N*'-ethylcarbodiimid-Hydrochlorid (EDC), Phosgen-Derivate wie *N,N*'-Carbonyldiimidazol (CDI) oder Isobutylchlorformiat, 1,2-Oxazolium-Verbindungen wie 2-Ethyl-5-phenyl-1,2-oxazolium-3-sulfat oder 2-*tert*.-Butyl-5-methylisoxazolium-perchlorat, Acylamino-Verbindungen wie 2-Ethoxy-1-ethoxycarbonyl-1,2-di-hydrochinolin, α-Chlorenamine wie 1-Chlor-*N,N*,2-trimethylprop-1-en-1-amin, 1,3,5-Triazin-Derivate wie 4-(4,6-Dimethoxy-1,3,5-triazin-2-yl)-4-methylmorpholiniumchlorid, Phosphor-Verbindungen wie *n*-Propanphosphonsäureanhydrid (PPA), Cyanophosphonsäurediethylester, Diphenylphosphorylazid (DPPA), Bis-(2-oxo-3-oxazolidinyl)-phosphorylchlorid, Benzotriazol-1-yloxy-tris-(dimethylamino)phosphonium-hexafluorophosphat oder Benzotriazol-1-yloxy-tris(pyrrolidino)-phosphonium-hexafluorophosphat (PyBOP), oder Uronium-Verbindungen wie *O*-(Benzotriazol-1-yl)-*N,N,N',N*'-tetramethyluronium-tetrafluoroborat (TBTU), *O*-(1*H*-6-Chlorbenzotriazol-1-yl)-1,1,3,3-tetramethyluronium-tetrafluoroborat (TCTU), *O*-(Benzotriazol-1-yl)-*N,N,N',N*'-tetramethyl-uronium-hexafluorophosphat (HBTU), *O*-(7-Azabenzotriazol-1-yl)-*N*,*N*,*N*',*N*'-tetramethyluronium-hexafluorophosphat (HATU) oder 2-(2-Oxo-1-(2*H*)-pyridyl)-1,1,3,3-tetramethyluronium-tetrafluoroborat (TPTU), gegebenenfalls in Kombination mit weiteren Hilfsstoffen wie 1-Hydroxybenzotriazol (HOBt) oder N-Hydroxysuccinimid (HOSu), sowie als Base einem Alkalicarbonat, z.B. Natrium- oder Kaliumcarbonat, oder einer tertiären Aminbase, wie Triethylamin, *N,N*-Diisopropylethylamin, *N*-Methylmorpholin (NMM), N-Methylpiperidin (NMP), Pyridin oder 4-*N,N-*Dimethylaminopyridin (DMAP). Als Kondensations- oder Aktivierungsmittel bevorzugt eingesetzt wird *O*-(7-Azabenzotriazol-1-yl)-*N,N,N',N*'-tetramethyluronium-hexafluorophosphat (HATU) in Kombination mit N,N-Diisopropylethylamin als Base.

Das alternative Verfahren über das Carbonsäurechlorid (VIII) [(VI) + (VIII) —> (I-B)] erfolgt im Allgemeinen in Gegenwart einer Base wie beispielsweise Natriumcarbonat, Kaliumcarbonat, Triethylamin, *N,N*-Diisopropylethylamin, *N*-Methylmorpholin (NMM), *N*-Methylpiperidin (NMP), Pyridin, 2,6-Dimethylpyridin, 4-*N,N*-Dimethylaminopyridin (DMAP), 1,5-Diazabicyclo[4.3.0]non-5-en (DBN) oder 1,8-Diazabicyclo[5.4.0]undec-7-en (DBU); bevorzugt wird Triethylamin oder *N,N*-Diisopropylethylamin verwendet.

Geeignete inerte Lösungsmittel für diese Amidbildungsreaktionen sind beispielsweise Ether wie Diethylether, Diisopropylether, Methyl-*tert*.-butylether, Tetrahydrofuran, 1,4-Dioxan, 1,2-Dimethoxyethan oder Bis(2-methoxyethyl)ether, Kohlenwasserstoffe wie Benzol, Toluol, Xylol, Pentan, Hexan oder Cyclohexan, Halogenkohlenwasserstoffe wie Dichlormethan, Trichlormethan, Tetrachlormethan, 1,2-Dichlorethan, Trichlorethylen oder Chlorbenzol, oder polar-aprotische Lösungsmittel wie Aceton, Methylethylketon, Ethylacetat, Acetonitril, Butyronitril, Pyridin, Dimethylsulfoxid (DMSO), *N,N*-Dimethylformamid (DMF), *N,N*'-Dimethylpropylenharnstoff (DMPU) oder N-Methylpyrrolidinon (NMP); auch können Gemische solcher Lösungsmittel eingesetzt werden. Bevorzugt werden Dichlormethan, 1,2-Dichlorethan, Tetrahydrofuran, *N,N*-Dimethylformamid oder Gemische hiervon verwendet. Die Umsetzungen werden in der Regel in einem Temperaturbereich von -20°C bis +60°C, bevorzugt bei 0°C bis +40°C durchgeführt.

Das Verfahren [B-2] (VI) + (IX) —> (I-C) [Bildung von Urethanen bzw. substituierten Harnstoffen] wird unter ähnlichen Reaktionsbedingungen bezüglich Lösungsmittel, Basenzusatz und Temperatur durchgeführt, wie zuvor für die Amid-Bildung [B-1] (VI) + (VIII) —> (I-B) beschrieben.

Die Umsetzung [B-3] (VI) + (X) —> (I-D) erfolgt gleichfalls in einem der zuvor aufgeführten inerten Lösungsmittel oder Lösungsmittelgemische bei einer Temperatur im Bereich von 0°C bis +60°C; auf den Zusatz einer Base kann bei dieser Reaktion gegebenenfalls verzichtet werden.

Die Amin-Verbindung (VI) kann bei den Verfahrensschritten [B-1] (VI) + (VII) bzw. (VIII) → (I-B), [B-2] (VI) + (IX) → (I-C) und [B-3] (VI) + (X) → (I-D) auch in Form eines Salzes, beispielsweise als Hydrochlorid oder Trifluoracetat, eingesetzt werden. In einem solchen Fall erfolgt die Umsetzung in Gegenwart einer entsprechend erhöhten Menge der jeweils verwendeten Hilfsbase.

Erfindungsgemäße Verbindungen der Formel (I), in welcher der Rest R² für Methyl steht, können dadurch erhalten werden, dass man den oben bereits erwähnten Carbaldehyd der Formel (II) 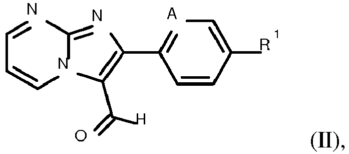
in welcher A und R¹ die oben angegebenen Bedeutungen haben,
zunächst mit Methylmagnesiumbromid zum sekundären Alkohol der Formel (XI) 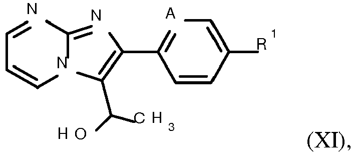
in welcher A und R¹ die oben angegebenen Bedeutungen haben,
umsetzt, diesen dann mit Hilfe von Triphenylphosphin und Tetrabromkohlenstoff in das korrespondierende Bromid der Formel (XII) 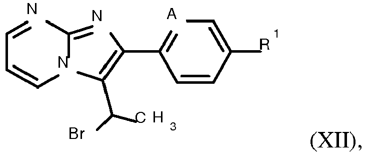
in welcher A und R¹ die oben angegebenen Bedeutungen haben,
überführt, anschließend mit einem geschützten Diaza-Heterobicyclus der Formel (IV) 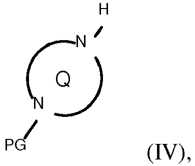
in welcher der Ring Q die oben angegebene Bedeutung hat
   und
   - PG: für eine geeignete Amino-Schutzgruppe wie beispielsweise *tert*.-Butoxycarbonyl, Benzyloxycarbonyl oder (9*H*-Fluoren-9-ylmethoxy)carbonyl steht,
zu einer Verbindung der Formel (XIII) 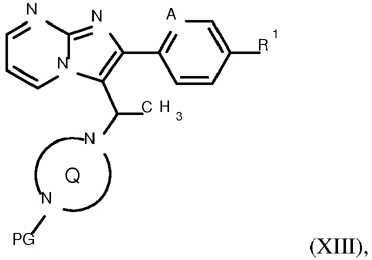
in welcher A, PG, R¹ und der Ring Q die oben angegebenen Bedeutungen haben,
umsetzt, nachfolgend die Schutzgruppe PG abspaltet und die resultierende Verbindung der Formel (XIV) 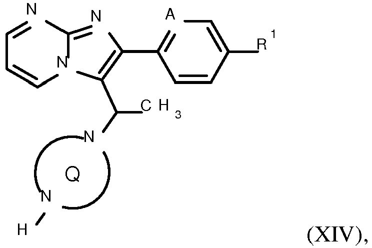
in welcher A, R¹ und der Ring Q die oben angegebenen Bedeutungen haben,
dann in Abhängigkeit von der spezifischen Bedeutung des Restes R³ gemäß einem der oben beschriebenen Verfahren [B-1], [B-2] bzw. [B-3] in die Zielverbindung der Formel (I-E) 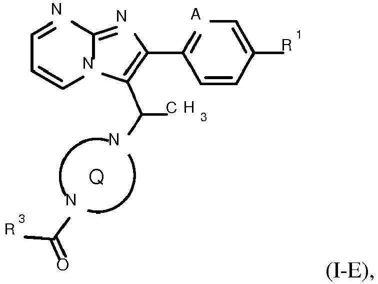
in welcher A, R¹, R³ und der Ring Q die oben angegebenen Bedeutungen haben,
überführt und letztere gegebenenfalls in ihre Enantiomere und/oder Diastereomere trennt und/oder gegebenenfalls mit den entsprechenden (*i*) Lösungsmitteln und/oder (*ii*) Säuren zu ihren Solvaten, Salzen und/oder Solvaten der Salze umsetzt.

Die Umsetzung des Carbaldehyds (II) mit Methylmagnesiumbromid zum sekundären Alkohol (XI) erfolgt typischerweise in einem etherischen Lösungsmittel wie Diethylether, Diisopropylether, Methyl-*tert*.-butylether, Tetrahydrofuran oder einem Gemisch hiervon in einem Temperaturbereich von -20°C bis +40°C. Die nachfolgende Überführung in das Bromid (XII) wird vorteilhaft unter milden Bedingungen mit Hilfe der Reagens-Kombination aus Triphenylphosphin und Tetrabromkohlenstoff in Gegenwart von Triethylamin als Base ("Appel-Reaktion") bewerkstelligt. Die Reaktion wird bevorzugt in Dichlormethan als inertem Lösungsmittel in einem Temperaturbereich von -10°C bis +30°C durchgeführt. Für die anschließende Umsetzung mit dem Diaza-Heterobicyclus (IV) wird das Bromid (XII) vorzugsweise nicht vorab isoliert, sondern direkt als Rohprodukt in einem Eintopf-Verfahren unter Wechsel des Lösungsmittels eingesetzt. Für diese Umsetzung (XII) + (IV) —> (XIII) wird bevorzugt Acetonitril als Lösungsmittel verwendet, und die Reaktion erfolgt im Allgemeinen in einem Temperaturbereich von +20°C bis +60°C.

Die Verfahrensschritte (XIII) —> (XIV) und (XIV) —> (I-E) schließlich werden auf analoge Weise durchgeführt wie zuvor für die Verfahren [B] (V) —> (VI) sowie [B-1], [B-2] bzw. [B-3] beschrieben.

Die zuvor beschriebenen Verfahren können bei normalem, bei erhöhtem oder bei erniedrigtem Druck durchgeführt werden (z.B. im Bereich von 0.5 bis 5 bar); im Allgemeinen arbeitet man jeweils bei Normaldruck.

Eine Trennung der erfindungsgemäßen Verbindungen in die entsprechenden Enantiomere und/ oder Diastereomere kann gegebenenfalls, je nach Zweckmäßigkeit, auch bereits auf der Stufe der Verbindungen (III), (IV), (V) oder (VI) bzw. (XI), (XIII) oder (XIV) erfolgen, welche dann in separierter Form gemäß den zuvor beschriebenen Verfahrensschritten weiter umgesetzt werden. Eine solche Auftrennung der Stereoisomeren läßt sich nach üblichen, dem Fachmann bekannten Methoden durchführen. Im Rahmen der vorliegenden Erfindung werden vorzugsweise chromatographische Verfahren an chiralen bzw. achiralen Trennphasen angewandt; im Falle von chiralen Aminen als Zwischen- oder Endprodukten kann alternativ auch eine Trennung über diastereomere Salze mit Hilfe enantiomerenreiner Carbonsäuren erfolgen.

Die Verbindungen der Formel (II) ihrerseits können nach literaturbekanntem Verfahren dadurch hergestellt werden, dass man 2-Aminopyrimidin (XV) 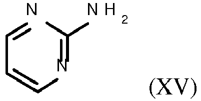
unter dem Einfluss einer Base mit einer Verbindung der Formel (XVI) 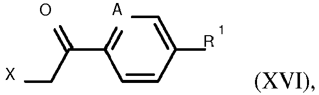
in welcher A und R¹ die oben angegebenen Bedeutungen haben
   und
   - X: für eine geeignete Fluchtgruppe wie beispielsweise Chlor, Brom oder Iod steht,
zu einem Imidazo[1,2-a]pyrimidin-Derivat der Formel (XVII) 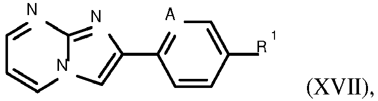
in welcher A und R¹ die oben angegebenen Bedeutungen haben,
kondensiert und dieses dann mit einer Mischung aus *N,N*-Dimethylformamid und Phosphoroxychlorid zu (II) formyliert.

Die Kondensationsreaktion (XV) + (XVI) —> (XVII) wird üblicherweise in einem alkoholischen Lösungsmittel wie Methanol, Ethanol, n-Propanol, Isopropanol oder n-Butanol, in einem Ether wie Diethylether, Diisopropylether, Methyl-tert.-butylether, Tetrahydrofuran, 1,4-Dioxan, 1,2-Dimethoxyethan oder Bis(2-methoxyethyl)ether, in einem dipolar-aprotischen Lösungsmittel wie *N,N*-Dimethylformamid (DMF), *N,N*'-Dimethylpropylenharnstoff (DMPU) oder *N*-Methylpyrrolidinon (NMP), oder auch in Wasser bei einer Temperatur im Bereich von +50°C bis +150°C durchgeführt; bevorzugt wird Ethanol oder Wasser als Lösungsmittel verwendet.

Für diese Reaktion geeignete Basen sind insbesondere Alkalihydrogencarbonate oder -carbonate wie Natrium- oder Kaliumhydrogencarbonat oder Lithium-, Natrium-, Kalium- oder Cäsiumcarbonat, Alkalihydroxide wie Natrium- oder Kaliumhydroxid, oder auch Aluminiumoxid; bevorzugt wird Natriumhydrogencarbonat oder Natriumhydroxid eingesetzt. Gegebenenfalls kann die Umsetzung - bei entsprechender Erhöhung der Reaktionstemperatur - auch ohne Zusatz einer Base erfolgen.

Die regioselektive Formylierung (XVII) —> (II) erfolgt unter den üblichen Bedingungen einer Vilsmaier-Haack-Reaktion durch Behandlung von (XVII) mit einer vorgebildeten Mischung aus *N,N-*Dimethylformamid und Phosphoroxychlorid, welche im großen Überschuss eingesetzt wird und gleichzeitig auch als Lösungsmittel dient. Die Umsetzung wird im Allgemeinen in einem Temperaturbereich von 0°C bis +100°C durchgeführt.

Die Verbindungen der Formeln (III), (IV), (VII), (VIII), (IX), (X), (XV) und (XVI) sind entweder kommerziell erhältlich oder als solche in der Literatur beschrieben, oder sie können, ausgehend von anderen kommerziell erhältlichen Verbindungen, auf einfache Weise nach dem Fachmann geläufigen, literaturbekannten Methoden hergestellt werden. Zahlreiche detaillierte Vorschriften und weitere Literaturangaben befinden sich auch im Experimentellen Teil im Abschnitt zur Herstellung der Ausgangsverbindungen und Intermediate.

Die Herstellung der erfindungsgemäßen Verbindungen kann durch die folgenden Reaktionsschemata beispielhaft veranschaulicht werden:

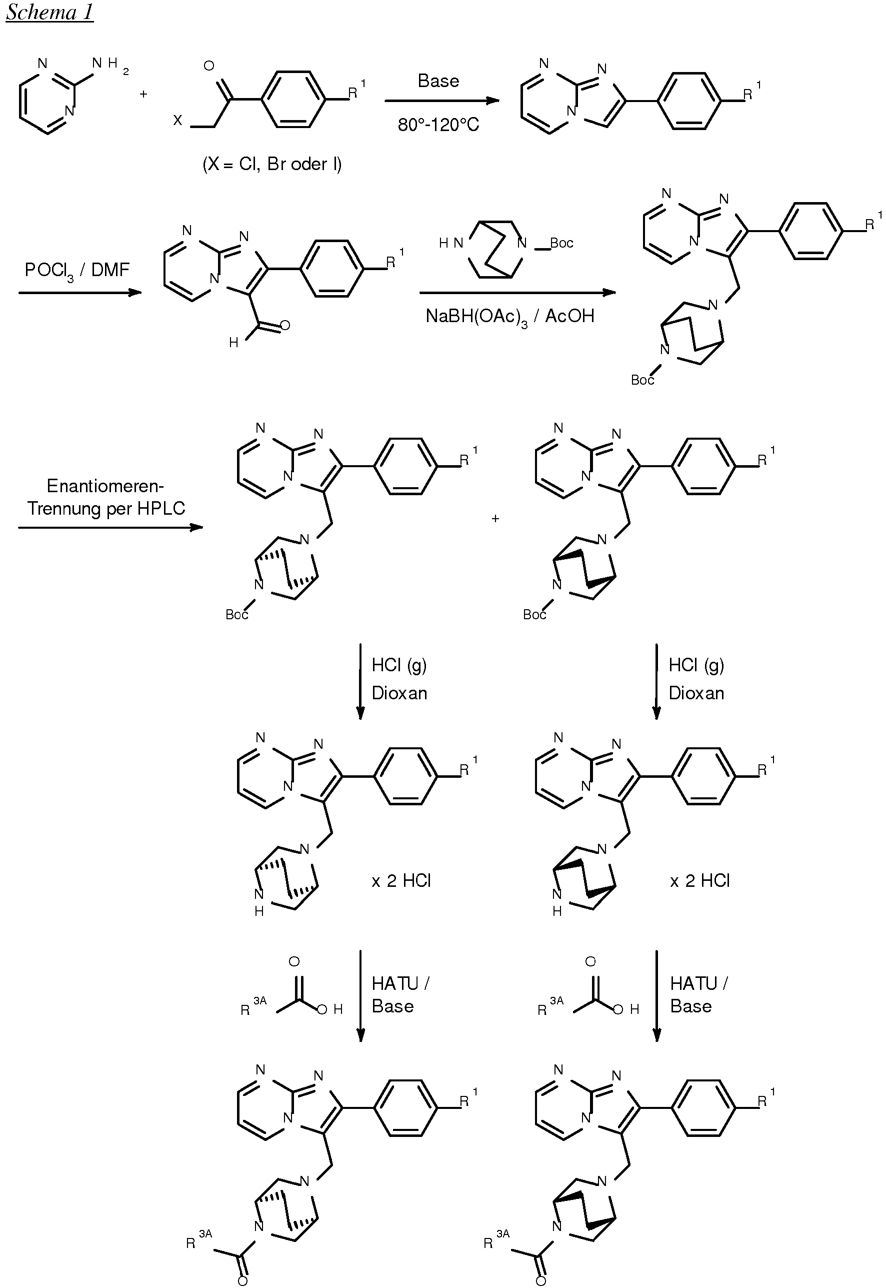

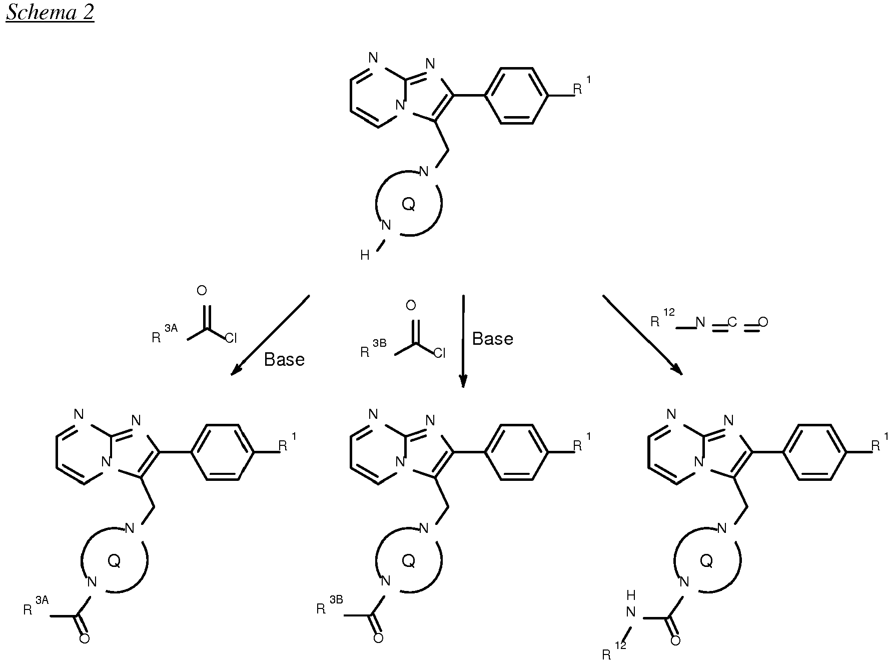

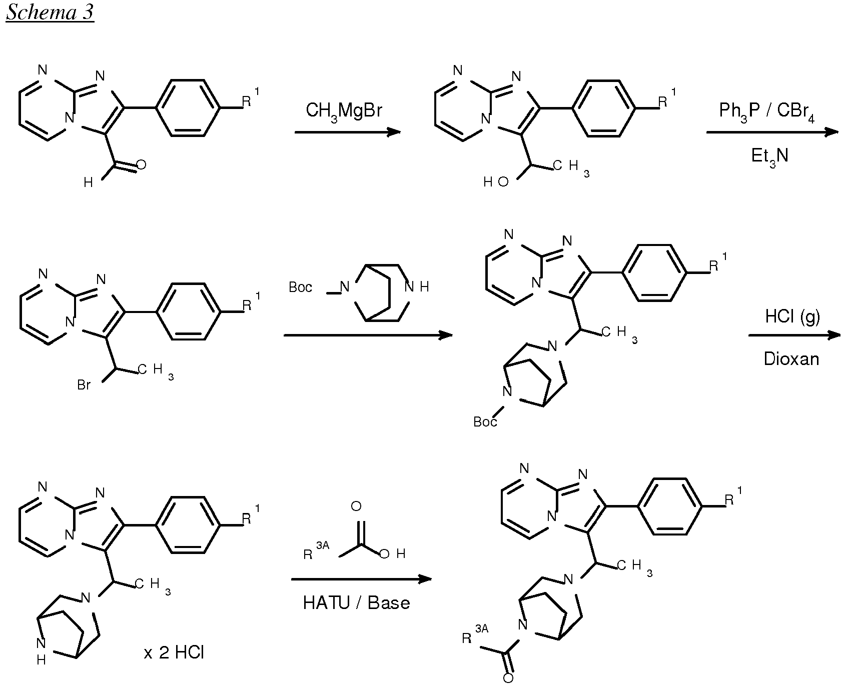

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen potente und selektive Blocker von TASK-1- und TASK-3-Kanälen dar und eignen sich daher zur Behandlung und/oder Prävention von Erkrankungen und pathologischen Prozessen, insbesondere solcher, die durch eine Aktivierung von TASK-1 und/oder TASK-3 oder durch aktiviertes TASK-1 und/oder TASK-3 hervorgerufen werden, sowie von Erkrankungen, die auf sekundärem Wege durch TASK-1- und/oder TASK-3-bedingte Schädigungen induziert werden.

Dazu zählen im Sinne der vorliegenden Erfindung insbesondere Erkrankungen aus der Gruppe der Atemstörungen und schlafbedingten Atemstörungen, wie die obstruktive Schlafapnoe (bei Erwachsenen und Kindern), primäres Schnarchen, obstruktives Schnarchen (*upper airway resistance syndrome*, *heavy snoring*, Hypopnoe-Syndrom), zentrale Schlafapnoe, gemischte Schlafapnoen, Cheyne-Stoke'sche Atmung, primäre Schlafapnoe in der Kindheit, Frühgeborenenapnoe, zentrale Schlafapnoe infolge Medikamenteneinnahme oder Gebrauch anderer Substanzen, Obesitas-Hypoventilationssyndrom, gestörter zentraler Atemantrieb, plötzlicher Kindstod, primäres alveoläres Hypoventilationssyndrom, postoperative Hypoxie und Apnoe, muskulär bedingte Atemstörungen, Atemstörungen nach Langzeitbeatmung, Atemstörungen bei Adaptation im Hochgebirge, akute und chronische Lungenkrankheiten mit Hypoxie und Hyperkapnie, schlafbezogene nicht-obstruktive alveoläre Hypoventilation und das kongenitale zentrale alveoläre Hypoventilationssyndrom.

Die erfindungsgemäßen Verbindungen können weiterhin verwendet werden zur Behandlung und/ oder Prävention von neurodegenerativen Erkrankungen, wie beispielsweise Demenz, Demenz mit Lewy-Körpern, Morbus Alzheimer, Morbus Parkinson, Morbus Huntington, Morbus Pick, Morbus Wilson, progressive supranukleäre Parese, kortikobasale Degeneration, Silberkornkrankheit, frontotemporale Demenz und Parkinsonismus des Chromosoms 17, Multisystematrophie, spinozerebelläre Ataxien, spinobulbäre Muskelatrophie Typ Kennedy, Friedreich-Ataxie, dentatorubropallidoluysische Atrophie, amyotrophe Lateralsklerose, primäre Lateralsklerose, spinale Muskelatrophie, Creutzfeldt-Jakob-Krankheit und Varianten der Creutzfeldt-Jakob-Krankheit, infantile neuroaxonale Dystrophie, Neurodegeneration mit Eisenablagerung im Gehirn, Frontotemporallappen-Degeneration mit Ubiquitin-Proteasom-System und die familiäre Enzephalopathie mit Neuroserpin-Einschlüssen.

Die erfindungsgemäßen Verbindungen können darüber hinaus zur Behandlung und/oder Prävention von neuroinflammatorischen und neuroimmunologischen Erkrankungen des zentralen Nervensystems (ZNS) eingesetzt werden, wie beispielsweise multiple Sklerose (Encephalomyelitis disseminata), transverse Myelitis, Neuromyelitis optica, akute disseminierte Enzephalomyelitis, Optikusneuritis, Meningitis, Enzephalitis, demyelinisierende Erkrankungen sowie entzündliche Gefäßveränderungen des zentralen Nervensystems.

Die erfindungsgemäßen Verbindungen sind zudem zur Behandlung und/oder Prävention von Krebserkrankungen geeignet, wie beispielsweise von Hautkrebs, Brustkrebs, Lungenkrebs, Kolonkrebs und Prostatakrebs.

Die erfindungsgemäßen Verbindungen eignen sich außerdem zur Behandlung und/oder Prävention von Herzrhythmusstörungen und Arrhythmien, wie beispielsweise Rhythmusstörungen der Vorhöfe und der Kammern, Überleitungsstörungen wie atrio-ventrikuläre Blockaden des Grades I-III, supraventrikuläre Tachyarrhythmie, Vorhofflimmern, Vorhofflattern, Kammerflimmern, Kammerflattern, ventrikuläre Tachyarrhythmie, Torsade de pointes-Tachykardie, Extrasystolen des Vorhofs und des Ventrikels, AV-junktionale Extrasystolen, Sick-Sinus-Syndrom, Synkopen und AV-Knoten-Reentry-Tachykardie.

Weitere kardiovaskuläre Erkrankungen, zu deren Behandlung und/oder Prävention die erfindungsgemäßen Verbindungen eingesetzt werden können, sind beispielsweise Herzinsuffizienz, koronare Herzerkrankung, stabile und instabile Angina pectoris, Bluthochdruck (Hypertonie), pulmonalarterielle Hypertonie (PAH) und andere Formen der pulmonalen Hypertonie (PH), renale Hypertonie, periphere und kardiale Gefäßerkrankungen, Wolff-Parkinson-White-Syndrom, akutes Koronarsyndrom (ACS), autoimmune Herzerkrankungen (Perikarditis, Endokarditis, Valvolitis, Aortitis, Kardiomyopathien), Boxerkardiomyopathie, Aneurysmen, Schock wie kardiogener Schock, septischer Schock und anaphylaktischer Schock, ferner thromboembolische Erkrankungen und Ischämien, wie myokardiale Ischämie, Myokardinfarkt, Hirnschlag, Herzhypertrophie, transistorische und ischämische Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, periphere Durchblutungsstörungen, Reperfusionsschäden, arterielle und venöse Thrombosen, Mikroalbuminurie, Herzmuskelschwäche, endotheliale Dysfunktion, mikro- und makrovaskuläre Schädigungen (Vaskulitis), sowie zur Verhinderung von Restenosen beispielsweise nach Thrombolyse-Therapien, percutan-transluminalen Angioplastien (PTA), percutan-transluminalen Koronarangioplastien (PTCA), Herztransplantationen und Bypass-Operationen.

Im Sinne der vorliegenden Erfindung umfasst der Begriff Herzinsuffizienz sowohl akute als auch chronische Erscheinungsformen der Herzinsuffizienz wie auch spezifische oder verwandte Krankheitsformen hiervon, wie akute dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz.

Die erfindungsgemäßen Verbindungen können weiterhin verwendet werden zur Behandlung und/ oder Prävention von asthmatischen Erkrankungen unterschiedlicher Schweregrade mit intermittierendem oder persistierendem Verlauf (refraktäres Asthma, bronchiales Asthma, allergisches Asthma, intrinsisches Asthma, extrinsisches Asthma, durch Medikamente oder durch Staub induziertes Asthma), von verschiedenen Formen der Bronchitis (chronische Bronchitis, infektiöse Bronchitis, eosinophile Bronchitis), von Bronchiektasien, Pneumonie, Farmerlunge und verwandten Krankheiten, Husten- und Erkältungskrankheiten (chronischer entzündlicher Husten, iatrogener Husten), Nasenschleimhautentzündungen (einschließlich medikamentöse Rhinitis, vasomotorische Rhinitis und jahreszeitabhängige, allergische Rhinitis, z.B. Heuschnupfen) und von Polypen.

Zudem eignen sich die erfindungsgemäßen Verbindungen zur Behandlung und/oder Prävention von Nierenerkrankungen, insbesondere von Niereninsuffizienz und Nierenversagen. Im Sinne der vorliegenden Erfindung umfassen die Begriffe Niereninsuffizienz und Nierenversagen sowohl akute als auch chronische Erscheinungsformen hiervon wie auch diesen zugrundeliegende oder verwandte Nierenerkrankungen, wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantat-Abstoßung und Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prävention von Folgeerscheinungen einer Niereninsuffizienz, wie beispielsweise Hypertonie, Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Darüber hinaus sind die erfindungsgemäßen Verbindungen zur Behandlung und/oder Prävention von Erkrankungen des Urogenitalsystems geeignet, wie beispielsweise benignes Prostata-Syndrom (BPS), benigne Prostatahyperplasie (BPH), benigne Prostatavergrößerung (BPE), Blasenentleerungsstörungen (BOO), untere Harnwegssyndrome (LUTS), neurogene überaktive Blase (OAB), Inkontinenz wie beispielsweise Misch-, Drang-, Stress- oder Überlauf-Inkontinenz (MUI, UUI, SUI, OUI), Beckenschmerzen sowie erektile Dysfunktion und weibliche sexuelle Dysfunktion.

Die erfindungsgemäßen Verbindungen sind ferner zur Behandlung und/oder Prävention von entzündlichen Erkrankungen und Autoimmunerkrankungen, wie zum Beispiel rheumatoiden Erkrankungen, entzündlichen Augenerkrankungen, chronisch-obstruktiver Lungenerkrankung (COPD), akutem Atemwegssyndrom (ARDS), akuter Lungenschädigung (ALI), alpha-1-Antitrypsin-Defizienz (AATD), Lungenemphysem (z.B. durch Zigarettenrauch induziertes Lungenemphysem), zystischer Fibrose (CF), Sepsis (SIRS), multiplem Organversagen (MODS, MOF), entzündlichen Erkrankungen der Niere, chronischen Darmentzündungen (IBD, Morbus Crohn, Colitis ulcerosa), Pankreatitis, Peritonitis, Cystitis, Urethritis, Prostatitis, Epidimytitis, Oophoritis, Salpingitis und Vulvovaginitis, sowie zur Behandlung und/oder Prävention von fibrotischen Erkrankungen der inneren Organe, wie beispielsweise der Lunge, des Herzens, der Niere, des Knochenmarks und insbesondere der Leber, von dermatologischen Fibrosen und von fibrotischen Erkrankungen des Auges geeignet. Im Sinne der vorliegenden Erfindung umfasst der Begriff fibrotische Erkrankungen insbesondere solche Erkrankungen wie Leberfibrose, Leberzirrhose, Lungenfibrose, Endomyokardfibrose, Nephropathie, Glomerulonephritis, interstitielle Nierenfibrose, fibrotische Schäden in Folge von Diabetes, Knochenmarksfibrose, Peritonealfibrose und ähnliche fibrotische Erkrankungen, Sklerodermie, Morphaea, Keloide, hypertrophe Narbenbildung, Naevi, diabetische Retinopathie, proliferative Vitroretinopathie und Erkrankungen des Bindegewebes (z.B. Sarkoidose). Die erfindungsgemäßen Verbindungen können ebenso verwendet werden zur Förderung der Wundheilung, zur Bekämpfung postoperativer Narbenbildung, z.B. nach Glaukom-Operationen, und zu kosmetischen Zwecken bei alternder oder verhornender Haut.

Darüber hinaus können die erfindungsgemäßen Verbindungen eingesetzt werden zur Behandlung und/oder Prävention von Arteriosklerose, Lipidstoffwechselstörungen und Dyslipidämien (Hypolipoproteinämie, Hypertriglyceridämie, Hyperlipidämie, kombinierte Hyperlipidämien, Hypercholesterolämie, Abetalipoproteinämie, Sitosterolämie), Xanthomatose, Tangier-Krankheit, Fettsucht (Adipositas), Fettleibigkeit (Obesitas), metabolischen Erkrankungen (Metabolisches Syndrom, Hyperglykämie, Insulin-abhängiger Diabetes, nicht-Insulin-abhängiger Diabetes, Gestationsdiabetes, Hyperinsulinämie, Insulinresistenz, Glukose-Intoleranz und diabetische Spätfolgen wie Retinopathie, Nephropathie und Neuropathie), Anämien wie hämolytischen Anämien, insbesondere Hämoglobinopathien wie Sichelzellanämie und Thalassämien, megaloblastären Anämien, Eisenmangel-Anämien, Anämien durch akuten Blutverlust, Verdrängungsanämien und aplastischen Anämien, von Erkrankungen des Gastrointestinaltrakts und des Abdomen (Glossitis, Gingivitis, Periodontitis, Oesophagitis, eosinophile Gastroenteritis, Mastocytose, Morbus Crohn, Colitis, Proctitis, Pruritis ani, Diarrhöe, Zöliakie, Hepatitis, Leberfibrose, Leberzirrhose, Pankreatitis und Cholecystitis), Erkrankungen des zentralen Nervensystems (Schlaganfall, Epilepsie, Depressionen), Immunerkrankungen, Schilddrüsenerkrankungen (Hyperthyreose), Hauterkrankungen (Psoriasis, Akne, Ekzeme, Neurodermitis, vielfältige Formen der Dermatitis, Keratitis, Bullosis, Vasculitis, Cellulitis, Panniculitis, Lupus erythematodes, Erythema, Lymphome, Hautkrebs, Sweet-Syndrom, Weber-Christian-Syndrom, Narbenbildung, Warzenbildung, Frostbeulen), entzündlichen Augenerkrankungen (Saccoidosis, Blepharitis, Conjunctivitis, Iritis, Uveitis, Chorioiditis, Ophthalmitis), viralen Erkrankungen (durch Influenza-, Adeno- und Coronaviren, wie z.B. HPV, HCMV, HIV, SARS), von Erkrankungen des Skelettknochens, der Gelenke und der Skelettmuskel, von entzündlichen Arterienveränderungen (vielfältige Formen der Arteritis wie z.B. Endarteritis, Mesarteritis, Periarteritis, Panarteritis, Arteritis rheumatica, Arteritis deformans, Arteritis temporalis, Arteritis cranialis, Arteritis gigantocellularis und Arteritis granulomatosa, sowie das Horton-Syndrom, Churg-Strauss-Syndrom und die Takayasu-Arteritis), des Muckle-Well-Syndroms, der Kikuchi-Krankheit, von Polychondritis, Sklerodermia sowie von weiteren Erkrankungen mit einer entzündlichen oder immunologischen Komponente, wie beispielsweise Katarakt, Kachexie, Osteoporose, Gicht, Inkontinenz, Lepra, Sezary-Syndrom und paraneoplastisches Syndrom, bei Abstossungsreaktionen nach Organtransplantationen und zur Wundheilung und Angiogenese insbesondere bei chronischen Wunden.

Aufgrund ihres Eigenschaftsprofils eignen sich die erfindungsgemäßen Verbindungen bevorzugt zur Behandlung und/oder Prävention von Atemstörungen, insbesondere von schlafbedingten Atemstörungen wie obstruktiven und zentralen Schlafapnoen sowie primärem und obstruktivem Schnarchen, zur Behandlung und/oder Prävention von Herzrhythmusstörungen und Arrhythmien sowie zur Behandlung und/oder Prävention von neurodegenerativen, neuroinflammatorischen und neuroimmunologischen Erkrankungen.

Die zuvor genannten, gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Arzneimittel, enthaltend mindestens eine der erfindungsgemäßen Verbindungen, zur Behandlung und/oder Prävention von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prävention von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als hierfür geeignete Kombinationswirkstoffe seien beispielhaft und vorzugsweise genannt:
- Atemstimulantien, wie beispielhaft und vorzugsweise Theophyllin, Doxapram, Nicethamid oder Coffein;
- psychostimulierende Verbindungen, wie beispielhaft und vorzugsweise Modafinil oder Armodafinil;
- Amphetamine und Amphetamin-Derivate, wie beispielhaft und vorzugsweise Amphetamin, Metamphetamin oder Methylphenidat;
- Serotonin-Wiederaufnahmehemmer, wie beispielhaft und vorzugsweise Fluoxetin, Paroxetin, Citalopram, Escitalopram, Sertralin, Fluvoxamin oder Trazodon;
- Serotonin-Präkursoren, wie beispielhaft und vorzugsweise L-Tryptophan;
- selektive Serotonin-Noradrenalin-Wiederaufnahmehemmer, wie beispielhaft und vorzugsweise Venlafaxin oder Duloxetin;
- noradrenerge und spezifisch serotonerge Antidepressiva, wie beispielhaft und vorzugsweise Mirtazapin;
- selektive Noradrenalin-Wiederaufnahmehemmer, wie beispielhaft und vorzugsweise Reboxetin;
- tricyclische Antidepressiva, wie beispielhaft und vorzugsweise Amitriptylin, Protriptylin, Doxepin, Trimipramin, Imipramin, Clomipramin oder Desipramin;
- alpha2-adrenerge Agonisten, wie beispielhaft und vorzugsweise Clonidin;
- GABA-Agonisten, wie beispielhaft und vorzugsweise Baclofen;
- alpha-Sympathomimetika, wie beispielhaft und vorzugsweise Xylometazolin, Oxymetazolin, Phenylephrin, Naphazolin, Tetryzolin oder Tramazolin;
- Glucocorticoide, wie beispielhaft und vorzugsweise Fluticason, Budesonid, Beclometason, Mometason, Tixocortol oder Triamcinolon;
- Cannabinoid-Rezeptor-Agonisten;
- Carboanhydrase-Hemmer, wie beispielhaft und vorzugsweise Acetazolamid, Methazolamid oder Diclofenamid;
- Opioid- und Benzodiazepin-Rezeptor-Antagonisten, wie beispielhaft und vorzugsweise Flumazenil, Naloxon oder Naltrexon;
- Cholinesterase-Hemmer, wie beispielhaft und vorzugsweise Neostigmin, Pyridostigmin, Physostigmin, Donepezil, Galantamin oder Rivastigmin;
- *N*-Methyl-D-Aspartat- und Glutamat-Antagonisten, wie beispielhaft und vorzugsweise Amantadin, Memantin oder Sabeluzol;
- Nikotin-Rezeptor-Agonisten;
- Leukotrien-Rezeptor-Antagonisten, wie beispielhaft und vorzugsweise Montelukast oder Tripelukast;
- Dopamin-Rezeptor-Antagonisten, wie beispielhaft und vorzugsweise Dromperidon, Metoclopramid oder Benzamid-, Butyrophenon- oder Phenothiazin-Derivate;
- Appetitzügler, wie beispielhaft und vorzugsweise Sibutramin, Topiramat, Phentermin, Lipase-Inhibitoren oder Cannabinoid-Rezeptor-Antagonisten;
- Protonenpumpen-Inhibitoren, wie beispielhaft und vorzugsweise Pantoprazol, Omeprazol, Esomeprazol, Lansoprazol oder Rabeprazol;
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil, Tadalafil, Udenafil, Dasantafil, Avanafil, Mirodenafil oder Lodenafil;
- NO- und Häm-unabhängige Aktivatoren der löslichen Guanylatcyclase (sGC), wie insbesondere die in WO 01/19355, WO 01/19776, WO 01/19778, WO 01/19780, WO 02/070462 und WO 02/070510 beschriebenen Verbindungen;
- NO-unabhängige, jedoch Häm-abhängige Stimulatoren der löslichen Guanylatcyclase (sGC), wie insbesondere Riociguat, Vericiguat sowie die in WO 00/06568, WO 00/06569, WO 02/ 42301, WO 03/095451, WO 2011/147809, WO 2012/004258, WO 2012/028647 und WO 2012/ 059549 beschriebenen Verbindungen;
- Prostacyclin-Analoga und IP-Rezeptor-Agonisten, wie beispielhaft und vorzugsweise Iloprost, Beraprost, Treprostinil, Epoprostenol oder Selexipag;
- Endothelin-Rezeptor-Antagonisten, wie beispielhaft und vorzugsweise Bosentan, Darusentan, Ambrisentan oder Sitaxsentan;
- Verbindungen, die die humane neutrophile Elastase (HNE) inhibieren, wie beispielhaft und vorzugsweise Sivelestat oder DX-890 (Reltran);
- Verbindungen, die den Ab- und Umbau der Extrazellulärmatrix inhibieren, beispielhaft und vorzugsweise Inhibitoren der Matrix-Metalloproteasen (MMPs), insbesondere Inhibitoren von Stromelysin, Kollagenasen, Gelatinasen und Aggrecanasen (hierbei vor allem von MMP-1, MMP-3, MMP-8, MMP-9, MMP-10, MMP-11 und MMP-13) sowie der Metallo-Elastase (MMP-12);
- Verbindungen, die die Bindung von Serotonin an dessen Rezeptor blockieren, beispielhaft und vorzugsweise Antagonisten des 5-HT_{2B}-Rezeptors wie PRX-08066;
- Antagonisten von Wachstumsfaktoren, Zytokinen und Chemokinen, beispielhaft und vorzugsweise Antagonisten von TGF-β, CTGF, IL-1, IL-4, IL-5, IL-6, IL-8, IL-13 und Integrinen;
- die Rho-Kinase inhibierende Verbindungen, wie beispielhaft und vorzugsweise Fasudil, Y-27632, SLx-2119, BF-66851, BF-66852, BF-66853, KI-23095 oder BA-1049;
- den Energiestoffwechsel des Herzens beeinflussende Verbindungen, wie beispielhaft und vorzugsweise Etomoxir, Dichloracetat, Ranolazin oder Trimetazidin;
- die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase-Inhibitoren, wie beispielhaft und vorzugsweise Nintedanib, Dasatinib, Nilotinib, Bosutinib, Regorafenib, Sorafenib, Sunitinib, Cediranib, Axitinib, Telatinib, Imatinib, Brivanib, Pazopanib, Vatalanib, Gefitinib, Erlotinib, Lapatinib, Canertinib, Lestaurtinib, Pelitinib, Semaxanib oder Tandutinib;
- anti-obstruktiv wirkende Mittel, wie sie z.B. zur Therapie der chronisch-obstruktiven Lungenerkrankung (COPD) oder eines Asthma bronchiale eingesetzt werden, beispielhaft und vorzugsweise aus der Gruppe der inhalativ oder systemisch angewendeten beta-adrenergen Rezeptor-Agonisten (beta-Mimetika) und der inhalativ angewendeten anti-muscarinergen Substanzen;
- entzündungshemmende, immunmodulierende, immunsuppressive und/oder zytotoxische Mittel, beispielhaft und vorzugsweise aus der Gruppe der systemisch oder inhalativ angewendeten Corticosteroide sowie Dimethylfumarat, Fingolimod, Glatirameracetat, β-Interferone, Natalizumab, Teriflunomid, Mitoxantron, Immunglobuline, Acetylcystein, Montelukast, Tripelukast, Azathioprin, Cyclophosphamid, Hydroxycarbamid, Azithromycin, Interferon-γ, Pirfenidon oder Etanercept;
- antifibrotisch wirkende Mittel, wie beispielhaft und vorzugsweise Lysophosphatidsäure-Rezeptor 1 (LPA-1)-Antagonisten, CTGF-Inhibitoren, IL-4-Antagonisten, IL-13-Antagonisten, TGF-β-Antagonisten oder Pirfenidon;
- antithrombotisch wirkende Mittel, beispielhaft und vorzugsweise aus der Gruppe der Thrombozytenaggregationshemmer, der Antikoagulantien und der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Vasopeptidase-Inhibitoren, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Bei einer bevorzugten Ausführungsform der Erfindung werden die erfindungsgemäßen Verbindungen in Kombination mit einem beta-adrenergen Rezeptor-Agonisten, wie beispielhaft und vorzugsweise Albuterol, Isoproterenol, Metaproterenol, Terbutalin, Fenoterol, Formoterol, Reproterol, Salbutamol oder Salmeterol, verabreicht.

Bei einer bevorzugten Ausführungsform der Erfindung werden die erfindungsgemäßen Verbindungen in Kombination mit einer anti-muscarinergen Substanz, wie beispielhaft und vorzugsweise Ipratropiumbromid, Tiotropiumbromid oder Oxitropiumbromid, verabreicht.

Bei einer bevorzugten Ausführungsform der Erfindung werden die erfindungsgemäßen Verbindungen in Kombination mit einem Corticosteroid, wie beispielhaft und vorzugsweise Prednison, Prednisolon, Methylprednisolon, Triamcinolon, Dexamethason, Betamethason, Beclometason, Flunisolid, Budesonid oder Fluticason, verabreicht.

Unter antithrombotisch wirkenden Mittel werden vorzugsweise Verbindungen aus der Gruppe der Thrombozytenaggregationshemmer, der Antikoagulantien und der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton, Eplerenon oder Finerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapid, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazon oder Rosiglitazon, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Besonders bevorzugt sind Kombinationen der erfindungsgemäßen Verbindungen mit einem oder mehreren weiteren Wirkstoffen ausgewählt aus der Gruppe bestehend aus Atemstimulantien, psychostimulierenden Verbindungen, Serotonin-Wiederaufnahmehemmern, noradrenergen, serotonergen und tricyclischen Antidepressiva, sGC-Stimulatoren, Mineralocorticoid-Rezeptor-Antagonisten, entzündungshemmend wirkenden Mitteln, immunmodulierend wirkenden Mitteln, immunsuppressiv wirkenden Mitteln und zytotoxisch wirkenden Mitteln.

Die erfindungsgemäßen Substanzen können bei Bedarf auch in Zusammenhang mit dem Einsatz eines oder mehrerer medizinisch-technischer Geräte oder Hilfsmittel verwendet werden, solange dies nicht zu unerwünschten und inakzeptablen Nebeneffekten führt. Für eine solche Kombinationsanwendung in Betracht kommende medizinische Geräte und Hilfsmittel sind beispielhaft und vorzugsweise:
- Geräte zur Atemwegs-Überdruckbeatmung, wie beispielhaft und vorzugsweise CPAP (*continuous positive airway pressure*)-Geräte, BiPAP (*bilevel positive airway pressure*)-Geräte und IPPV (*intermittent positive pressure ventilation*)-Geräte;
- Neurostimulatoren des *Nervus hypoglossus*;
- intraorale Hilfsmittel, wie beispielhaft und vorzugsweise Protrusionsspangen;
- nasale Einwegventile;
- Nasenstents.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, intrapulmonal (inhalativ), nasal, intranasal, pharyngeal, lingual, sublingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Dosieraerosole), Nasentropfen, -lösungen oder -sprays, Rachensprays, lingual, sublingual oder buccal zu applizierende Tabletten, Filme/Oblaten oder Kapseln, Suppositorien, Augentropfen, -salben oder -bäder, okulare Inserte, Ohrentropfen, -sprays, -pulver, -spülungen oder -tampons, Vaginalkapseln, wässrige Suspensionen (Lotionen, Schüttelmixturen), lipophile Suspensionen, Emulsionen, Mikroemulsionen, Salben, Cremes, transdermale therapeutische Systeme (z.B. Pflaster), Milch, Pasten, Schäume, Streupuder, Implantate oder Stents.

Bevorzugt sind die orale, die intravenöse, die intranasale und die pharyngeale Applikation.

Gemäß einer Ausführungsform erfolgt die Applikation intranasal. Gemäß einer Ausführungsform erfolgt die intranasale Applikation mit Hilfe von Nasentropfen oder eines Nasensprays. Gemäß einer Ausführungsform erfolgt die intranasale Applikation mit Hilfe eines Nasensprays.

Die erfindungsgemäßen Verbindungen können in die angeführten Applikationsformen überführt werden. Dies kann in an sich bekannter Weise durch Mischen mit inerten, nicht-toxischen, pharmazeutisch geeigneten Hilfsstoffen geschehen. Zu diesen Hilfsstoffen zählen u.a.
- Füll- und Trägerstoffe (beispielsweise Cellulose, mikrokristalline Cellulose wie z.B. Avicel^{®}, Lactose, Mannitol, Stärke, Calciumphosphate wie z.B. Di-Cafos^{®});
- Salbengrundlagen (beispielsweise Vaseline, Paraffine, Triglyceride, Wachse, Wollwachs, Wollwachsalkohole, Lanolin, hydrophile Salbe, Polyethylenglycole);
- Suppositoriengrundlagen (zum Beispiel Polyethylenglycole, Kakaobutter, Hartfett);
- Lösungsmittel (z.B. Wasser, Ethanol, Isopropanol, Glycerol, Propylenglycol, mittelkettige Triglyceride, Fettöle, flüssige Polyethylenglycole, Paraffine);
- Tenside, Emulgatoren, Dispergier- oder Netzmittel (beispielsweise Natriumdodecylsulfat, Lecithin, Phospholipide, Fettalkohole wie z.B. Lanette^{®}, Sorbitanfettsäureester wie z.B. Span^{®}, Polyoxyethylen-Sorbitanfettsäureester wie z.B. Tween^{®}, Polyoxyethylen-Fettsäureglyceride wie z.B. Cremophor^{®}, Polyoxyethylen-Fettsäureester, Polyoxyethylen-Fettalkoholether, Glycerolfettsäureester, Poloxamere wie z.B. Pluronic^{®});
- Puffersubstanzen sowie Säuren und Basen (beispielsweise Phosphate, Carbonate, Citronensäure, Essigsäure, Salzsäure, Natronlauge, Ammoniumcarbonat, Trometamol, Triethanolamin);
- Isotonisierungsmittel (beispielsweise Glucose, Natriumchlorid);
- Adsorptionsmittel (beispielsweise hochdisperse Siliziumdioxide);
- viskositätserhöhende Mittel, Gelbildner, Verdickungs- bzw. Bindemittel (beispielsweise Polyvinylpyrrolidon, Methylcellulose, Hydroxypropylcellulose, Hydroxypropylmethylcellulose, Carboxymethylcellulose-Natrium, Stärke, Carbomere, Polyacrylsäuren wie z.B. Carbopol^{®}, Alginate, Gelatine);
- Sprengmittel (beispielsweise modifizierte Stärke, Carboxymethylcellulose-Natrium, Natriumstärkeglycolat wie z.B. Explotab^{®}, quervernetztes Polyvinylpyrrolidon, Croscarmellose-Natrium wie z.B. AcDiSol^{®});
- Fließregulier-, Schmier-, Gleit- und Formtrennmittel (beispielsweise Magnesiumstearat, Stearinsäure, Talkum, hochdisperse Siliziumdioxide wie z.B. Aerosil^{®});
- Überzugsmittel (beispielsweise Zucker, Schellack) sowie Filmbildemittel für sich schnell oder modifiziert auflösende Filme bzw. Diffusionsmembranen (beispielsweise Polyvinylpyrrolidone wie z.B. Kollidon^{®}, Polyvinylalkohol, Ethylcellulose, Hydroxypropylcellulose, Hydroxypropylmethylcellulose, Hydroxypropylmethylcellulosephthalat, Celluloseacetat, Celluloseacetatphthalat, Polyacrylate, Polymethacrylate wie z.B. Eudragit^{®});
- Kapselmaterialien (z.B. Gelatine, Hydroxypropylmethylcellulose);
- natürliche Polymere (beispielsweise Albumine);
- synthetische Polymere (beispielsweise Polylactide, Polyglycolide, Polyacrylate, Polymethacrylate wie z.B. Eudragit^{®}, Polyvinylpyrrolidone wie z.B. Kollidon^{®}, Polyvinylalkohole, Polyvinylacetate, Polyethylenoxide, Polyethylenglycole und deren Copolymere und Block-Copolymere) ;
- Weichmacher (beispielsweise Polyethylenglycole, Propylenglycol, Glycerol, Triacetin, Triacetylcitrat, Dibutylphthalat);
- Penetrationsverstärker;
- Stabilisatoren (z.B. Antioxidantien wie beispielsweise Ascorbinsäure, Natriumascorbat, Ascorbylpalmitat, Butylhydroxyanisol, Butylhydroxytoluol, Propylgallat);
- Konservierungsmittel (beispielsweise Parabene, Sorbinsäure, Natriumbenzoat, Thiomersal, Benzalkoniumchlorid, Chlorhexidinacetat);
- Farbstoffe (z.B. anorganische Pigmente wie beispielsweise Eisenoxide, Titandioxid);
- Aromen, Süßungsmittel, Geschmacks- und/oder Geruchskorrigentien.

Im Allgemeinen hat es sich als vorteilhaft erwiesen, bei parenteraler Applikation Wirkstoffmengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht. Bei intrapulmonaler Applikation beträgt die Wirkstoffmenge im Allgemeinen etwa 0.1 bis 50 mg je Inhalation.

Gemäß einer Ausführungsform beträgt die Dosierung bei intranasaler Applikation etwa 0.1 µg bis 500 µg pro Tag. Gemäß einer weiteren Ausführungsform beträgt die Dosierung bei intranasaler Applikation etwa 1 µg bis 250 µg pro Tag. Gemäß einer weiteren Ausführungsform beträgt die Dosierung bei intranasaler Applikation etwa 1 µg bis 120 µg pro Tag. Gemäß einer weiteren Ausführungsform wird die Dosis von etwa 0.1 µg bis 500 µg pro Tag, oder von etwa 1 µg bis 250 µg pro Tag, oder von etwa 1 µg bis 120 µg pro Tag, einmal täglich vor dem Schlafen intranasal appliziert. Gemäß einer Ausführungsform wird die Dosis von etwa 0.1 µg bis 500 µg pro Tag, oder von etwa 1 µg bis 250 µg pro Tag, oder von etwa 1 µg bis 120 µg pro Tag, einmal täglich je zur Hälfte in jede Nasenöffnung appliziert. Gemäß einer Ausführungsform wird die Dosis von etwa 0.1 µg bis 500 µg pro Tag, oder von etwa 1 µg bis 250 µg pro Tag, oder von etwa 1 µg bis 120 µg pro Tag, einmal täglich vor dem Schlafen je zur Hälfte in jede Nasenöffnung appliziert.

Trotzdem kann es gegebenenfalls erforderlich sein, von den genannten Wirkstoffmengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

### A. Beispiele

### Abkürzungen und Akronyme:

- abs.: absolut
- Ac: Acetyl
- aq.: wässrig, wässrige Lösung
- Boc: *tert*.-Butoxycarbonyl
- br.: breit (bei NMR-Signal)
- Bsp.: Beispiel
- Bu: Butyl
- c: Konzentration
- ca.: *circa*, ungefähr
- cat.: katalytisch
- CI: chemische Ionisation (bei MS)
- d: Dublett (bei NMR)
- d: Tag(e)
- DCI: direkte chemische Ionisation (bei MS)
- dd: Dublett von Dublett (bei NMR)
- DMF: *N,N*-Dimethylformamid
- DMSO: Dimethylsulfoxid
- dq: Dublett von Quartett (bei NMR)
- dt: Dublett von Triplett (bei NMR)
- d. Th.: der Theorie (bei chemischer Ausbeute)
- EI: Elektronenstoß-Ionisation (bei MS)
- eq.: Äquivalent(e)
- ESI: Elektrospray-Ionisation (bei MS)
- Et: Ethyl
- h: Stunde(n)
- HATU: *O*-(7-Azabenzotriazol-1 -yl)-*N,N,N',N*'-tetramethyluroniumhexafluorophosphat
- HOBt: 1-Hydroxy-1*H*-benzotriazol-Hydrat
- HPLC: Hochdruck-, Hochleistungsflüssigchromatographie
- iPr: Isopropyl
- konz.: konzentriert (bei Lösung)
- LC: Flüssigchromatographie
- LC-MS: Flüssigchromatographie-gekoppelte Massenspektrometrie
- Lit.: Literatur(stelle)
- m: Multiplett (bei NMR)
- Me: Methyl
- min: Minute(n)
- MS: Massenspektrometrie
- NMR: Kernresonanzspektrometrie
- Ph: Phenyl
- Pr: Propyl
- q: Quartett (bei NMR)
- quant.: quantitativ (bei chemischer Ausbeute)
- RP: reverse phase (Umkehrphase, bei HPLC)
- RT: Raumtemperatur
- Rₜ: Retentionszeit (bei HPLC, LC-MS)
- s: Singulett (bei NMR)
- SFC: superkritische Flüssigchromatographie
- t: Triplett (bei NMR)
- tBu: *tert*.-Butyl
- TFA: Trifluoressigsäure
- THF: Tetrahydrofuran
- UV: Ultraviolett-Spektrometrie
- v/v: Volumen zu Volumen-Verhältnis (einer Lösung)
- zus.: zusammen

### LC-MS- und HPLC-Methoden:

### Methode 1 (LC-MS):

Instrument: Waters Acquity SQD UPLC System; Säule: Waters Acquity UPLC HSS T3 1.8 µm, 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A ----> 2.0 min 5% A; Temperatur: 50°C; Fluss: 0.40 ml/min; UV-Detektion: 208-400 nm.

### Methode 2 (LC-MS):

Instrument MS: Thermo Scientific FT-MS; Gerätetyp UHPLC: Thermo Scientific UltiMate 3000; Säule: Waters HSS T3 C18 1.8 µm, 75 mm x 2.1 mm; Eluent A: 1 1 Wasser + 0.01% Ameisensäure, Eluent B: 1 1 Acetonitril + 0.01% Ameisensäure; Gradient: 0.0 min 10% B ----> 2.5 min 95% B ----> 3.5 min 95% B; Temperatur: 50°C; Fluss: 0.90 ml/min; UV-Detektion: 210 nm / optimum integration path 210-300 nm.

### Methode 3 (LC-MS):

Instrument MS: Waters Micromass QM; Instrument HPLC: Agilent 1100 Serie; Säule: Agilent ZORBAX Extend-C18 3.5 µm, 50 mm x 3.0 mm; Eluent A: 1 1 Wasser + 0.01 mol Ammoniumcarbonat, Eluent B: 1 1 Acetonitril; Gradient: 0.0 min 98% A → 0.2 min 98% A → 3.0 min 5% A ----> 4.5 min 5% A; Temperatur: 40°C; Fluss: 1.75 ml/min; UV-Detektion: 210 nm.

### Methode 4 (LC-MS):

Instrument MS: Waters Micromass Quattro Micro; Instrument HPLC: Waters UPLC Acquity; Säule: Waters BEH C18 1.7 µm, 50 mm x 2.1 mm; Eluent A: 1 1 Wasser + 0.01 mol Ammoniumformiat, Eluent B: 1 l Acetonitril; Gradient: 0.0 min 95% A ----> 0.1 min 95% A ----> 2.0 min 15% A ----> 2.5 min 15% A ----> 2.51 min 10% A ----> 3.0 min 10% A; Temperatur: 40°C; Fluss: 0.5 ml/min; UV-Detektion: 210 nm.

### Methode 5 (LC-MS):

Instrument: Agilent MS Quad 6150 mit HPLC Agilent 1290; Säule: Waters Acquity UPLC HSS T3 1.8 µm, 50 mm x 2.1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 0.3 min 90% A → 1.7 min 5% A ----> 3.0 min 5% A; Fluss: 1.20 ml/min; Temperatur: 50°C; UV-Detektion: 205-305 nm.

### Methode 6 (LC-MS):

Instrument MS: Waters Single Quad MS-System; Instrument HPLC: Waters UPLC Acquity; Säule: Waters BEH C18 1.7 µm, 50 mm x 2.1 mm; Eluent A: 11 Wasser + 1.0 ml 25% Ammoniak, Eluent B: 1 1 Acetonitril; Gradient: 0.0 min 92% A ----> 0.1 min 92% A → 1.8 min 5% A → 3.5 min 5% A; Temperatur: 50°C; Fluss: 0.45 ml/min; UV-Detektion: 210 nm (208-400 nm).

### Methode 7 (LC-MS):

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril + 0.025% Ameisensäure; Gradient: 0.0 min 98% A ----> 0.9 min 25% A ----> 1.0 min 5% A ----> 1.4 min 5% A ----> 1.41 min 98% A ----> 1.5 min 98% A; Temperatur: 40°C; Fluss: 0.60 ml/min; UV-Detektion: DAD, 210 nm.

### Methode 8 (präparative HPLC):

Instrument: Abimed Gilson 305; Säule: Reprosil C18 10 µm, 250 mm x 30 mm; Eluent A: Wasser, Eluent B: Acetonitril; Gradient: 0-3 min 10% B, 3-27 min 10% B ----> 95% B, 27-34.5 min 95% B, 34.5-35.5 min 95% B ----> 10% B, 35.5-36.5 min 10% B; Fluss: 50 ml/min; Raumtemperatur; UV-Detektion: 210 nm.

### Methode 9 (präparative HPLC):

Instrument: Waters Prep LC/MS-System; Säule: XBridge C18 5 µm, 100 mm x 30 mm; Eluent A: Wasser, Eluent B: Acetonitril; Gradientenprofil: 0-2 min 10% B, 2-2.2 min auf 30% B, 2.2-7 min auf 70% B, 7-7.5 min auf 92% B, 7.5-9 min 92% B; Fluss: 65 ml/min + 5 ml 2% Ammoniak in Wasser; Raumtemperatur; UV-Detektion: 200-400 nm; at-column-Injektion (Komplettinjektion).

### Weitere Angaben:

Die Prozentangaben in den folgenden Beispiel- und Testbeschreibungen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Reinheitsangaben beziehen sich in der Regel auf entsprechende Peak-Integrationen im LC-MS-Chromatogramm, können aber zusätzlich auch unter Zuhilfenahme des ¹H-NMR-Spektrums ermittelt worden sein. Wenn keine Reinheit angegeben ist, handelt es sich in der Regel um eine Reinheit >95% laut automatischer Peak-Integration im LC-MS-Chromatogramm oder die Reinheit wurde nicht explizit ermittelt.

Angaben zu Ausbeuten in % d. Th. sind in der Regel reinheitskorrigiert, sofern eine Reinheit <100% angegeben ist. Bei lösungsmittelhaltigen oder verunreinigten Chargen kann die Ausbeute formal ">100%" betragen; in diesen Fällen ist die Ausbeute nicht lösungsmittel- bzw. reinheitskorrigiert.

In den Fällen, in denen Reaktionsprodukte durch Ausrühren, Verrühren oder Umkristallisieren gewonnen wurden, war es oft möglich, weitere Produktmengen aus der jeweiligen Mutterlauge durch Chromatographie zu isolieren. Auf die Beschreibung dieser Chromatographie wird im Folgenden jedoch verzichtet, es denn, ein großer Teil der Gesamtausbeute konnte erst in diesem Schritt isoliert werden.

Schmelzpunkte und Schmelzbereiche, soweit angegeben, sind nicht korrigiert.

Die nachfolgenden Beschreibungen der Kopplungsmuster von ¹H-NMR-Signalen wurden teilweise direkt den Vorschlägen des ACD SpecManagers (ACD/Labs Release 12.00, Product version 12.5) entnommen und nicht notwendigerweise streng hinterfragt. Teilweise wurden die Vorschläge des SpecManagers manuell angepasst. Manuell angepasste bzw. zugewiesene Beschreibungen orientieren sich in der Regel an dem optischen Erscheinungsbild der betreffenden Signale und entsprechen nicht notwendigerweise einer strengen, physikalisch korrekten Interpretation. In der Regel bezieht sich die Angabe zur chemischen Verschiebung auf das Zentrum des betreffenden Signals. Bei breiten Multipletts erfolgt die Angabe eines Intervalls. Durch Lösungsmittel oder Wasser verdeckte Signale wurden entweder tentativ zugeordnet oder sind nicht aufgeführt.

Die ¹H-NMR-Daten von Synthese-Intermediaten und Ausführungsbeispielen können auch in Form von ¹H-NMR-Peaklisten angegeben sein. Hierin wird zu jedem Signalpeak erst der δ-Wert in ppm und dann die Signalintensität in runden Klammem aufgeführt. Die δ-Wert/Signalintensität-Zahlenpaare von verschiedenen Signalpeaks werden durch Kommata voneinander getrennt aufgelistet; die Peakliste zu einer Verbindung hat daher die Form: δ₁ (Intensität₁), δ₂ (Intensität₂), ... , δᵢ (Intensitätᵢ), ... , δₙ (Intensitätₙ).

Die Intensität scharfer Signale korreliert mit der Höhe der Signale (in cm) in einem ausgedruckten Beispiel eines NMR-Spektrums und zeigt im Vergleich mit anderen Signalen die wirklichen Verhältnisse der Signalintensitäten. Bei breiten Signalen können mehrere Peaks oder die Mitte des Signals sowie deren relative Intensität im Vergleich zum intensivsten Signal im Spektrum aufgeführt sein. Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden. Darüber hinaus können sie wie klassische ¹H-NMR-Ausdrucke Lösungsmittel-Signale, Signale von Stereoisomeren der betreffenden Zielverbindung, Peaks von Verunreinigungen, ¹³C-Satelliten-Peaks und/oder Rotations-Seitenbanden aufweisen. Peaks von Stereoisomeren der Zielverbindung und/oder Peaks von Verunreinigungen haben gewöhnlich im Durchschnitt eine geringere Intensität als die Peaks der Zielverbindung (zum Beispiel bei einer Reinheit von >90%). Solche Stereoisomere und/oder Verunreinigungen können typisch für das jeweilige Herstellungsverfahren sein. Ihre Peaks können insofern dabei helfen, eine Reproduktion des Herstellungsverfahrens anhand von "Nebenprodukt-Fingerabdrücken" zu erkennen. Ein Experte, der die Peaks einer Zielverbindung mit Hilfe bekannter Verfahren (MestreC, ACD-Simulation oder unter Verwendung von empirisch bestimmten Erwartungswerten) berechnet, kann je nach Bedarf die Peaks der Zielverbindung isolieren, wobei gegebenenfalls zusätzliche Intensitätsfilter eingesetzt werden. Diese Isolierung wäre ähnlich dem betreffenden "Peak-Picking" bei der klassischen ¹H-NMR-Interpretation.

Eine detaillierte Beschreibung der Darstellung von NMR-Daten in Form von Peaklisten kann der Publikation "Citation of NMR Peaklist Data within Patent Applications" entnommen werden (siehe http://www.researchdisclosure.com/searching-disclosures, Research Disclosure Database Number 605005, 2014, 1. August 2014). In der Peak Picking-Routine, die in der angegebenen Research Disclosure beschrieben ist, kann der Parameter "MinimumHeight" zwischen 1% und 4% eingestellt werden. In Abhängigkeit von der Art der chemischen Struktur und/oder der Konzentration der zu vermessenden Verbindung kann es jedoch auch sinnvoll sein, den Parameter "MinimumHeight" auf Werte <1% einzustellen.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist, und die nicht kommerziell erhältlich waren, oder von Quellen bezogen wurden, die nicht allgemein zugänglich sind, ist ein Verweis auf die veröffentlichte Literatur angegeben, in der ihre Herstellung beschrieben ist.

### Ausgangsverbindungen und Intermediate:

### Beispiel 1A

### 2-(4-Chlorphenyl)imidazo[1,2-a]pyrimidin

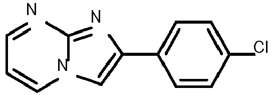

Eine Lösung von 2-Brom-1-(4-chlorphenyl)ethanon (20.0 g, 85.7 mmol) und Pyrimidin-2-amin (8.96 g, 94.2 mmol) in 200 ml Ethanol wurde mit Natriumhydrogencarbonat (10.8 g, 128 mmol) versetzt und 5 Stunden bei 80°C gerührt. Anschließend wurde der Ansatz auf 0°C abgekühlt (Eisbad). Der erhaltene Niederschlag wurde abfiltriert und mit einem Ethanol/Wasser-Gemisch (1:1) zweimal gewaschen. Der Feststoff wurde dann im Vakuum über Nacht bei 40°C getrocknet. Es wurden 15.9 g (69.23 mmol, 80.8% d. Th.) des Zielprodukts erhalten.

LC-MS (Methode 2): Rₜ = 1.25 min; m/z = 230 (M+H)⁺.

¹H-NMR (400 MHz, DMSO-*d*₆, δ/ppm): 7.07 (dd, 1H), 7.53 (d, 2H), 8.03 (d, 2H), 8.41 (s, 1H), 8.54 (dd, 1H), 8.97 (dd, 1H).

### Beispiel 2A

### 2-(4-Isopropylphenyl)imidazo[1,2-a]pyrimidin

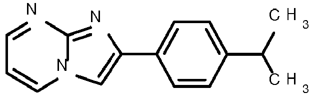

Eine Lösung von 2-Brom-1-(4-isopropylphenyl)ethanon (1.0 g, 4.15 mmol) und Pyrimidin-2-amin (0.43 g, 4.6 mmol) in 50 ml Ethanol wurde mit Natriumhydrogencarbonat (0.52 g, 6.22 mmol) versetzt und 5 Stunden bei 80°C gerührt. Anschließend wurde der Ansatz bis zur Trockene eingeengt. Der Rückstand wurde mit Diethylether verrührt und der verbliebene Feststoff abfiltriert und im Vakuum über Nacht bei 40°C getrocknet. Es wurden 1.15 g des rohen Zielprodukts erhalten, welches ohne weitere Aufreinigung in Folgereaktionen eingesetzt wurde.

LC-MS (Methode 2): Rₜ = 1.48 min; m/z = 238 (M+H)⁺.

¹H-NMR (400 MHz, DMSO-*d*₆, δ/ppm): 1.24 (d, 6H), 2.87-3.00 (m, 1H), 7.04 (dd, 1H), 7.34 (d, 2H), 7.92 (d, 2H), 8.33 (s, 1H), 8.51 (dd, 1H), 8.95 (dd, 1H).

Analog zu den Beispielen 1A und 2A wurde die folgende Verbindung aus den angegebenen Edukten hergestellt:

| **Beispiel** | **Name / Struktur / Edukte** | **Analytische Daten** |
|---|---|---|
| **3A** | 2-(4-Bromphenyl)imidazo[1,2-a]pyrimidin | ¹H-NMR (400 MHz, DMSO-d₆): δ [ppm] = 7.07 (dd, 1H), 7.67 (d, 2H), 7.97 (d, 2H), 8.42 (s, 1H), 8.54 (dd, 1H), 8.97 (dd, 1H). |
| | 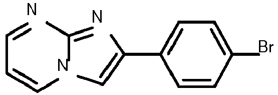 aus 2-Brom-1-(4-bromphenyl)ethanon und Pyrimidin-2-amin | |
| | | LC-MS (Methode 2): Rₜ = 1.34 min; m/z = 274/276 (M+H)⁺. |

### Beispiel 4A

### 2-(4-Chlorphenyl)imidazo[1,2-a]pyrimidin-3-carbaldehyd

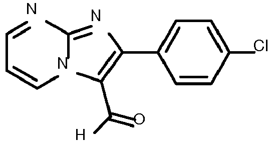

300 ml DMF wurden vorgelegt und auf 0°C gekühlt. Anschließend wurde langsam Phosphoroxychlorid (16 ml, 173 mmol) zugetropft. Die Lösung wurde danach langsam auf Raumtemperatur erwärmt und eine Stunde bei dieser Temperatur nachgerührt. Dann wurde portionsweise 2-(4-Chlorphenyl)imidazo[1,2-a]pyrimidin (15.9 g, 69.2 mmol) zugegeben. Nach Beendigung der Zugabe wurde das Reaktionsgemisch auf 80°C erhitzt und 1 Stunde bei dieser Temperatur gerührt. Anschließend wurde der Ansatz auf 0°C abgekühlt (Eisbad). Der erhaltene Feststoff wurde abgesaugt, mehrmals mit Wasser gewaschen und über Nacht im Hochvakuum-Trockenschrank bei 40°C getrocknet. Es wurden 13.75 g (53.36 mmol, 77% d. Th.) des Zielprodukts erhalten.

LC-MS (Methode 2): Rₜ = 1.44 min; m/z = 258 (M+H)⁺.

¹H-NMR (400 MHz, DMSO-d₆): δ [ppm] = 7.46 (dd, 1H), 7.65 (d, 2H), 8.01 (d, 2H), 8.91 (dd, 1H), 9.83 (dd, 1H), 10.07 (s, 1H).

### Beispiel 5A

### 2-(4-Isopropylphenyl)imidazo[1,2-a]pyrimidin-3-carbaldehyd

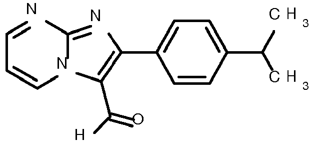

50 ml DMF wurden vorgelegt und auf 0°C gekühlt. Anschließend wurde langsam Phosphoroxychlorid (2.86 ml, 30.66 mmol) zugetropft. Die Lösung wurde danach langsam auf Raumtemperatur erwärmt und eine Stunde bei dieser Temperatur nachgerührt. Dann wurde portionsweise 2-(4-Isopropylphenyl)imidazo[1,2-a]pyrimidin (2.91 g, 12.26 mmol) zugegeben. Nach Beendigung der Zugabe wurde das Reaktionsgemisch auf 80°C erhitzt und 1 Stunde bei dieser Temperatur gerührt. Anschließend wurde der Ansatz auf 0°C abgekühlt (Eisbad). Der erhaltene Feststoff wurde abgesaugt und im Vakuum getrocknet. Das resultierende Rohprodukt wurde nachfolgend mittels zweimaliger Säulenchromatographie gereinigt (Biotage Isolera, Biotage SNAP-KP-NH-Säule, Laufmittel Cyclohexan/Essigsäureethylester-Gradient). Es wurden 3 g (11.3 mmol, 92% d. Th.) der Zielverbindung erhalten.

LC-MS (Methode 2): Rₜ = 1.75 min; m/z = 266 (M+H)⁺.

¹H-NMR (400 MHz, DMSO-d₆): δ [ppm] = 1.27 (d, 6H), 2.92-3.07 (m, 1H), 7.39-7.52 (m, 3H), 7.90 (d, 2H), 8.89 (dd, 1H), 9.83 (dd, 1H), 10.08 (s, 1H).

Analog zu den Beispielen 4A und 5A wurde die folgende Verbindung aus dem angegebenen Edukt hergestellt:

| **Beispiel** | **Name / Struktur / Edukt** | **Analytische Daten** |
|---|---|---|
| **6A** | 2-(4-Bromphenyl)imidazo[1,2-a]pyrimidin-3-carbaldehyd | ¹H-NMR (400 MHz, DMSO-d₆): δ [ppm] = 7.46 (dd, 1H), 7.79 (d, 2H), 7.94 (d, 2H), 8.91 (dd, 1H), 9.83 (dd, 1H), 10.07 (s, 1H). |
| | 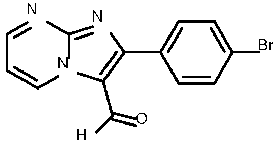 aus 2-(4-Bromphenyl)imidazo[1,2-a]pyrimidin | |
| | | LC-MS (Methode 1): Rₜ = 0.78 min; m/z = 302/304 (M+H)⁺. |

### Beispiel 7A

### 7- {[2-(4-Chlorphenyl)imidazo[1,2-a]pyrimidin-3-yl]methyl}-3-oxa-7,9-diazabicyclo[3.3.1]nonan-Dihydrochlorid

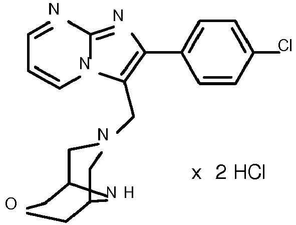

*tert.*-Butyl-7-{[2-(4-chlorphenyl)imidazo[1,2-a]pyrimidin-3-yl]methyl}-3-oxa-7,9-diazabicyclo-[3.3.1]nonan-9-carboxylat (1.52 g, 3.23 mmol) wurde unter Rühren mit 12 ml einer 4 M Lösung von Chlorwasserstoff in Dioxan versetzt. Das Gemisch wurde über Nacht bei Raumtemperatur gerührt. Anschließend wurde der erhaltene Feststoff abgesaugt, mehrfach mit Diethylether gewaschen und im Hochvakuum bei 40°C getrocknet. Es wurden 1.76 g des Zielprodukts erhalten.

LC-MS (Methode 2): Rₜ = 0.71 min; m/z = 370 (M+H)⁺.

### Beispiel 8A

### 7-{[2-(4-Isopropylphenyl)imidazo[l,2-a]pyrimidin-3-yl]methyl}-3-oxa-7,9-diazabicyclo[3.3.1]-nonan-Dihydrochlorid

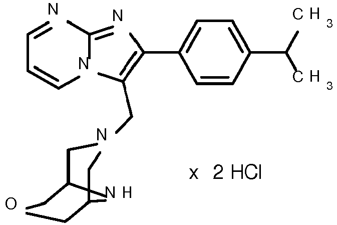

*tert.*-Butyl-7-{[2-(4-isopropylphenyl)imidazo[1,2-a]pyrimidin-3-yl]methyl}-3-oxa-7,9-diaza-bicyclo[3.3.1]nonan-9-carboxylat (420 mg, 0.88 mmol) wurde unter Rühren mit 2.2 ml einer 4 M Lösung von Chlorwasserstoff in Dioxan versetzt. Das Gemisch wurde über Nacht bei Raumtemperatur gerührt. Anschließend wurde der erhaltene Feststoff abgesaugt, mehrfach mit Diethylether gewaschen und im Hochvakuum bei 40°C getrocknet. Es wurden 430 mg des Zielprodukts erhalten.

LC-MS (Methode 2): Rₜ = 0.87 min; m/z = 378 (M+H)⁺.

### Beispiel 9A

### 2-(4-Chlorphenyl)-3-(3,8-diazabicyclo[3.2.1]oct-3-ylmethyl)imidazo[1,2-a]pyrimidin-Dihydrochlorid

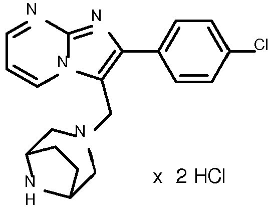

*tert.-*Butyl-3-{[2-(4-chlorphenyl)imidazo[1,2-a]pyrimidin-3-yl]methyl}-3,8-diazabicyclo[3.2.1]-octan-8-carboxylat (2.72 g, 6.00 mmol) wurde unter Rühren mit 15 ml einer 4 M Lösung von Chlorwasserstoff in Dioxan versetzt. Das Gemisch wurde über Nacht bei Raumtemperatur gerührt. Anschließend wurde der erhaltene Feststoff abgesaugt, mehrfach mit Diethylether gewaschen und im Hochvakuum bei 40°C getrocknet. Es wurden 3.5 g des Zielprodukts erhalten.

LC-MS (Methode 6): Rₜ = 1.36 min; m/z = 354 (M+H)⁺.

### Beispiel 10A

### 3-(3,8-Diazabicyclo[3.2.1]oct-3-ylmethyl)-2-(4-isopropylphenyl)imidazo[1,2-a]pyrimidin-Dihydrochlorid

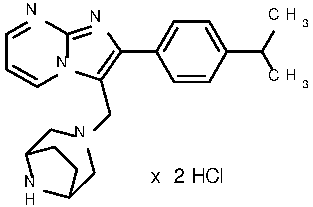

*tert.*-Butyl-3-{[2-(4-isopropylphenyl)imidazo[1,2-a]pyrimidin-3-yl] methyl } -3,8-diazabicyclo-[3.2.1]octan-8-carboxylat (493 mg, 1.03 mmol) wurde unter Rühren mit 2.57 ml einer 4 M Lösung von Chlorwasserstoff in Dioxan versetzt. Das Gemisch wurde über Nacht bei Raumtemperatur gerührt. Anschließend wurde die Reaktionslösung bis zur Trockene eingeengt und der resultierende Rückstand im Hochvakuum bei 40°C getrocknet. Es wurden 393 mg des Zielprodukts erhalten.

LC-MS (Methode 2): Rₜ = 0.93 min; m/z = 362 (M+H)⁺.

### Beispiel 11A

### 2-(4-Chlorphenyl)-3-(2,5-diazabicyclo[2.2.2]oct-2-ylmethyl)imidazo[l,2-a]pyrimidin-Dihydrochlorid (Enantiomer 1)

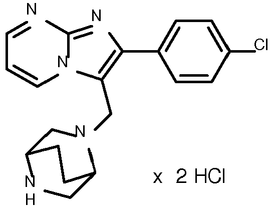

*tert*.-Butyl-5-{[2-(4-chlorphenyl)imidazo[1,2-a]pyrimidin-3-yl]methyl}-2,5-diazabicyclo[2.2.2]-octan-2-carboxylat (Enantiomer 1; 1.29 g, 2.84 mmol) wurde unter Rühren mit 7.1 ml einer 4 M Lösung von Chlorwasserstoff in Dioxan versetzt. Das Gemisch wurde über Nacht bei Raumtemperatur gerührt. Anschließend wurde der erhaltene Feststoff abgesaugt, mehrfach mit Diethylether gewaschen und im Hochvakuum bei 40°C getrocknet. Es wurden 1.4 g des Zielprodukts erhalten.

LC-MS (Methode 2): Rₜ = 0.79 min; m/z = 354 (M+H)⁺.

### Beispiel 12A

### 2-(4-Chlorphenyl)-3-(2,5-diazabicyclo[2.2.2]oct-2-ylmethyl)imidazo[1,2-a]pyrimidin-Dihydrochlorid (Enantiomer 2)

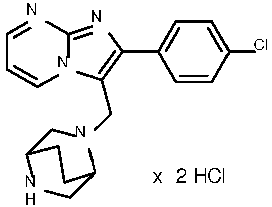

*tert*.-Butyl-5-{[2-(4-chlorphenyl)imidazo[1,2-a]pyrimidin-3-yl]methyl}-2,5-diazabicyclo[2.2.2]-octan-2-carboxylat (Enantiomer 2; 710 mg, 1.56 mmol) wurde unter Rühren mit 3.9 ml einer 4 M Lösung von Chlorwasserstoff in Dioxan versetzt. Das Gemisch wurde über Nacht bei Raumtemperatur gerührt. Anschließend wurde der erhaltene Feststoff abgesaugt, mehrfach mit Diethylether gewaschen und im Hochvakuum bei 40°C getrocknet. Es wurden 740 mg des Zielprodukts erhalten.

LC-MS (Methode 1): Rₜ = 0.49 min; m/z = 354 (M+H)⁺.

### Beispiel 13A

### 3-(2,5-Diazabicyclo[2.2.2]oct-2-ylmethyl)-2-(4-isopropylphenyl)imidazo[1,2-a]pyrimidin-Dihydrochlorid (Enantiomer 1)

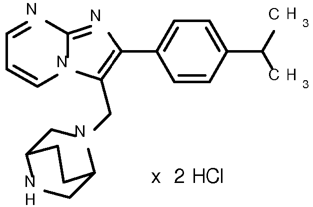

*tert.*-Butyl-5- { [2-(4-isopropylphenyl)imidazo [1,2-a]pyrimidin-3-yl] methyl } -2,5-diazabicyclo-[2.2.2]octan-2-carboxylat (Enantiomer 1; 774 mg, 1.88 mmol) wurde unter Rühren mit 4.2 ml einer 4 M Lösung von Chlorwasserstoff in Dioxan versetzt. Das Gemisch wurde über Nacht bei Raumtemperatur gerührt. Anschließend wurde der erhaltene Feststoff abgesaugt, mehrfach mit Diethylether gewaschen und im Hochvakuum bei 40°C getrocknet. Es wurden 850 mg des Zielprodukts erhalten.

LC-MS (Methode 1): Rₜ = 0.54 min; m/z = 362 (M+H)⁺.

### Beispiel 14A

### 3-(2,5-Diazabicyclo[2.2.2]oct-2-ylmethyl)-2-(4-isopropylphenyl)imidazo[1,2-a]pyrimidin-Dihydrochlorid (Enantiomer 2)

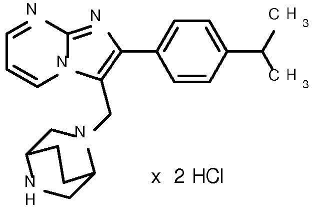

*tert*.-Butyl-5-{[2-(4-isopropylphenyl)imidazo[1,2-a]pyrimidin-3-yl]methyl}-2,5-diazabicyclo-[2.2.2]octan-2-carboxylat (Enantiomer 2; 734 mg, 1.59 mmol) wurde unter Rühren mit 4.0 ml einer 4 M Lösung von Chlorwasserstoff in Dioxan versetzt. Das Gemisch wurde über Nacht bei Raumtemperatur gerührt. Anschließend wurde der erhaltene Feststoff abgesaugt, mehrfach mit Diethylether gewaschen und im Hochvakuum bei 40°C getrocknet. Es wurden 761 mg des Zielprodukts erhalten.

LC-MS (Methode 1): Rₜ = 0.55 min; m/z = 362 (M+H)⁺.

### Beispiel 15A

### 1-[2-(4-Chlorphenyl)imidazo[1,2-a]pyrimidin-3-yl]ethanol (Racemat)

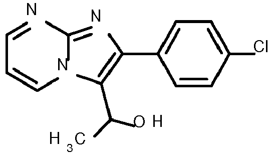

2-(4-Chlorphenyl)imidazo[1,2-a]pyrimidin-3-carbaldehyd (500 mg, 1.94 mmol) wurde in 5 ml THF suspendiert. Anschließend wurde unter Eiskühlung mit Methylmagnesiumbromid in Diethylether (3.0 M, 710 µl,2.1 mmol) versetzt und für 1 h bei Raumtemperatur gerührt. Danach wurden weitere 4 ml THF und nochmals Methylmagnesiumbromid in Diethylether (3.0 M, 237 µl, 0.7 mmol) hinzugefügt. Das Gemisch wurde über Nacht bei Raumtemperatur gerührt. Dann wurde wässrige Ammoniumchlorid-Lösung hinzugefügt und mit Wasser und Essigsäureethylester versetzt. Die resultierende organische Phase wurde abgetrennt, mit gesättigter Natriumchlorid-Lösung gewaschen, über Magnesiumsulfat getrocknet, filtriert und im Vakuum am Rotationsverdampfer bis zur Trockene eingeengt. Der Rückstand wurde in Diethylether verrührt. Der verbliebene Feststoff wurde abgesaugt und über Nacht im Hochvakuum-Trockenschrank bei 40°C getrocknet. Es wurden 370 mg (1.35 mmol, 70% d. Th.) des Zielprodukts erhalten.

LC-MS (Methode 2): Rₜ = 1.22 min; m/z = 274 (M+H)⁺.

### Beispiel 16A

### 2-(4-Chlorphenyl)-3-[1-(3,8-diazabicyclo[3.2.1]oct-3-yl)ethyl]imidazo[1,2-a]pyrimidin-Dihydrochlorid (Racemat)

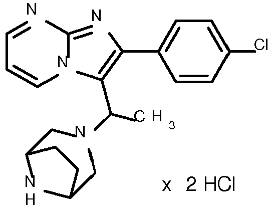

*tert.*-Butyl-3-{1-[2-(4-chlorphenyl)imidazo[1,2-a]pyrimidin-3-yl]ethyl}-3,8-diazabicyclo[3.2.1]-octan-8-carboxylat (Racemat; 39.8 mg, 0.09 mmol) wurde unter Rühren mit 0.21 ml einer 4 M Lösung von Chlorwasserstoff in Dioxan und mit 0.2 ml Dioxan versetzt. Das Gemisch wurde über Nacht bei Raumtemperatur gerührt. Anschließend wurde die Reaktionslösung bis zur Trockene eingeengt und der resultierende Rückstand im Hochvakuum bei 40°C getrocknet. Es wurden 41 mg des Zielprodukts erhalten.

LC-MS (Methode 2): Rₜ = 0.86 min; m/z = 256/258 (M+H)⁺.

Analog zu den Beispielen 7A-14A wurden die folgenden Verbindungen aus dem jeweils angegebenen Edukt hergestellt:

| **Beispiel** | **Name / Struktur / Edukt** | **Analytische Daten** |
|---|---|---|
| **17A** | 2-(4-Bromphenyl)-3-(2,5-diazabicyclo[2.2.2]-oct-2-ylmethyl)imidazo[1,2-a]pyrimidin-Dihydrochlorid (*Racemat*) | LC-MS (Methode 6): |
| | | Rₜ = 1.65 min; m/z = 398/400 (M+H)⁺. |
| | 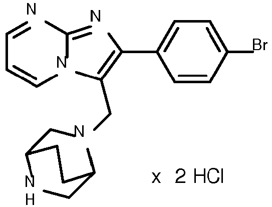 aus *tert*.-Butyl-5-{ [2-(4-bromphenyl)imidazo-[1,2-a]pyrimidin-3-yl]methyl}-2,5-diaza-bicyclo[2.2.2]octan-2-carboxylat (*Racemat*) | |
| **18A** | 2-(4-Bromphenyl)-3-(3,8-diazabicyclo[3.2.1]-oct-3-ylmethyl)imidazo[1,2-a]pyrimidin-Dihydrochlorid | LC-MS (Methode 6): |
| | | Rₜ = 1.56 min; m/z = 398/400 (M+H)⁺. |
| | 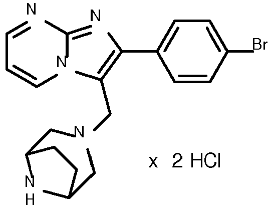 aus *tert*.-Butyl-3-{ [2-(4-bromphenyl)imidazo-[1,2-a]pyrimidin-3-yl]methyl}-3,8-diaza-bicyclo[3.2.1]octan-8-carboxylat | |

### Beispiel 19A

### 2-(4-Cyclopropylphenyl)-3-(3,8-diazabicyclo[3.2.1]octan-3-ylmethyl)imidazo[l,2-a]pyrimidin-Dihydrochlorid

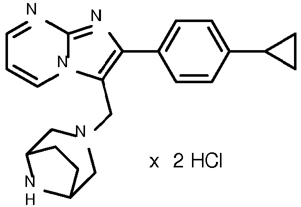

*tert.*-Butyl-3-{[2-(4-cyclopropylphenyl)imidazo[1,2-a] pyrimidin-3-yl] methyl} -3,8-diazabicyclo-[3.2.1]octan-8-carboxylat (720 mg, 1.57 mmol) wurde in 3 ml Dioxan gelöst und unter Rühren mit 3.92 ml einer 4 M Lösung von Chlorwasserstoff in Dioxan versetzt. Das Gemisch wurde über Nacht bei Raumtemperatur gerührt. Anschließend wurde die Reaktionslösung bis zur Trockene eingeengt und der resultierende Rückstand im Hochvakuum bei 40°C getrocknet. Es wurden 808 mg des Zielprodukts erhalten.

LC-MS (Methode 1): Rₜ = 0.48 min; m/z = 360 (M+H)⁺.

### Ausführungsbeispiele_{:}

### Beispiel 1

### tert.-Butyl-7-{[2-(4-chlorphenyl)imidazo[1,2-a]pyrimidin-3-yl]methyl}-3-oxa-7,9-diazabicyclo-[3.3.1]nonan-9-carboxylat

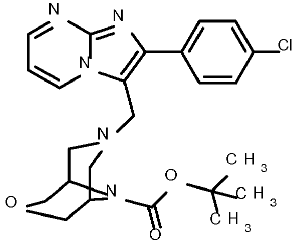

Unter Argon wurde bei Raumtemperatur 2-(4-Chlorphenyl)imidazo[1,2-a]pyrimidin-3-carbaldehyd (1.50 g, 5.82 mmol) in 25 ml THF gelöst und mit tert.-Butyl-3-oxa-7,9-diazabicyclo[3.3.1]nonan-9-carboxylat (1.59 g, 6.99 mmol) und Essigsäure (670 µl, 12 mmol) versetzt. Anschließend wurde portionsweise Natriumtriacetoxyborhydrid (1.85 g, 8.73 mmol) hinzugegeben und die Reaktionslösung über Nacht bei Raumtemperatur gerührt. Dann wurde langsam und vorsichtig Wasser zugetropft (Vorsicht: Gasentwicklung) und anschließend mit Essigsäureethylester versetzt. Die resultierende organische Phase wurde abgetrennt und die wässrige Phase zweimal mit Essigsäureethylester extrahiert. Die vereinigten organischen Phasen wurden mit gesättigter Natriumchlorid-Lösung gewaschen, über Magnesiumsulfat getrocknet, filtriert und im Vakuum am Rotationsverdampfer bis zur Trockene eingeengt. Der erhaltene Rückstand wurde aus Diethylether kristallisiert. Die entstandenen Kristalle wurden abgesaugt und über Nacht im Hochvakuum-Trockenschrank bei 40°C getrocknet. Es wurden 1.52 g (3.23 mmol, 56% d. Th.) der Zielverbindung erhalten.

LC-MS (Methode 2): Rₜ = 1.65 min; m/z = 470/472 (M+H)⁺.

¹H-NMR (400 MHz, DMSO-d₆): δ [ppm] = 1.40 (s, 9H), 2.42 (br. d, 2H), 2.87 (br. d, 2H), 3.57 (br. d, 2H), 3.72 (br. dd, 2H), 3.84 (br. d, 2H), 3.92 (s, 2H), 7.08 (dd, 1H), 7.55 (d, 2H), 7.96 (d, 2H), 8.58 (dd, 1H), 9.28 (dd, 1H).

### Beispiel 2

### tert.-Butyl-7-{[2-(4-isopropylphenyl)imidazo[1,2-a]pyrimidin-3-yl]methyl}-3-oxa-7,9-diaza-bicyclo[3.3.1]nonan-9-carboxylat

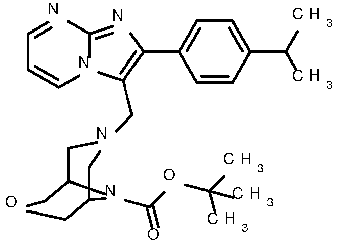

Unter Argon wurde bei Raumtemperatur 2-(4-Isopropylphenyl)imidazo[1,2-a]pyrimidin-3-carb-aldehyd (500 mg, 1.89 mmol) in 10 ml THF gelöst und mit tert.-Butyl-3-oxa-7,9-diazabicyclo-[3.3.1]nonan-9-carboxylat (516 mg, 2.26 mmol) und Essigsäure (220 µl, 3.77 mmol) versetzt. Anschließend wurde portionsweise Natriumtriacetoxyborhydrid (599 mg, 2.83 mmol) hinzugegeben und die Reaktionslösung über Nacht bei Raumtemperatur gerührt. Dann wurde langsam und vorsichtig Wasser zugetropft (Vorsicht: Gasentwicklung) und anschließend mit Essigsäureethylester versetzt. Die resultierende organische Phase wurde abgetrennt und die wässrige Phase zweimal mit Essigsäureethylester extrahiert. Die vereinigten organischen Phasen wurden mit gesättigter Natriumchlorid-Lösung gewaschen, über Magnesiumsulfat getrocknet, filtriert und im Vakuum am Rotationsverdampfer bis zur Trockene eingeengt. Der erhaltene Rückstand wurde mittels Säulenchromatographie gereinigt (Biotage Isolera, Biotage SNAP-KP-NH-Säule, Laufmittel Cyclohexan/ Essigsäureethylester-Gradient). Es wurden 431 mg (0.9 mmol, 48% d. Th.) der Zielverbindung erhalten.

LC-MS (Methode 2): Rₜ = 1.79 min; m/z = 478 (M+H)⁺.

¹H-NMR (400 MHz, DMSO-d₆): δ [ppm] = 1.25 (d, 6H), 1.40 (s, 9H), 2.39 (br. d, 2H), 2.87 (br. d, 2H), 2.90 (m, 1H), 3.57 (br. d, 2H), 3.72 (br. dd, 2H), 3.84 (br. d, 2H), 3.95 (s, 2H), 7.05 (dd, 1H), 7.36 (d, 2H), 7.80 (d, 2H), 8.55 (dd, 1H), 9.27 (dd, 1H).

### Beispiel 3

### tert.-Butyl-5-{[2-(4-chlorphenyl)imidazo[1,2-a]pyrimidin-3-yl]methyl}-2,5-diazabicyclo[2.2.2]-octan-2-carboxylat (Racemat)

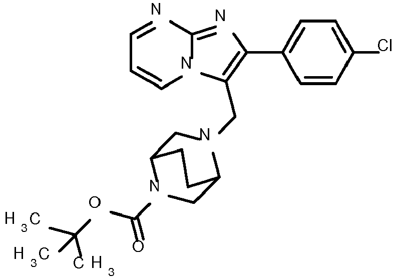

Unter Argon wurde bei Raumtemperatur 2-(4-Chlorphenyl)imidazo[1,2-a]pyrimidin-3-carbaldehyd (4.00 g, 15.5 mmol) in 100 ml THF gelöst und mit *tert.*-Butyl-2,5-diazabicyclo[2.2.2]octan-2-carboxylat (3.95 g, 18.6 mmol) und Essigsäure (1.8 ml, 31 mmol) versetzt. Anschließend wurde portionsweise Natriumtriacetoxyborhydrid (4.93 g, 23.3 mmol) hinzugegeben und die Reaktionslösung über Nacht bei Raumtemperatur gerührt. Danach wurde weiteres *tert*.-Butyl-2,5-diaza-bicyclo[2.2.2]octan-2-carboxylat (1.6 g, 7.76 mmol) und Natriumtriacetoxyborhydrid (1.2 g, 5.8 mmol) hinzugegeben und die Reaktionslösung nochmals über Nacht bei Raumtemperatur gerührt. Dann wurde langsam und vorsichtig Wasser zugetropft (Vorsicht: Gasentwicklung) und anschließend mit Essigsäureethylester versetzt. Die resultierende organische Phase wurde abgetrennt und die wässrige Phase zweimal mit Essigsäureethylester extrahiert. Die vereinigten organischen Phasen wurden über Magnesiumsulfat getrocknet, filtriert und im Vakuum am Rotationsverdampfer bis zur Trockene eingeengt. Der erhaltene Rückstand wurde mittels Säulenchromatographie gereinigt (Biotage Isolera, Biotage SNAP-KP-NH-Säule, Laufmittel Cyclohexan/Essigsäureethylester-Gradient). Es wurden 3.17 g (6.7 mmol, 43% d. Th.) der Zielverbindung erhalten.

LC-MS (Methode 2): Rₜ = 1.55 min; m/z = 454/456 (M+H)⁺.

### Beispiel 4 und Beispiel 5

### tert.-Butyl-5-{[2-(4-chlorphenyl)imidazo[1,2-a]pyrimidin-3-yl]methyl}-2,5-diazabicyclo[2.2.2]-octan-2-carboxylat (Enantiomer 1 und 2)

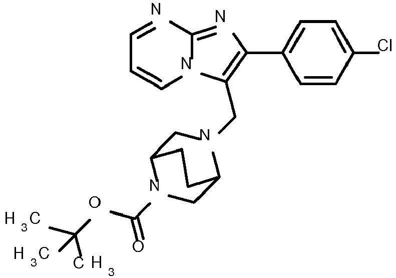

3.17 g (6.70 mmol) des racemischen *tert*.-Butyl-5-{[2-(4-chlorphenyl)imidazo[1,2-a]pyrimidin-3-yl]methyl}-2,5-diazabicyclo[2.2.2]octan-2-carboxylats (Beispiel 3) wurden durch präparative SFC-HPLC an chiraler Phase in die Enantiomere aufgetrennt [Säule: Daicel Chiralpak OJ-H, 5 µm, 250 mm x 30 mm; Elutionsmittel: Kohlendioxid/Ethanol 85:15 (v/v); Fluss: 150 ml/min; Druck: 135 bar; UV-Detektion: 210 nm; Temperatur: 38°C]:

### Beispiel 4 (Enantiomer 1):

Ausbeute: 1.29 g
Rₜ = 4.15 min; chemische Reinheit >99%; >99% ee
[Säule: Daicel Chiralpak OJ-H, 3 µm, 100 mm x 4.6 mm; Elutionsmittel: Kohlendioxid/Ethanol 85:15 (v/v); Fluss: 3 ml/min; Druck: 130 bar; Temperatur: 40°C; UV-Detektion: 210 nm].

LC-MS (Methode 2): Rₜ = 1.55 min; m/z = 454/456 (M+H)⁺.

¹H-NMR (400 MHz, DMSO-d₆): δ [ppm] = 1.36 (d, 9H), 1.42-1.55 (m, 1H), 1.57-1.73 (m, 2H), 1.79-1.92 (m, 1H), 2.63-2.80 (m, 3H), 3.09-3.17 (m, 1H), 3.47-3.56 (m, 1H), 3.80 (br. d, 1H), 4.18-4.29 (m, 2H), 7.12 (dd, 1H), 7.56 (d, 2H), 7.84-7.93 (m, 2H), 8.59 (dd, 1H), 9.02 (br. d, 1H).

### Beispiel 5 (Enantiomer 2):

Ausbeute: 720 mg
Rₜ = 6.6 min; chemische Reinheit >99%; >99% ee
[Säule: Daicel Chiralpak OJ-H, 3 µm, 100 mm x 4.6 mm; Elutionsmittel: Kohlendioxid/Ethanol 85:15 (v/v); Fluss: 3 ml/min; Druck: 130 bar; Temperatur: 40°C; UV-Detektion: 210 nm].

LC-MS (Methode 2): Rₜ = 1.56 min; m/z = 454/456 (M+H)⁺.

¹H-NMR (400 MHz, DMSO-d₆): δ [ppm] = 1.36 (d, 9H), 1.43-1.55 (m, 1H), 1.57-1.73 (m, 2H), 1.80-1.92 (m, 1H), 2.63-2.80 (m, 3H), 3.14 (br. dd, 1H), 3.47-3.56 (m, 1H), 3.80 (br. d, 1H), 4.18-4.29 (m, 2H), 7.12 (dd, 1H), 7.56 (d, 2H), 7.84-7.94 (m, 2H), 8.59 (dd, 1H), 9.02 (br. d, 1H).

### Beispiel 6

### tert.-Butyl-3-{[2-(4-chlorphenyl)imidazo[1,2-a]pyrimidin-3-yl]methyl}-3,8-diazabicyclo[3.2.1]-octan-8-carboxylat

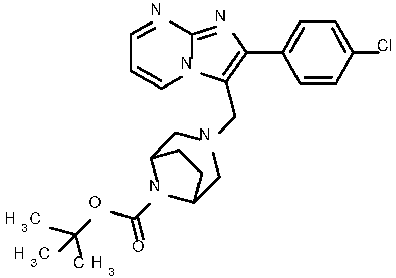

Unter Argon wurde bei Raumtemperatur 2-(4-Chlorphenyl)imidazo[1,2-a]pyrimidin-3-carbaldehyd (1.50 g, 5.82 mmol) in 25 ml THF gelöst und mit *tert.*-Butyl-3,8-diazabicyclo[3.2.1]octan-8-carboxylat (1.48 g, 6.99 mmol) und Essigsäure (670 µl, 12 mmol) versetzt. Anschließend wurde portionsweise Natriumtriacetoxyborhydrid (1.85 g, 8.73 mmol) hinzugegeben und die Reaktionslösung über Nacht bei Raumtemperatur gerührt. Dann wurde langsam und vorsichtig Wasser zugetropft (Vorsicht: Gasentwicklung) und anschließend mit Essigsäureethylester versetzt. Die resultierende organische Phase wurde abgetrennt und die wässrige Phase zweimal mit Essigsäureethylester extrahiert. Die vereinigten organischen Phasen wurden mit gesättigter Natriumchlorid-Lösung gewaschen, über Magnesiumsulfat getrocknet, filtriert und im Vakuum am Rotationsverdampfer bis zur Trockene eingeengt. Der erhaltene Rückstand wurde aus Diethylether kristallisiert. Die entstandenen Kristalle wurden in Acetonitril aufgenommen, und der verbliebene Niederschlag wurde abgesaugt und über Nacht im Hochvakuum-Trockenschrank bei 40°C getrocknet. Es wurden 840 mg (1.85 mmol, 32% d. Th.) der Zielverbindung erhalten.

LC-MS (Methode 2): Rₜ = 2.06 min; m/z = 454/456 (M+H)⁺

¹H-NMR (400 MHz, DMSO-d₆): δ [ppm] = 1.39 (s, 9H), 1.64 (br. s, 4H), 2.26 (br. d, 2H), 2.42-2.60 (m, 2H, verdeckt durch DMSO-Signal), 3.96-4.05 (m, 4H), 7.14 (dd, 1H), 7.56 (d, 2H), 7.95 (d, 2H), 8.59 (dd, 1H), 9.03 (dd, 1H).

### Beispiel 7

### tert.-Butyl-5-{[2-(4-isopropylphenyl)imidazo[1,2-a]pyrimidin-3-yl]methyl}-2,5-diazabicyclo-[2.2.2]octan-2-carboxylat (Racemat)

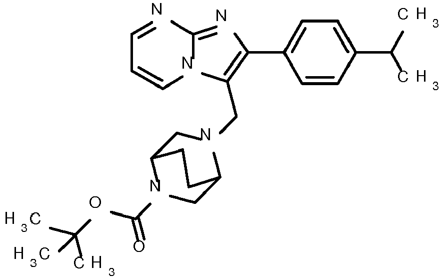

Unter Argon wurde bei Raumtemperatur 2-(4-Isopropylphenyl)imidazo[1,2-a]pyrimidin-3-carbaldehyd (1.50 g, 5.65 mmol) in 20 ml THF gelöst und mit *tert*.-Butyl-2,5-diazabicyclo[2.2.2]octan-2-carboxylat (Racemat; 1.44 g, 6.78 mmol) und Essigsäure (650 µl, 11.31 mmol) versetzt. Anschließend wurde portionsweise Natriumtriacetoxyborhydrid (1.8 g, 8.48 mmol) hinzugegeben und die Reaktionslösung über Nacht bei Raumtemperatur gerührt. Dann wurde langsam und vorsichtig Wasser zugetropft (Vorsicht: Gasentwicklung) und anschließend mit Essigsäureethylester versetzt. Die resultierende organische Phase wurde abgetrennt und die wässrige Phase zweimal mit Essigsäureethylester extrahiert. Die vereinigten organischen Phasen wurden mit gesättigter Natriumchlorid-Lösung gewaschen, über Magnesiumsulfat getrocknet, filtriert und im Vakuum am Rotationsverdampfer bis zur Trockene eingeengt. Der erhaltene Rückstand wurde mittels Säulenchromatographie gereinigt (Biotage Isolera, Biotage SNAP-KP-NH-Säule, Laufmittel Cyclohexan/ Essigsäureethylester-Gradient). Es wurden 1760 mg (3.81 mmol, 67% d. Th.) der Zielverbindung erhalten.

LC-MS (Methode 2): Rₜ = 1.71 min; m/z = 462 (M+H)⁺.

### Beispiel 8 und Beispiel 9

### tert.-Butyl-5-{[2-(4-isopropylphenyl)imidazo[1,2-a]pyrimidin-3-yl]methyl}-2,5-diazabicyclo-[2.2.2]octan-2-carboxylat (Enantiomer 1 und 2)

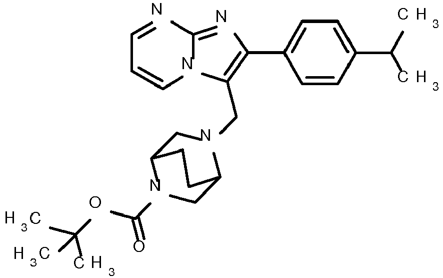

1.66 g (3.59 mmol) des racemischen *tert*.-Butyl-5-{[2-(4-isopropylphenyl)imidazo[1,2-a]pyrimidin-3-yl]methyl}-2,5-diazabicyclo[2.2.2]octan-2-carboxylats (Beispiel 7) wurden durch präparative SFC-HPLC an chiraler Phase in die Enantiomere aufgetrennt [Säule: Daicel Chiralpak OX-H (SFC), 5 µm, 250 mm x 30 mm; Elutionsmittel: Kohlendioxid/Methanol 62:38 (v/v); Fluss: 80 g/min; Druck: 120 bar; UV-Detektion: 210 nm; Temperatur: 38°C]:

### Beispiel 8 (Enantiomer 1):

Ausbeute: 774 mg
Rₜ = 4.91 min; chemische Reinheit >99%; >99% ee
[Säule: Daicel Chiralpak OX-3 (SFC), 3 µm, 100 mm x 4.6 mm; Elutionsmittel: Kohlendioxid/ Ethanol 70:30 (v/v); Fluss: 3 ml/min; Druck: 130 bar; Temperatur: 40°C; UV-Detektion: 210 nm].

LC-MS (Methode 1): Rₜ = 0.85 min; m/z = 462 (M+H)⁺.

[α]_{D}²⁰ = +16.21° (c = 0.270, Methanol).

¹H-NMR (400 MHz, DMSO-d₆): δ [ppm] = 1.25 (d, 6H), 1.36 (2s, 9H), 1.44-1.56 (m, 1H), 1.66 (br. s, 2H), 1.79-1.95 (m, 1H), 2.65-2.83 (m, 3H), 2.89-3.03 (m, 1H), 3.09-3.20 (m, 1H), 3.53 (br. d, 1H), 3.81 (br. d, 1H), 4.24 (s, 2H), 7.10 (dd, 1H), 7.37 (d, 2H), 7.78 (dd, 2H), 8.56 (dd, 1H), 8.99 (br. d, 1H).

### Beispiel 9 (Enantiomer 2):

Ausbeute: 734 mg
Rₜ = 6.88 min; chemische Reinheit >99%; >99% ee
[Säule: Daicel Chiralpak OX-3 (SFC), 3 µm, 100 mm x 4.6 mm; Elutionsmittel: Kohlendioxid/ Ethanol 70:30 (v/v); Fluss: 3 ml/min; Druck: 130 bar; Temperatur: 40°C; UV-Detektion: 210 nm].

LC-MS (Methode 1): Rₜ = 0.85 min; m/z = 462 (M+H)⁺.

[α]_{D}²⁰ = -15.67° (c = 0.270, Methanol).

¹H-NMR (400 MHz, DMSO-d₆): δ [ppm] = 1.25 (d, 6H), 1.36 (2s, 9H), 1.44-1.56 (m, 1H), 1.66 (br. s, 2H), 1.79-1.94 (m, 1H), 2.64-2.83 (m, 3H), 2.95 (dt, 1H), 3.09-3.20 (m, 1H), 3.53 (br. d, 1H), 3.81 (br. d, 1H), 4.24 (s, 2H), 7.10 (dd, 1H), 7.37 (d, 2H), 7.78 (dd, 2H), 8.56 (dd, 1H), 8.99 (br. d, 1H).

### Beispiel 10

### tert.-Butyl-3-{1-[2-(4-chlorphenyl)imidazo[1,2-a]pyrimidin-3-yl]ethyl}-3,8-diazabicyclo[3.2.1]-octan-8-carboxylat (Racemat)

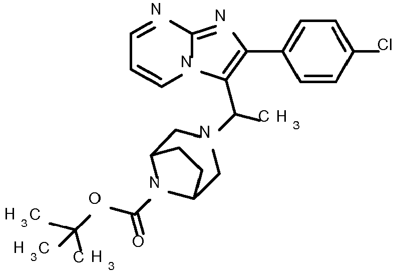

1-[2-(4-Chlorphenyl)imidazo[1,2-a]pyrimidin-3-yl]ethanol (473 mg, 1.73 mmol) und Triphenylphosphin (906 mg, 3.46 mmol) wurden in 10 ml Dichlormethan vorgelegt und unter Kühlung (Eisbad) portionsweise mit Tetrabromkohlenstoff (1.15 g, 3.46 mmol) versetzt. Anschließend wurde Triethylamin (480 µl, 3.5 mmol) hinzugefügt und das Gemisch 1 h bei Raumtemperatur nachgerührt. Der Ansatz wurde danach eingedampft und der Rückstand in 10 ml Acetonitril gelöst, *tert.-*Butyl-3,8-diazabicyclo[3.2.1]octan-8-carboxylat (734 mg, 3.46 mmol) wurde hinzugefügt, und das Reaktionsgemisch wurde über Nacht bei 40°C gerührt. Das Gemisch wurde dann wieder zur Trockene eingeengt. 400 mg des so erhaltenen Rückstands wurden direkt über präparative HPLC (Methode 8) in die Komponenten aufgetrennt. Die restliche Menge des Rückstands wurde auf Kieselgel aufgezogen und durch Säulenchromatographie vorgereinigt (Biotage Isolera, Biotage SNAP-KP-NH-Säule, Laufmittel Cyclohexan/Essigsäureethylester-Gradient). Das so vorgereinigte Produkt wurde dann über präparative HPLC (Methode 8) nachgereinigt. Es wurden auf diese Weise 50 mg (0.11 mmol, 6% d. Th.) der Titelverbindung erhalten.

LC-MS (Methode 2): Rε = 2.14 min; MS (ESIpos): m/z = 468/470 [M+H]⁺.

Analog zu den Beispielen 1-3 sowie 6-7 wurden die folgenden Verbindungen aus den jeweils angegebenen Edukten hergestellt:

| **Beispiel** | **Name / Struktur / Edukte** | **Analytische Daten** |
|---|---|---|
| **11** | *tert*.-Butyl-5-{[2-(4-bromphenyl)imidazo[1,2-a]-pyrimidin-3-yl]methyl}-2,5-diaza-bicyclo[2.2.2]octan-2-carboxylat (*Racemat*) | ¹H-NMR (400 MHz, DMSO-d₆): δ [ppm] = -0.149 (0.44), -0.008 (3.64), 0.008 (3.55), 0.146 (0.45), 1.345 (14.48), 1.374 (16.00), 1.402 (4.45), 1.489 (0.55), 1.654 (0.95), 1.859 (0.54), 2.328 (0.58), 2.670 (1.91), 2.709 (1.58), 2.774 (0.64), 3.118 (0.57), 3.146 (0.65), 3.155 (0.60), 3.515 (0.56), 3.774 (0.61), 3.827 (0.68), 4.235 (4.16), 5.754 (5.06), 7.108 (1.24), 7.119 (1.32), 7.125 (1.31), 7.136 (1.29), 7.679 (3.52), 7.700 (4.83), 7.812 (1.99), 7.824 (1.97), 7.833 (1.62), 7.845 (1.39), 8.579 (1.48), 8.584 (1.62), 8.590 (1.53), 8.594 (1.45), 9.013 (1.24), 9.030 (1.21). |
| | 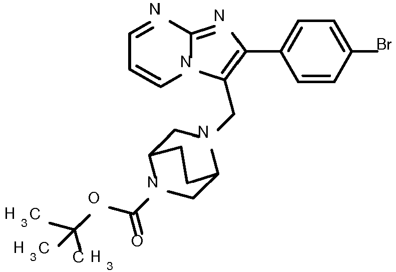 aus *tert*.-Butyl-2,5-diazabicyclo[2.2.2]octan-2-carboxylat (*Racemat*) und 2-(4-Bromphenyl)-imidazo[1,2-a]pyrimidin-3-carbaldehyd | |
| | | LC-MS (Methode 2): Rₜ = 1.68 min; m/z = 498/500 (M+H)⁺. |
| **12** | *tert*.-Butyl-3-{[2-(4-bromphenyl)imidazo[1,2-a]-pyrimidin-3-yl]methyl}-3,8-diazabicyclo-[3.2.1]octan-8-carboxylat | ¹H-NMR (400 MHz, DMSO-d₆): δ [ppm] = 1.39 (s, 9H), 1.56-1.73 (m, 4H), 2.26 (br. d, 2H), 2.46-2.60 (m, 2H, verdeckt durch DMSO-Signal), 3.98 (s, 2H), 4.02 (br. s, 2H), 7.14 (dd, 1H), 7.70 (d, 2H), 7.88 (d, 2H), 8.59 (dd, 1H), 9.03 (dd, 1H). |
| | 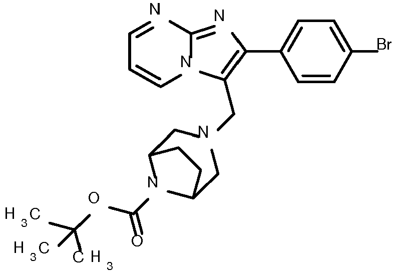 aus *tert*.-Butyl-3,8-diazabicyclo[3.2.1]octan-8-carboxylat und 2-(4-Bromphenyl)imidazo-[1,2-a]pyrimidin-3-carbaldehyd | |
| | | LC-MS (Methode 5): Rₜ = 1.42 min; m/z = 498/500 (M+H)⁺. |

### Beispiel 13

### (7-{[2-(4-Chlorphenyl)imidazo[1,2-a]pyrimidin-3-yl]methyl}-3-oxa-7,9-diazabicyclo[3.3.1]non-9-yl)(6-methoxypyridin-2-yl)methanon

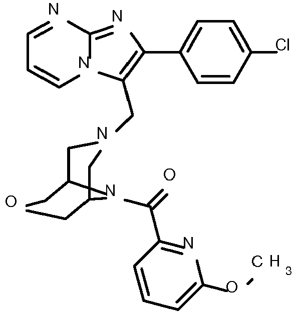

6-Methoxypyridin-2-carbonsäure (35.1 mg, 230 µmol) wurde in 1.5 ml DMF gelöst, mit 2-(7-Aza-1*H*-benzotriazol-1-yl)-1,1,3,3-tetramethyluronium-Hexafluorophosphat (HATU) (119 mg, 313 µmol) versetzt und 30 min bei Raumtemperatur gerührt. Anschließend wurde 7-{[2-(4-Chlorphenyl)imidazo[1,2-a]pyrimidin-3-yl]methyl}-3-oxa-7,9-diazabicyclo[3.3.1]nonan-Dihydrochlorid (100 mg) sowie *N,N*-Diisopropylethylamin (180 µl, 1.0 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 74 mg (0.15 mmol, 70% d. Th.) der Titelverbindung erhalten.

LC-MS (Methode 2): Rₜ = 1.48 min; m/z = 505/507 (M+H)⁺.

¹H-NMR (400 MHz, DMSO-d₆): δ [ppm] = 2.46-2.66 (m, 2H, teilweise verdeckt durch DMSO-Signal), 2.91 (br. d, 1H), 3.05 (br. d, 1H), 3.66-3.83 (m, 3H), 3.80 (s, 3H), 3.89 (d, 1H), 3.93-4.03 (m, 2H), 4.20 (br. s, 1H), 4.44 (br. s, 1H), 6.93 (d, 1H), 7.09 (dd, 1H), 7.29 (d, 1H), 7.54 (d, 2H), 7.83 (t, 1H), 7.97 (d, 2H), 8.58 (dd, 1H), 9.28 (dd, 1H).

### Beispiel 14

### (3-Chlor-6-methoxypyridin-2-yl)(7-{[2-(4-chlorphenyl)imidazo[1,2-a]pyrimidin-3-yl]methyl}-3-oxa-7,9-diazabicyclo[3.3.1]non-9-yl)methanon

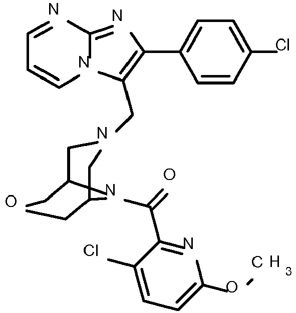

3-Chlor-6-methoxypyridin-2-carbonsäure (43.1 mg, 230 µmol) wurde in 1.5 ml DMF gelöst, mit 2-(7-Aza-1*H*-benzotriazol-1-yl)-1,1,3,3-tetramethyluronium-Hexafluorophosphat (HATU) (119 mg, 313 µmol) versetzt und 30 min bei Raumtemperatur gerührt. Anschließend wurde 7-{[2-(4-Chlorphenyl)imidazo[1,2-a]pyrimidin-3-yl]methyl}-3-oxa-7,9-diazabicyclo[3.3.1]nonan-Dihydrochlorid (100 mg) sowie *N,N*-Diisopropylethylamin (180 µl, 1.0 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 78 mg (0.15 mmol, 70% d. Th.) der Titelverbindung erhalten.

LC-MS (Methode 1): Rₜ = 0.86 min; m/z = 539/541 (M+H)⁺.

### Beispiel 15

### (7-{[2-(4-Chlorphenyl)imidazo[1,2-a]pyrimidin-3-yl]methyl}-3-oxa-7,9-diazabicyclo[3.3.1]non-9-yl)(3-fluor-6-methoxypyridin-2-yl)methanon

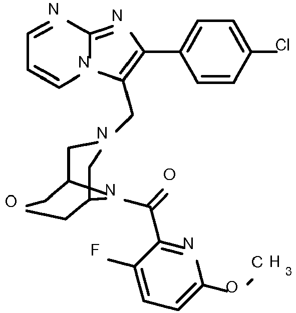

3-Fluor-6-methoxypyridin-2-carbonsäure (39.3 mg, 230 µmol) wurde in 1.5 ml DMF gelöst, mit 2-(7-Aza-1*H*-benzotriazol-1-yl)-1,1,3,3-tetramethyluronium-Hexafluorophosphat (HATU) (119 mg, 313 µmol) versetzt und 30 min bei Raumtemperatur gerührt. Anschließend wurde 7-{[2-(4-Chlorphenyl)imidazo[1,2-a]pyrimidin-3-yl]methyl}-3-oxa-7,9-diazabicyclo[3.3.1]nonan-Dihydrochlorid (100 mg) sowie *N,N*-Diisopropylethylamin (180 µl, 1.0 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 82 mg (0.16 mmol, 76% d. Th.) der Titelverbindung erhalten.

LC-MS (Methode 1): Rₜ = 0.82 min; m/z = 523/525 (M+H)⁺.

¹H-NMR (400 MHz, DMSO-d₆): δ [ppm] = 2.45-2.60 (m, 2H, verdeckt durch DMSO-Signal), 2.90 (br. d, 1H), 3.05 (br. d, 1H), 3.58-3.70 (m, 3H), 3.72-3.84 (m, 1H), 3.80 (s, 3H), 3.89 (d, 1H), 3.98 (s, 2H), 4.45 (br. s, 1H), 6.97 (dd, 1H), 7.08 (dd, 1H), 7.55 (d, 2H), 7.80 (t, 1H), 7.97 (d, 2H), 8.58 (dd, 1H), 9.28 (dd, 1H).

### Beispiel 16

### (7-{[2-(4-Chlorphenyl)imidazo[1,2-a]pyrimidin-3-yl]methyl}-3-oxa-7,9-diazabicyclo[3.3.1]non-9-yl) [6-(methylsulfanyl)pyridin-2-yl]methanon

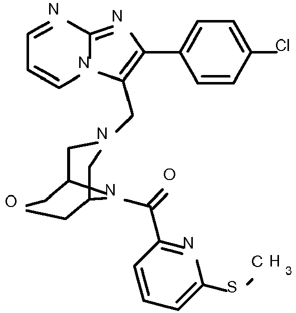

6-(Methylsulfanyl)pyridin-2-carbonsäure (38.8 mg, 230 µmol) wurde in 1.5 ml DMF gelöst, mit 2-(7-Aza-1*H*-benzotriazol-1-yl)-1,1,3,3-tetramethyluronium-Hexafluorophosphat (HATU) (119 mg, 313 µmol) versetzt und 30 min bei Raumtemperatur gerührt. Anschließend wurde 7-{[2-(4-Chlorphenyl)imidazo[1,2-a]pyrimidin-3-yl]methyl}-3-oxa-7,9-diazabicyclo[3.3.1]nonan-Dihydrochlorid (100 mg) sowie *N,N*-Diisopropylethylamin (180 µl, 1.0 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 84 mg (0.16 mmol, 77% d. Th.) der Titelverbindung erhalten.

LC-MS (Methode 1): Rₜ = 0.85 min; m/z = 521/523 (M+H)⁺.

¹H-NMR (400 MHz, DMSO-d₆): δ [ppm] = 2.46 (s, 3H), 2.56-2.65 (m, 2H), 2.91 (br. d, 1H), 3.06 (br. d, 1H), 3.65-3.81 (m, 3H), 3.86-4.03 (m, 3H), 4.15 (br. s, 1H), 4.46 (br. s, 1H), 7.09 (dd, 1H), 7.40 (dd, 2H), 7.55 (d, 2H), 7.77 (t, 1H), 7.98 (d, 2H), 8.58 (dd, 1H), 9.28 (dd, 1H).

### Beispiel 17

### (7-{[2-(4-Chlorphenyl)imidazo[1,2-a]pyrimidin-3-yl]methyl}-3-oxa-7,9-diazabicyclo[3.3.1]non-9-yl)(cyclopentyl)methanon

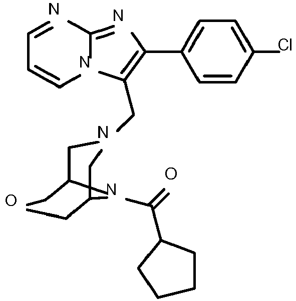

Cyclopentancarbonsäure (18 µl, 230 µmol) wurde in 1.5 ml DMF gelöst, mit 2-(7-Aza-1*H*-benzotriazol-1-yl)-1,1,3,3-tetramethyluronium-Hexafluorophosphat (HATU) (119 mg, 313 µmol) versetzt und 30 min bei Raumtemperatur gerührt. Anschließend wurde 7-{[2-(4-Chlorphenyl)imidazo-[1,2-a]pyrimidin-3-yl]methyl}-3-oxa-7,9-diazabicyclo[3.3.1]nonan-Dihydrochlorid (100 mg) sowie *N,N*-Diisopropylethylamin (180 µl, 1.0 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 72 mg (0.15 mmol, 74% d. Th.) der Titelverbindung erhalten.

LC-MS (Methode 1): Rₜ = 0.81 min; m/z = 466/468 (M+H)⁺.

¹H-NMR (400 MHz, DMSO-d₆): δ [ppm] = 1.43-1.81 (m, 8H), 2.31-2.61 (m, 2H, teilweise verdeckt durch DMSO-Signal), 2.86-2.97 (m, 3H), 3.47-3.54 (m, 1H), 3.56-3.63 (m, 1H), 3.77 (dd, 2H), 3.94 (s, 2H), 4.04 (br. s, 1H), 4.32 (br. s, 1H), 7.08 (dd, 1H), 7.55 (d, 2H), 7.97 (d, 2H), 8.59 (dd, 1H), 9.27 (dd, 1H).

### Beispiel 18

### (3-Fluor-6-methoxypyridin-2-yl)(7-{[2-(4-isopropylphenyl)imidazo[1,2-a]pyrimidin-3-yl]methyl}-3-oxa-7,9-diazabicyclo[3.3.1]non-9-yl)methanon

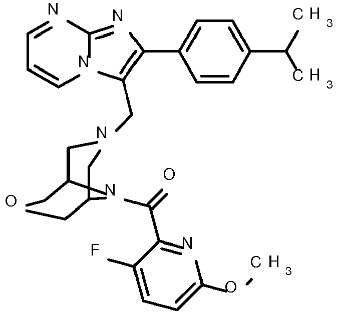

3-Fluor-6-methoxypyridin-2-carbonsäure (39 mg, 0.23 mmol) wurde in 1.5 ml DMF gelöst, mit 2-(7-Aza-1*H*-benzotriazol-1-yl)-1,1,3,3-tetramethyluronium-Hexafluorophosphat (HATU) (117 mg, 0.31 mmol) versetzt und 30 min bei Raumtemperatur gerührt. Anschließend wurde 7-{ [2-(4-Isopropylphenyl)imidazo[1,2-a]pyrimidin-3-yl]methyl}-3-oxa-7,9-diazabicyclo[3.3.1]nonan-Dihydrochlorid (100 mg) sowie *N,N*-Diisopropylethylamin (180 µl,1.0 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 83 mg (0.16 mmol, 76% d. Th.) der Titelverbindung erhalten.

LC-MS (Methode 1): Rₜ = 0.84 min; m/z = 531 (M+H)⁺.

¹H-NMR (400 MHz, DMSO-d₆): δ [ppm] = 1.26 (d, 6H), 2.46-2.58 (m, 2H, verdeckt durch DMSO-Signal), 2.86-3.01 (m, 2H), 3.06 (br. d, 1H), 3.57-3.70 (m, 3H), 3.75 (br. d, 1H), 3.79 (s, 3H), 3.89 (d, 1H), 3.99 (s, 2H), 4.46 (br. s, 1H), 6.97 (dd, 1H), 7.06 (dd, 1H), 7.36 (d, 2H), 7.74-7.84 (m, 3H), 8.55 (dd, 1H), 9.26 (dd, 1H).

### Beispiel 19

### [6-(Difluormethoxy)pyridin-2-yl](7-{[2-(4-isopropylphenyl)imidazo[1,2-a]pyrimidin-3-yl]methyl}-3-oxa-7,9-diazabicyclo[3.3.1]non-9-yl)methanon

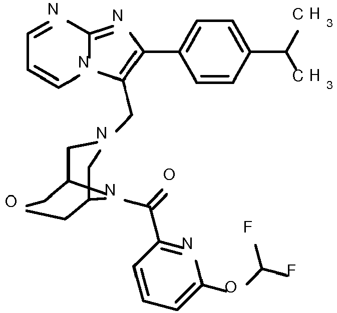

6-(Difluormethoxy)pyridin-2-carbonsäure (43 mg, 0.23 mmol) wurde in 1.5 ml DMF gelöst, mit 2-(7-Aza-1*H*-benzotriazol-1-yl)-1,1,3,3-tetramethyluronium-Hexafluorophosphat (HATU) (117 mg, 0.31 mmol) versetzt und 30 min bei Raumtemperatur gerührt. Anschließend wurde 7-{[2-(4-Isopropylphenyl)imidazo[1,2-a]pyrimidin-3-yl]methyl}-3-oxa-7,9-diazabicyclo[3.3.1]nonan-Dihydrochlorid (100 mg) sowie *N,N*-Diisopropylethylamin (180 µl, 1.0 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 72 mg (0.13 mmol, 61% d. Th.) der Titelverbindung erhalten.

LC-MS (Methode 1): Rₜ = 0.89 min; m/z = 549 (M+H)⁺.

¹H-NMR (400 MHz, DMSO-d₆): δ [ppm] = 1.25 (d, 6H), 2.45-2.64 (m, 3H, teilweise verdeckt durch DMSO-Signal), 2.86-2.99 (m, 2H), 3.05 (br. d, 1H), 3.65-3.79 (m, 3H), 3.89 (d, 1H), 4.00 (s, 2H), 4.09 (br. s, 1H), 4.44 (br. s, 1H), 7.06 (dd, 1H), 7.21 (d, 1H), 7.36 (d, 2H), 7.54-7.62 (m, 1H), 7.81 (d, 2H), 8.06 (t, 1H), 8.55 (dd, 1H), 9.28 (dd, 1H).

### Beispiel 20

### (3-{[2-(4-Chlorphenyl)imidazo[1,2-a]pyrimidin-3-yl]methyl}-3,8-diazabicyclo[3.2.1]oct-8-yl)-(6-methoxypyridin-2-yl)methanon

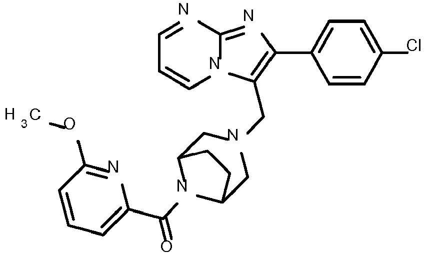

6-Methoxypyridin-2-carbonsäure (36.4 mg, 237 µmol) wurde in 1.5 ml DMF gelöst, mit 2-(7-Aza-1*H*-benzotriazol-1-yl)-1,1,3,3-tetramethyluronium-Hexafluorophosphat (HATU) (119 mg, 313 µmol) versetzt und 30 min bei Raumtemperatur gerührt. Anschließend wurde 2-(4-Chlorphenyl)-3-(3,8-diazabicyclo[3.2.1]oct-3-ylmethyl)imidazo[1,2-a]pyrimidin-Dihydrochlorid (100 mg) sowie *N,N*-Diisopropylethylamin (190 µl, 1.1 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 79 mg (0.16 mmol, 74% d. Th.) der Titelverbindung erhalten.

LC-MS (Methode 1): Rₜ = 1.76 min; m/z = 489/491 (M+H)⁺.

¹H-NMR (400 MHz, DMSO-d₆): δ [ppm] = 1.63-1.84 (m, 4H), 2.45 (br. d, 1H), 2.56-2.65 (m, 2H), 2.73 (dd, 1H), 3.77 (s, 3H), 4.00-4.12 (m, 2H), 4.67 (br. d, 2H), 6.93 (d, 1H), 7.15 (dd, 1H), 7.35 (d, 1H), 7.57 (d, 2H), 7.82 (t, 1H), 7.96 (d, 2H), 8.59 (dd, 1H), 9.06 (dd, 1H).

### Beispiel 21

### (3-Chlor-6-methoxypyridin-2-yl)(3-{[2-(4-chlorphenyl)imidazo[1,2-a]pyrimidin-3-yl]methyl}-3,8-diazabicyclo[3.2.1]oct-8-yl)methanon

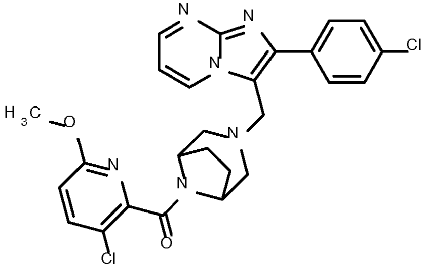

3-Chlor-6-methoxypyridin-2-carbonsäure (44.5 mg, 237 µmol) wurde in 1.5 ml DMF gelöst, mit 2-(7-Aza-1*H*-benzotriazol-1-yl)-1,1,3,3-tetramethyluronium-Hexafluorophosphat (HATU) (119 mg, 313 µmol) versetzt und 30 min bei Raumtemperatur gerührt. Anschließend wurde 2-(4-Chlorphenyl)-3-(3,8-diazabicyclo[3.2.1]oct-3-ylmethyl)imidazo[1,2-a]pyrimidin-Dihydrochlorid (100 mg) sowie *N,N*-Diisopropylethylamin (190 µl, 1.1 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 56 mg (0.11 mmol, 49% d. Th.) der Titelverbindung erhalten.

LC-MS (Methode 2): Rₜ = 1.81 min; m/z = 523/524/525 (M+H)⁺.

¹H-NMR (400 MHz, DMSO-d₆): δ [ppm] = 1.62-1.82 (m, 4H), 2.34-2.46 (m, 2H), 2.47-2.59 (m, 1H, verdeckt durch DMSO-Signal), 2.69-2.78 (m, 1H), 3.62 (br. s, 1H), 3.79 (s, 3H), 4.06 (s, 2H), 4.59 (br. s, 1H), 6.92 (d, 1H), 7.15 (dd, 1H), 7.57 (d, 2H), 7.87 (d, 1H), 7.94 (d, 2H), 8.59 (dd, 1H), 9.04 (dd, 1H).

### Beispiel 22

### (3-{[2-(4-Chlorphenyl)imidazo[1,2-a]pyrimidin-3-yl]methyl}-3,8-diazabicyclo[3.2.1]oct-8-yl)-(3-fluor-6-methoxypyridin-2-yl)methanon

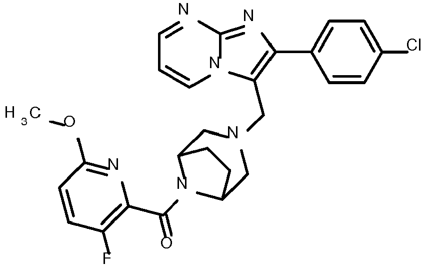

3-Fluor-6-methoxypyridin-2-carbonsäure (40.6 mg, 237 µmol) wurde in 1.5 ml DMF gelöst, mit 2-(7-Aza-1*H*-benzotriazol-1-yl)-1,1 ,3,3-tetramethyluronium-Hexafluorophosphat (HATU) (123 mg, 324 µmol) versetzt und 30 min bei Raumtemperatur gerührt. Anschließend wurde 2-(4-Chlorphenyl)-3-(3,8-diazabicyclo[3.2.1]oct-3-ylmethyl)imidazo[1,2-a]pyrimidin-Dihydrochlorid (100 mg) sowie *N,N*-Diisopropylethylamin (190 µl, 1.1 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 93 mg (0.18 mmol, 85% d. Th.) der Titelverbindung erhalten.

LC-MS (Methode 2): Rₜ = 1.73 min; m/z = 507/509 (M+H)⁺.

¹H-NMR (400 MHz, DMSO-d₆): δ [ppm] = 1.62-1.83 (m, 4H), 2.44 (br. t, 2H), 2.48-2.58 (m, 1H, teilweise verdeckt durch DMSO-Signal), 2.75 (dd, 1H), 3.76 (s, 3H), 3.92 (br. s, 1H), 4.01-4.12 (m, 2H), 4.61 (br. s, 1H), 6.95 (dd, 1H), 7.14 (dd, 1H), 7.57 (d, 2H), 7.77 (t, 1H), 7.95 (d, 2H), 8.59 (dd, 1H), 9.06 (dd, 1H).

### Beispiel 23

### (3-Chlor-6-methoxypyridin-2-yl)(5-{[2-(4-chlorphenyl)imidazo[1,2-a]pyrimidin-3-yl]methyl}-2,5-diazabicyclo[2.2.2]oct-2-yl)methanon (Enantiomer 1)

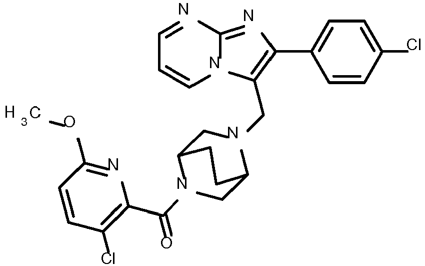

3-Chlor-6-methoxypyridin-2-carbonsäure (44.5 mg, 237 µmol) wurde in 1.5 ml DMF gelöst, mit 2-(7-Aza-1*H*-benzotriazol-1-yl)-1,1,3,3-tetramethyluronium-Hexafluorophosphat (HATU) (123 mg, 324 µmol) versetzt und 30 min bei Raumtemperatur gerührt. Anschließend wurde 2-(4-Chlorphenyl)-3-(2,5-diazabicyclo[2.2.2]oct-2-ylmethyl)imidazo[1,2-a]pyrimidin-Dihydrochlorid (Enantiomer 1; 100 mg) sowie *N,N*-Diisopropylethylamin (190 µl, 1.1 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 87 mg (0.16 mmol, 74% d. Th.) der Titelverbindung erhalten.

LC-MS (Methode 2): Rₜ = 1.62 min; m/z = 523/524/525 (M+H)⁺.

¹H-NMR (400 MHz, DMSO-d₆): δ [ppm] = 1.52-2.00 (m, 4H), 2.57-3.24 (m, 3.75H), 3.35-3.46 (m, 1.25H), 3.70-3.86 (m, 3.75H), 4.20-4.40 (m, 2.25H), 6.84-6.96 (m, 1H), 7.08-7.19 (m, 1H), 7.49-7.61 (m, 2H), 7.79-7.93 (m, 3H), 8.56-8.64 (m, 1H), 8.98-9.07 (m, 1H).

### Beispiel 24

### (5-{[2-(4-Chlorphenyl)imidazo[1,2-a]pyrimidin-3-yl]methyl}-2,5-diazabicyclo[2.2.2]oct-2-yl)-(3-fluor-6-methoxypyridin-2-yl)methanon (Enantiomer 2)

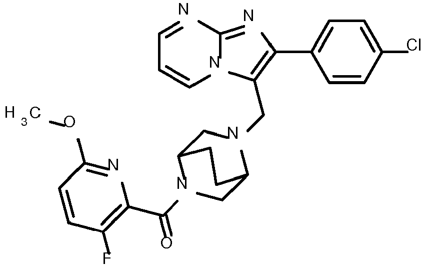

3-Fluor-6-methoxypyridin-2-carbonsäure (40.6 mg, 237 µmol) wurde in 1.5 ml DMF gelöst, mit 2-(7-Aza-1*H*-benzotriazol-1-yl)-1,1 ,3,3-tetramethyluronium-Hexafluorophosphat (HATU) (123 mg, 324 µmol) versetzt und 30 min bei Raumtemperatur gerührt. Anschließend wurde 2-(4-Chlorphenyl)-3-(2,5-diazabicyclo[2.2.2]oct-2-ylmethyl)imidazo[1,2-a]pyrimidin-Dihydrochlorid (Enantiomer 2; 100 mg) sowie *N,N*-Diisopropylethylamin (190 µl, 1.1 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 84 mg (0.17 mmol, 77% d. Th.) der Titelverbindung erhalten.

LC-MS (Methode 6): Rε = 1.52 min; MS (ESIpos): m/z = 507/509 [M+H]⁺.

¹H-NMR (400 MHz, DMSO-d₆): δ [ppm] = 1.50-2.00 (m, 4H), 2.62-2.87 (m, 2.25H), 2.92 (br. s, 0.75H), 3.15 (br. d, 0.25H), 3.38-3.50 (m, 1.5H), 3.56 (br. d, 0.25H), 3.70-3.83 (m, 3.75H), 4.20-4.35 (m, 2H), 4.38 (br. s, 0.25H), 6.89-6.99 (m, 1H), 7.07-7.17 (m, 1H), 7.49-7.60 (m, 2H), 7.70-7.83 (m, 1H), 7.84-7.95 (m, 2H), 8.56-8.63 (m, 1H), 8.99-9.09 (m, 1H).

### Beispiel 25

### (5-{[2-(4-Chlorphenyl)imidazo[1,2-a]pyrimidin-3-yl]methyl}-2,5-diazabicyclo[2.2.2]oct-2-yl)-(6-methoxy-3-methylpyridin-2-yl)methanon (Enantiomer 1)

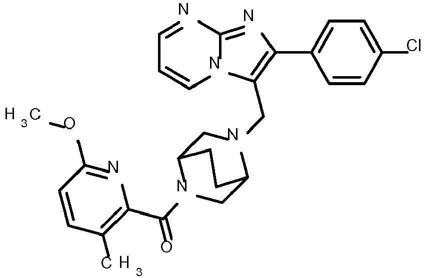

6-Methoxy-3-methylpyridin-2-carbonsäure (39.7 mg, 237 µmol) wurde in 1.5 ml DMF gelöst, mit 2-(7-Aza-1*H*-benzotriazol-1-yl)-1,1 ,3,3-tetramethyluronium-Hexafluorophosphat (HATU) (123 mg, 324 µmol) versetzt und 30 min bei Raumtemperatur gerührt. Anschließend wurde 2-(4-Chlorphenyl)-3-(2,5-diazabicyclo[2.2.2]oct-2-ylmethyl)imidazo[1,2-a]pyrimidin-Dihydrochlorid (Enantiomer 1; 100 mg) sowie *N,N*-Diisopropylethylamin (190 µl, 1.1 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 25 mg (0.05 mmol, 23% d. Th.) der Titelverbindung erhalten.

LC-MS (Methode 2): Rε = 1.55 min; MS (ESIpos): m/z = 503/505 [M+H]⁺.

¹H-NMR (400 MHz, DMSO-d₆): δ [ppm] = 1.49-1.97 (m, 4H), 2.02-2.12 (m, 3H), 2.58-2.84 (m, 2.25H), 2.91-3.02 (m, 1H), 3.23 (br. s, 0.75H), 3.34-3.45 (m, 1H), 3.65-3.83 (m, 3.75H), 4.19-4.42 (m, 2.25H), 6.71-6.81 (m, 1H), 7.06-7.17 (m, 1H), 7.49-7.65 (m, 3H), 7.82-7.94 (m, 2H), 8.56-8.63 (m, 1H), 8.97-9.08 (m, 1H).

### Beispiel 26

### (3-Chlor-6-methoxypyridin-2-yl)(5-{[2-(4-isopropylphenyl)imidazo[1,2-a]pyriinidin-3-yl]methyl}-2,5-diazabicyclo[2.2.2]oct-2-yl)methanon (Enantiomer 2)

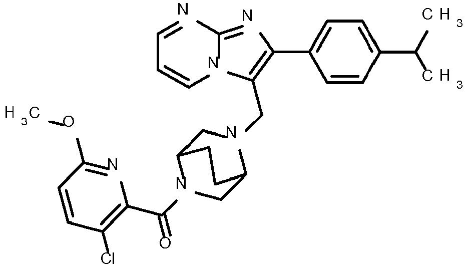

3-Chlor-6-methoxypyridin-2-carbonsäure (43 mg, 0.21 mmol) wurde in 1.5 ml DMF gelöst, mit 2-(7-Aza-1*H*-benzotriazol-1-yl)-1,1,3,3-tetramethyluronium-Hexafluorophosphat (HATU) (121 mg, 0.32 mmol) versetzt und 30 min bei Raumtemperatur gerührt. Anschließend wurde 3-(2,5-Diazabicyclo[2.2.2]oct-2-ylmethyl)-2-(4-isopropylphenyl)imidazo[1,2-a]pyrimidin-Dihydrochlorid (Enantiomer 2; 100 mg) sowie *N,N*-Diisopropylethylamin (190 µl, 1.1 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 82 mg (0.15 mmol, Gehalt 96%, 70% d. Th.) der Titelverbindung erhalten.

LC-MS (Methode 2): Rₜ = 1.73 min; m/z = 531/533 (M+H)⁺.

¹H-NMR (400 MHz, DMSO-d₆): δ [ppm] = 1.20-1.31 (m, 6H), 1.53-2.01 (m, 4H), 2.62 (br. d, 0.75H), 2.69-2.85 (m, 1.5H), 2.89-3.03 (m, 2H), 3.21 (br. s, 0.75H), 3.43 (br. d, 1H), 3.70-3.85 (m, 3.75H), 4.21-4.35 (m, 2H), 4.39 (br. s, 0.25H), 6.85-6.96 (m, 1H), 7.05-7.14 (m, 1H), 7.30-7.43 (m, 2H), 7.70-7.87 (m, 2.75H), 7.90 (d, 0.25H), 8.56 (dd, 1H), 8.95-9.04 (m, 1H).

### Beispiel 27

### (5-Cyclopropyl-1,3-oxazol-4-yl)(5-{[2-(4-isopropylphenyl)imidazo[1,2-a]pyrimidin-3-yl]methyl}-2,5-diazabicyclo[2.2.2]oct-2-yl)methanon (Enantiomer 2)

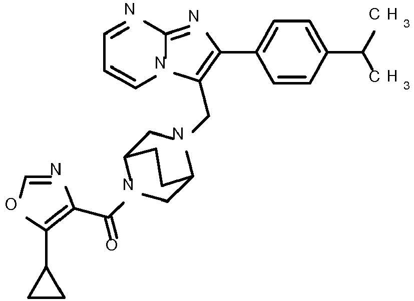

5-Cyclopropyl-1,3-oxazol-4-carbonsäure (32 mg, 0.21 mmol) wurde in 1.35 ml DMF gelöst, mit 2-(7-Aza-1*H*-benzotriazol-1-yl)-1,1,3,3-tetramethyluronium-Hexafluorophosphat (HATU) (109 mg, 0.29 mmol) versetzt und 30 min bei Raumtemperatur gerührt. Anschließend wurde 3-(2,5-Diazabicyclo[2.2.2]oct-2-ylmethyl)-2-(4-isopropylphenyl)imidazo[1,2-a]pyrimidin-Dihydrochlorid (Enantiomer 2; 90 mg) sowie *N,N*-Diisopropylethylamin (170 µl, 0.96 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 67 mg (0.14 mmol, 71% d. Th.) der Titelverbindung erhalten.

LC-MS (Methode 2): Rₜ = 1.51 min; m/z = 497 (M+H)⁺.

¹H-NMR (400 MHz, DMSO-d₆): δ [ppm] = 0.83-0.95 (m, 2H), 0.96-1.08 (m, 2H), 1.25 (d, 6H), 1.51-1.61 (m, 1H), 1.67-1.99 (m, 3H), 2.44-2.57 (m, 0.7H, teilweise verdeckt durch DMSO-Signal), 2.57-2.66 (m, 0.3H), 2.73-3.01 (m, 4H), 3.37 (dd, 0.7H), 3.64-3.76 (m, 1H), 4.03 (br. d, 0.3H), 4.23-4.33 (m, 2H), 4.37 (br. s, 0.3H), 4.59 (br. s, 0.7H), 7.06-7.14 (m, 1H), 7.31-7.40 (m, 2H), 7.75-7.83 (m, 2H), 8.12-8.20 (m, 1H), 8.53-8.59 (m, 1H), 8.98-9.06 (m, 1H).

### Beispiel 28

### (3-Fluor-6-methoxypyridin-2-yl)(3-{[2-(4-isopropylphenyl)imidazo[1,2-a]pyrimidin-3-yl]methyl}-3,8-diazabicyclo[3.2.1]oct-8-yl)methanon

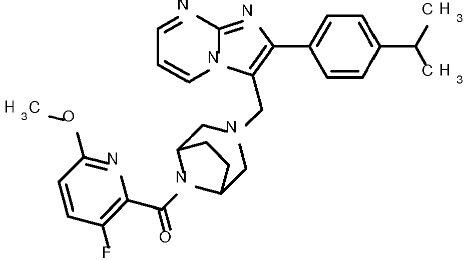

3-Fluor-6-methoxypyridin-2-carbonsäure (39.2 mg, 0.21 mmol) wurde in 1.5 ml DMF gelöst, mit 2-(7-Aza-1*H*-benzotriazol-1-yl)-1,1,3,3-tetramethyluronium-Hexafluorophosphat (HATU) (119 mg, 0.31 mmol) versetzt und 30 min bei Raumtemperatur gerührt. Anschließend wurde 3-(3,8-Diazabicyclo[3.2.1]oct-3-ylmethyl)-2-(4-isopropylphenyl)imidazo[1,2-a]pyrimidin-Dihydrochlorid (98 mg) sowie *N,N*-Diisopropylethylamin (180 µl, 1.04 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 65 mg (0.13 mmol, 61% d. Th.) der Titelverbindung erhalten.

LC-MS (Methode 5): Rε = 1.22 min; MS (ESIpos): m/z = 515 [M+H]⁺.

¹H-NMR (400 MHz, DMSO-d₆): δ [ppm] = 1.26 (d, 6H), 1.63-1.86 (m, 4H), 2.44 (br. t, 2H), 2.48-2.60 (m, 1H, teilweise verdeckt durch DMSO-Signal), 2.76 (dd, 1H), 2.96 (quin, 1H), 3.76 (s, 3H), 3.92 (br. s, 1H), 4.00-4.11 (m, 2H), 4.61 (br. s, 1H), 6.95 (dd, 1H), 7.12 (dd, 1H), 7.36 (d, 2H), 7.77 (t, 1H), 7.83 (d, 2H), 8.57 (dd, 1H), 9.02 (dd, 1H).

### Beispiel 29

### (3-Chlor-6-methoxypyridin-2-yl)(3-{[2-(4-isopropylphenyl)imidazo[1,2-a]pyrimidin-3-yl]methyl}-3,8-diazabicyclo[3.2.1]oct-8-yl)methanon

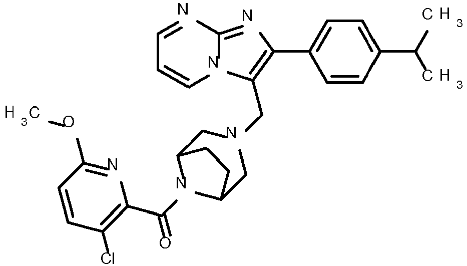

3-Chlor-6-methoxypyridin-2-carbonsäure (43 mg, 0.23 mmol) wurde in 1.5 ml DMF gelöst, mit 2-(7-Aza-1*H*-benzotriazol-1-yl)-1,1,3,3-tetramethyluronium-Hexafluorophosphat (HATU) (118 mg, 0.31 mmol) versetzt und 30 min bei Raumtemperatur gerührt. Anschließend wurde 3-(3,8-Diazabicyclo[3.2.1]oct-3-ylmethyl)-2-(4-isopropylphenyl)imidazo[1,2-a]pyrimidin-Dihydrochlorid (98 mg) sowie *N,N*-Diisopropylethylamin (180 µl, 1.04 mmol) hinzugegeben und das Gemisch über Nacht bei Raumtemperatur gerührt. Danach wurde das Reaktionsgemisch direkt über präparative HPLC (Methode 8) in seine Komponenten aufgetrennt. Es wurden 67 mg (0.13 mmol, 60% d. Th.) der Titelverbindung erhalten.

LC-MS (Methode 5): Rε = 1.27 min; MS (ESIpos): m/z = 531/533 [M+H]⁺.

¹H-NMR (400 MHz, DMSO-d₆): δ [ppm] = 1.26 (d, 6H), 1.63-1.84 (m, 4H), 2.41 (br. t, 2H), 2.46-2.57 (m, 1H, verdeckt durch DMSO-Signal), 2.75 (br. d, 1H), 2.96 (quin, 1H), 3.63 (br. s, 1H), 3.79 (s, 3H), 4.00-4.11 (m, 2H), 4.60 (br. s, 1H), 6.92 (d, 1H), 7.12 (dd, 1H), 7.37 (d, 2H), 7.82 (d, 2H), 7.87 (d, 1H), 8.56 (dd, 1H), 9.01 (dd, 1H).

Analog zu den Beispielen 13-29 wurden die folgenden Verbindungen aus den jeweils angegebenen Edukten hergestellt:

| **Beispiel** | **Name / Struktur / Edukte** | **Analytische Daten** |
|---|---|---|
| **30** | (7-{[2-(4-Chlorphenyl)imidazo[1,2-a]pyrimidin-3-yl]methyl}-3-oxa-7,9-diazabicyclo[3.3.1]-non-9-yl)(2-fluorphenyl)methanon | ¹H-NMR (400 MHz, DMSO-d₆): δ [ppm] = 2.43 (br. d, 1H), 2.47-2.59 (m, 1H, teilweise verdeckt durch DMSO-Signal), 2.87 (br. d, 1H), 3.02 (br. d, 1H), 3.37 (br. s, 1H), 3.59 (br. d, 1H), 3.65-3.76 (m, 2H), 3.87 (d, 1H), 3.97 (s, 2H), 4.47 (br. s, 1H), 7.08 (dd, 1H), 7.24-7.34 (m, 2H), 7.41-7.53 (m, 2H), 7.55 (d, 2H), 7.95 (d, 2H), 8.58 (dd, 1H), 9.27 (dd, 1H). |
| | 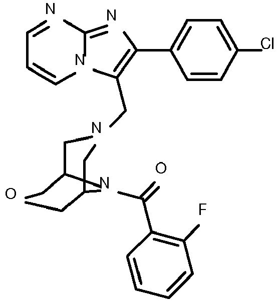 aus 7-{[2-(4-Chlorphenyl)imidazo[1,2-a]-pyrimidin-3-yl]methyl}-3-oxa-7,9-diazabicyclo-[3.3.1]nonan-Dihydrochlorid und 2-Fluorbenzoesäure | |
| | | LC-MS (Methode 2): |
| | | Rₜ = 1.53 min; m/z = 492/494 (M+H)⁺. |
| **31** | (7-{[2-(4-Chlorphenyl)imidazo[1,2-a]pyrimidin-3-yl]methyl}-3-oxa-7,9-diazabicyclo[3.3.1]-non-9-yl)(3-methoxyphenyl)methanon | ¹H-NMR (400 MHz, DMSO-d₆): δ [ppm] = 2.46-2.55 (m, 1H, verdeckt durch DMSO-Signal), 2.59 (br. d, 1H), 2.86 (br. d, 1H), 2.99 (br. d, 1H), 3.54-3.75 (m, 4H), 3.77 (s, 3H), 3.85 (d, 1H), 3.98 (s, 2H), 4.39 (br. s, 1H), 6.91-7.12 (m, 4H), 7.36 (t, 1H), 7.55 (d, 2H), 7.97 (d, 2H), 8.57 (dd, 1H), 9.29 (dd, 1H). |
| | 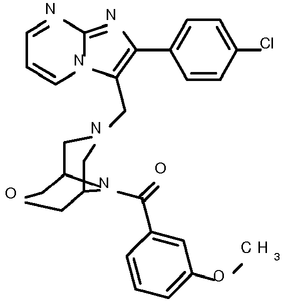 aus 7-{[2-(4-Chlorphenyl)imidazo[1,2-a]-pyrimidin-3-yl]methyl}-3-oxa-7,9-diazabicyclo-[3.3.1]nonan-Dihydrochlorid und 3-Methoxybenzoesäure | |
| | | LC-MS (Methode 2): |
| | | Rₜ = 1.53 min; m/z = 504/506 (M+H)⁺. |
| **32** | (3-{[2-(4-Chlorphenyl)imidazo[1,2-a]pyrimidin-3-yl]methyl}-3,8-diazabicyclo[3.2.1]oct-8-yl)-(2-fluorphenyl)methanon | ¹H-NMR (400 MHz, DMSO-d₆): δ [ppm] = 1.60-1.79 (m, 4H), 2.25 (br. d, 1H), 2.42 (br. d, 1H), 2.47-2.60 (m, 1H, teilweise verdeckt durch DMSO-Signal), 2.68 (br. d, 1H), 3.66 (br. s, 1H), 4.04 (s, 2H), 4.59 (br. s, 1H), 7.14 (dd, 1H), 7.24-7.32 (m, 2H), 7.40-7.53 (m, 2H), 7.57 (d, 2H), 7.95 (d, 2H), 8.59 (dd, 1H), 9.04 (dd, 1H). |
| | 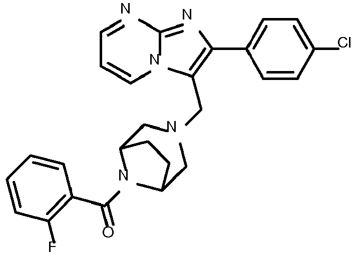 aus 2-(4-Chlorphenyl)-3-(3,8-diazabicyclo-[3.2.1]oct-3-ylmethyl)imidazo[1,2-a]pyrimidin-Dihydrochlorid und 2-Fluorbenzoesäure | |
| | | LC-MS (Methode 2): |
| | | Rₜ = 1.73 min; m/z = 476/478 (M+H)⁺. |
| **33** | (3-{[2-(4-Chlorphenyl)imidazo[1,2-a]pyrimidin-3-yl]methyl}-3,8-diazabicyclo[3.2.1]oct-8-yl)-[6-(methylsulfanyl)pyridin-2-yl]methanon | ¹H-NMR (400 MHz, DMSO-d₆): δ [ppm] = 1.64-1.83 (m, 4H), 2.40-2.63 (m, 3H, teilweise verdeckt durch DMSO-Signal), 2.43 (s, 3H), 2.74 (br. d, 1H), 3.99-4.11 (m, 2H), 4.63 (br. s, 2H), 7.15 (dd, 1H), 7.42 (dd, 2H), 7.57 (d, 2H), 7.75 (t, 1H), 7.97 (d, 2H), 8.55-8.62 (m, 1H), 9.06 (d, 1H). |
| | 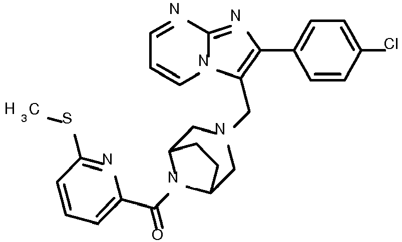 aus 2-(4-Chlorphenyl)-3-(3,8-diazabicyclo-[3.2.1]oct-3-ylmethyl)imidazo[1,2-a]pyrimidin-Dihydrochlorid und 6-(Methylsulfanyl)pyridin-2-carbonsäure | |
| | | LC-MS (Methode 2): |
| | | Rₜ = 1.86 min; m/z = 505/507 (M+H)⁺. |
| **34** | (3-{[2-(4-Chlorphenyl)imidazo[1,2-a]pyrimidin-3-yl]methyl}-3,8-diazabicyclo[3.2.1]oct-8-yl)-(cyclopentyl)methanon | ¹H-NMR (400 MHz, DMSO-d₆): δ [ppm] = 1.44-1.66 (m, 7H), 1.66-1.80 (m, 5H), 2.26 (br. d, 2H), 2.47-2.66 (m, 2H, teilweise verdeckt durch DMSO-Signal), 2.80-2.92 (m, 1H), 3.94-4.06 (m, 2H), 4.28 (br. s, 1H), 4.41 (br. d, 1H), 7.14 (dd, 1H), 7.56 (d, 2H), 7.96 (d, 2H), 8.59 (dd, 1H), 9.05 (dd, 1H). |
| | 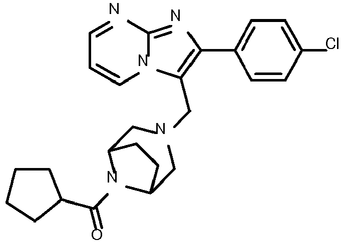 aus 2-(4-Chlorphenyl)-3-(3,8-diazabicyclo-[3.2.1]oct-3-ylmethyl)imidazo[1,2-a]pyrimidin-Dihydrochlorid und Cyclopentancarbonsäure | |
| | | LC-MS (Methode 2): |
| | | Rₜ = 1.83 min; m/z = 450/452 (M+H)⁺. |
| **35** | (3-{[2-(4-Chlorphenyl)imidazo[1,2-a]pyrimidin-3-yl]methyl}-3,8-diazabicyclo[3.2.1]oct-8-yl)-[6-(methylamino)pyridin-2-yl]methanon | ¹H-NMR (400 MHz, DMSO-d₆): δ [ppm] = 1.63-1.81 (m, 4H), 2.42 (br. d, 1H), 2.47-2.62 (m, 2H, teilweise verdeckt durch DMSO-Signal), 2.64-2.75 (m, 1H), 2.67 (d, 3H), 3.97-4.09 (m, 2H), 4.60 (br. s, 1H), 4.76 (br. s, 1H), 6.51 (d, 1H), 6.65 (q, 1H), 6.82 (d, 1H), 7.14 (dd, 1H), 7.44 (t, 1H), 7.56 (d, 2H), 7.96 (d, 2H), 8.59 (dd, 1H), 9.05 (dd, 1H). |
| | 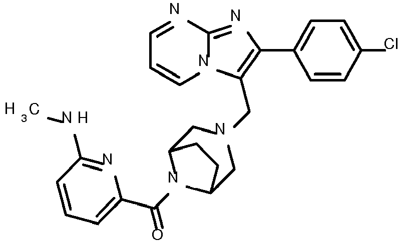 aus 2-(4-Chlorphenyl)-3-(3,8-diazabicyclo-[3.2.1]oct-3-ylmethyl)imidazo[1,2-a]pyrimidin-Dihydrochlorid und 6-(Methylamino)pyridin-2-carbonsäure | |
| | | LC-MS (Methode 2): |
| | | Rₜ = 1.51 min; m/z = 532/534 (M-H+HCOOH)⁻. |
| **36** | (3-{[2-(4-Chlorphenyl)imidazo[1,2-a]pyrimidin-3-yl]methyl}-3,8-diazabicyclo[3.2.1]oct-8-yl)-(3-methoxyphenyl)methanon | ¹H-NMR (400 MHz, DMSO-d₆): δ [ppm] = 1.64-1.80 (m, 4H), 2.30-2.47 (m, 2H), 2.57-2.74 (m, 2H), 3.78 (s, 3H), 3.92 (br. s, 1H), 4.05 (s, 2H), 4.54 (br. s, 1H), 6.92-7.08 (m, 3H), 7.30 (br. t, 1H), 7.35 (t, 1H), 7.61 (d, 2H), 7.93 (d, 2H), 8.71 (br. d, 1H), 9.15 (br. d, 1H). |
| | 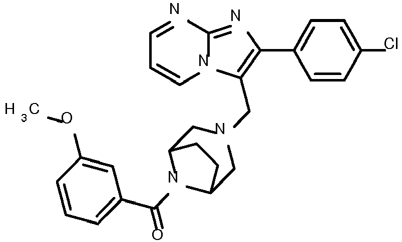 aus 2-(4-Chlorphenyl)-3-(3,8-diazabicyclo-[3.2.1]oct-3-ylmethyl)imidazo[1,2-a]pyrimidin-Dihydrochlorid und 3-Methoxybenzoesäure | |
| | | LC-MS (Methode 2): |
| | | Rₜ = 1.73 min; m/z = 488/490 (M+H)⁺. |
| **37** | (5-{[2-(4-Chlorphenyl)imidazo[1,2-a]pyrimidin-3-yl]methyl}-2,5-diazabicyclo[2.2.2]oct-2-yl)-(cyclopentyl)methanon (*Enantiomer 1*) | ¹H-NMR (400 MHz, DMSO-d₆): δ [ppm] = 1.38-1.79 (m, 11H), 1.82-1.96 (m, 1H), 2.63-2.86 (m, 4H), 3.19 (dd, 0.5H), 3.37 (dd, 0.5H), 3.54 (br. d, 0.5H), 3.75 (br. d, 0.5H), 3.93 (br. d, 0.5H), 4.18-4.31 (m, 2.5H), 7.12 (dd, 1H), 7.56 (d, 2H), 7.89 (d, 2H), 8.59 (dd, 1H), 9.03 (dt, 1H). |
| | 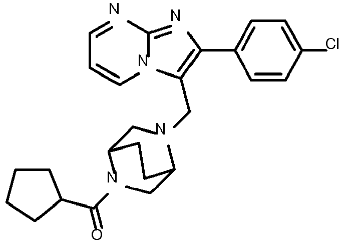 aus 2-(4-Chlorphenyl)-3-(2,5-diazabicyclo-[2.2.2]oct-2-ylmethyl)imidazo[1,2-a]pyrimidin-Dihydrochlorid (*Enantiomer 1*) und Cyclopentancarbonsäure | |
| | | LC-MS (Methode 2): |
| | | Rₜ = 1.42 min; m/z = 450/452 (M+H)⁺. |
| **38** | (5-{[2-(4-Chlorphenyl)imidazo[1,2-a]pyrimidin-3-yl]methyl}-2,5-diazabicyclo[2.2.2]oct-2-yl)-(3-methoxyphenyl)methanon (*Enantiomer 1*) | ¹H-NMR (400 MHz, DMSO-d₆): δ [ppm] = 1.44-1.99 (m, 4H), 2.61-2.72 (m, 1H), 2.76-2.84 (m, 1H), 2.90 (br. s, 1H), 3.17 (br. d, 0.25H), 3.37 (br. d, 0.75H), 3.53 (br. s, 0.75H), 3.63 (br. d, 0.25H), 3.69-3.82 (m, 3.75H), 4.21-4.34 (m, 2.25H), 6.80-6.88 (m, 1.5H), 6.94-7.06 (m, 1.5H), 7.08-7.17 (m, 1H), 7.26-7.39 (m, 1H), 7.50-7.61 (m, 2H), 7.86-7.96 (m, 2H), 8.55-8.63 (m, 1H), 9.00-9.09 (m, 1H). |
| | 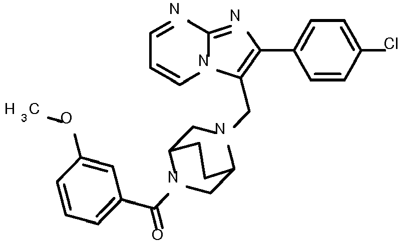 aus 2-(4-Chlorphenyl)-3-(2,5-diazabicyclo-[2.2.2]oct-2-ylmethyl)imidazo[1,2-a]pyrimidin-Dihydrochlorid (*Enantiomer 1*) und 3-Methoxybenzoesäure | |
| | | LC-MS (Methode 2): |
| | | Rₜ = 1.46 min; m/z = 488/490 (M+H)⁺. |
| **39** | (5-{[2-(4-Chlorphenyl)imidazo[1,2-a]pyrimidin-3-yl]methyl}-2,5-diazabicyclo [2.2.2] oct-2-yl)-(2-fluorphenyl)methanon (*Enantiomer 1*) | ¹H-NMR (400 MHz, DMSO-d₆): δ [ppm] = 1.47-1.82 (m, 3H), 1.84-1.98 (m, 1H), 2.46-2.57 (m, 0.75H, verdeckt durch DMSO-Signal), 2.60-2.87 (m, 2.25H), 2.92 (br. s, 0.75H), 3.02 (br. d, 0.25H), 3.42 (br. d, 1H), 3.77 (br. d, 0.75H), 4.20-4.33 (m, 2H), 4.37 (br. s, 0.25H), 7.09-7.17 (m, 1H), 7.20-7.40 (m, 3H), 7.41-7.61 (m, 3H), 7.83-7.95 (m, 2H), 8.56-8.63 (m, 1H), 8.98-9.08 (m, 1H). |
| | 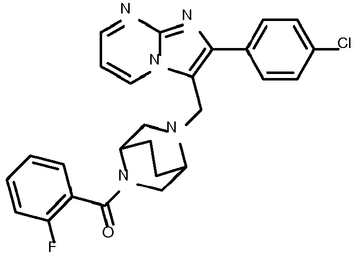 aus 2-(4-Chlorphenyl)-3-(2,5-diazabicyclo-[2.2.2]oct-2-ylmethyl)imidazo[1,2-a]pyrimidin-Dihydrochlorid (*Enantiomer 1*) und 2-Fluorbenzoesäure | |
| | | LC-MS (Methode 2): Rₜ = 1.49 min; m/z = 476/478 (M+H)⁺. |
| **40** | (5-{[2-(4-Chlorphenyl)imidazo[1,2-a]pyrimidin-3-yl]methyl}-2,5-diazabicyclo[2.2.2]oct-2-yl)-(6-methoxypyridin-2-yl)methanon (*Enantiomer 1*) | ¹H-NMR (400 MHz, DMSO-d₆): δ [ppm] = 1.46-1.99 (m, 4H), 2.63-2.73 (m, 1H), 2.80-2.94 (m, 2H), 3.39 (dd, 0.75H), 3.48 (br. d, 0.25H), 3.70-3.83 (m, 3.75H), 3.92 (br. d, 0.25H), 3.98 (br. s, 0.75H), 4.24-4.35 (m, 2H), 4.38 (br. s, 0.25H), 6.84-6.94 (m, 1H), 7.08-7.20 (m, 1.75H), 7.29 (d, 0.25H), 7.49-7.60 (m, 2H), 7.74-7.94 (m, 3H), 8.55-8.62 (m, 1H), 9.00-9.08 (m, 1H). |
| | 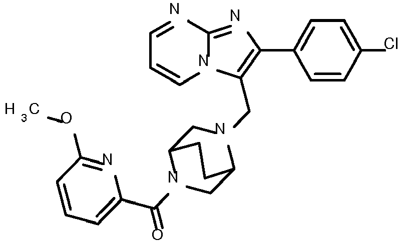 aus 2-(4-Chlorphenyl)-3-(2,5-diazabicyclo-[2.2.2]oct-2-ylmethyl)imidazo[1,2-a]pyrimidin-Dihydrochlorid (*Enantiomer 1*) und 6-Methoxypyridin-2-carbonsäure | |
| | | LC-MS (Methode 2): Rₜ = 1.41 min; m/z = 489/491 (M+H)⁺. |
| **41** | (5-{[2-(4-Chlorphenyl)imidazo[1,2-a]pyrimidin-3-yl]methyl}-2,5-diazabicyclo[2.2.2]oct-2-yl)-(3-fluor-6-methoxypyridin-2-yl)methanon (*Enantiomer 1*) | ¹H-NMR (400 MHz, DMSO-d₆): δ [ppm] = 1.49-2.01 (m, 4H), 2.62-2.87 (m, 2.25H), 2.92 (br. s, 0.75H), 3.15 (br. d, 0.25H), 3.38-3.50 (m, 1.5H), 3.56 (br. d, 0.25H), 3.69-3.84 (m, 3.75H), 4.20-4.35 (m, 2H), 4.38 (br. s, 0.25H), 6.87-6.99 (m, 1H), 7.08-7.17 (m, 1H), 7.49-7.59 (m, 2H), 7.70-7.82 (m, 1H), 7.83-7.95 (m, 2H), 8.56-8.63 (m, 1H), 8.98-9.08 (m, 1H). |
| | 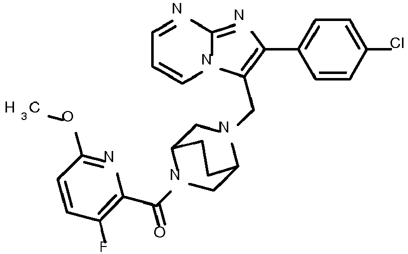 aus 2-(4-Chlorphenyl)-3-(2,5-diazabicyclo-[2.2.2]oct-2-ylmethyl)imidazo[1,2-a]pyrimidin-Dihydrochlorid (*Enantiomer 1*) und 3-Fluor-6-methoxypyridin-2-carbonsäure | |
| | | LC-MS (Methode 2): Rₜ = 1.51 min; m/z = 507/509 (M+H)⁺. |
| **42** | (5-{[2-(4-Chlorphenyl)imidazo[1,2-a]pyrimidin-3-yl]methyl}-2,5-diazabicyclo[2.2.2]oct-2-yl)-(2-fluorphenyl)methanon (*Enantiomer 2*) | ¹H-NMR (400 MHz, DMSO-d₆): δ [ppm] = 1.48-1.83 (m, 3H), 1.83-1.98 (m, 1H), 2.46-2.57 (m, 0.75H, verdeckt durch DMSO-Signal), 2.60-2.88 (m, 2.25H), 2.92 (br. s, 0.75H), 3.02 (br. d, 0.25H), 3.43 (br. d, 1H), 3.77 (br. d, 0.75H), 4.20-4.34 (m, 2H), 4.38 (br. s, 0.25H), 7.08-7.17 (m, 1H), 7.19-7.40 (m, 3H), 7.41-7.60 (m, 3H), 7.83-7.95 (m, 2H), 8.55-8.63 (m, 1H), 8.97-9.08 (m, 1H). |
| | 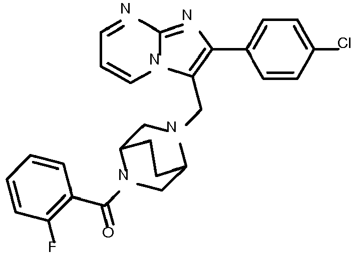 aus 2-(4-Chlorphenyl)-3-(2,5-diazabicyclo-[2.2.2]oct-2-ylmethyl)imidazo[1,2-a]pyrimidin-Dihydrochlorid (*Enantiomer 2*) und 2-Fluorbenzoesäure | |
| | | LC-MS (Methode 2): Rₜ = 1.50 min; m/z = 476/478 (M+H)⁺. |
| **43** | (5-{[2-(4-Chlorphenyl)imidazo[1,2-a]pyrimidin-3-yl]methyl}-2,5-diazabicyclo[2.2.2]oct-2-yl)-(3-methoxyphenyl)methanon (*Enantiomer 2*) | ¹H-NMR (400 MHz, DMSO-d₆): δ [ppm] = 1.44-1.99 (m, 4H), 2.61-2.72 (m, 1H), 2.74-2.84 (m, 1H), 2.90 (br. s, 1H), 3.17 (br. d, 0.25H), 3.37 (br. d, 0.75H), 3.53 (br. s, 0.75H), 3.63 (br. d, 0.25H), 3.67-3.82 (m, 3.75H), 4.18-4.35 (m, 2.25H), 6.76-6.87 (m, 1.5H), 6.92-7.05 (m, 1.5H), 7.08-7.17 (m, 1H), 7.25-7.39 (m, 1H), 7.49-7.61 (m, 2H), 7.84-7.96 (m, 2H), 8.52-8.63 (m, 1H), 8.98-9.09 (m, 1H). |
| | 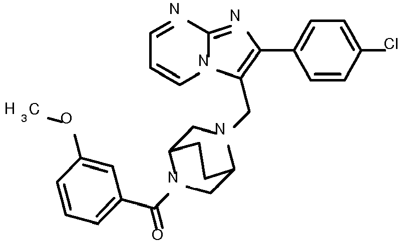 aus 2-(4-Chlorphenyl)-3-(2,5-diazabicyclo-[2.2.2]oct-2-ylmethyl)imidazo[1,2-a]pyrimidin-Dihydrochlorid (*Enantiomer 2*) und 3-Methoxybenzoesäure | |
| | | LC-MS (Methode 2): |
| | | Rₜ = 1.46 min; m/z = 488/490 (M+H)⁺. |
| **44** | (5-{[2-(4-Chlorphenyl)imidazo[1,2-a]pyrimidin-3-yl]methyl}-2,5-diazabicyclo[2.2.2]oct-2-yl)-(6-methoxypyridin-2-yl)methanon (*Enantiomer 2*) | ¹H-NMR (400 MHz, DMSO-d₆): δ [ppm] = 1.46-1.99 (m, 4H), 2.63-2.74 (m, 1H), 2.80-2.94 (m, 2H), 3.39 (dd, 0.75H), 3.49 (br. d, 0.25H), 3.69-3.83 (m, 3.75H), 3.92 (br. d, 0.25H), 3.98 (br. s, 0.75H), 4.24-4.35 (m, 2H), 4.38 (br. s, 0.25H), 6.84-6.94 (m, 1H), 7.08-7.20 (m, 1.75H), 7.29 (d, 0.25H), 7.48-7.60 (m, 2H), 7.73-7.95 (m, 3H), 8.54-8.63 (m, 1H), 9.00-9.09 (m, 1H). |
| | 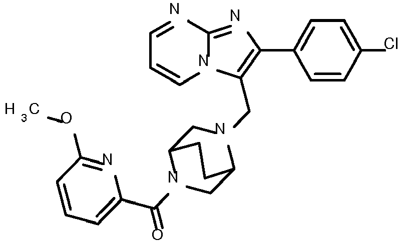 aus 2-(4-Chlorphenyl)-3-(2,5-diazabicyclo-[2.2.2]oct-2-ylmethyl)imidazo[1,2-a]pyrimidin-Dihydrochlorid (*Enantiomer 2*) und 6-Methoxypyridin-2-carbonsäure | |
| | | LC-MS (Methode 2): |
| | | Rₜ = 1.41 min; m/z = 489/491 (M+H)⁺. |
| **45** | (3-Chlor-6-methoxypyridin-2-yl)(5-{[2-(4-chlorphenyl)imidazo[1,2-a]pyrimidin-3-yl]methyl}-2,5-diazabicyclo[2.2.2]oct-2-yl)methanon (*Enantiomer 2*) | ¹H-NMR (400 MHz, DMSO-d₆): δ [ppm] = 1.52-2.00 (m, 4H), 2.72 (br. d, 0.75H), 2.73 (br. d, 1H), 2.80 (br. s, 0.5H), 2.94-3.01 (m, 1H), 3.20 (br. s, 0.75H), 3.35-3.47 (m, 1H), 3.70-3.86 (m, 3.75H), 4.20-4.33 (m, 2H), 4.38 (br. s, 0.25H), 6.85-6.97 (m, 1H), 7.08-7.19 (m, 1H), 7.49-7.61 (m, 2H), 7.79-7.94 (m, 3H), 8.56-8.64 (m, 1H), 8.97-9.08 (m, 1H). |
| | 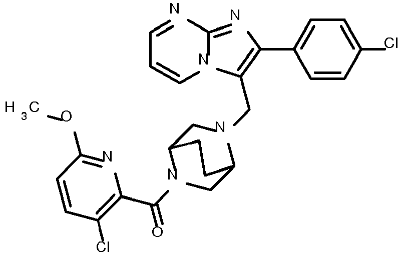 aus 2-(4-Chlorphenyl)-3-(2,5-diazabicyclo-[2.2.2]oct-2-ylmethyl)imidazo[1,2-a]pyrimidin-Dihydrochlorid (*Enantiomer 2*) und 3-Chlor-6-methoxypyridin-2-carbonsäure | |
| | | LC-MS (Methode 2): Rₜ = 1.63 min; MS (ESIpos): m/z = 523/524/525 [M+H]⁺. |
| **46** | (7-{[2-(4-Chlorphenyl)imidazo[1,2-a]pyrimidin-3-yl]methyl}-3-oxa-7,9-diazabicyclo[3.3.1]non-9-yl)(5-cyclopropyl-1,3-oxazol-4-yl)methanon | ¹H-NMR (400 MHz, DMSO-d₆): δ [ppm] = 0.88-0.97 (m, 2H), 1.03-1.13 (m, 2H), 2.44-2.65 (m, 3H, teilweise verdeckt durch DMSO-Signal), 2.92-3.06 (m, 2H), 3.62-3.73 (m, 2H), 3.77-3.90 (m, 2H), 3.96 (s, 2H), 4.41 (br. s, 1H), 4.75 (br. s, 1H), 7.08 (dd, 1H), 7.54 (d, 2H), 7.98 (d, 2H), 8.20 (s, 1H), 8.59 (dd, 1H), 9.29 (dd, 1H). |
| | 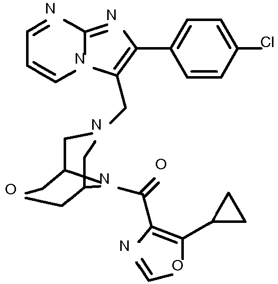 aus 7-{[2-(4-Chlorphenyl)imidazo[1,2-a]-pyrimidin-3-yl]methyl}-3-oxa-7,9-diazabicyclo-[3.3.1]nonan-Dihydrochlorid und 5-Cyclopropyl-1,3-oxazol-4-carbonsäure | |
| | | LC-MS (Methode 1): Rₜ = 0.76 min; MS (ESIpos): m/z = 505/507 [M+H]⁺. |
| **47** | (3-{[2-(4-Chlorphenyl)imidazo[1,2-a]pyrimidin-3-yl]methyl}-3,8-diazabicyclo[3.2.1]oct-8-yl)-(2-cyclopropyl-1,3-oxazol-4-yl)methanon | ¹H-NMR (400 MHz, DMSO-d₆): δ [ppm] = 0.89-0.98 (m, 2H), 1.00-1.09 (m, 2H), 1.57-1.87 (m, 4H), 2.08-2.18 (m, 1H), 2.30-2.42 (m, 2H), 2.58-2.70 (m, 2H), 4.02 (s, 2H), 4.53 (br. s, 1H), 5.15 (br. s, 1H), 7.15 (dd, 1H), 7.56 (d, 2H), 7.97 (d, 2H), 8.37 (s, 1H), 8.59 (dd, 1H), 9.05 (dd, 1H). |
| | 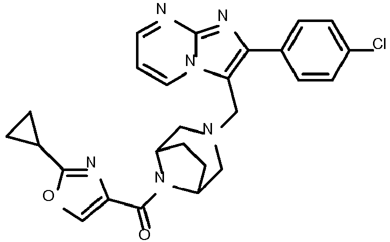 aus 2-(4-Chlorphenyl)-3-(3,8-diazabicyclo-[3.2.1]oct-3-ylmethyl)imidazo[1,2-a]pyrimidin-Dihydrochlorid und 2-Cyclopropyl-1,3-oxazol-4-carbonsäure | |
| | | LC-MS (Methode 2): |
| | | Rₜ = 1.75 min; MS (ESIpos): m/z = 489/491 [M+H]⁺. |
| **48** | (3-{[2-(4-Chlorphenyl)imidazo[1,2-a]pyrimidin-3-yl]methyl}-3,8-diazabicyclo[3.2.1]oct-8-yl)-(5-methyl-1,3-oxazol-4-yl)methanon | ¹H-NMR (400 MHz, DMSO-d₆): δ [ppm] = 1.59-1.84 (m, 4H), 2.35-2.44 (m, 2H), 2.52 (s, 3H, teilweise verdeckt durch DMSO-Signal), 2.60-2.69 (m, 2H), 4.02 (s, 2H), 4.57 (br. s, 1H), 5.12 (br. s, 1H), 7.14 (dd, 1H), 7.56 (d, 2H), 7.97 (d, 2H), 8.29 (s, 1H), 8.56 (dd, 1H), 9.06 (dd, 1H). |
| | 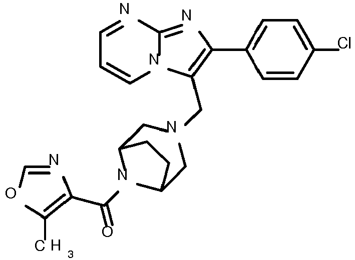 aus 2-(4-Chlorphenyl)-3-(3,8-diazabicyclo-[3.2.1]oct-3-ylmethyl)imidazo[1,2-a]pyrimidin-Dihydrochlorid und 5-Methyl-1,3-oxazol-4-carbonsäure | |
| | | LC-MS (Methode 2): |
| | | Rₜ = 1.59 min; MS (ESIpos): m/z = 463/465 [M+H]⁺. |
| **49** | (3-{[2-(4-Chlorphenyl)imidazo[1,2-a]pyrimidin-3-yl]methyl}-3,8-diazabicyclo[3.2.1]oct-8-yl)-(5-isopropyl-1,3-oxazol-4-yl)methanon | ¹H-NMR (400 MHz, DMSO-d₆): δ [ppm] = 1.21 (t, 6H), 1.63-1.82 (m, 4H), 2.39 (br. t, 2H), 2.64 (br. t, 2H), 3.62 (quin, 1H), 4.02 (s, 2H), 4.57 (br. s, 1H), 4.97 (br. s, 1H), 7.15 (dd, 1H), 7.56 (d, 2H), 7.96 (d, 2H), 8.29 (s, 1H), 8.59 (dd, 1H), 9.05 (dd, 1H). |
| | 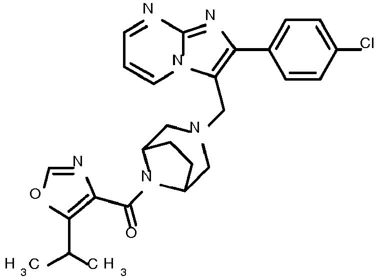 aus 2-(4-Chlorphenyl)-3-(3,8-diazabicyclo-[3.2.1]oct-3-ylmethyl)imidazo[1,2-a]pyrimidin-Dihydrochlorid und 5-Isopropyl-1,3-oxazol-4-carbonsäure | |
| | | LC-MS (Methode 2): |
| | | Rₜ = 1.86 min; MS (ESIpos): m/z = 491/493 [M+H]⁺. |
| **50** | (3-{[2-(4-Chlorphenyl)imidazo[1,2-a]pyrimidin-3-yl]methyl}-3,8-diazabicyclo[3.2.1]oct-8-yl)-(2,4-dimethyl-1,3-oxazol-5-yl)methanon | ¹H-NMR (400 MHz, DMSO-d₆): δ [ppm] = 1.74 (br. s, 4H), 2.27 (s, 3H), 2.34-2.45 (m, 2H), 2.40 (s, 3H), 2.60-2.70 (m, 2H), 4.04 (s, 2H), 4.58 (br. s, 2H), 7.17 (dd, 1H), 7.56 (d, 2H), 7.97 (d, 2H), 8.59 (dd, 1H), 9.07 (dd, 1H). |
| | 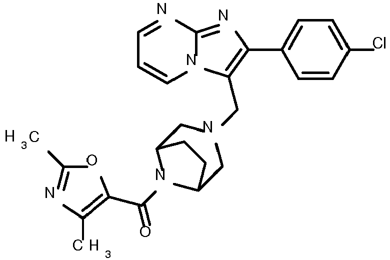 aus 2-(4-Chlorphenyl)-3-(3,8-diazabicyclo-[3.2.1]oct-3-ylmethyl)imidazo[1,2-a]pyrimidin-Dihydrochlorid und 2,4-Dimethyl-1,3-oxazol-5-carbonsäure | |
| | | LC-MS (Methode 2): |
| | | Rₜ = 1.53 min; MS (ESIpos): m/z = 477/479 [M+H]⁺. |
| **51** | (3-{[2-(4-Chlorphenyl)imidazo[1,2-a]pyrimidin-3-yl]methyl}-3,8-diazabicyclo[3.2.1]oct-8-yl)-(5-ethyl-1,3-oxazol-4-yl)methanon | ¹H-NMR (400 MHz, DMSO-d₆): δ [ppm] = 1.17 (t, 3H), 1.61-1.83 (m, 4H), 2.39 (br. d, 2H), 2.64 (br. d, 2H), 2.95 (q, 2H), 4.02 (s, 2H), 4.57 (br. s, 1H), 5.07 (br. s, 1H), 7.15 (dd, 1H), 7.56 (d, 2H), 7.96 (d, 2H), 8.30 (s, 1H), 8.59 (dd, 1H), 9.05 (dd, 1H). |
| | 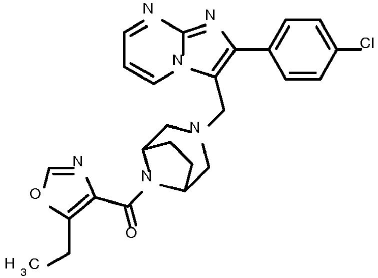 aus 2-(4-Chlorphenyl)-3-(3,8-diazabicyclo-[3.2.1]oct-3-ylmethyl)imidazo[1,2-a]pyrimidin-Dihydrochlorid und 5-Ethyl-1,3-oxazol-4-carbonsäure | |
| | | LC-MS (Methode 2): |
| | | Rₜ = 1.73 min; MS (ESIpos): m/z = 477/479 [M+H]⁺. |
| **52** | (4-Brom-5-methyl-1,3-thiazol-2-yl)(3-{[2-(4-chlorphenyl)imidazo[1,2-a]pyrimidin-3-yl]-methyl}-3,8-diazabicyclo[3.2.1]oct-8-yl)-methanon | ¹H-NMR (400 MHz, DMSO-d₆): δ [ppm] = 1.67-1.77 (m, 2H), 1.79-1.91 (m, 2H), 2.39-2.48 (m, 2H), 2.43 (s, 3H), 2.66 (br. d, 1H), 2.72 (br. d, 1H), 4.04 (s, 2H), 4.57 (br. s, 1H), 5.46 (br. s, 1H), 7.15 (dd, 1H), 7.56 (d, 2H), 7.97 (d, 2H), 8.59 (dd, 1H), 9.06 (dd, 1H). |
| | 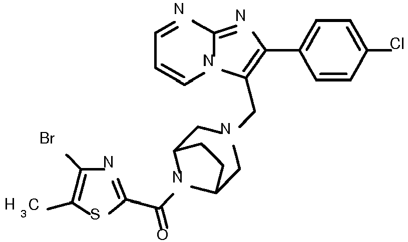 aus 2-(4-Chlorphenyl)-3-(3,8-diazabicyclo-[3.2.1]oct-3-ylmethyl)imidazo[1,2-a]pyrimidin-Dihydrochlorid und 4-Brom-5-methyl-1,3-thiazol-2-carbonsäure | |
| | | LC-MS (Methode 1): |
| | | Rₜ = 1.10 min; MS (ESIpos): m/z = 557/559 [M+H]⁺. |
| **53** | (3-{[2-(4-Chlorphenyl)imidazo[1,2-a]pyrimidin-3-yl]methyl}-3,8-diazabicyclo[3.2.1]oct-8-yl)-(5-cyclopropyl-1,3-oxazol-4-yl)methanon | ¹H-NMR (400 MHz, DMSO-d₆): δ [ppm] = 0.86-0.99 (m, 2H), 1.02-1.13 (m, 2H), 1.61-1.85 (m, 4H), 2.36-2.46 (m, 2H), 2.60-2.75 (m, 3H), 4.03 (s, 2H), 4.58 (br. s, 1H), 5.12 (br. s, 1H), 7.15 (dd, 1H), 7.56 (d, 2H), 7.97 (d, 2H), 8.18 (s, 1H), 8.59 (dd, 1H), 9.06 (dd, 1H). |
| | 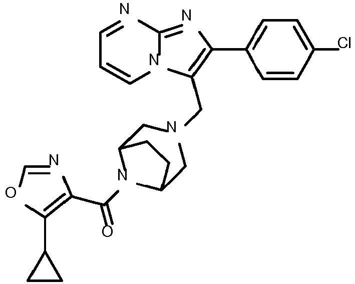 aus 2-(4-Chlorphenyl)-3-(3,8-diazabicyclo-[3.2.1]oct-3-ylmethyl)imidazo[1,2-a]pyrimidin-Dihydrochlorid und 5-Cyclopropyl-1,3-oxazol-4-carbonsäure | |
| | | LC-MS (Methode 1): |
| | | Rₜ = 0.91 min; MS (ESIpos): m/z = 489/491 [M+H]⁺. |
| **54** | (3-{[2-(4-Chlorphenyl)imidazo[1,2-a]pyrimidin-3-yl]methyl}-3,8-diazabicyclo[3.2.1]oct-8-yl)-(2-isopropyl-1,3-thiazol-4-yl)methanon | ¹H-NMR (400 MHz, DMSO-d₆): δ [ppm] = 1.31 (d, 6H), 1.63-1.85 (m, 4H), 2.40-2.48 (m, 2H), 2.65 (br. d, 2H), 3.23-3.33 (m, 1H, teilweise verdeckt durch H₂O-Signal), 4.04 (s, 2H), 4.59 (br. s, 1H), 4.99 (br. s, 1H), 7.14 (dd, 1H), 7.55 (d, 2H), 7.96 (d, 2H), 8.06 (s, 1H), 8.59 (dd, 1H), 9.05 (dd, 1H). |
| | 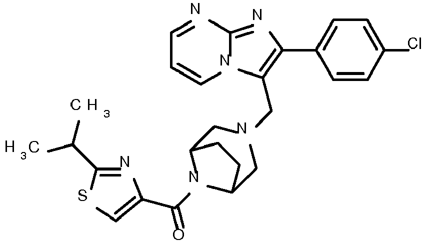 aus 2-(4-Chlorphenyl)-3-(3,8-diazabicyclo-[3.2.1]oct-3-ylmethyl)imidazo[1,2-a]pyrimidin-Dihydrochlorid und 2-Isopropyl-1,3-thiazol-4-carbonsäure | |
| | | LC-MS (Methode 1): |
| | | Rₜ = 1.01 min; MS (ESIpos): m/z = 507/509 [M+H]⁺. |
| **55** | (3-{[2-(4-Chlorphenyl)imidazo[1,2-a]pyrimidin-3-yl]methyl}-3,8-diazabicyclo[3.2.1]oct-8-yl)-(1,3-thiazol-5-yl)methanon | ¹H-NMR (400 MHz, DMSO-d₆): δ [ppm] = 1.62-1.94 (m, 4H), 2.39-2.59 (m, 2H, teilweise verdeckt durch DMSO-Signal), 2.68 (dd, 2H), 4.06 (s, 2H), 4.37-4.65 (m, 2H), 7.15 (dd, 1H), 7.57 (d, 2H), 7.97 (d, 2H), 8.29 (s, 1H), 8.60 (dd, 1H), 9.07 (dd, 1H), 9.23 (s, 1H). |
| | 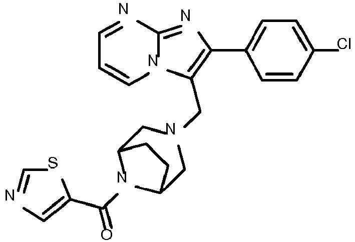 aus 2-(4-Chlorphenyl)-3-(3,8-diazabicyclo-[3.2.1]oct-3-ylmethyl)imidazo[1,2-a]pyrimidin-Dihydrochlorid und 1,3-Thiazol-5-carbonsäure | |
| | | LC-MS (Methode 1): |
| | | Rₜ = 0.77 min; MS (ESIpos): m/z = 465/467 [M+H]⁺. |
| **56** | (3-{[2-(4-Chlorphenyl)imidazo[1,2-a]pyrimidin-3-yl]methyl}-3,8-diazabicyclo[3.2.1]oct-8-yl)-(2,5-dimethyl-1,3-oxazol-4-yl)methanon | ¹H-NMR (400 MHz, DMSO-d₆): δ [ppm] = 1.60-1.81 (m, 4H), 2.31-2.42 (m, 2H), 2.36 (s, 3H), 2.47 (s, 3H), 2.63 (br. d, 2H), 4.02 (s, 2H), 4.54 (br. s, 1H), 5.18 (br. s, 1H), 7.14 (dd, 1H), 7.56 (d, 2H), 7.97 (d, 2H), 8.59 (dd, 1H), 9.05 (dd, 1H). |
| | 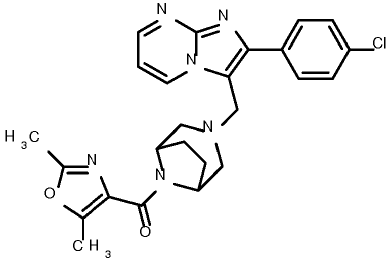 aus 2-(4-Chlorphenyl)-3-(3,8-diazabicyclo-[3.2.1]oct-3-ylmethyl)imidazo[1,2-a]pyrimidin-Dihydrochlorid und 2,5-Dimethyl-1,3-oxazol-4-carbonsäure | |
| | | LC-MS (Methode 5): |
| | | Rₜ = 1.16 min; MS (ESIpos): m/z = 477/479 [M+H]⁺. |
| **57** | (3-{[2-(4-Chlorphenyl)imidazo[1,2-a]pyrimidin-3-yl]methyl}-3,8-diazabicyclo[3.2.1]oct-8-yl)-[2-methoxy-4-(trifluormethyl)-1,3-thiazol-5-yl]-methanon | ¹H-NMR (400 MHz, DMSO-d₆): δ [ppm] = 1.62-1.77 (m, 4H), 2.21-2.40 (m, 2H), 2.63 (br. d, 2H), 3.92 (br. s, 1H), 4.04 (s, 2H), 4.10 (s, 3H), 4.51 (br. s, 1H), 7.15 (dd, 1H), 7.57 (d, 2H), 7.93 (d, 2H), 8.59 (dd, 1H), 9.03 (dd, 1H). |
| | 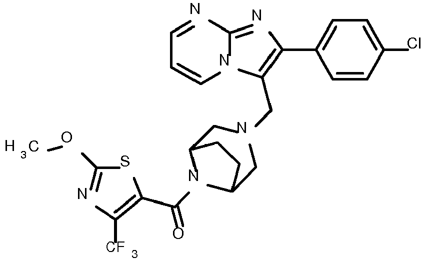 aus 2-(4-Chlorphenyl)-3-(3,8-diazabicyclo-[3.2.1]oct-3-ylmethyl)imidazo[1,2-a]pyrimidin-Dihydrochlorid und 2-Methoxy-4-(trifluormethyl)-1,3-thiazol-5-carbonsäure | |
| | | LC-MS (Methode 5): |
| | | Rₜ = 1.30 min; MS (ESIpos): m/z = 563/565 [M+H]⁺. |
| **58** | (3-{[2-(4-Chlorphenyl)imidazo[1,2-a]pyrimidin-3-yl]methyl}-3,8-diazabicyclo[3.2.1]oct-8-yl)-[2-(trifluormethyl)-1,3-thiazol-4-yl]methanon | ¹H-NMR (400 MHz, DMSO-d₆): δ [ppm] = 1.65-1.89 (m, 4H), 2.45 (br. d, 2H), 2.67 (br. t, 2H), 4.05 (s, 2H), 4.61 (br. s, 1H), 4.74 (br. s, 1H), 7.15 (dd, 1H), 7.56 (d, 2H), 7.97 (d, 2H), 8.59 (dd, 1H), 8.62 (s, 1H), 9.06 (dd, 1H). |
| | 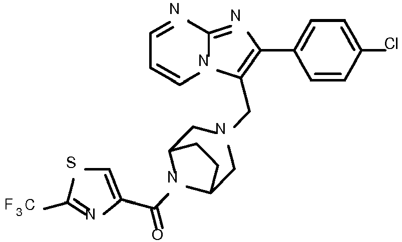 aus 2-(4-Chlorphenyl)-3-(3,8-diazabicyclo-[3.2.1]oct-3-ylmethyl)imidazo[1,2-a]pyrimidin-Dihydrochlorid und 2-(Trifluormethyl)-1,3-thiazol-4-carbonsäure | |
| | | LC-MS (Methode 5): |
| | | Rₜ = 1.31 min; MS (ESIpos): m/z = 533/535 [M+H]⁺. |
| **59** | (3-{[2-(4-Chlorphenyl)imidazo[1,2-a]pyrimidin-3-yl]methyl}-3,8-diazabicyclo[3.2.1]oct-8-yl)-(5-methyl-1,3-thiazol-4-yl)methanon | ¹H-NMR (400 MHz, DMSO-d₆): δ [ppm] = 1.63-1.79 (m, 4H), 2.34-2.45 (m, 2H), 2.46-2.61 (m, 1H, teilweise verdeckt durch DMSO-Signal), 2.55 (s, 3H), 2.68 (dd, 1H), 4.04 (s, 2H), 4.34 (br. s, 1H), 4.60 (br. s, 1H), 7.15 (dd, 1H), 7.57 (d, 2H), 7.96 (d, 2H), 8.59 (dd, 1H), 8.89 (s, 1H), 9.06 (dd, 1H). |
| | 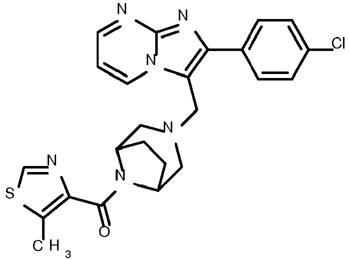 aus 2-(4-Chlorphenyl)-3-(3,8-diazabicyclo-[3.2.1]oct-3-ylmethyl)imidazo[1,2-a]pyrimidin-Dihydrochlorid und 5-Methyl-1,3-thiazol-4-carbonsäure | |
| | | LC-MS (Methode 5): Rₜ = 1.11 min; MS (ESIpos): m/z = 479/481 [M+H]⁺. |
| **60** | (3-{[2-(4-Chlorphenyl)imidazo[1,2-a]pyrimidin-3-yl]methyl}-3,8-diazabicyclo[3.2.1]oct-8-yl)-[4-(trifluormethyl)-1,3-thiazol-2-yl]methanon | ¹H-NMR (400 MHz, DMSO-d₆): δ [ppm] = 1.68-1.80 (m, 2H), 1.83-1.95 (m, 2H), 2.45 (br. d, 1H), 2.48-2.57 (m, 1H, teilweise verdeckt durch DMSO-Signal), 2.65 (br. d, 1H), 2.80 (br. d, 1H), 4.05 (s, 2H), 4.61 (br. s, 1H), 5.43 (br. s, 1H), 7.15 (dd, 1H), 7.55 (d, 2H), 7.97 (d, 2H), 8.60 (dd, 1H), 8.79 (s, 1H), 9.06 (dd, 1H). |
| | 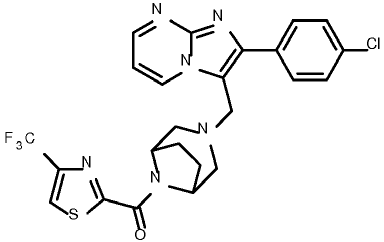 aus 2-(4-Chlorphenyl)-3-(3,8-diazabicyclo-[3.2.1]oct-3-ylmethyl)imidazo[1,2-a]pyrimidin-Dihydrochlorid und 4-(Trifluormethyl)-1,3-thiazol-2-carbonsäure | |
| | | LC-MS (Methode 2): Rₜ = 2.00 min; MS (ESIpos): m/z = 533/535 [M+H]⁺. |
| **61** | (3-{[2-(4-Chlorphenyl)imidazo[1,2-a]pyrimidin-3-yl]methyl}-3,8-diazabicyclo[3.2.1]oct-8-yl)-(1,3-thiazol-4-yl)methanon | ¹H-NMR (400 MHz, DMSO-d₆): δ [ppm] = 1.75 (br. d, 4H), 2.44 (br. t, 2H), 2.66 (br. t, 2H), 4.03 (s, 2H), 4.62 (br. s, 1H), 5.02 (br. s, 1H), 7.14 (dd, 1H), 7.56 (d, 2H), 7.97 (d, 2H), 8.27 (d, 1H), 8.59 (dd, 1H), 9.06 (dd, 1H), 9.15 (d, 1H). |
| | 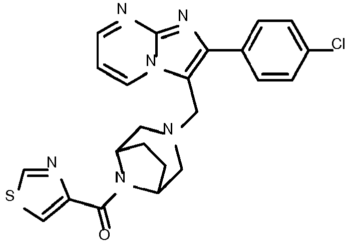 aus 2-(4-Chlorphenyl)-3-(3,8-diazabicyclo-[3.2.1]oct-3-ylmethyl)imidazo[1,2-a]pyrimidin-Dihydrochlorid und 1,3-Thiazol-4-carbonsäure | |
| | | LC-MS (Methode 5): |
| | | Rₜ = 1.07 min; MS (ESIpos): m/z = 465/467 [M+H]⁺. |
| **62** | (3-{[2-(4-Isopropylphenyl)imidazo[1,2-a]-pyrimidin-3-yl]methyl}-3,8-diazabicyclo-[3.2.1]oct-8-yl)[6-(methylamino)pyridin-2-yl]-methanon | ¹H-NMR (400 MHz, DMSO-d₆): δ [ppm] = 1.25 (d, 6H), 1.64-1.81 (m, 4H), 2.41 (br. d, 1H), 2.58 (br. s, 1.75H), 2.65-2.76 (m, 4.25H), 2.88-3.01 (m, 1H), 3.98-4.09 (m, 2H), 4.60 (br. s, 1H), 4.76 (br. s, 1H), 6.51 (d, 1H), 6.65 (q, 1H), 6.80 (d, 1H), 7.12 (dd, 1H), 7.37 (d, 2H), 7.74 (d, 1H), 7.84 (d, 2H), 8.56 (dd, 1H), 9.02 (dd, 1H). |
| | 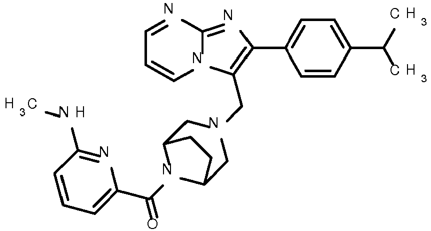 aus 3-(3,8-Diazabicyclo[3.2.1]oct-3-ylmethyl)-2-(4-isopropylphenyl)imidazo[1,2-a]pyrimidin-Dihydrochlorid und 6-(Methylamino)pyridin-2-carbonsäure | |
| | | LC-MS (Methode 2): |
| | | Rₜ = 1.58 min; MS (ESIpos): m/z = 496 [M+H]⁺. |
| **63** | (3-{[2-(4-Isopropylphenyl)imidazo[1,2-a]-pyrimidin-3-yl]methyl}-3,8-diazabicyclo-[3.2.1]oct-8-yl)(6-methoxypyridin-2-yl)methanon | ¹H-NMR (400 MHz, DMSO-d₆): δ [ppm] = 1.25 (d, 6H), 1.66-1.84 (m, 4H), 2.44 (br. d, 1H), 2.56-2.66 (m, 2H), 2.74 (dd, 1H), 2.95 (quin, 1H), 3.77 (s, 3H), 3.99-4.11 (m, 2H), 4.64 (br. s, 1H), 4.69 (br. s, 1H), 6.93 (d, 1H), 7.12 (dd, 1H), 7.31-7.40 (m, 3H), 7.77-7.88 (m, 3H), 8.57 (dd, 1H), 9.03 (dd, 1H). |
| | 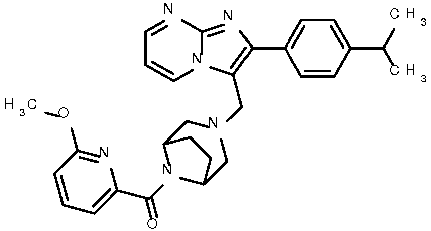 aus 3-(3,8-Diazabicyclo[3.2.1]oct-3-ylmethyl)-2-(4-isopropylphenyl)imidazo[1,2-a]pyrimidin-Dihydrochlorid und 6-Methoxypyridin-2-carbonsäure | |
| | | LC-MS (Methode 5): |
| | | Rₜ = 1.24 min; MS (ESIpos): m/z = 497 [M+H]⁺. |
| **64** | (2-Fluorphenyl)(3-{[2-(4-isopropylphenyl)-imidazo[1,2-a]pyrimidin-3-yl]methyl}-3,8-diazabicyclo[3.2.1]oct-8-yl)methanon | ¹H-NMR (400 MHz, DMSO-d₆): δ [ppm] = 1.26 (d, 6H), 1.64-1.81 (m, 4H), 2.26 (br. d, 1H), 2.42 (br. d, 1H), 2.58 (br. d, 1H), 2.68 (br. d, 1H), 2.96 (quin, 1H), 3.67 (br. s, 1H), 4.04 (s, 2H), 4.59 (br. s, 1H), 7.12 (dd, 1H), 7.23-7.32 (m, 2H), 7.38 (d, 2H), 7.41-7.54 (m, 2H), 7.83 (d, 2H), 8.56 (dd, 1H), 9.01 (dd, 1H). |
| | 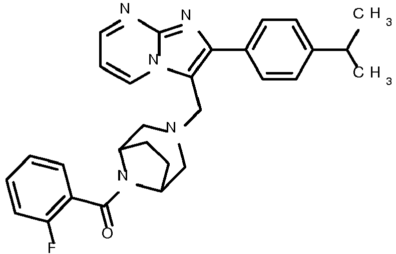 aus 3-(3,8-Diazabicyclo[3.2.1]oct-3-ylmethyl)-2-(4-isopropylphenyl)imidazo[1,2-a]pyrimidin-Dihydrochlorid und 2-Fluorbenzoesäure | |
| | | LC-MS (Methode 5): |
| | | Rₜ = 1.23 min; MS (ESIpos): m/z = 484 [M+H]⁺. |
| **65** | (3-{1-[2-(4-Chlorphenyl)imidazo[1,2-a]-pyrimidin-3-yl]ethyl}-3,8-diazabicyclo[3.2.1]oct-8-yl)(3-fluor-6-methoxypyridin-2-yl)methanon (*Racemat*) | ¹H-NMR (400 MHz, DMSO-d₆): δ [ppm] = 1.41-1.60 (m, 4H), 1.61-1.75 (m, 1H), 1.76-1.90 (m, 1H), 2.01 (br. d, 0.5H), 2.06-2.24 (m, 2.5H), 2.34 (br. d, 1H), 3.12 (br. d, 0.5H), 3.23 (br. dd, 0.5H), 3.59 (s, 1.5H), 3.75 (br. d, 0.5H), 3.85 (s, 1.5H), 4.04 (br. d, 0.5H), 4.07-4.17 (m, 1H), 4.43 (br. d, 0.5H), 4.71 (br. d, 0.5H), 6.89 (dd, 0.5H), 6.98 (dd, 0.5H), 7.08-7.15 (m, 1H), 7.52-7.61 (m, 2H), 7.66-7.84 (m, 3H), 8.53-8.63 (m, 1H), 9.27 (d, 1H). |
| | 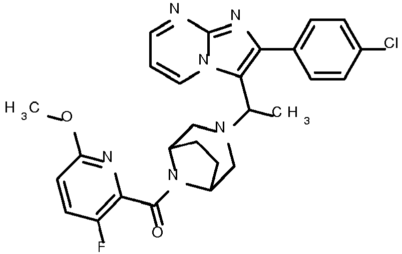 aus 2-(4-Chlorphenyl)-3-[1-(3,8-diazabicyclo-[3.2.1]oct-3-yl)ethyl]imidazo[1,2-a]pyrimidin-Dihydrochlorid (*Racemat*) und 3-Fluor-6-methoxypyridin-2-carbonsäure | |
| | | LC-MS (Methode 2): |
| | | Rₜ = 1.77 min; MS (ESIpos): m/z = 521/523 [M+H]⁺. |
| **66** | (7-{[2-(4-Isopropylphenyl)imidazo[1,2-a]-pyrimidin-3-yl]methyl}-3-oxa-7,9-diazabicyclo-[3.3.1]non-9-yl)(6-methoxypyridin-2-yl)-methanon | ¹H-NMR (400 MHz, DMSO-d₆): δ [ppm] = 1.25 (d, 6H), 2.44-2.65 (m, 2H, teilweise verdeckt durch DMSO-Signal), 2.86-3.00 (m, 2H), 3.05 (br. d, 1H), 3.66-3.82 (m, 3H), 3.80 (s, 3H), 3.89 (d, 1H), 3.94-4.06 (m, 2H), 4.20 (br. s, 1H), 4.45 (br. s, 1H), 6.92 (d, 1H), 7.06 (dd, 1H), 7.29 (d, 1H), 7.36 (d, 2H), 7.75-7.87 (m, 3H), 8.55 (dd, 1H), 9.28 (dd, 1H). |
| | 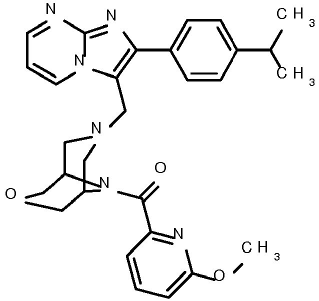 aus 7-{[2-(4-Isopropylphenyl)imidazo[1,2-a]-pyrimidin-3-yl]methyl}-3-oxa-7,9-diazabicyclo-[3.3.1]nonan-Dihydrochlorid und 6-Methoxypyridin-2-carbonsäure | |
| | | LC-MS (Methode 1): |
| | | Rₜ = 0.82 min; m/z = 513 (M+H)⁺. |
| **67** | (3-Chlor-6-methoxypyridin-2-yl)(7-{[2-(4-isopropylphenyl)imidazo[1,2-a]pyrimidin-3-yl]-methyl}-3-oxa-7,9-diazabicyclo[3.3.1]non-9-yl)-methanon | ¹H-NMR (400 MHz, DMSO-d₆): δ [ppm] = 1.26 (d, 6H), 2.44-2.57 (m, 2H, verdeckt durch DMSO-Signal), 2.84-3.00 (m, 2H), 3.06 (br. d, 1H), 3.34 (br. s, 1H, teilweise verdeckt H₂O-Signal), 3.61-3.75 (m, 3H), 3.82 (s, 3H), 3.88 (d, 1H), 3.99 (s, 2H), 4.44 (br. s, 1H), 6.93 (d, 1H), 7.05 (dd, 1H), 7.36 (d, 2H), 7.80 (d, 2H), 8.55 (dd, 1H), 9.28 (dd, 1H). |
| | 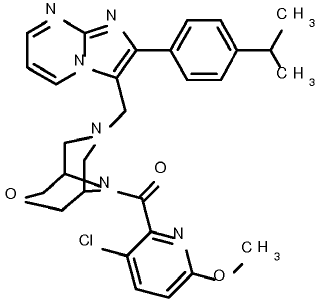 aus 7-{[2-(4-Isopropylphenyl)imidazo[1,2-a]-pyrimidin-3-yl]methyl}-3-oxa-7,9-diazabicyclo-[3.3.1]nonan-Dihydrochlorid und 3-Chlor-6-methoxypyridin-2-carbonsäure | |
| | | LC-MS (Methode 1): |
| | | Rₜ = 0.88 min; m/z = 547/549 (M+H)⁺. |
| **68** | (2-Fluorphenyl)(7-{[2-(4-isopropylphenyl)-imidazo[1,2-a]pyrimidin-3-yl]methyl}-3-oxa-7,9-diazabicyclo[3.3.1]non-9-yl)methanon | ¹H-NMR (400 MHz, DMSO-d₆): δ [ppm] = 1.26 (d, 6H), 2.41 (br. d, 1H), 2.46-2.58 (m, 1H, verdeckt durch DMSO-Signal), 2.84-3.06 (m, 3H), 3.37 (br. s, 1H), 3.59 (br. d, 1H), 3.71 (br. t, 2H), 3.87 (d, 1H), 3.99 (s, 2H), 4.47 (br. s, 1H), 7.05 (dd, 1H), 7.24-7.33 (m, 2H), 7.37 (d, 2H), 7.42-7.54 (m, 2H), 7.79 (d, 2H), 8.55 (dd, 1H), 9.28 (dd, 1H). |
| | 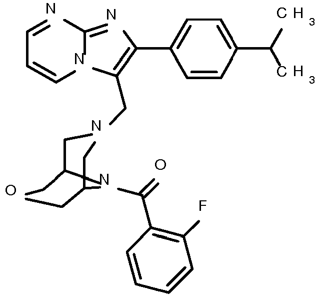 aus 7-{[2-(4-Isopropylphenyl)imidazo[1,2-a]-pyrimidin-3-yl]methyl}-3-oxa-7,9-diazabicyclo-[3.3.1]nonan-Dihydrochlorid und 2-Fluorbenzoesäure | |
| | | LC-MS (Methode 1): |
| | | Rₜ = 0.84 min; m/z = 500 (M+H)⁺. |
| **69** | (3-Chlor-6-methoxypyridin-2-yl)(5-{[2-(4-isopropylphenyl)imidazo[1,2-a]pyrimidin-3-yl]-methyl}-2,5-diazabicyclo[2.2.2]oct-2-yl)-methanon (*Enantiomer 1*) | ¹H-NMR (400 MHz, DMSO-d₆): δ [ppm] = 1.19-1.31 (m, 6H), 1.52-2.00 (m, 4H), 2.62 (br. dd, 0.75H), 2.70-2.85 (m, 1.5H), 2.89-3.03 (m, 2H), 3.21 (br. s, 0.75H), 3.43 (br. d, 1H), 3.70-3.86 (m, 3.75H), 4.22-4.35 (m, 2H), 4.38 (br. s, 0.25H), 6.85-6.96 (m, 1H), 7.05-7.15 (m, 1H), 7.30-7.40 (m, 2H), 7.70-7.86 (m, 2.7H), 7.90 (d, 0.3H), 8.56 (dd, 1H), 8.94-9.04 (m, 1H). |
| | 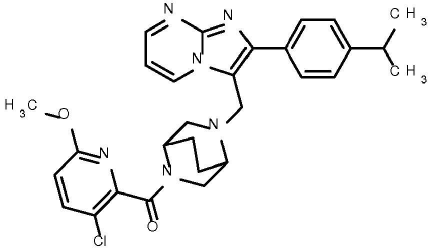 aus 3-(2,5-Diazabicyclo[2.2.2]oct-2-ylmethyl)-2-(4-isopropylphenyl)imidazo[1,2-a]pyrimidin-Dihydrochlorid (*Enantiomer 1*) und 3-Chlor-6-methoxypyridin-2-carbonsäure | |
| | | LC-MS (Methode 2): |
| | | Rₜ = 1.73 min; m/z = 531/533 (M+H)⁺. |
| **70** | (5-Cyclopropyl-1,3-oxazol-4-yl)(5-{[2-(4-isopropylphenyl)imidazo[1,2-a]pyrimidin-3-yl]-methyl}-2,5-diazabicyclo[2.2.2]oct-2-yl)-methanon (*Enantiomer 1*) | ¹H-NMR (400 MHz, DMSO-d₆): δ [ppm] = 0.81-0.95 (m, 2H), 0.96-1.09 (m, 2H), 1.25 (d, 6H), 1.49-1.63 (m, 1H), 1.66-2.00 (m, 3H), 2.44-2.57 (m, 0.7H, teilweise verdeckt durch DMSO-Signal), 2.57-2.65 (m, 0.3H), 2.73-3.02 (m, 4H), 3.36 (dd, 0.7H), 3.63-3.76 (m, 1H), 4.04 (br. d, 0.3H), 4.22-4.33 (m, 2H), 4.36 (br. s, 0.3H), 4.59 (br. s, 0.7H), 7.06-7.14 (m, 1H), 7.31-7.40 (m, 2H), 7.75-7.83 (m, 2H), 8.12-8.19 (m, 1H), 8.53-8.60 (m, 1H), 8.98-9.06 (m, 1H). |
| | 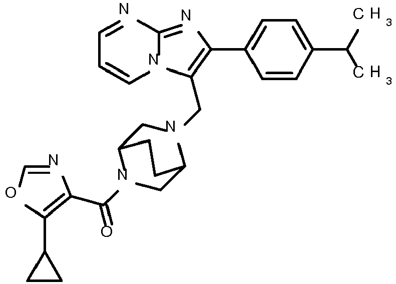 aus 3-(2,5-Diazabicyclo[2.2.2]oct-2-ylmethyl)-2-(4-isopropylphenyl)imidazo[1,2-a]pyrimidin-Dihydrochlorid (*Enantiomer 1*) und 5-Cyclopropyl-1,3-oxazol-4-carbonsäure | |
| | | LC-MS (Methode 2): |
| | | Rₜ = 1.52 min; m/z = 497 (M+H)⁺. |
| **71** | (3-Fluor-6-methoxypyridin-2-yl)(5-{ [2-(4-isopropylphenyl)imidazo[1,2-a]pyrimidin-3-yl]-methyl}-2,5-diazabicyclo[2.2.2]oct-2-yl)-methanon (*Enantiomer 1*) | ¹H-NMR (400 MHz, DMSO-d₆): δ [ppm] = 1.20-1.30 (m, 6H), 1.50-2.01 (m, 4H), 2.69 (br. dd, 0.75H), 2.74 (br. s, 0.25H), 2.77-2.88 (m, 1.25H), 2.90-3.01 (m, 1.75H), 3.13 (br. d, 0.25H), 3.39-3.52 (m, 1.5H), 3.61 (d, 0.25H), 3.69-3.84 (m, 3.75H), 4.21-4.35 (m, 2H), 4.36-4.42 (m, 0.25H), 6.88-6.98 (m, 1H), 7.05-7.15 (m, 1H), 7.30-7.40 (m, 2H), 7.70-7.83 (m, 3H), 8.52-8.60 (m, 1H), 8.96-9.06 (m, 1H). |
| | 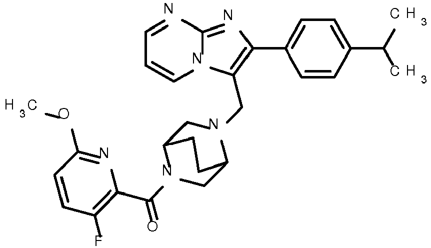 aus 3-(2,5-Diazabicyclo[2.2.2]oct-2-ylmethyl)-2-(4-isopropylphenyl)imidazo[1,2-a]pyrimidin-Dihydrochlorid (*Enantiomer 1*) und 3-Fluor-6-methoxypyridin-2-carbonsäure | |
| | | LC-MS (Methode 2): |
| | | Rₜ = 1.62 min; m/z = 515 (M+H)⁺. |
| **72** | (3-Fluor-6-methoxypyridin-2-yl)(5-{ [2-(4-isopropylphenyl)imidazo[1,2-a]pyrimidin-3-yl]-methyl}-2,5-diazabicyclo[2.2.2]oct-2-yl)-methanon (*Enantiomer 2*) | ¹H-NMR (400 MHz, DMSO-d₆): δ [ppm] = 1.20-1.31 (m, 6H), 1.51-2.01 (m, 4H), 2.69 (br. dd, 0.75H), 2.74 (br. s, 0.25H), 2.77-2.87 (m, 1.25H), 2.89-3.02 (m, 1.75H), 3.14 (br. d, 0.25H), 3.39-3.52 (m, 1.5H), 3.61 (d, 0.25H), 3.69-3.83 (m, 3.75H), 4.21-4.35 (m, 2H), 4.36-4.42 (m, 0.25H), 6.88-6.98 (m, 1H), 7.05-7.15 (m, 1H), 7.30-7.41 (m, 2H), 7.70-7.84 (m, 3H), 8.53-8.60 (m, 1H), 8.96-9.05 (m, 1H). |
| | 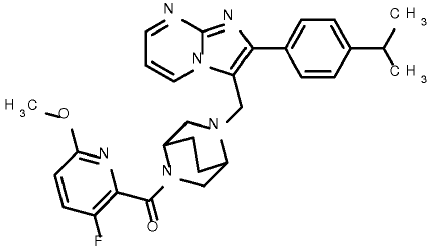 aus 3-(2,5-Diazabicyclo[2.2.2]oct-2-ylmethyl)-2-(4-isopropylphenyl)imidazo[1,2-a]pyrimidin-Dihydrochlorid (*Enantiomer 2*) und 3-Fluor-6-methoxypyridin-2-carbonsäure | |
| | | LC-MS (Methode 2): |
| | | Rₜ = 1.63 min; m/z = 515 (M+H)⁺. |
| **73** | (5-{[2-(4-Isopropylphenyl)imidazo[1,2-a]-pyrimidin-3-yl]methyl}-2,5-diazabicyclo[2.2.2]-oct-2-yl)(6-methoxypyridin-2-yl)methanon (*Enantiomer 1*) | ¹H-NMR (400 MHz, DMSO-d₆): δ [ppm] = 1.25 (d, 6H), 1.49-2.01 (m, 4H), 2.69-2.76 (m, 1H), 2.83-3.02 (m, 3H), 3.39 (dd, 0.75H), 3.47 (br. d, 0.25H), 3.70-3.86 (m, 3.75H), 3.93-4.04 (m, 1H), 4.23-4.35 (m, 2H), 4.39 (br. s, 0.25H), 6.84-6.95 (m, 1H), 7.05-7.14 (m, 1H), 7.17 (d, 0.75H), 7.26-7.40 (m, 2.25H), 7.73-7.85 (m, 3H), 8.52-8.60 (m, 1H), 8.97-9.06 (m, 1H). |
| | 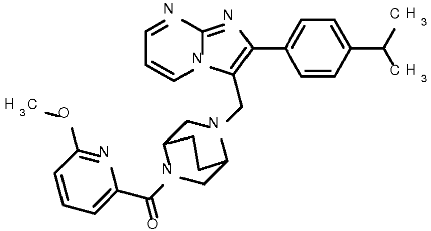 aus 3-(2,5-Diazabicyclo[2.2.2]oct-2-ylmethyl)-2-(4-isopropylphenyl)imidazo[1,2-a]pyrimidin-Dihydrochlorid (*Enantiomer 1*) und 6-Methoxypyridin-2-carbonsäure | |
| | | LC-MS (Methode 2): |
| | | Rₜ = 1.56 min; m/z = 497 (M+H)⁺. |
| **74** | (5-{[2-(4-Isopropylphenyl)imidazo[1,2-a]-pyrimidin-3-yl]methyl}-2,5-diazabicylo[2.2.2]-oct-2yl)(6-methoxypyridin-2-yl)methanon (*Enantiomer 2*) | ¹H-NMR (400 MHz, DMSO-d₆): δ [ppm] = 1.25 (d, 6H), 1.49-2.01 (m, 4H), 2.69-2.76 (m, 1H), 2.82-3.01 (m, 3H), 3.39 (dd, 0.75H), 3.47 (br. d, 0.25H), 3.70-3.85 (m, 3.75H), 3.93-4.02 (m, 1H), 4.23-4.35 (m, 2H), 4.39 (br. s, 0.25H), 6.84-6.94 (m, 1H), 7.05-7.14 (m, 1H), 7.17 (d, 0.75H), 7.25-7.40 (m, 2.25H), 7.73-7.85 (m, 3H), 8.52-8.60 (m, 1H), 8.97-9.05 (m, 1H). |
| | 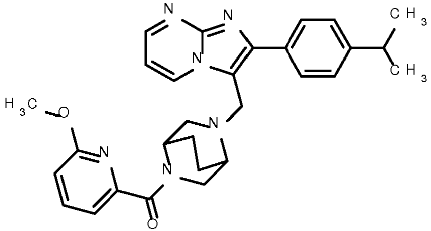 aus 3-(2,5-Diazabicyclo[2.2.2]oct-2-ylmethyl)-2-(4-isopropylphenyl)imidazo[1,2-a]pyrimidin-Dihydrochlorid (*Enantiomer 2*) und 6-Methoxypyridin-2-carbonsäure | |
| | | LC-MS (Methode 2): |
| | | Rₜ = 1.55 min; m/z = 497 (M+H)⁺. |
| **75** | [6-(Difluormethoxy)pyridin-2-yl](5-{[2-(4-isopropylphenyl)imidazo[1,2-a]pyrimidin-3-yl]-methyl}-2,5-diazabicyclo[2.2.2]oct-2-yl)-methanon (*Enantiomer 2*) | ¹H-NMR (400 MHz, DMSO-d₆): δ [ppm] = 1.20-1.29 (m, 6H), 1.50-2.01 (m, 4H), 2.71 (dd, 0.75H), 2.75 (br. s, 0.25H), 2.83-3.01 (m, 3H), 3.39 (dd, 0.75H), 3.47 (br. d, 0.25H), 3.73 (d, 0.75H), 3.93 (br. s, 1H), 4.23-4.34 (m, 2H), 4.39 (br. s, 0.25H), 7.05-7.13 (m, 1H), 7.14-7.22 (m, 1H), 7.31-7.41 (m, 2.25H), 7.45 (d, 0.75H), 7.48-7.71 (m, 1H), 7.75-7.82 (m, 2H), 7.97-8.09 (m, 1H), 8.53-8.60 (m, 1H), 8.96-9.05 (m, 1H). |
| | 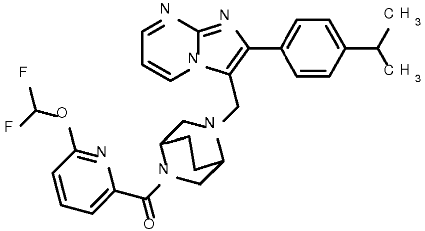 aus 3-(2,5-Diazabicyclo[2.2.2]oct-2-ylmethyl)-2-(4-isopropylphenyl)imidazo[1,2-a]pyrimidin-Dihydrochlorid (*Enantiomer 2*) und 6-(Difluormethoxy)pyridin-2-carbonsäure | |
| | | LC-MS (Methode 2): |
| | | Rₜ = 1.68 min; m/z = 533 (M+H)⁺. |
| **76** | [6-(Difluormethoxy)pyridin-2-yl](5-{[2-(4-isopropylphenyl)imidazo[1,2-a]pyrimidin-3-yl]-methyl}-2,5-diazabicyclo[2.2.2]oct-2-yl)-methanon (*Enantiomer 1*) | ¹H-NMR (400 MHz, DMSO-d₆): δ [ppm] = 1.20-1.30 (m, 6H), 1.50-2.01 (m, 4H), 2.71 (dd, 0.75H), 2.75 (br. s, 0.25H), 2.82-3.02 (m, 3H), 3.39 (dd, 0.75H), 3.47 (br. d, 0.25H), 3.73 (d, 0.75H), 3.93 (br. s, 1H), 4.23-4.35 (m, 2H), 4.38 (br. s, 0.25H), 7.05-7.13 (m, 1H), 7.14-7.23 (m, 1H), 7.31-7.41 (m, 2.25H), 7.45 (d, 0.75H), 7.49-7.71 (m, 1H), 7.75-7.83 (m, 2H), 7.97-8.09 (m, 1H), 8.52-8.60 (m, 1H), 8.96-9.07 (m, 1H). |
| | 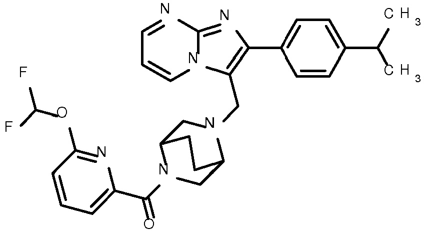 aus 3-(2,5-Diazabicyclo[2.2.2]oct-2-ylmethyl)-2-(4-isopropylphenyl)imidazo[1,2-a]pyrimidin-Dihydrochlorid (*Enantiomer 1*) und 6-(Difluormethoxy)pyridin-2-carbonsäure | |
| | | LC-MS (Methode 2): |
| | | Rₜ = 1.69 min; m/z = 533 (M+H)⁺. |
| **77** | (5-{[2-(4-Isopropylphenyl)imidazo[1,2-a]-pyrimidin-3-yl]methyl}-2,5-diazabicyclo[2.2.2]-oct-2-yl) (6-methoxy-3 -methylpyridin-2-yl) - methanon (*Enantiomer 2*) | ¹H-NMR (400 MHz, DMSO-d₆): δ [ppm] = 1.20-1.31 (m, 6H), 1.51-2.00 (m, 4H), 2.03-2.12 (m, 3H), 2.63 (dd, 0.75H), 2.70-2.77 (m, 1H), 2.81 (d, 0.5H), 2.89-3.03 (m, 2H), 3.24 (br. s, 0.75H), 3.42 (br. d, 1H), 3.65-3.84 (m, 3.75H), 4.20-4.34 (m, 2H), 4.37-4.43 (m, 0.25H), 6.71-6.80 (m, 1H), 7.06-7.14 (m, 1H), 7.29-7.41 (m, 2H), 7.54-7.65 (m, 1H), 7.72-7.83 (m, 2H), 8.52-8.60 (m, 1H), 8.95-9.05 (m, 1H). |
| | 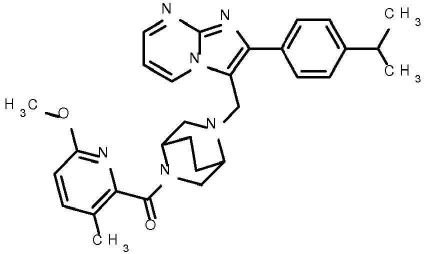 aus 3-(2,5-Diazabicyclo[2.2.2]oct-2-ylmethyl)-2-(4-isopropylphenyl)imidazo[1,2-a]pyrimidin-Dihydrochlorid (*Enantiomer 2*) und 6-Methoxy-3-methylpyridin-2-carbonsäure | |
| | | LC-MS (Methode 6): |
| | | Rₜ = 1.68 min; m/z = 511 (M+H)⁺. |
| **78** | (5-{[2-(4-Isopropylphenyl)imidazo[1,2-a]-pyrimidin-3-yl]methyl}-2,5-diazabicyclo[2.2.2]-oct-2-yl) (6-methoxy-3 -methylpyridin-2-yl) - methanon (*Enantiomer 1*) | ¹H-NMR (400 MHz, DMSO-d₆): δ [ppm] = 1.19-1.31 (m, 6H), 1.51-2.00 (m, 4H), 2.02-2.14 (m, 3H), 2.63 (dd, 0.75H), 2.69-2.78 (m, 1H), 2.81 (d, 0.5H), 2.89-3.03 (m, 2H), 3.24 (br. s, 0.75H), 3.42 (br. d, 1H), 3.65-3.84 (m, 3.75H), 4.20-4.35 (m, 2H), 4.37-4.43 (m, 0.25H), 6.70-6.80 (m, 1H), 7.06-7.15 (m, 1H), 7.30-7.40 (m, 2H), 7.54-7.64 (m, 1H), 7.72-7.83 (m, 2H), 8.53-8.60 (m, 1H), 8.94-9.05 (m, 1H). |
| | 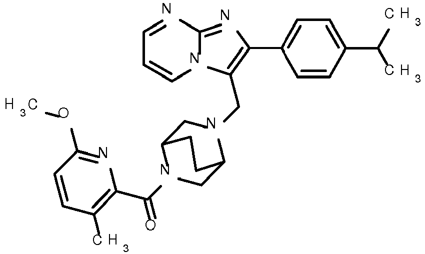 aus 3-(2,5-Diazabicyclo[2.2.2]oct-2-ylmethyl)-2-(4-isopropylphenyl)imidazo[1,2-a]pyrimidin-Dihydrochlorid (*Enantiomer 1*) und 6-Methoxy-3-methylpyridin-2-carbonsäure | |
| | | LC-MS (Methode 6): |
| | | Rₜ = 1.68 min; m/z = 511 (M+H)⁺. |
| **79** | (5-{[2-(4-Bromphenyl)imidazo[1,2-a]pyrimidin-3-yl]methyl}-2,5-diazabicyclo[2.2.2]oct-2-yl)-(2-fluorphenyl)methanon (*Racemat*) | ¹H-NMR (400 MHz, DMSO-d₆): δ [ppm] = 1.45-2.01 (m, 4H), 2.44-2.57 (m, 0.5H), 2.59-2.86 (m, 2.5H), 2.89-3.07 (m, 1H), 3.36-3.49 (m, 1H), 3.77 (br. d, 0.7H), 4.17-4.43 (m, 2.3H), 7.04-7.19 (m, 1H), 7.18-7.39 (m, 3H), 7.40-7.58 (m, 1H), 7.62-7.75 (m, 2H), 7.76-7.91 (m, 2H), 8.54-8.63 (m, 1H), 8.96-9.07 (m, 1H). |
| | 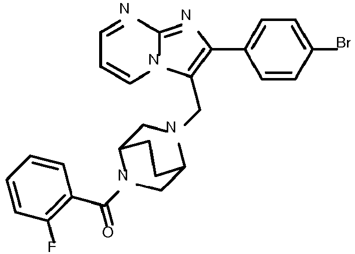 aus 2-(4-Bromphenyl)-3-(2,5-diazabicyclo-[2.2.2]oct-2-ylmethyl)imidazo[1,2-a]pyrimidin-Dihydrochlorid (*Racemat*) und 2-Fluorbenzoesäure | |
| | | LC-MS (Methode 2): |
| | | Rₜ = 1.54 min; m/z = 520/522 (M+H)⁺. |
| **80** | (5-{[2-(4-Bromphenyl)imidazo[1,2-a]pyrimidin-3-yl]methyl}-2,5-diazabicyclo[2.2.2]oct-2-yl)(cyclopentyl)methanon *(Racemat)* | |
| | 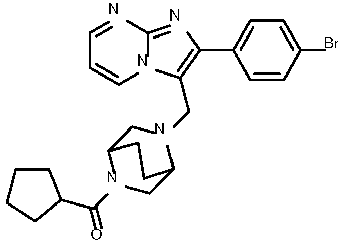 aus 2-(4-Bromphenyl)-3-(2,5-diazabicyclo[2.2.2]oct-2-ylmethyl)imidazo[1,2-a]-pyrimidin-Dihydrochlorid (*Racemat*) und Cyclopentancarbonsäure | |
| | ¹H-NMR (400 MHz, DMSO-d₆): δ [ppm] = -0.149 (0.54), -0.008 (4.35), 0.008 (3.57), 0.146 (0.49), 1.468 (2.71), 1.487 (3.20), 1.501 (3.94), 1.516 (4.35), 1.528 (4.98), 1.538 (4.91), 1.552 (4.91), 1.578 (3.45), 1.589 (3.88), 1.605 (4.88), 1.624 (5.35), 1.643 (3.91), 1.685 (3.00), 1.707 (4.06), 1.726 (3.25), 1.750 (1.24), 1.876 (1.41), 2.328 (0.63), 2.646 (0.95), 2.671 (2.30), 2.714 (7.31), 2.746 (4.33), 2.775 (1.34), 2.793 (1.64), 2.822 (3.00), 3.172 (2.01), 3.199 (2.37), 3.359 (1.56), 3.382 (1.69), 3.527 (1.95), 3.558 (1.66), 3.731 (1.41), 3.757 (1.20), 3.926 (2.76), 4.199 (1.20), 4.236 (6.26), 4.243 (9.84), 4.251 (6.91), 4.287 (1.22), 7.110 (4.45), 7.120 (4.69), 7.127 (4.62), 7.138 (4.61), 7.680 (11.67), 7.701 (16.00), 7.815 (8.75), 7.818 (11.65), 7.836 (7.72), 7.839 (7.96), 8.585 (4.37), 8.591 (4.13), 8.595 (4.20), 9.014 (3.08), 9.018 (5.06), 9.023 (2.68), 9.031 (3.23), 9.036 (4.89), 9.040 (2.42). | |
| | LC-MS (Methode 2): Rₜ = 1.45 min; m/z = 494/496 (M+H)⁺. | |
| **81** | (5-{[2-(4-Bromphenyl)imidazo[1,2-a]pyrimidin-3-yl]methyl}-2,5-diazabicyclo[2.2.2]oct-2-yl)-(3-fluor-6-methoxypyridin-2-yl)methanon (*Racemat*) | ¹H-NMR (400 MHz, DMSO-d₆): δ [ppm] = 1.52-1.99 (m, 4H), 2.67 (dd, 0.75H), 2.72 (br. s, 0.25H), 2.77-2.86 (m, 1.25H), 2.93 (br. s, 0.75H), 3.15 (br. d, 0.25H), 3.43 (dd, 0.75H), 3.48 (br. s, 0.75H), 3.57 (br. d, 0.25H), 3.70-3.83 (m, 3.75H), 4.22-4.34 (m, 2H), 4.36-4.41 (m, 0.25H), 6.89-6.99 (m, 1H), 7.08-7.17 (m, 1H), 7.64-7.88 (m, 5H), 8.57-8.62 (m, 1H), 9.00-9.08 (m, 1H). |
| | 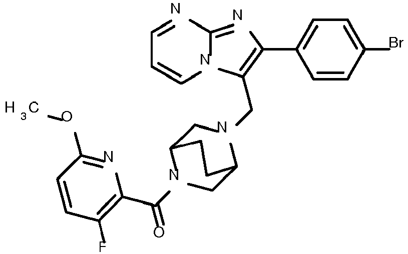 aus 2-(4-Bromphenyl)-3-(2,5-diazabicyclo-[2.2.2]oct-2-ylmethyl)imidazo[1,2-a]pyrimidin-Dihydrochlorid (*Racemat*) und 3-Fluor-6-methoxypyridin-2-carbonsäure | |
| | | LC-MS (Methode 2): |
| | | Rₜ = 1.55 min; m/z = 551/553 (M+H)⁺. |
| **82** | (3-{ [2-(4-Bromphenyl)imidazo[1,2-a]pyrimidin-3-yl]methyl}-3,8-diazabicyclo[3.2.1]oct-8-yl)-(3-fluor-6-methoxypyridin-2-yl)methanon | ¹H-NMR (400 MHz, DMSO-d₆): δ [ppm] = 1.60-1.83 (m, 4H), 2.39-2.58 (m, 3H, teilweise verdeckt durch DMSO-Signal), 2.75 (dd, 1H), 3.76 (s, 3H), 3.91 (br. s, 1H), 4.00-4.13 (m, 2H), 4.60 (br. s, 1H), 6.95 (dd, 1H), 7.14 (dd, 1H), 7.70 (d, 2H), 7.77 (t, 1H), 7.89 (d, 2H), 8.59 (dd, 1H), 9.06 (dd, 1H). |
| | 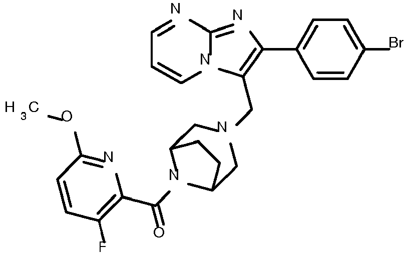 aus 2-(4-Bromphenyl)-3-(3,8-diazabicyclo-[3.2.1]oct-3-ylmethyl)imidazo[1,2-a]pyrimidin-Dihydrochlorid und 3-Fluor-6-methoxypyridin-2-carbonsäure | |
| | | LC-MS (Methode 1): |
| | | Rₜ = 0.93 min; m/z = 551/553 (M+H)⁺. |
| **83** | (3-{[2-(4-Bromphenyl)imidazo[1,2-a]pyrimidin-3-yl]methyl}-3,8-diazabicyclo[3.2.1]oct-8-yl)-(2-fluorphenyl)methanon | ¹H-NMR (400 MHz, DMSO-d₆): δ [ppm] = 1.64-1.81 (m, 4H), 2.24 (br. d, 1H), 2.42 (br. d, 1H), 2.47-2.60 (m, 1H, teilweise verdeckt durch DMSO-Signal), 2.68 (br. d, 1H), 3.66 (br. s, 1H), 4.04 (s, 2H), 4.59 (br. s, 1H), 7.14 (dd, 1H), 7.24-7.32 (m, 2H), 7.40-7.55 (m, 2H), 7.70 (d, 2H), 7.88 (d, 2H), 8.59 (dd, 1H), 9.04 (dd, 1H). |
| | 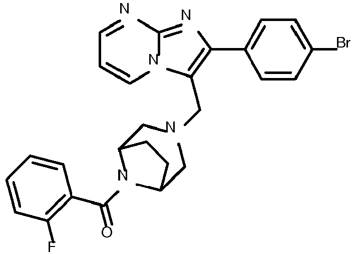 aus 2-(4-Bromphenyl)-3-(3,8-diazabicyclo-[3.2.1]oct-3-ylmethyl)imidazo[1,2-a]pyrimidin-Dihydrochlorid und 2-Fluorbenzoesäure | |
| | | LC-MS (Methode 1): |
| | | Rₜ = 0.93 min; m/z = 520/522 (M+H)⁺. |
| **84** | (3-{[2-(4-Bromphenyl)imidazo[1,2-a]pyrimidin-3-yl]methyl}-3,8-diazabicyclo[3.2.1]oct-8-yl)-(cyclopentyl)methanon | ¹H-NMR (400 MHz, DMSO-d₆): δ [ppm] = 1.44-1.66 (m, 7H), 1.66-1.80 (m, 5H), 2.26 (br. dd, 2H), 2.46-2.65 (m, 2H, teilweise verdeckt durch DMSO-Signal), 2.80-2.91 (m, 1H), 3.95-4.05 (m, 2H), 4.28 (br. s, 1H), 4.37-4.44 (m, 1H), 7.14 (dd, 1H), 7.70 (d, 2H), 7.90 (d, 2H), 8.59 (dd, 1H), 9.05 (dd, 1H). |
| | 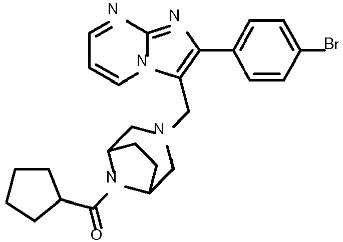 aus 2-(4-Bromphenyl)-3-(3,8-diazabicyclo-[3.2.1]oct-3-ylmethyl)imidazo[1,2-a]pyrimidin-Dihydrochlorid und Cyclopentancarbonsäure | |
| | | LC-MS (Methode 1): |
| | | Rₜ = 0.97 min; m/z = 494/496 (M+H)⁺. |

### Beispiel 85

### 3-{[2-(4-Chlorphenyl)imidazo[1,2-a]pyrimidin-3-yl]methyl}-N-(2,4-difluorphenyl)-3,8-diaza-bicyclo[3.2.1]octan-8-carboxamid

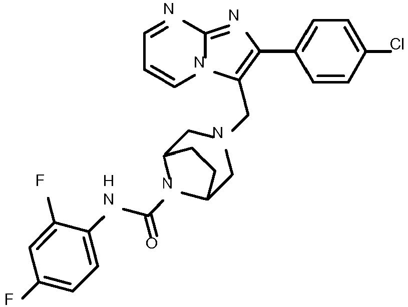

15.5 mg (0.10 mmol) 2,4-Difluorphenylisocyanat wurden in einer Vertiefung einer 96er-Multititerplatte vorgelegt und auf 0°C gekühlt. Separat wurden 46.3 mg 2-(4-Chlorphenyl)-3-(3,8-diaza-bicyclo[3.2.1]oct-3-ylmethyl)imidazo[1,2-a]pyrimidin-Dihydrochlorid in 0.8 ml 1,2-Dichlorethan gelöst, mit 0.052 ml (0.3 mmol) *N,N*-Diisopropylethylamin versetzt und auf 8°C gekühlt. Die beiden Lösungen wurden auf der Multititerplatte vereint, mit 4Å-Molekularsieb versetzt und zunächst 1 h bei 0°C geschüttelt. Anschließend wurde auf RT erwärmen gelassen und weiter bei RT über Nacht geschüttelt. Danach wurde das Lösungsmittel mittels Zentrifugaltrockner vollständig entfernt. Der Rückstand wurde in 0.6 ml DMF gelöst, filtriert und das Filtrat per präparativer LC-MS nach einer der folgenden Methoden in seine Komponenten aufgetrennt:
Instrument MS: Waters, Instrument HPLC: Waters; Säule: Phenomenex Luna 5µ C18(2) 100A, AXIA Tech., 50 mm x 21.2 mm; Eluent A: Wasser, Eluent B: Acetonitril, mit Eluentengradient; Fluss: 38.5 ml/min + 1.5 ml/min 10% aq. Ameisensäure; UV-Detektion: DAD, 210-400 nm bzw.
Instrument MS: Waters, Instrument HPLC: Waters; Säule: Phenomenex Luna 5µ C18(2) 100A, AXIA Tech., 50 mm x 21.2 mm; Eluent A: Wasser, Eluent B: Methanol, mit Eluentengradient; Fluss: 38.5 ml/min + 1.5 ml/min 10% Ammoniak in Wasser; UV-Detektion: DAD, 210-400 nm.

Es wurden so 17.9 mg (35% d. Th., Reinheit 100%) der Titelverbindung erhalten.

LC-MS (Methode 7, ESIpos): Rₜ = 1.14 min; m/z = 509 (M+H)⁺.

Auf zu Beispiel 85 analoge, parallel-synthetische Weise wurden die folgenden Verbindungen ausgehend von 2-(4-Chlorphenyl)-3-(3,8-diazabicyclo[3.2.1]oct-3-ylmethyl)imidazo[1,2-a]pyrimidin-Dihydrochlorid und dem entsprechenden Isocyanat, Carbamoylchlorid bzw. Chlorformiat hergestellt:

| **Beispiel** | **Name / Struktur (Ausbeute, Reinheit)** | **LC-MS (Methode 7)** |
|---|---|---|
| **86** | 3-{[2-(4-Chlorphenyl)imidazo[1,2-a]pyrimidin-3-yl]methyl}-*N*-isopropyl-3,8-diazabicyclo-[3.2.1]octan-8-carboxamid | Rₜ = 1.03 min; m/z = 439 [M+H]⁺ |
| | 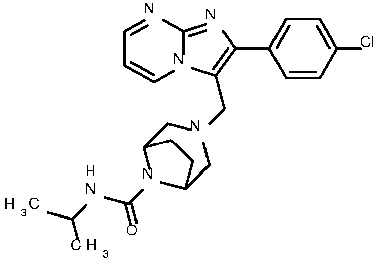 11.1 mg (90% Reinheit, 23% d. Th.) | |
| **87** | 3-{[2-(4-Chlorphenyl)imidazo[1,2-a]pyrimidin-3-yl]methyl}-*N*-cyclopropyl-3,8-diazabicyclo-[3.2.1]octan-8-carboxamid | Rₜ = 0.99 min; m/z = 437 [M+H]⁺ |
| | 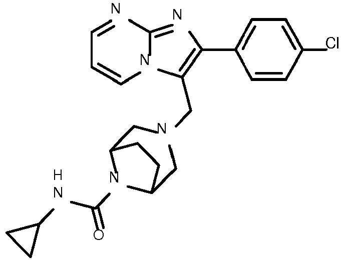 900 µg (100% Reinheit, 2% d. Th.) | |
| **88** | 3-{[2-(4-Chlorphenyl)imidazo[1,2-a]pyrimidin-3-yl]methyl}-*N*-(2,5-dichlor-4-methoxyphenyl)-3,8-diazabicyclo[3.2.1]octan-8-carboxamid | Rₜ = 1.22 min; m/z = 571 [M+H]⁺ |
| | 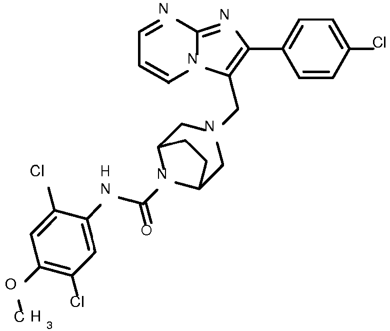 8.3 mg (90% Reinheit, 13% d. Th.) | |
| **89** | *N*-(3-Chlorphenyl)-3-{[2-(4-chlorphenyl)-imidazo[1,2-a]pyrimidin-3-yl]methyl}-3,8-diazabicyclo[3.2.1]octan-8-carboxamid | Rₜ = 1.19 min; m/z = 507 [M+H]⁺ |
| | 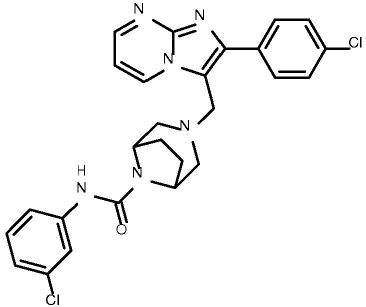 9.7 mg (100% Reinheit, 19% d. Th.) | |
| **90** | 3-{[2-(4-Chlorphenyl)imidazo[1,2-a]pyrimidin-3-yl]methyl}-*N*-(2,6-difluorbenzyl)-3,8-diaza-bicyclo[3.2.1]octan-8-carboxamid | Rₜ = 1.14 min; m/z = 523 [M+H]⁺ |
| | 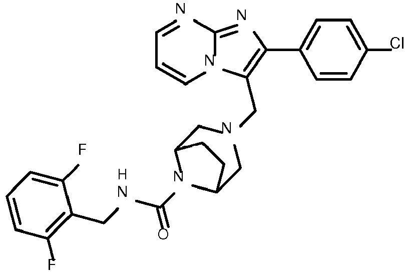 17.2 mg (100% Reinheit, 33% d. Th.) | |
| **91** | 3-{[2-(4-Chlorphenyl)imidazo[1,2-a]pyrimidin-3-yl]methyl}-*N*-(2,6-dichlorphenyl)-3,8-diaza-bicyclo[3.2.1]octan-8-carboxamid | Rₜ = 1.15 min; m/z = 541 [M+H]⁺ |
| | 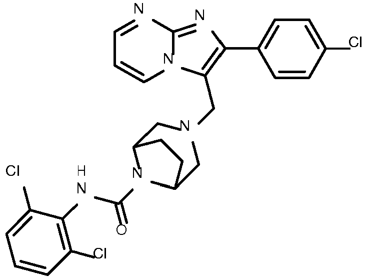 16.4 mg (98% Reinheit, 30% d. Th.) | |
| **92** | 3-{[2-(4-Chlorphenyl)imidazo[1,2-a]pyrimidin-3-yl]methyl}-*N*-(2,6-dimethylphenyl)-3,8-diaza-bicyclo[3.2.1]octan-8-carboxamid | Rₜ = 1.14 min; m/z = 501 [M+H]⁺ |
| | 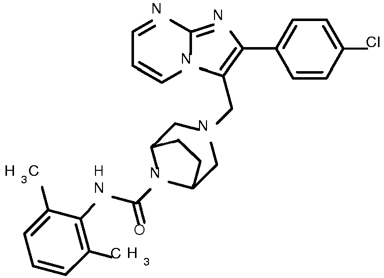 18.5 mg (99% Reinheit, 37% d. Th.) | |
| **93** | 3-{[2-(4-Chlorphenyl)imidazo[1,2-a]pyrimidin-3-yl]methyl}-*N*-(2-fluorphenyl)-3,8-diaza-bicyclo[3.2.1]octan-8-carboxamid | Rₜ = 1.13 min; m/z = 491 [M+H]⁺ |
| | 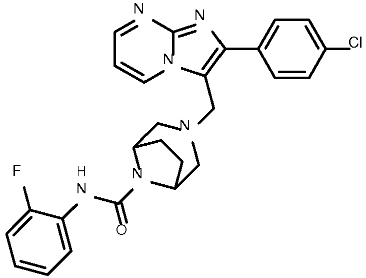 24.8 mg (98% Reinheit, 49% d. Th.) | |
| **94** | 3-{ [2-(4-Chlorphenyl)imidazo[1,2-a]pyrimidin-3-yl]methyl}-*N*-(2,3-dichlorphenyl)-3,8-diaza-bicyclo[3.2.1]octan-8-carboxamid | Rₜ = 1.24 min; m/z = 541 [M+H]⁺ |
| | 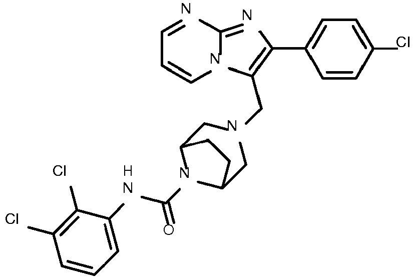 20.8 mg (99% Reinheit, 38% d. Th.) | |
| **95** | 3-{[2-(4-Chlorphenyl)imidazo[1,2-a]pyrimidin-3-yl]methyl}-*N*-(2-ethylphenyl)-3,8-diaza-bicyclo[3.2.1]octan-8-carboxamid | Rₜ = 1.17 min; m/z = 501 [M+H]⁺ |
| | 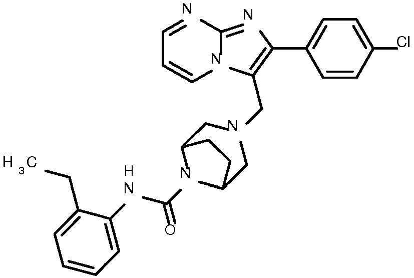 6.0 mg (100% Reinheit, 12% d. Th.) | |
| **96** | *N*-(2-Chlorphenyl)-3-{[2-(4-chlorphenyl)-imidazo[1,2-a]pyrimidin-3-yl]methyl}-3,8-diaza-bicyclo[3.2.1]octan-8-carboxamid | Rₜ = 1.17 min; m/z = 507 [M+H]⁺ |
| | 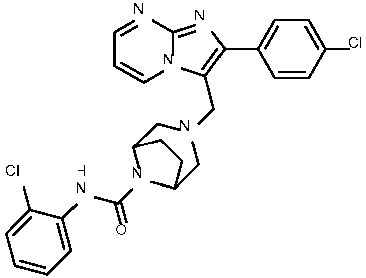 600 µg (100% Reinheit, 1% d. Th.) | |
| **97** | 3-{ [2-(4-Chlorphenyl)imidazo[1,2-a]pyrimidin-3-yl]methyl}-*N*-[2-chlor-5-(trifluormethyl)-phenyl]-3,8-diazabicyclo[3.2.1]octan-8-carboxamid | Rₜ = 1.29 min; m/z = 575 [M+H]⁺ |
| | 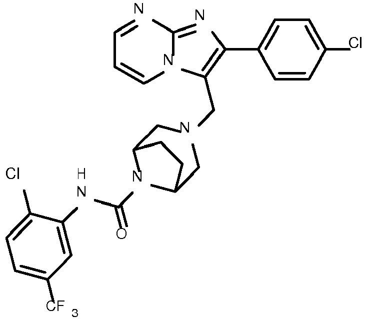 26.9 mg (99% Reinheit, 47% d. Th.) | |
| **98** | 3-{[2-(4-Chlorphenyl)imidazo[1,2-a]pyrimidin-3-yl]methyl}-*N*-(2-ethyl-6-methylphenyl)-3,8-diazabicyclo[3.2.1]octan-8-carboxamid | Rₜ = 1.18 min; m/z = 515 [M+H]⁺ |
| | 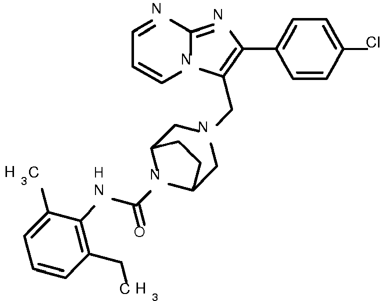 7.8 mg (97% Reinheit, 15% d. Th.) | |
| **99** | 3-{[2-(4-Chlorphenyl)imidazo[1,2-a]pyrimidin-3-yl] methyl } -*N*-(2, 5-dimethylphenyl)-3 , 8-diazabicyclo[3.2.1]octan-8-carboxamid | Rₜ = 1.17 min; m/z = 501 [M+H]⁺ |
| | 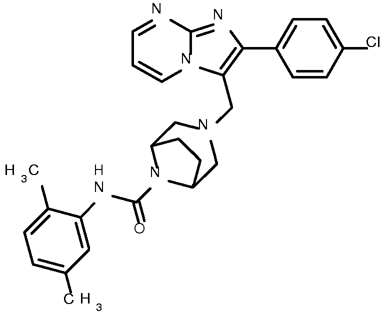 16.1 mg (94% Reinheit, 30% d. Th.) | |
| **100** | 3-{[2-(4-Chlorphenyl)imidazo[1,2-a]pyrimidin-3-yl]methyl}-*N*-cyclohexyl-3,8-diaza-bicyclo[3.2.1]octan-8-carboxamid | Rₜ = 1.14 min; m/z = 479 [M+H]⁺ |
| | 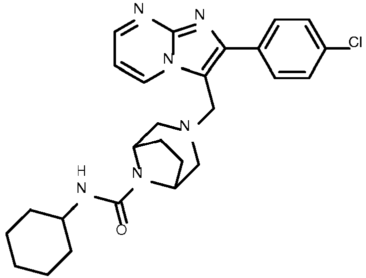 27.3 mg (100% Reinheit, 57% d. Th.) | |
| **101** | 3-{[2-(4-Chlorphenyl)imidazo[1,2-a]pyrimidin-3-yl]methyl}-*N*-isobutyl-3,8-diazabicyclo-[3.2.1]octan-8-carboxamid | Rₜ = 1.09 min; m/z = 453 [M+H]⁺ |
| | 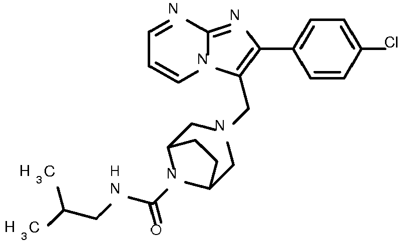 5.1 mg (100% Reinheit, 11% d. Th.) | |
| **102** | 3-{[2-(4-Chlorphenyl)imidazo[1,2-a]pyrimidin-3-yl]methyl}-*N*-(3,4-dimethoxyphenyl)-3,8-diazabicyclo[3.2.1]octan-8-carboxamid | Rₜ = 1.07 min; m/z = 533 [M+H]⁺ |
| | 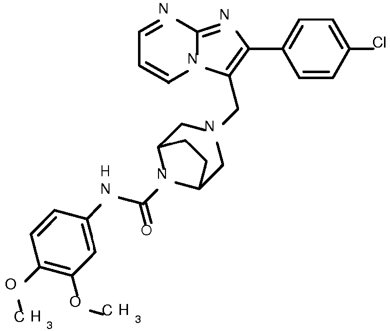 14.6 mg (90% Reinheit, 25% d. Th.) | |
| **103** | 3-{ [2-(4-Chlorphenyl)imidazo[1,2-a]pyrimidin-3-yl]methyl}-*N*-{4-[(trifluormethyl)sulfanyl]-phenyl}-3,8-diazabicyclo[3.2.1]octan-8-carboxamid | Rₜ = 1.27 min; m/z = 573 [M+H]⁺ |
| | 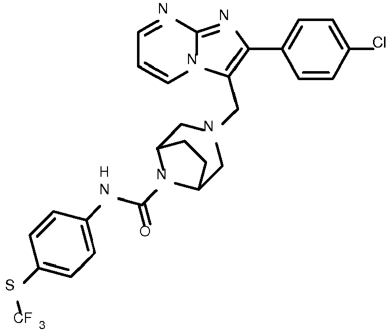 2.0 mg (100% Reinheit, 3% d. Th.) | |
| **104** | 3-{[2-(4-Chlorphenyl)imidazo[1,2-a]pyrimidin-3-yl]methyl}-*N*-(3-fluorphenyl)-3,8-diaza-bicyclo[3.2.1]octan-8-carboxamid | Rₜ = 1.16 min; m/z = 491 [M+H]⁺ |
| | 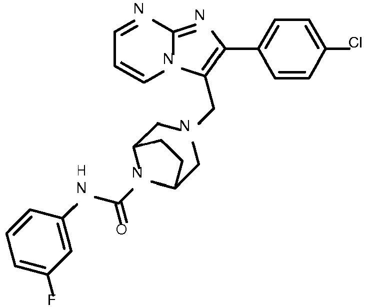 26.2 mg (97% Reinheit, 52% d. Th.) | |
| **105** | 3-{[2-(4-Chlorphenyl)imidazo[1,2-a]pyrimidin-3-yl]methyl}-*N*-(2,6-difluorphenyl)-3,8-diaza-bicyclo[3.2.1]octan-8-carboxamid | Rₜ = 1.10 min; m/z = 509 [M+H]⁺ |
| | 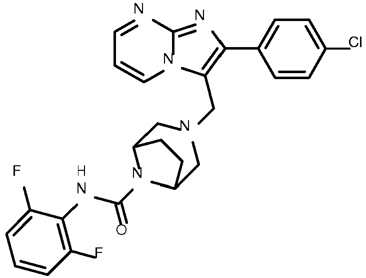 12.8 mg (100% Reinheit, 25% d. Th.) | |
| **106** | 3-{[2-(4-Chlorphenyl)imidazo[1,2-a]pyrimidin-3-yl]methyl}-*N*-[4-chlor-2-(trifluormethyl)-phenyl]-3,8-diazabicyclo[3.2.1]octan-8-carboxamid | Rₜ = 1.25 min; m/z = 575 [M+H]⁺ |
| | 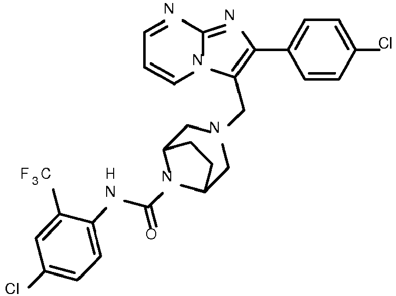 20.6 mg (92% Reinheit, 33% d. Th.) | |
| **107** | 3-{[2-(4-Chlorphenyl)imidazo[1,2-a]pyrimidin-3-yl]methyl}-*N*-(2-methylbenzyl)-3,8-diaza-bicyclo[3.2.1]octan-8-carboxamid | Rₜ = 1.16 min; m/z = 501 [M+H]⁺ |
| | 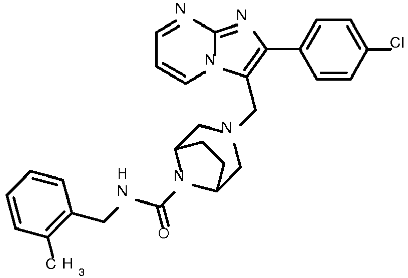 20.1 mg (100% Reinheit, 40% d. Th.) | |
| **108** | 3-{[2-(4-Chlorphenyl)imidazo[1,2-a]pyrimidin-3-yl]methyl}-*N*-methyl-N-phenyl-3,8-diaza-bicyclo[3.2.1]octan-8-carboxamid | Rₜ = 1.22 min; m/z = 487 [M+H]⁺ |
| | 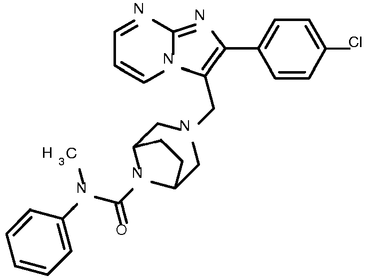 16.6 mg (100% Reinheit, 34% d. Th.) | |
| **109** | 3-{[2-(4-Chlorphenyl)imidazo[1,2-a]pyrimidin-3-yl]methyl}-*N,N*-diethyl-3,8-diazabicyclo-[3.2.1]octan-8-carboxamid | Rₜ = 1.16 min; m/z = 453 [M+H]⁺ |
| | 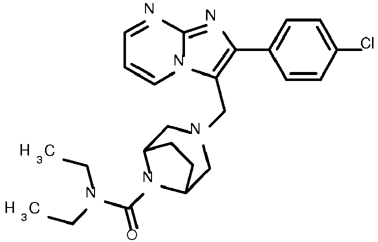 21.3 mg (100% Reinheit, 47% d. Th.) | |
| **110** | (3-{[2-(4-Chlorphenyl)imidazo[1,2-a]pyrimidin-3-yl]methyl}-3,8-diazabicyclo[3.2.1]oct-8-yl)-(morpholin-4-yl)methanon | Rₜ = 1.03 min; m/z = 467 [M+H]⁺ |
| | 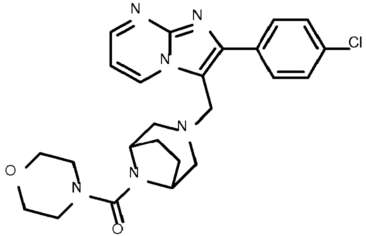 2.7 mg (100% Reinheit, 6% d. Th.) | |
| **111** | 3-{ [2-(4-Chlorphenyl)imidazo[1,2-a]pyrimidin-3-yl]methyl}-*N,N*-diisopropyl-3,8-diaza-bicyclo[3.2.1]octan-8-carboxamid | Rₜ = 1.23 min; m/z = 481 [M+H]⁺ |
| | 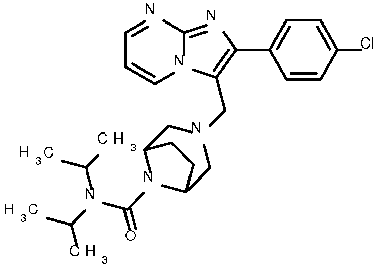 2.4 mg (100% Reinheit, 5% d. Th.) | |
| **112** | 3-{[2-(4-Chlorphenyl)imidazo[1,2-a]pyrimidin-3-yl]methyl}-N-cyclohexyl-*N*-ethyl-3,8-diaza-bicyclo[3.2.1]octan-8-carboxamid | Rₜ = 1.30 min; m/z = 507 [M+H]⁺ |
| | 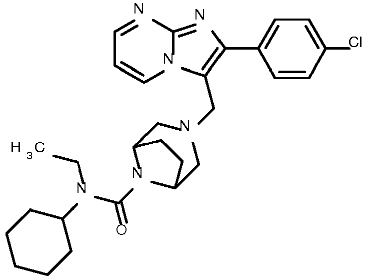 16.8 mg (100% Reinheit, 33% d. Th.) | |
| **113** | (3-{[2-(4-Chlorphenyl)imidazo[1,2-a]pyrimidin-3-yl]methyl}-3,8-diazabicyclo[3.2.1]oct-8-yl)-(pyrrolidin-1-yl)methanon | Rₜ = 1.10 min; m/z = 451 [M+H]⁺ |
| | 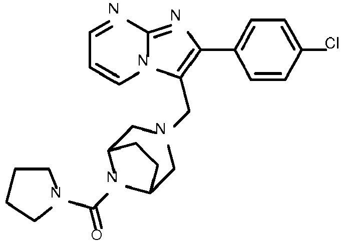 22.8 mg (98% Reinheit, 50% d. Th.) | |
| **114** | 3-{ [2-(4-Chlorphenyl)imidazo[1,2-a]pyrimidin-3-yl]methyl}-*N*-ethyl-*N*-phenyl-3,8-diaza-bicyclo[3.2.1]octan-8-carboxamid | Rₜ = 1.25 min; m/z = 501 [M+H]⁺ |
| | 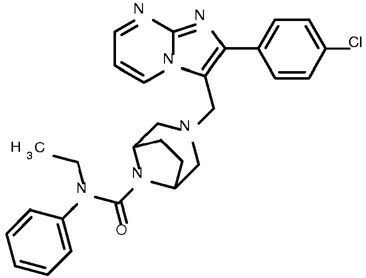 14.9 mg (100% Reinheit, 30% d. Th.) | |
| **115** | 3-{[2-(4-Chlorphenyl)imidazo[1,2-a]pyrimidin-3-yl]methyl}-*N*-isopropyl-*N*-methyl-3,8-diaza-bicyclo[3.2.1]octan-8-carboxamid | Rₜ = 1.14 min; m/z = 453 [M+H]⁺ |
| | 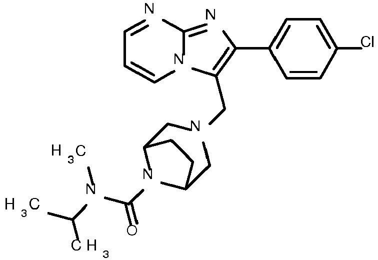 4.3 mg (100% Reinheit, 9% d. Th.) | |
| **116** | (3-{[2-(4-Chlorphenyl)imidazo[1,2-a]pyrimidin-3-yl]methyl}-3,8-diazabicyclo[3.2.1]oct-8-yl)-(piperidin-1-yl)methanon | Rₜ = 1.16 min; m/z = 465 [M+H]⁺ |
| | 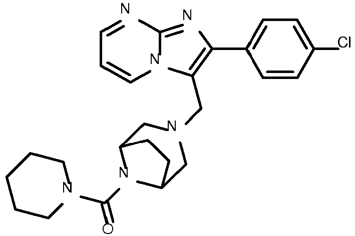 3.0 mg (100% Reinheit, 6% d. Th.) | |
| **117** | 3-{[2-(4-Chlorphenyl)imidazo[1,2-a]pyrimidin-3-yl]methyl}-*N*-ethyl-*N*-(4-methylphenyl)-3,8-diazabicyclo[3.2.1]octan-8-carboxamid | Rₜ = 1.30 min; m/z = 515 [M+H]⁺ |
| | 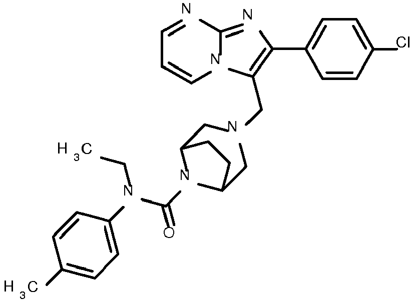 5.2 mg (98% Reinheit, 10% d. Th.) | |
| **118** | *N*-(4-Chlorphenyl)-3-{[2-(4-chlorphenyl)-imidazo[1,2-a]pyrimidin-3-yl]methyl}-*N*-isopropyl-3,8-diazabicyclo[3.2.1]octan-8-carboxamid | Rₜ = 1.33 min; m/z = 549 [M+H]⁺ |
| | 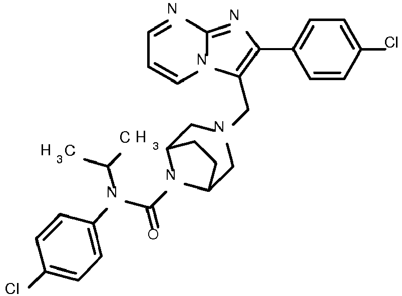 3.4 mg (100% Reinheit, 6% d. Th.) | |
| **119** | 3-{ [2-(4-Chlorphenyl)imidazo[1,2-a]pyrimidin-3-yl]methyl}-*N,N-*dimethyl-3,8-diazabicyclo-[3.2.1]octan-8-carboxamid | Rₜ = 1.05 min; m/z = 425 [M+H]⁺ |
| | 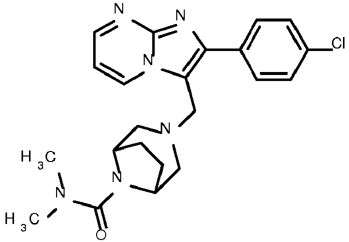 6.6 mg (100% Reinheit, 16% d. Th.) | |
| **120** | 3-{[2-(4-Chlorphenyl)imidazo[1,2-a]pyrimidin-3-yl]methyl}-*N*-(4-ethoxyphenyl)-*N*-methyl-3,8-diazabicyclo[3.2.1]octan-8-carboxamid | Rₜ = 1.26 min; m/z = 531 [M+H]⁺ |
| | 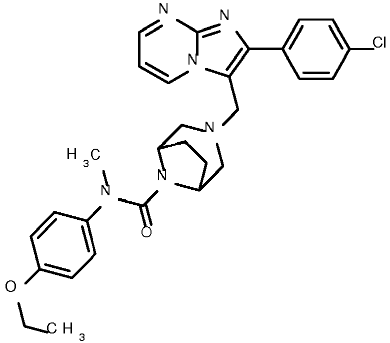 35.4 mg (100% Reinheit, 67% d. Th.) | |
| **121** | 3-{[2-(4-Chlorphenyl)imidazo[1,2-a]pyrimidin-3-yl]methyl}-*N*-(3-methoxybenzyl)-*N*-methyl-3 ,8-diazabicyclo[3.2.1]octan-8-carboxamid | Rₜ = 1.23 min; m/z = 531 [M+H]⁺ |
| | 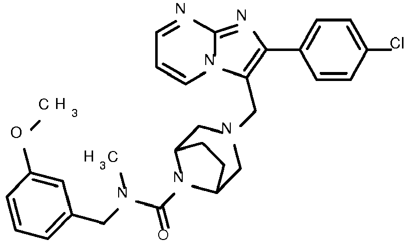 35.0 mg (96% Reinheit, 63% d. Th.) | |
| **122** | (3-{[2-(4-Chlorphenyl)imidazo[1,2-a]pyrimidin-3-yl]methyl}-3,8-diazabicyclo[3.2.1]oct-8-yl)-(thiomorpholin-4-yl)methanon | Rₜ = 1.13 min; m/z = 483 [M+H]⁺ |
| | 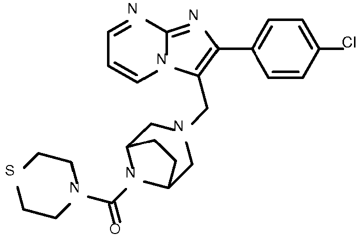 5.4 mg (100% Reinheit, 11% d. Th.) | |
| **123** | Methyl-3-{[2-(4-chlorphenyl)imidazo[1,2-a]-pyrimidin-3-yl]methyl}-3,8-diazabicyclo-[3.2.1]octan-8-carboxylat | Rₜ = 1.10 min; m/z = 412 [M+H]⁺ |
| | 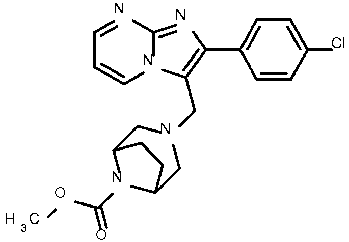 20.7 mg (100% Reinheit, 50% d. Th.) | |
| **124** | Ethyl-3-{[2-(4-chlorphenyl)imidazo[1,2-a]-pyrimidin-3-yl]methyl}-3,8-diazabicyclo-[3.2.1]octan-8-carboxylat | Rₜ = 1.15 min; m/z = 426 [M+H]⁺ |
| | 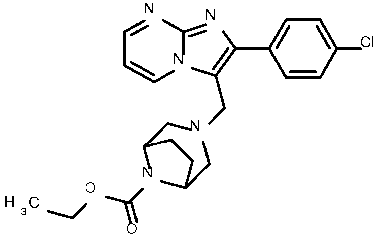 17.8 mg (98% Reinheit, 41% d. Th.) | |
| **125** | Cyclopentyl-3-{[2-(4-chlorphenyl)imidazo-[1,2-a]pyrimidin-3-yl]methyl}-3,8-diaza-bicyclo[3.2.1]octan-8-carboxylat | Rₜ = 1.26 min; m/z = 466 [M+H]⁺ |
| | 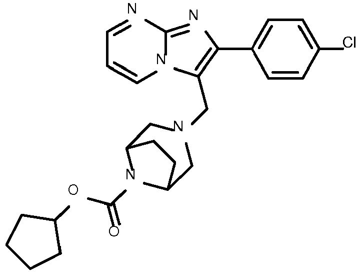 19.7 mg (100% Reinheit, 42% d. Th.) | |
| **126** | Propyl-3-{[2-(4-chlorphenyl)imidazo[1,2-a]-pyrimidin-3-yl]methyl}-3,8-diazabicyclo-[3.2.1]octan-8-carboxylat | Rₜ = 1.21 min; m/z = 440 [M+H]⁺ |
| | 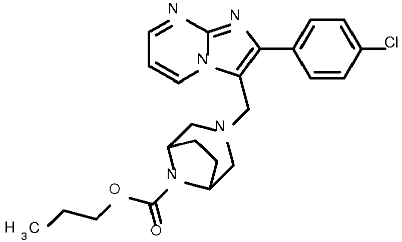 22.5 mg (98% Reinheit, 50% d. Th.) | |
| **127** | Cyclohexylmethyl-3-{ [2-(4-chlorphenyl)-imidazo[1,2-a]pyrimidin-3-yl]methyl}-3,8-diazabicyclo[3.2.1]octan-8-carboxylat | Rₜ = 1.35 min; m/z = 494 [M+H]⁺ |
| | 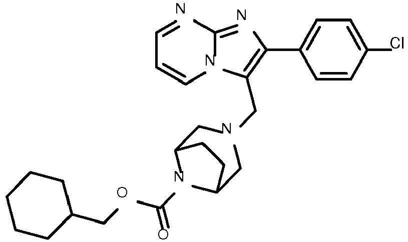 11.7 mg (92% Reinheit, 22% d. Th.) | |
| **128** | Cyclohexyl-3-{[2-(4-chlorphenyl)imidazo[1,2-a]-pyrimidin-3-yl]methyl}-3,8-diazabicyclo-[3.2.1]octan-8-carboxylat | Rₜ = 1.30 min; m/z = 480 [M+H]⁺ |
| | 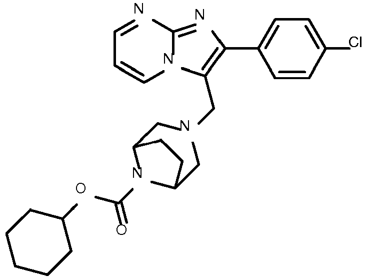 4.0 mg (91% Reinheit, 8% d. Th.) | |
| **129** | 2,2-Dimethylpropyl-3- { [2-(4-chlorphenyl)-imidazo[1,2-a]pyrimidin-3-yl]methyl}-3,8-diazabicyclo[3.2.1]octan-8-carboxylat | Rₜ = 1.29 min; m/z = 468 [M+H]⁺ |
| | 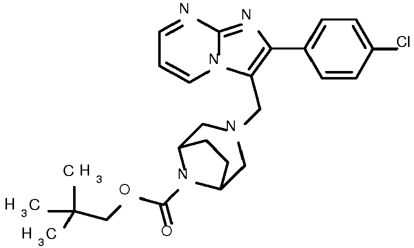 10.9 mg (96% Reinheit, 22% d. Th.) | |

### Beispiel 130

### tert.-Butyl-3-{[2-(4-isopropylphenyl)imidazo[1,2-a]pyrimidin-3-yl]methyl}-3,8-diazabicyclo-[3.2.1]octan-8-carboxylat

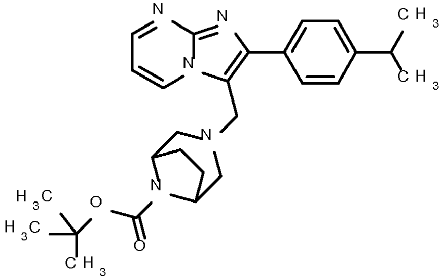

Unter Argon wurde bei Raumtemperatur 2-(4-Isopropylphenyl)imidazo[1,2-a]pyrimidin-3-carb-aldehyd (700 mg, 2.64 mmol) in 14 ml THF gelöst und mit tert.-Butyl-3,8-diazabicyclo[3.2.1]-octan-8-carboxylat (672 mg, 3.17 mmol) versetzt. Anschließend wurde portionsweise Natriumtriacetoxyborhydrid (839 mg, 3.96 mmol) hinzugegeben und die Reaktionslösung über Nacht bei Raumtemperatur gerührt. Dann wurde langsam und vorsichtig Wasser zugetropft (Vorsicht: Gasentwicklung) und anschließend mit Essigsäureethylester versetzt. Die resultierende organische Phase wurde abgetrennt und die wässrige Phase zweimal mit Essigsäureethylester extrahiert. Die vereinigten organischen Phasen wurden mit gesättigter Natriumchlorid-Lösung gewaschen, über Magnesiumsulfat getrocknet, filtriert und im Vakuum am Rotationsverdampfer bis zur Trockene eingeengt. Der erhaltene Rückstand wurde mittels Säulenchromatographie gereinigt (Biotage Isolera, Biotage SNAP-KP-NH-Säule, Laufmittel Cyclohexan/Essigsäureethylester-Gradient). Es wurden 896 mg (1.94 mmol, 74% d. Th.) der Zielverbindung erhalten.

LC-MS (Methode 2): Rₜ = 2.14 min; m/z = 462 (M+H)⁺.

¹H-NMR (400 MHz, DMSO-d₆): δ [ppm] = 1.25 (d, 6H), 1.39 (s, 9H), 1.65 (br. s, 4H), 2.26 (br. d, 2H), 2.47-2.60 (m, 2H, teilweise verdeckt durch DMSO-Signal), 2.95 (quin, 1H), 3.98 (s, 2H), 4.02 (br. s, 2H), 7.12 (dd, 1H), 7.37 (d, 2H), 7.83 (d, 2H), 8.56 (dd, 1H), 8.99 (dd, 1H).

### Beispiel 131

### (5-Cyclopropyl-1,3-oxazol-4-yl)(3-{[2-(4-isopropylphenyl)imidazo[1,2-a]pyrimidin-3-yl]methyl}-3,8-diazabicyclo[3.2.1]oct-8-yl)methanon

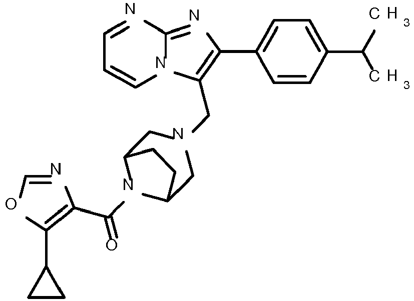

5-Cyclopropyl-1,3-oxazol-4-carbonsäure (39 mg, 0.26 mmol) wurde in 1.5 ml DMF gelöst, mit 2-(7-Aza-1*H*-benzotriazol-1-yl)-1,1 ,3,3-tetramethyluronium-Hexafluorophosphat (HATU) (121 mg, 0.32 mmol) versetzt und 30 min bei Raumtemperatur gerührt. Anschließend wurde 3-(3,8-Diazabicyclo[3.2.1]oct-3-ylmethyl)-2-(4-isopropylphenyl)imidazo[1,2-a]pyrimidin-Dihydrochlorid (100 mg) sowie *N,N*-Diisopropylethylamin (190 µl, 1.06 mmol) hinzugegeben und das Gemisch über Nacht bei Raumtemperatur gerührt. Danach wurde das Reaktionsgemisch direkt über präparative HPLC (Methode 9) in seine Komponenten aufgetrennt. Es wurden 65 mg (0.13 mmol, 61% d. Th.) der Titelverbindung erhalten.

LC-MS (Methode 2): Rₜ = 1.81 min; MS (ESIpos): m/z = 497 [M+H]⁺.

¹H-NMR (400 MHz, DMSO-d₆): δ [ppm] = 0.85-0.99 (m, 2H), 1.01-1.13 (m, 2H), 1.25 (d, 6H), 1.63-1.85 (m, 4H), 2.35-2.45 (m, 2H), 2.60-2.74 (m, 3H), 2.88-3.01 (m, 1H), 4.03 (s, 2H), 4.53-4.64 (m, 1H), 5.12 (br. s, 1H), 7.12 (dd, 1H), 7.37 (d, 2H), 7.85 (d, 2H), 8.17 (s, 1H), 8.57 (dd, 1H), 9.03 (dd, 1H).

### Beispiel 132

### tert.-Butyl-3-{[2-(4-cyclopropylphenyl)imidazo[1,2-a]pyrimidin-3-yl]methyl}-3,8-diazabicyclo-[3.2.1]octan-8-carboxylat

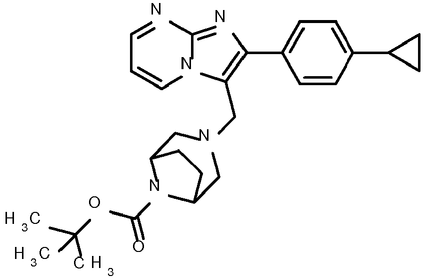

Unter Argon wurden bei Raumtemperatur 1090 mg (2.19 mmol) *tert*.-Butyl-3-{[2-(4-bromphenyl)-imidazo[1,2-a]pyrimidin-3-yl]methyl}-3,8-diazabicyclo[3.2.1]octan-8-carboxylat in 15 ml Toluol und 3 ml Wasser in einem 30 ml-Mikrowellengefäß vorgelegt und anschließend mit Cyclopropylboronsäure (376 mg, 4.37 mmol), Kaliumphosphat (1625 mg, 7.65 mmol), Palladium(II)acetat (49 mg, 0.22 mmol) und Tricyclohexylphosphin (123 mg, 0.44 mmol) versetzt. Das Mikrowellengefäß wurde danach verschlossen, und das Gemisch wurde auf 120°C erhitzt und über Nacht bei dieser Temperatur gerührt. Nach Abkühlen auf Raumtemperatur wurde das Reaktionsgemisch über Kieselgur filtriert und der Rückstand portionsweise mit Essigsäureethylester nachgewaschen. Zu dem erhaltenen Filtrat wurde eine weitere Menge Essigsäureethylester sowie Wasser hinzugegeben und die Phasen getrennt. Die organische Phase wurde mit gesättigter Natriumchlorid-Lösung gewaschen, über Magnesiumsulfat getrocknet und bis zur Trockene eingeengt. Der Rückstand wurde danach mit Diethylether verrührt. Nach Filtration wurde der erhaltene Feststoff über Nacht im Hochvakuum getrocknet. Es wurden 667 mg (1.36 mmol, 62% d. Th.) der Zielverbindung erhalten.

LC-MS (Methode 2): Rₜ = 2.00 min; m/z = 460 (M+H)⁺.

¹H-NMR (400 MHz, DMSO-d₆): δ [ppm] = 0.69-0.77 (m, 2H), 0.95-1.03 (m, 2H), 1.39 (s, 9H), 1.65 (br. s, 4H), 1.93-2.03 (m, 1H), 2.24 (br. d, 2H), 2.45-2.61 (m, 2H, teilweise verdeckt durch DMSO-Signal), 3.97 (s, 2H), 4.02 (br. s, 2H), 7.11 (dd, 1H), 7.20 (d, 2H), 7.78 (d, 2H), 8.55 (dd, 1H), 8.99 (dd, 1H).

Analog zu den Beispielen 13-29 wurden die folgenden Verbindungen aus den jeweils angegebenen Edukten hergestellt:

| **Beispiel** | **Name / Struktur / Edukte** | **Analytische Daten** |
|---|---|---|
| **133** | (3-{[2-(4-Cyclopropylphenyl)imidazo[1,2-a]-pyrimidin-3-yl]methyl}-3,8-diazabicyclo[3.2.1]-octan-8-yl)(2-fluorphenyl)methanon | ¹H-NMR (400 MHz, DMSO-d₆): δ [ppm] = 0.70-0.77 (m, 2H), 0.95-1.02 (m, 2H), 1.65-1.79 (m, 4H), 1.93-2.02 (m, 1H), 2.24 (br. d, 1H), 2.41 (br. d, 1H), 2.56 (dd, 1H), 2.68 (dd, 1H), 3.66 (br. s, 1H), 4.03 (s, 2H), 4.59 (br. s, 1H), 7.12 (dd, 1H), 7.20 (d, 2H), 7.25-7.32 (br. s, 1H), 7.41-7.53 (m, 2H), 7.78 (d, 2H), 8.55 (dd, 1H), 9.00 (dd, 1H). |
| | 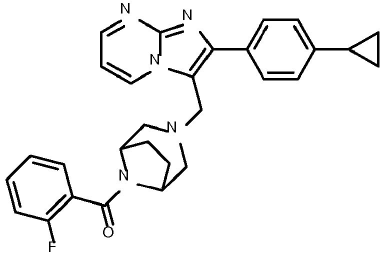 | |
| | aus 2-(4-Cyclopropylphenyl)-3-(3,8-diaza-bicyclo[3.2.1]octan-3-ylmethyl)imidazo[1,2-a]-pyrimidin-Dihydrochlorid und 2-Fluorbenzoesäure | LC-MS (Methode 1): |
| | | Rₜ = 0.87 min; m/z = 482 (M+H)⁺. |
| **134** | Cyclopentyl(3-{[2-(4-cyclopropylphenyl)-imidazo[1,2-a]pyrimidin-3-yl]methyl}-3,8-diazabicyclo[3.2.1]octan-8-yl)methanon | ¹H-NMR (400 MHz, DMSO-d₆): δ [ppm] = 0.68-0.79 (m, 2H), 0.94-1.03 (m, 2H), 1.44-1.68 (m, 7H), 1.68-1.78 (m, 5H), 1.97 (tt, 1H), 2.18-2.30 (m, 2H), 2.53-2.65 (m, 2H), 2.80-2.90 (m, 1H), 3.94-4.03 (m, 2H), 4.28 (br. s, 1H), 4.41 (br. d, 1H), 7.11 (dd, 1H), 7.20 (d, 2H), 7.79 (d, 2H), 8.56 (dd, 1H), 9.01 (dd, 1H). |
| | 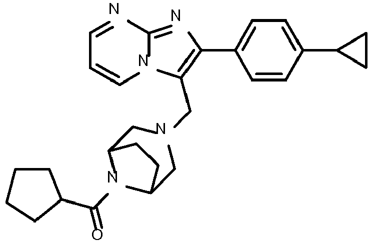 | |
| | aus 2-(4-Cyclopropylphenyl)-3-(3,8-diaza-bicyclo[3.2.1]octan-3-ylmethyl)imidazo[1,2-a]-pyrimidin-Dihydrochlorid und Cyclopentancarbonsäure | LC-MS (Methode 1): |
| | | Rₜ = 0.90 min; m/z = 456 (M+H)⁺. |
| **135** | (3-{[2-(4-Cyclopropylphenyl)imidazo[1,2-a]-pyrimidin-3-yl]methyl}-3,8-diazabicyclo[3.2.1]-octan-8-yl)(3-fluor-6-methoxypyridin-2-yl)-methanon | ¹H-NMR (400 MHz, DMSO-d₆): δ [ppm] = 0.69-0.78 (m, 2H), 0.95-1.04 (m, 2H), 1.63-1.84 (m, 4H), 1.93-2.02 (m, 1H), 2.43 (br. t, 2H), 2.52-2.57 (m, 1H), 2.76 (dd, 1H), 3.75 (s, 3H), 3.91 (br. s, 1H), 3.99-4.09 (m, 2H), 4.61 (br. s, 1H), 6.95 (dd, 1H), 7.12 (dd, 1H), 7.20 (d, 2H), 7.73-7.81 (m, 3H), 8.56 (dd, 1H), 9.01 (dd, 1H). |
| | 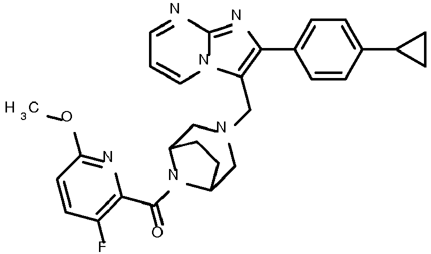 | |
| | aus 2-(4-Cyclopropylphenyl)-3-(3,8-diaza-bicyclo[3.2.1]octan-3-ylmethyl)imidazo[1,2-a]-pyrimidin-Dihydrochlorid und 3-Fluor-6-methoxypyridin-2-carbonsäure | LC-MS (Methode 1): |
| | | Rₜ = 0.86 min; m/z = 513 (M+H)⁺. |
| **136** | (3-Chlor-6-methoxypyridin-2-yl)(3-{[2-(4-cyclo-propylphenyl)imidazo[1,2-a]pyrimidin-3-yl]-methyl}-3,8-diazabicyclo[3.2.1]octan-8-yl)-methanon | ¹H-NMR (400 MHz, DMSO-d₆): δ [ppm] = 0.68-0.78 (m, 2H), 0.94-1.03 (m, 2H), 1.62-1.83 (m, 4H), 1.93-2.02 (m, 1H), 2.33-2.44 (m, 2H), 2.45-2.56 (m, 1H, teilweise verdeckt durch DMSO-Signal), 2.75 (dd, 1H), 3.62 (br. s, 1H), 3.78 (s, 3H), 3.99-4.10 (m, 2H), 4.60 (br. s, 1H), 6.92 (d, 1H), 7.12 (dd, 1H), 7.19 (d, 2H), 7.77 (d, 2H), 7.87 (d, 1H), 8.56 (dd, 1H), 9.00 (dd, 1H). |
| | 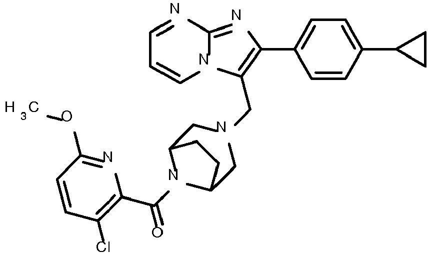 | |
| | aus 2-(4-Cyclopropylphenyl)-3-(3,8-diaza-bicyclo[3.2.1]octan-3-ylmethyl)imidazo[1,2-a]-pyrimidin-Dihydrochlorid und 3-Chlor-6-methoxypyridin-2-carbonsäure | LC-MS (Methode 1): |
| | | Rₜ = 0.90 min; m/z = 529/531 (M+H)⁺. |
| **137** | (3-{[2-(4-Cyclopropylphenyl)imidazo[1,2-a]-pyrimidin-3-yl]methyl}-3,8-diazabicyclo[3.2.1]-octan-8-yl)(6-methoxypyridin-2-yl)methanon | ¹H-NMR (400 MHz, DMSO-d₆): δ [ppm] = 0.65-0.79 (m, 2H), 0.94-1.04 (m, 2H), 1.65-1.82 (m, 4H), 1.93-2.01 (m, 1H), 2.43 (br. d, 1H), 2.55-2.64 (m, 2H), 2.73 (dd, 1H), 3.77 (s, 3H), 3.98-4.09 (m, 2H), 4.63 (br. s, 1H), 4.69 (br. s, 1H), 6.92 (dd, 1H), 7.12 (dd, 1H), 7.20 (d, 2H), 7.35 (dd, 1H), 7.75-7.85 (m, 3H), 8.56 (dd, 1H), 9.02 (dd, 1H). |
| | 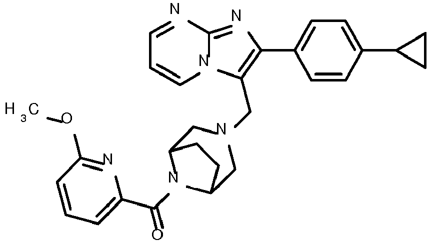 | |
| | aus 2-(4-Cyclopropylphenyl)-3-(3,8-diaza-bicyclo[3.2.1]octan-3-ylmethyl)imidazo[1,2-a]-pyrimidin-Dihydrochlorid und 6-Methoxy-pyridin-2-carbonsäure | LC-MS (Methode 1): |
| | | Rₜ = 0.87 min; m/z = 495 (M+H)⁺. |
| **138** | (3-{[2-(4-Cyclopropylphenyl)imidazo[1,2-a]-pyrimidin-3-yl]methyl}-3,8-diazabicyclo[3.2.1]-octan-8-yl)[6-(difluormethoxy)pyridin-2-yl]-methanon | ¹H-NMR (400 MHz, DMSO-d₆): δ [ppm] = 0.68-0.76 (m, 2H), 0.94-1.02 (m, 2H), 1.67-1.83 (m, 4H), 1.92-2.01 (m, 1H), 2.42 (br. d, 1H), 2.53-2.59 (m, 1H), 2.60-2.66 (m, 1H), 2.72 (dd, 1H), 4.04 (s, 2H), 4.57 (br. s, 1H), 4.64 (br. s, 1H), 7.11 (dd, 1H), 7.16-7.24 (m, 3H), 7.44 (s, 0.25H), 7.56-7.64 (m, 1.5H), 7.73 (s, 0.25H), 7.79 (d, 2H), 8.05 (t, 1H), 8.57 (dd, 1H), 9.03 (dd, 1H). |
| | 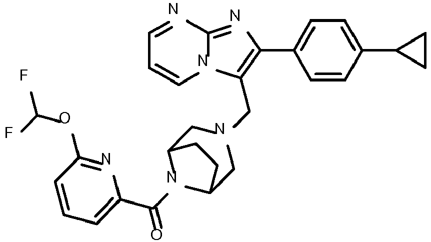 | |
| | aus 2-(4-Cyclopropylphenyl)-3-(3,8-diaza-bicyclo[3.2.1]octan-3-ylmethyl)imidazo[1,2-a]-pyrimidin-Dihydrochlorid und 6-(Difluor-methoxy)pyridin-2-carbonsäure | LC-MS (Methode 1): |
| | | Rₜ = 0.91 min; m/z = 531 (M+H)⁺. |
| **139** | (5-Cyclopropyl-1,3-oxazol-4-yl)(3-{[2-(4-cyclo-propylphenyl)imidazo[1,2-a]pyrimidin-3-yl]-methyl}-3,8-diazabicyclo[3.2.1]octan-8-yl)-methanon | ¹H-NMR (400 MHz, DMSO-d₆): δ [ppm] = 0.69-0.77 (m, 2H), 0.87-1.01 (m, 4H), 1.02-1.12 (m, 2H), 1.61-1.85 (m, 4H), 1.92-2.01 (m, 1H), 2.39 (br. t, 2H), 2.60-2.74 (m, 3H), 4.01 (s, 2H), 4.59 (br. d, 1H), 5.11 (br. s, 1H), 7.12 (dd, 1H), 7.20 (d, 2H), 7.80 (d, 2H), 8.17 (s, 1H), 8.56 (dd, 1H), 9.02 (dd, 1H). |
| | 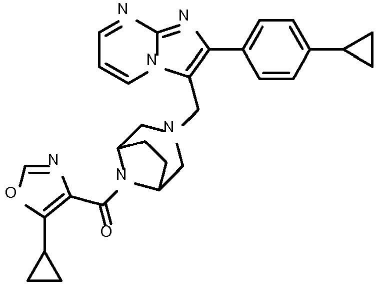 | |
| | | LC-MS (Methode 1): |
| | aus 2-(4-Cyclopropylphenyl)-3-(3,8-diaza-bicyclo[3.2.1]octan-3-ylmethyl)imidazo[1,2-a]-pyrimidin-Dihydrochlorid und 5-Cyclopropyl-1,3-oxazol-4-carbonsäure | Rₜ = 0.87 min; m/z = 495 (M+H)⁺. |

### B. Bewertung der pharmakologischen Wirksamkeit

Die pharmakologische Aktivität der erfindungsgemäßen Verbindungen kann durch *in vitro-* und *in vivo*-Untersuchungen, wie sie dem Fachmann bekannt sind, nachgewiesen werden. Die nachfolgenden Anwendungsbeispiele beschreiben die biologische Wirkung der erfindungsgemäßen Verbindungen, ohne die Erfindung auf diese Beispiele zu beschränken.

### B-1. In vitro-elektrophysiologische Analyse der humanen TASK-1- und TASK-3-Kanäle via Zwei-Elektroden-Voltage-Clamp-Technik in Xenopus laevis-Oozyten

*Xenopus laevis*-Oozyten wurden selektioniert wie andernorts beispielhaft beschrieben [Decher et al., FEBS Lett. 492, 84-89 (2001)]. Im Nachgang wurden die Oozyten mit 0.5-5 ng cRNA-Lösung, die TASK-1 oder TASK-3 codiert, injiziert. Zur elektrophysiologischen Analyse der in den Oozyten exprimierten Kanalproteine wurde die Zwei-Elektroden-Voltage-Clamp-Technik verwendet [Stühmer, Methods Enzymol. 207, 319-339 (1992)]. Die Messungen wurden wie beschrieben [Decher et al., FEBS Lett. 492, 84-89 (2001)] bei Raumtemperatur (21-22°C) mit einem Turbo-TEC-10CD-Verstärker (NPI) durchgeführt, mit 2 kHz aufgenommen und mit 0.4 kHz gefiltert. Die Substanzapplikation erfolgte durch ein gravitationsgetriebenes Perfusionssystem. Die Oozyte befindet sich hierbei in einer Messkammer und wird dem Lösungsstrom von 10 ml/min ausgesetzt. Der Pegel der Messkammer wird durch Absaugen der Lösung mit einer peristaltischen Pumpe kontrolliert und reguliert.

In der folgenden Tabelle 1 ist die in diesem Test bestimmte halbmaximale Inhibition der humanen TASK-1- und TASK-3-Kanäle (ICso-Wert) durch repräsentative Ausführungsbeispiele der Erfindung aufgeführt:

**Tabelle 1**

| **Beispiel Nr.** | **TASK-1 IC₅₀ [nM]** | **TASK-3 IC₅₀ [nM]** |
|---|---|---|
| 19 | 239.4 ± 2.7 | 774.2 ± 67.1 |
| 21 | 19.2 ± 4.3 | 32.9 ± 6.0 |
| 26 | 31.2 ± 5.8 | 140.0 ± 34.6 |
| 27 | 17.9 ± 2.2 | 367.1 ± 67.6 |
| 28 | 20.5 ± 2.7 | 6.6 ± 0.8 |
| 29 | 21.0 ± 4.1 | 42.7 ± 8.4 |
| 41 | 44.4 ± 4.4 | 71.8 ± 15.5 |
| 51 | 21.7 ± 4.6 | 35.9 ± 8.2 |

Aus den Daten in Tabelle 1 ist ersichtlich, dass eine Blockade sowohl an TASK-1 als auch an TASK-3 erreicht wird. Die Ergebnisse in Tabelle 1 belegen somit den Wirkmechanismus der erfindungsgemäßen Verbindungen als duale TASK-1/3-Inhibitoren.

### B-2. Inhibition des rekombinanten TASK-1 und TASK-3 in vitro

Die Untersuchungen zur Inhibition der rekombinanten TASK-1- und TASK-3-Kanäle wurden an stabil transfizierten CHO-Zellen durchgeführt. Die erfindungsgemäßen Verbindungen wurden hierbei unter Applikation von 40 mM Kaliumchlorid in Anwesenheit eines spannungssensitiven Farbstoffes nach der in folgenden Referenzen im Detail beschriebenen Methode getestet [Whiteaker et al., Validation of FLIPR membrane potential dye for high-throughput screening of potassium channel modulators, J. Biomol. Screen. 6 (5), 305-312 (2001); Molecular Devices FLIPR Application Note: Measuring membrane potential using the FLIPR^{®} membrane potential assay kit on Fluorometric Imaging Plate Reader (FLIPR^{®}) systems, http://www.moleculardevices.com/reagentssupplies/assay-kits/ion-channels/flipr-membrane-potential-assay-kits]. Die Aktivität der Testsubstanzen wurde bestimmt als ihre Fähigkeit, eine in den rekombinanten Zellen durch 40 mM Kaliumchlorid induzierte Depolarisation zu inhibieren. Die Konzentration, die diese Depolarisation zur Hälfte blockieren kann, wird als IC₅₀ bezeichnet.

In der folgenden Tabelle 2 sind die zu individuellen Ausführungsbeispielen der Erfindung ermittelten ICso-Werte aus diesem Assay aufgeführt (zum Teil als Mittelwerte aus mehreren unabhängigen Einzelbestimmungen):

**Tabelle 2**

| **Beispiel Nr.** | **TASK-1 IC₅₀ [nM]** | **TASK-3 IC₅₀ [nM]** |
|---|---|---|
| 1 | 1700 | 400 |
| 4 | 470 | 97 |
| 5 | 17000 | 1300 |
| 6 | 1400 | 41 |
| 7 | 8600 | 570 |
| 10 | 2200 | 130 |
| 11 | 2500 | 16 |
| 12 | 220 | 13 |
| 13 | 1500 | 33 |
| 14 | 7600 | 170 |
| 15 | 1100 | 19 |
| 16 | 670 | 12 |
| 17 | 1200 | 33 |
| 18 | 910 | 8.6 |
| 19 | 22000 | 59 |
| 20 | 160 | 38 |
| 21 | 140 | 4.2 |
| 22 | 340 | 5.2 |
| 23 | 1600 | 100 |
| 24 | 410 | 102 |
| 25 | 1100 | 71 |
| 26 | 1400 | 16 |
| 27 | 1200 | 10 |
| 28 | 290 | 3.2 |
| 29 | 280 | 1.8 |
| 30 | 3500 | 85 |
| 31 | 7100 | 140 |
| 32 | 370 | 29 |
| 33 | 190 | 130 |
| 34 | 76 | 29 |
| 35 | 6700 | 1500 |
| 36 | 310 | 80 |
| 37 | 1500 | 140 |
| 38 | 9600 | 320 |
| 39 | 1400 | 160 |
| 40 | 5700 | 210 |
| 41 | 1500 | 100 |
| 42 | 1000 | 340 |
| 43 | 1000 | 320 |
| 44 | 1000 | 190 |
| 45 | 1800 | 120 |
| 46 | 7600 | 140 |
| 47 | 2800 | 110 |
| 48 | 400 | 23 |
| 49 | 260 | 12 |
| 50 | 3300 | 430 |
| 51 | 250 | 8.7 |
| 52 | 1300 | 52 |
| 53 | 620 | 19 |
| 54 | 860 | 13 |
| 55 | 2900 | 170 |
| 56 | 5600 | 54 |
| 57 | 6400 | 57 |
| 58 | 1600 | 17 |
| 59 | 3000 | 39 |
| 60 | 670 | 430 |
| 61 | 3000 | 640 |
| 62 | 6900 | 70 |
| 63 | 1700 | 15 |
| 64 | 1100 | 8.4 |
| 65 | 3500 | 670 |
| 66 | 3700 | 20 |
| 67 | 1200 | 17 |
| 68 | 9400 | 87 |
| 69 | 2800 | 22 |
| 70 | 1900 | 20 |
| 71 | 14000 | 110 |
| 72 | 2100 | 29 |
| 73 | 9100 | 81 |
| 74 | 3400 | 61 |
| 75 | 3800 | 51 |
| 76 | 13000 | 56 |
| 77 | 720 | 4.9 |
| 78 | 3800 | 24 |
| 81 | 820 | 21 |
| 82 | 670 | 37 |
| 83 | 250 | 14 |
| 84 | 93 | 4.6 |
| 85 | 30000 | 1000 |
| 86 | 7700 | 430 |
| 89 | 30000 | 850 |
| 91 | 20000 | 410 |
| 92 | 15000 | 270 |
| 93 | 12000 | 260 |
| 94 | 30000 | 160 |
| 95 | 3000 | 41 |
| 97 | 31000 | 450 |
| 98 | 9000 | 160 |
| 99 | 30000 | 750 |
| 100 | 19000 | 630 |
| 101 | 19000 | 510 |
| 103 | 30000 | 690 |
| 104 | 19000 | 460 |
| 106 | 30000 | 89 |
| 107 | 30000 | 750 |
| 108 | 7000 | 90 |
| 109 | 24 | 1.5 |
| 110 | 13000 | 230 |
| 111 | 6300 | 290 |
| 112 | 15000 | 130 |
| 113 | 1100 | 62 |
| 114 | 6700 | 140 |
| 115 | 7400 | 330 |
| 116 | 2700 | 65 |
| 118 | 19000 | 220 |
| 119 | 5300 | 210 |
| 120 | 15000 | 230 |
| 121 | 12000 | 120 |
| 122 | 5000 | 120 |
| 123 | 14000 | 230 |
| 124 | 790 | 17 |
| 125 | 230 | 27 |
| 126 | 280 | 14 |
| 128 | 2000 | 110 |
| 130 | 1700 | 210 |
| 131 | 3500 | 410 |
| 132 | 1700 | 410 |
| 133 | 1600 | 180 |
| 134 | 770 | 97 |
| 135 | 420 | 34 |
| 136 | 320 | 41 |
| 137 | 1100 | 180 |
| 138 | 3200 | 290 |
| 139 | 4500 | 470 |

Aus den Daten in Tabelle 2 ist ersichtlich, dass eine Blockade sowohl an TASK-1 als auch insbesondere an TASK-3 erreicht wird. Die Ergebnisse in Tabelle 2 belegen somit den Wirkmechanismus der erfindungsgemäßen Verbindungen als duale TASK-1/3-Inhibitoren.

### B-3. Tiermodell der obstruktiven Schlafapnoe am Schwein

Durch den Einsatz von negativem Druck kann bei anästhesierten, spontan-atmenden Schweinen ein Kollaps und damit ein Verschluss der oberen Atemwege ausgelöst werden [Wirth et al., Sleep 36, 699-708 (2013)].

Für das Modell werden Deutsche Landschweine verwendet. Die Schweine werden anästhesiert und tracheotomiert. In den rostralen und kaudalen Teil der Trachea wird jeweils eine Kanüle eingebunden. Die rostrale Kanüle wird mittels eines T-Stückes einerseits mit einem Gerät verbunden, welches negative Drücke generiert, und andererseits mit der kaudalen Kanüle. Die kaudale Kanüle ist mittels eines T-Stückes mit der rostralen Kanüle verbunden sowie mit einem Schlauch, der unter Umgehung der oberen Atemwege eine spontane Atmung erlaubt. Durch entsprechendes Verschließen und Öffnen der Schläuche ist es so möglich, dass das Schwein von einer normalen Nasenatmung zu einer Atmung über die kaudale Kanüle wechseln kann, während die oberen Atemwege isoliert und mit dem Gerät zur Erzeugung der negativen Drücke verbunden sind. Die Muskelaktivität des *Musculus genioglossus* wird mittels Elektromyogramm (EMG) registriert.

Zu bestimmten Zeitpunkten wird die Kollapsibilität der oberen Atemwege getestet, indem das Schwein über die kaudale Kanüle atmet und an die oberen Atemwege Unterdrücke von -50, -100 und -150 cm Wassersäule (cmH₂O) angelegt werden. Hierdurch kollabieren die oberen Atemwege, was durch Unterbrechung des Luftstroms und einen Druckabfall im Schlauchsystem angezeigt wird. Dieser Test wird durchgeführt vor Gabe der Testsubstanz und in bestimmten Abständen nach Gabe die Testsubstanz. Eine entsprechend wirksame Testsubstanz kann diesen Kollaps der Atemwege in der inspiratorischen Phase verhindern.

Nach dem Wechsel von nasaler Atmung zur Atmung durch die kaudale Kanüle ist keine EMG-Aktivität des *Musculus genioglossus* beim anästhesierten Schwein messbar. Als ein weiterer Test wird dann der Unterdruck ermittelt, bei dem die EMG-Aktivität wieder einsetzt. Dieser Schwellenwert wird bei Wirksamkeit einer Testsubstanz zu positiveren Werten verschoben. Die Untersuchung wird ebenfalls vor Gabe der Testsubstanz und zu bestimmten Zeitpunkten nach Gabe der Testsubstanz durchgeführt. Die Gabe der Testsubstanz kann intranasal, intravenös, subkutan, intraperitoneal oder intragastral erfolgen.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung;

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

### Nasal applizierbare Lösung:

Die erfindungsgemäße Verbindung wird in einer Konzentration unterhalb der Sättigungslöslichkeit in einem physiologisch verträglichen Lösungsmittel (z.B. gereinigtes Wasser, Phosphatpuffer, Citratpuffer) gelöst. Die Lösung kann weitere Zusätze zur Isotonisierung, zur Konservierung, zur Einstellung des pH-Wertes, zur Verbesserung der Löslichkeit und/oder zur Stabilisierung enthalten.

## Patentansprüche

1. Verbindung der Formel (I) 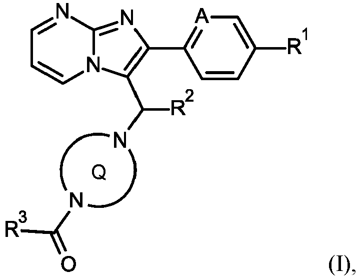 in welcher
der Ring Q für einen Diaza-Heterobicyclus der Formel 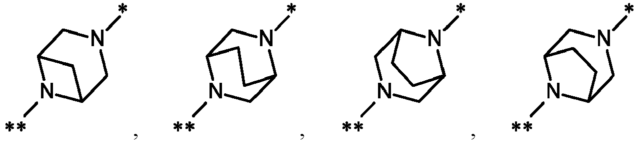 oder 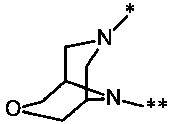 steht,
worin * die Verknüpfung zur angrenzenden CHR²-Gruppe und ** die Verknüpfung zur Carbonyl-Gruppe kennzeichnet,
A für CH oder N steht,
R¹ für Halogen, Cyano, (C₁-C₄)-Alkyl, Cyclopropyl oder Cyclobutyl steht, wobei (C₁-C₄)-Alkyl bis zu dreifach mit Fluor substituiert sein kann und Cyclopropyl und Cyclobutyl bis zu zweifach mit Fluor substituiert sein können,
R² für Wasserstoff oder Methyl steht,
und R³ für (C₄-C₆)-Cycloalkyl, worin eine Ring-CH₂-Gruppe gegen -O- ausgetauscht sein kann, steht
oder R³ für eine Phenyl-Gruppe der Formel (a), eine Pyridyl-Gruppe der Formel (b) oder (c) oder eine Azol-Gruppe der Formel (d), (e) oder (f) 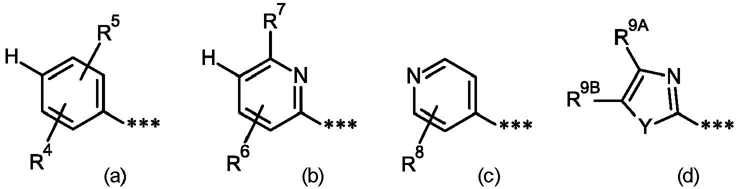 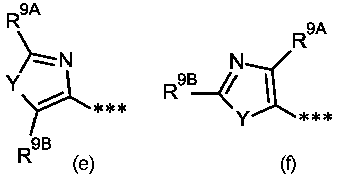 steht, worin *** die Verknüpfung zur angrenzenden Carbonyl-Gruppe markiert und
R⁴ Wasserstoff, Fluor, Chlor, Brom oder Methyl bedeutet,
R⁵ Wasserstoff, Fluor, Chlor, Brom, Cyano, (C₁-C₃)-Alkyl oder (C₁-C₃)-Alkoxy bedeutet, wobei (C₁-C₃)-Alkyl und (C₁-C₃)-Alkoxy jeweils bis zu dreifach mit Fluor substituiert sein können,
R⁶ Wasserstoff, Fluor, Chlor, Brom oder Methyl bedeutet,
R⁷ Wasserstoff, (C₁-C₃)-Alkoxy, Cyclobutyloxy, Oxetan-3-yloxy, Tetrahydrofuran-3-yloxy, Tetrahydro-2*H*-pyran-4-yloxy, Mono-(C₁-C₃)-alkylamino, Di-(C₁-C₃)-alkylamino oder (C₁-C₃)-Alkylsulfanyl bedeutet, wobei (C₁-C₃)-Alkoxy bis zu dreifach mit Fluor substituiert sein kann,
R⁸ Wasserstoff, Fluor, Chlor, Brom, (C₁-C₃)-Alkyl oder (C₁-C₃)-Alkoxy bedeutet,
R^{9A} und R^{9B} gleich oder verschieden sind und unabhängig voneinander Wasserstoff, Fluor, Chlor, Brom, (C₁-C₃)-Alkyl, Cyclopropyl oder (C₁-C₃)-Alkoxy bedeuten, wobei (C₁-C₃)-Alkyl und (C₁-C₃)-Alkoxy jeweils bis zu dreifach mit Fluor substituiert sein können,
und Y O oder S bedeutet, oder
R³ für eine Gruppe -OR¹⁰ oder -NR¹¹R¹² steht, worin
R¹⁰ (C₁-C₆)-Alkyl, (C₄-C₆)-Cycloalkyl oder [(C₃-C₆)-Cycloalkyl]methyl bedeutet,
R¹¹ Wasserstoff oder (C₁-C₃)-Alkyl bedeutet
und
R¹² (C₁-C₆)-Alkyl, (C₃-C₆)-Cycloalkyl, Phenyl oder Benzyl bedeutet, wobei (C₁-C₆)-Alkyl bis zu dreifach mit Fluor substituiert sein kann und wobei Phenyl sowie die Phenyl-Gruppe in Benzyl bis zu dreifach, gleich oder verschieden, mit einem Rest ausgewählt aus der Reihe Fluor, Chlor, Methyl, Ethyl, Trifluormethyl, Methoxy, Ethoxy, Trifluormethoxy und (Trifluormethyl)sulfanyl substituiert sein können,
oder
R¹¹ und R¹² miteinander verknüpft sind und gemeinsam mit dem Stickstoffatom, an das sie gebunden sind, einen Pyrrolidin-, Piperidin-, Morpholin- oder Thiomorpholin-Ring bilden,
sowie ihre Salze, Solvate und Solvate der Salze.

2. Verbindung der Formel (I) nach Anspruch 1, in welcher
der Ring Q für einen Diaza-Heterobicyclus der Formel 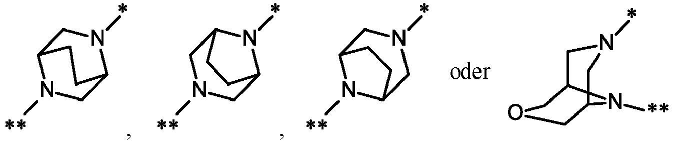 steht, worin * die Verknüpfung zur angrenzenden CHR²-Gruppe und ** die Verknüpfung zur Carbonyl-Gruppe kennzeichnet,
A für CH steht,
R¹ für Fluor, Chlor, Brom, Methyl, Isopropyl, *tert.-Butyl,* Cyclopropyl oder Cyclobutyl steht,
R² für Wasserstoff steht,
und
R³ für Cyclobutyl, Cyclopentyl oder Cyclohexyl steht
oder R³ für eine Phenyl-Gruppe der Formel (a), eine Pyridyl-Gruppe der Formel (b) oder eine Azol-Gruppe der Formel (d), (e) oder (f) 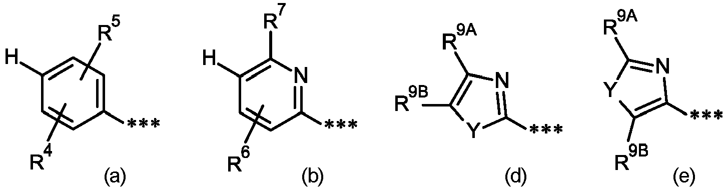 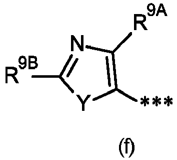 steht, worin *** die Verknüpfung zur angrenzenden Carbonyl-Gruppe markiert und
R⁴ Wasserstoff, Fluor oder Chlor bedeutet,
R⁵ Fluor, Chlor, Cyano, (C₁-C₃)-Alkyl, (C₁-C₃)-Alkoxy oder Trifluormethoxy bedeutet,
R⁶ Wasserstoff, Fluor, Chlor, Brom oder Methyl bedeutet,
R⁷ (C₁-C₃)-Alkoxy, Cyclobutyloxy oder (C₁-C₃)-Alkylsulfanyl bedeutet, wobei (C₁-C₃)-Alkoxy bis zu dreifach mit Fluor substituiert sein kann,
R^{9A} und R^{9B} gleich oder verschieden sind und unabhängig voneinander Wasserstoff, Chlor, Brom, (C₁-C₃)-Alkyl oder Cyclopropyl bedeuten, wobei (C₁-C₃)-Alkyl bis zu dreifach mit Fluor substituiert sein kann, und
Y O oder S bedeutet,
sowie ihre Salze, Solvate und Solvate der Salze.

3. Verbindung der Formel (I) nach Anspruch 1 oder 2, in welcher
der Ring Q für einen Diaza-Heterobicyclus der Formel 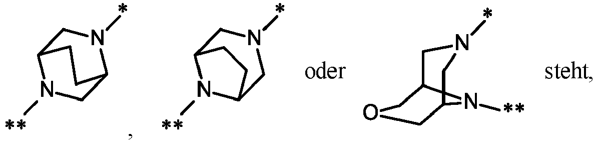 worin * die Verknüpfung zur angrenzenden CHR²-Gruppe und ** die Verknüpfung zur Carbonyl-Gruppe kennzeichnet,
A für CH steht,
R¹ für Chlor, Brom, Isopropyl oder Cyclopropyl steht,
R² für Wasserstoff steht, und
R³ für Cyclopentyl oder Cyclohexyl steht oder
R³ für eine Phenyl-Gruppe der Formel (a), eine Pyridyl-Gruppe der Formel (b) oder eine Azol-Gruppe der Formel (d), (e) oder (f) 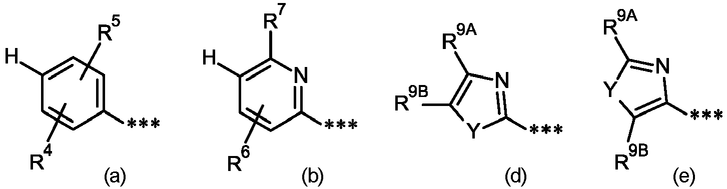 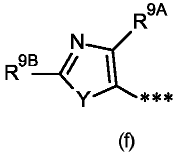 steht, worin *** die Verknüpfung zur angrenzenden Carbonyl-Gruppe markiert und
R⁴ Wasserstoff, Fluor oder Chlor bedeutet,
R⁵ Fluor, Chlor, Methyl, Isopropyl, Methoxy oder Ethoxy bedeutet,
R⁶ Wasserstoff, Fluor, Chlor, Brom oder Methyl bedeutet,
R⁷ Methoxy, Difluormethoxy, Trifluormethoxy, Isopropoxy, Cyclobutyloxy oder Methylsulfanyl bedeutet,
R^{9A} und R^{9B} gleich oder verschieden sind und unabhängig voneinander Wasserstoff, Methyl, Trifluormethyl, Ethyl, Isopropyl oder Cyclopropyl bedeuten
und
Y O oder S bedeutet,
sowie ihre Salze, Solvate und Solvate der Salze.

4. Verbindung der Formel (I) gemäß Anspruch 1, mit dem systematischen Namen (5 {[2-(4-Chlorphenyl)imidazo[1,2-a]pyrimidin-3-yl]methyl}-2,5-diazabicyclo[2.2.2]-oct-2-yl)(3-fluor-6-methoxypyridin-2-yl)methanon (Enantiomer 2) und der Strukturformel

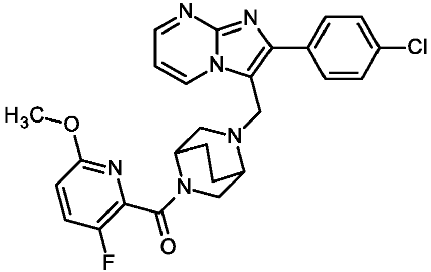

5. Verbindung der Formel (I) gemäß Anspruch 1, mit dem systematischen Namen (3-Fluor-6-methoxypyridin-2-yl)(3-{[2-(4-isopropylphenyl)imidazo[1,2-a]pyrimidin-3-yl]methyl}-3,8-diazabicyclo[3.2.1]oct-8-yl)methanon und der Strukturformel

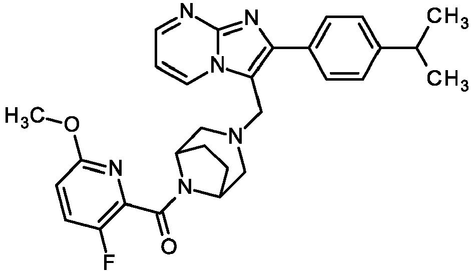

6. Verbindung der Formel (I) gemäß Anspruch 1, mit dem systematischen Namen ((3-Chlor-6-methoxypyridin-2-yl)(3-{[2-(4-isopropylphenyl)imidazo[1,2-a]pyrimidin-3-yl]methyl}-3,8-diazabicyclo[3.2.1]oct-8-yl)methanon und der Strukturformel

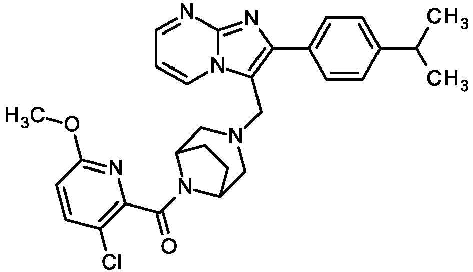

7. Verbindung der Formel (1) gemäß Anspruch 1, mit dem systematischen Namen (3-Chlor-6-methoxypyridin-2-yl)(5-{[2-(4-isopropylphenyl)imidazo[1,2-a]pyrimidin-3-yl]methyl}-2,5-diazabicyclo[2.2.2]oct-2-yl)methanon (Enantiomer 2) und der Strukturformel

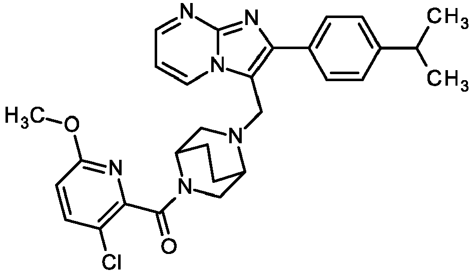

8. Verbindung der Formel (1) gemäß Anspruch 1, mit dem systematischen Namen (3-Chlor-6-methoxypyridin-2-yl)(3-{[2-(4-chlorophenyl)imidazo[1,2-a]pyrimidin-3-yl]methyl}-3,8-diazabicyclo[3.2.1]oct-8-yl)methanon und der Strukturformel

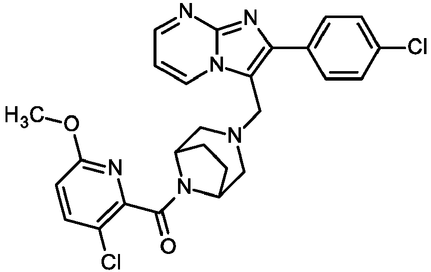

9. Verfahren zur Herstellung einer Verbindung der Formel (1), wie in den Ansprüchen 1 bis 3 definiert, in welcher der Rest R² für Wasserstoff steht, **dadurch gekennzeichnet, dass** man eine Verbindung der Formel (II) 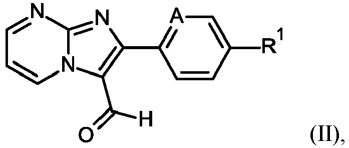 in welcher A und R¹ die in den Ansprüchen 1 bis 3 angegebenen Bedeutungen haben, in Gegenwart eines geeigneten Reduktionsmittels entweder
[A] mit einer Verbindung der Formel (III) 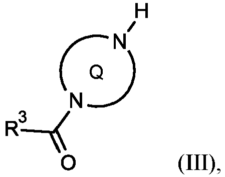 in welcher R³ und der Ring Q die in den Ansprüchen 1 bis 3 angegebenen Bedeutungen haben,
zu einer Verbindung der Formel (I-A) 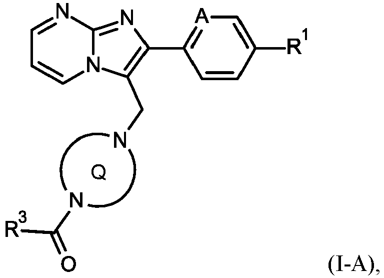 in welcher A, R¹, R³ und der Ring Q die oben angegebenen Bedeutungen haben, umsetzt
oder
[B] mit einem geschützten Diaza-Heterobicyclus der Formel (IV) 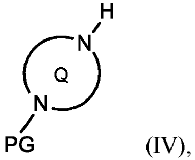 in welcher der Ring Q die in den Ansprüchen 1 bis 3 angegebene Bedeutung hat und
PG für eine geeignete Amino-Schutzgruppe, wie beispielsweise *tert*.-Butoxycarbonyl, Benzyloxycarbonyl oder (9*H*-Fluoren-9-ylmethoxy)carbonyl, steht,
zunächst zu einer Verbindung der Formel (V) 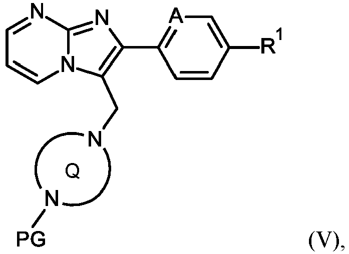 in welcher A, PG, R¹ und der Ring Q die oben angegebenen Bedeutungen haben, umsetzt, anschließend die Schutzgruppe PG abspaltet und die resultierende Verbindung der Formel (VI) 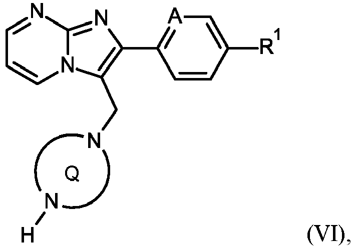 in welcher A, R¹ und der Ring Q die oben angegebenen Bedeutungen haben, dann in Abhängigkeit von der spezifischen Bedeutung des Restes R³
[B-1] mit einer Carbonsäure der Formel (VII) 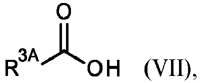 in welcher
R^{3A} für (C₄-C₆)-Cycloalkyl, worin eine Ring-CH₂-Gruppe gegen -O- ausgetauscht sein kann, oder für eine Phenyl-Gruppe der Formel (a), eine Pyridyl-Gruppe der Formel (b) oder (c) oder eine Azol-Gruppe der Formel (d), (e) oder (f), wie in den Ansprüchen 1 bis 3 beschrieben, steht,
unter Aktivierung der Carbonsäure-Funktion in (VII) oder mit dem korrespondierenden Säurechlorid der Formel (VIII) 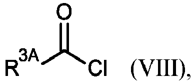 in welcher R^{3A} die oben angegebene Bedeutung hat,
zu einer Verbindung der Formel (I-B) 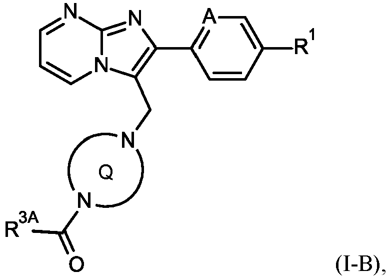 in welcher A, R¹, R^{3A} und der Ring Q die oben angegebenen Bedeutungen haben, umsetzt
oder
[B-2] mit einem Chlorformiat beziehungsweise Carbamoylchlorid der Formel (IX) 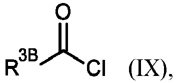 in welcher
R^{3B} für die Gruppe -OR¹⁰ oder -NR^{11A}R¹² steht, worin
R¹⁰ und R¹² die in den Ansprüchen 1 bis 3 angegebenen Bedeutungen haben und
R^{11A} die in den Ansprüchen 1 bis 3 angegebene Bedeutung von R¹¹ hat, jedoch ungleich Wasserstoff ist,
zu einer Verbindung der Formel (I-C) 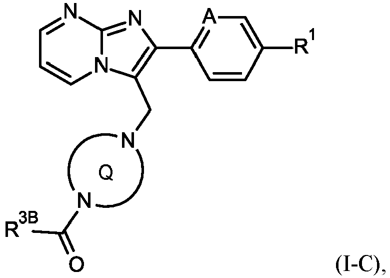 in welcher A, R¹, R^{3B} und der Ring Q die oben angegebenen Bedeutungen haben, umsetzt
oder
[B-3] mit einem Isocyanat der Formel (X)
R¹²-N=C=O (X),
in welcher R¹² die in den Ansprüchen 1 bis 3 angegebene Bedeutung hat,
zu einer Verbindung der Formel (I-D) 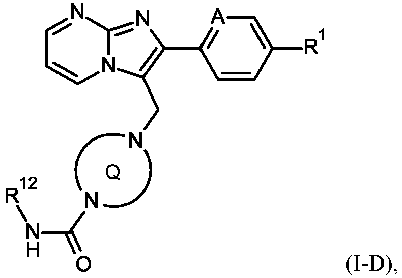
in welcher A, R¹, R¹² und der Ring Q die oben angegebenen Bedeutungen haben,
umsetzt
und die so erhaltenen Verbindungen der Formeln (I-A), (I-B), (I-C) beziehungsweise (I-D) gegebenenfalls in ihre Enantiomere und/oder Diastereomere trennt und/oder gegebenenfalls mit den entsprechenden (*i*) Lösungsmitteln und/oder (*ii*) Säuren in ihre Solvate, Salze und/oder Solvate der Salze überführt.

10. Verbindung, wie in einem der Ansprüche 1 bis 8 definiert, zur Behandlung und/oder Prävention von Krankheiten.

11. Verbindung, wie in einem der Ansprüche 1 bis 8 definiert, zur Verwendung in einem Verfahren zur Behandlung und/oder Prävention von Atemstörungen, schlafbedingten Atemstörungen, obstruktiven Schlafapnoen, zentralen Schlafapnoen, Schnarchen, Herzrhythmusstörungen, Arrhythmien, neurodegenerativen Erkrankungen, neuroinflammatorischen Erkrankungen und neuroimmunologischen Erkrankungen.

12. Verwendung einer Verbindung, wie in einem der Ansprüche 1 bis 8 definiert, zur Herstellung eines Arzneimittels zur Behandlung und/oder Prävention von Atemstörungen, schlafbedingten Atemstörungen, obstruktiven Schlafapnoen, zentralen Schlafapnoen, Schnarchen, Herzrhythmusstörungen, Arrhythmien, neurodegenerativen Erkrankungen, neuroinflammatorischen Erkrankungen und neuroimmunologischen Erkrankungen.

13. Arzneimittel enthaltend eine Verbindung, wie in einem der Ansprüche 1 bis 8 definiert, in Kombination mit einem oder mehreren inerten, nicht-toxischen, pharmazeutisch geeigneten Hilfsstoffen.

14. Arzneimittel enthaltend eine Verbindung, wie in einem der Ansprüche 1 bis 8 definiert, in Kombination mit einem oder mehreren weiteren Wirkstoffen ausgewählt aus der Gruppe bestehend aus Atemstimulantien, psychostimulierenden Verbindungen, Serotonin-Wiederaufnahmehemmern, noradrenergen, serotonergen und tricyclischen Antidepressiva, sGC-Stimulatoren, Mineralocorticoid-Rezeptor-Antagonisten, entzündungshemmend wirkenden Mitteln, immunmodulierend wirkenden Mitteln, immunsuppressiv wirkenden Mitteln und zytotoxisch wirkenden Mitteln.

15. Arzneimittel nach Anspruch 13 oder 14 zur Behandlung und/oder Prävention von Atemstörungen, schlafbedingten Atemstörungen, obstruktiven Schlafapnoen, zentralen Schlafapnoen, Schnarchen, Herzrhythmusstörungen, Arrhythmien, neurodegenerativen Erkrankungen, neuroinflammatorischen Erkrankungen und neuroimmunologischen Erkrankungen.

## Claims

1. Compound of the formula (I) 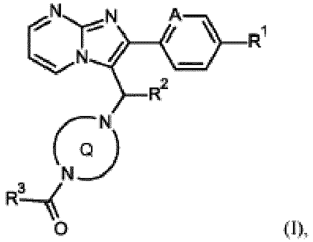 in which
the ring Q represents a diazaheterobicyclic system of the formula 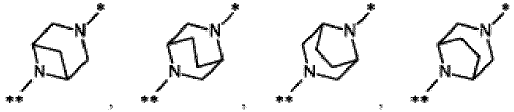 or 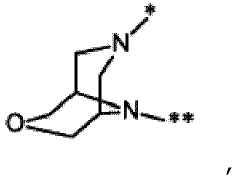
in which * denotes the bond to the adjacent CHR² group and ** the bond to the carbonyl group,
A represents CH or N,
R¹ represents halogen, cyano, (C₁-C₄)-alkyl, cyclopropyl or cyclobutyl,
where (C₁-C₄)-alkyl may be up to trisubstituted by fluorine and cyclopropyl and cyclobutyl may be up to disubstituted by fluorine,
R² represents hydrogen or methyl,
and
R³ represents (C₄-C₆) -cycloalkyl in which a ring CH₂ group may be replaced by -O-,
or
R³ represents a phenyl group of the formula (a), a pyridyl group of the formula (b) or (c) or an azole group of the formula (d), (e) or (f) 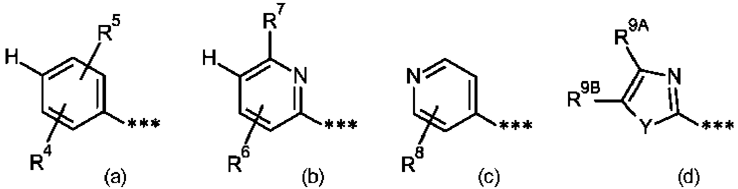 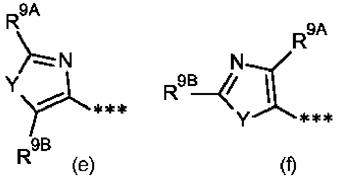
in which *** marks the bond to the adjacent carbonyl group and
R⁴ represents hydrogen, fluorine, chlorine, bromine or methyl,
R⁵ represents hydrogen, fluorine, chlorine, bromine, cyano, (C₁-C₃) -alkyl or (C₁-C₃)-alkoxy,
where (C₁-C₃)-alkyl and (C₁-C₃)-alkoxy may each be up to trisubstituted by fluorine,
R⁶ represents hydrogen, fluorine, chlorine, bromine or methyl,
R⁷ represents hydrogen, (C₁-C₃)-alkoxy, cyclobutyloxy, oxetan-3-yloxy, tetrahydrofuran-3-yloxy, tetrahydro-2H-pyran-4-yloxy, mono-(C₁-C₃)-alkylamino, di-(C₁-C₃)- alkylamino or (C₁-C₃)-alkylsulfanyl,
where (C₁-C₃)-alkoxy may be up to trisubstituted by fluorine,
R⁸ represents hydrogen, fluorine, chlorine, bromine, (C₁-C₃)-alkyl or (C₁-C₃)-alkoxy,
R^{9A} and R^{9B} are identical or different and independently of one another represent hydrogen, fluorine, chlorine, bromine, (C₁-C₃)-alkyl, cyclopropyl or (C₁-C₃)-alkoxy, where (C₁-C₃)-alkyl and (C₁-C₃)-alkoxy may each be up to trisubstituted by fluorine,
and
Y represents O or S,
or
R³ represents an -OR¹⁰ or -NR¹¹R¹² group in which R¹⁰ represents (C₁-C₆)-alkyl, (C₄-C₆)-cycloalkyl or [(C3-C6)-cycloalkyl]methyl,
R¹¹ represents hydrogen or (C₁-C₃)-alkyl
and
R¹² represents (C₁-C₆)-alkyl, (C₃-C₆)-cycloalkyl, phenyl or benzyl,
where (C₁-C₆)-alkyl may be up to trisubstituted by fluorine,
and
where phenyl and the phenyl group in benzyl may be up to trisubstituted by identical or different radicals selected from the group consisting of fluorine, chlorine, methyl, ethyl, trifluoromethyl, methoxy, ethoxy, trifluoromethoxy and (trifluoromethyl)sulfanyl,
or
R¹¹ and R¹² are attached to one another and, together with the nitrogen atom to which they are bonded, form a pyrrolidine, piperidine, morpholine or thiomorpholine ring,
and the salts, solvates and solvates of the salts thereof.

2. Compound of the formula (I) according to Claim 1, in which
the ring Q represents a diazaheterobicyclic system of the formula 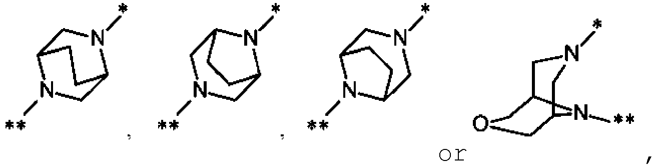
in which * denotes the bond to the adjacent CHR² group and ** the bond to the carbonyl group,
A represents CH,
R¹ represents fluorine, chlorine, bromine, methyl, isopropyl, *tert*-butyl, cyclopropyl or cyclobutyl,
R² represents hydrogen,
and
R³ represents cyclobutyl, cyclopentyl or cyclohexyl,
or
R³ represents a phenyl group of the formula (a), a pyridyl group of the formula (b) or an azole group of the formula (d), (e) or (f) 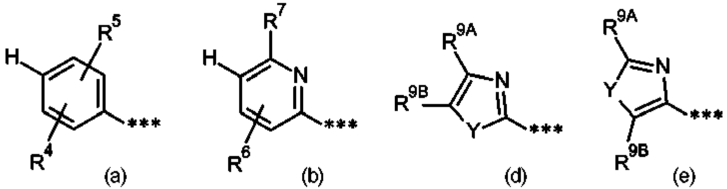 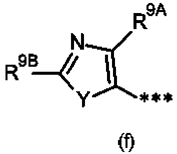
in which *** marks the bond to the adjacent carbonyl group and
R⁴ represents hydrogen, fluorine or chlorine,
R⁵ represents fluorine, chlorine, cyano, (C₁-C₃)-alkyl, (C₁-C₃)-alkoxy or trifluoromethoxy,
R⁶ represents hydrogen, fluorine, chlorine, bromine or methyl,
R⁷ represents (C₁-C₃)-alkoxy, cyclobutyloxy or (C₁-C₃)-alkylsulfanyl, where (C₁-C₃)-alkoxy may be up to trisubstituted by fluorine,
R^{9A} and R^{9B} are identical or different and independently of one another represent hydrogen, chlorine, bromine, (C₁-C₃)-alkyl or cyclopropyl,
where (C₁-C₃)-alkyl may be up to trisubstituted by fluorine,
and
Y represents O or S,
and the salts, solvates and solvates of the salts thereof.

3. Compound of the formula (I) according to Claim 1 or 2, in which
the ring Q represents a diazaheterobicyclic system of the formula 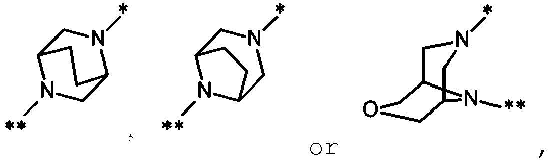
in which * denotes the bond to the adjacent CHR² group and ** the bond to the carbonyl group,
A represents CH,
R¹ represents chlorine, bromine, isopropyl or cyclopropyl,
R² represents hydrogen,
and
R³ represents cyclopentyl or cyclohexyl,
or
R³ represents a phenyl group of the formula (a), a pyridyl group of the formula (b) or an azole group of the formula (d), (e) or (f) 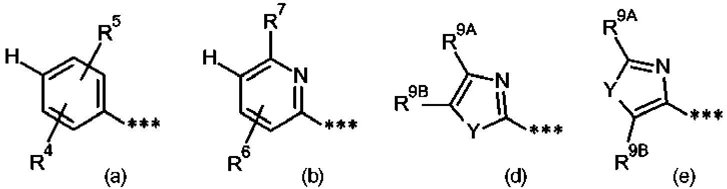 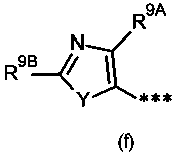
in which *** marks the bond to the adjacent carbonyl group and
R⁴ represents hydrogen, fluorine or chlorine,
R³ represents fluorine, chlorine, methyl, isopropyl, methoxy or ethoxy,
R⁶ represents hydrogen, fluorine, chlorine, bromine or methyl,
R⁷ represents methoxy, difluoromethoxy, trifluoromethoxy, isopropoxy, cyclobutyloxy or methylsulfanyl,
R^{9A} and R^{9B} are identical or different and independently of one another represent hydrogen, methyl, trifluoromethyl, ethyl, isopropyl or cyclopropyl,
and
Y represents O or S,
and the salts, solvates and solvates of the salts thereof.

4. Compound of the formula (I) according to Claim 1, with the systematic name (5{ [2- (4-chlorophenyl)imidazo[1,2-a]pyrimidin-3-yl]methyl}-2,5-diazabicyclo[2.2.2]-oct-2-yl)(3-fluoro-6-methoxypyridin-2-yl)methanone (enantiomer 2) and the structural formula

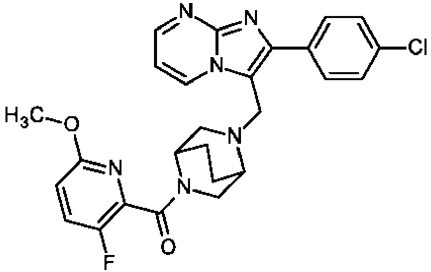

5. Compound of the formula (I) according to Claim 1, with the systematic name (3-fluoro-6-methoxypyridin-2-yl)(3-{[2-(4-isopropylphenyl)imidazo[1,2-a]pyrimidin-3-yl]methyl}-3,8-diazabicyclo[3.2.1]oct-8-yl)methanone and the structural formula

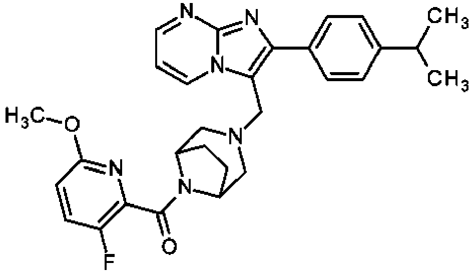

6. Compound of the formula (I) according to Claim 1, with the systematic name ((3-chloro-6-methoxypyridin-2-yl)(3-{[2-(4-isopropylphenyl)imidazo[1,2-a]pyrimidin-3-yl]methyl}-3,8-diazabicyclo[3.2.1]oct-8-yl)methanone and the structural formula

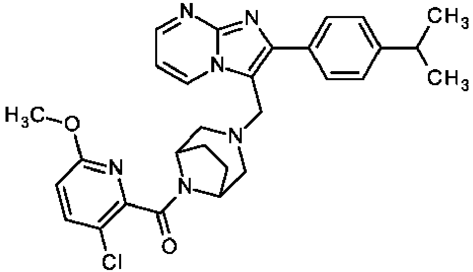

7. Compound of the formula (I) according to Claim 1, with the systematic name (3-chloro-6-methoxypyridin-2-yl)(5-{[2-(4-isopropylphenyl)imidazo[1,2-a]pyrimidin-3-yl]methyl}-2,5-diazabicyclo[2.2.2]oct-2-yl)methanone (enantiomer 2) and the structural formula

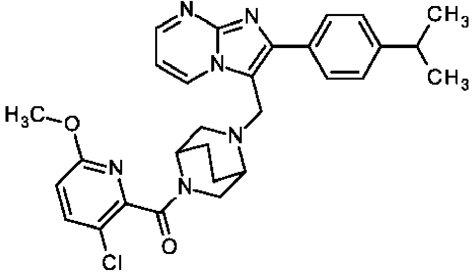

8. Compound of the formula (I) according to Claim 1, with the systematic name (3-chloro-6-methoxypyridin-2-yl)(3-{[2-(4-chlorophenyl)imidazo[1,2-a]pyrimidin-3-yl]methyl}-3,8-diazabicyclo[3.2.1]oct-8-yl)methanone and the structural formula

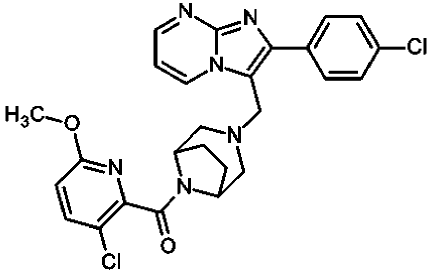

9. Process for preparing a compound of the formula (I) as defined in Claims 1 to 3 in which the radical R² represents hydrogen, **characterized in that** a compound of the formula (II) 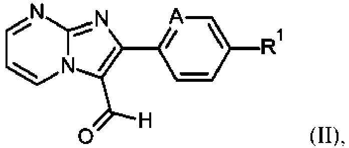 in which A and R¹ have the definitions given in Claims 1 to 3 is reacted in the presence of a suitable reducing agent either
[A] with a compound of the formula (III) 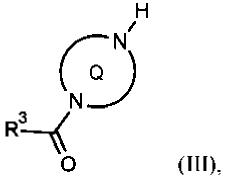
in which R³ and the ring Q have the definitions given in Claims 1 to 3
to give a compound of the formula (I-A) 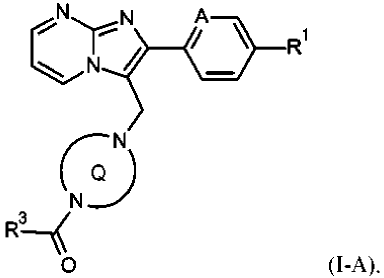
in which A, R¹, R³ and the ring Q have the meanings given above
or
[B] with a protected diazaheterobicyclic system of the formula (IV) 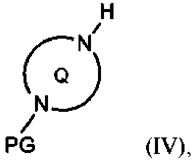
in which the ring Q has the definition given in Claims 1 to 3
and
PG represents a suitable amino protecting group, for example *tert*-butoxycarbonyl, benzyloxycarbonyl or (9H-fluoren-9-ylmethoxy) carbonyl
at first to give a compound of the formula (V) 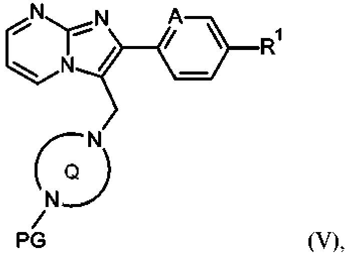
in which A, PG, R¹ and the ring Q have the meanings given above,
then the protecting group PG is cleaved and the resulting compound of the formula (VI) 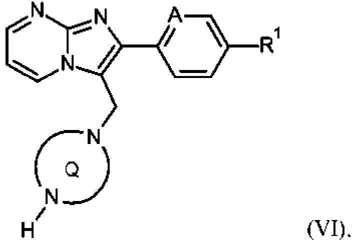
in which A, R¹ and the ring Q have the meanings given above is then reacted, depending on the specific definition of the R³ radical,
[B-1] with a carboxylic acid of the formula (VII) 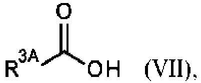 in which
R^{3A} represents (C₄-C₆)-cycloalkyl in which a ring CH₂ group may be replaced by -O-, or represents a phenyl group of the formula (a), a pyridyl group of the formula (b) or (c) or an azole group of the formula (d), (e) or (f), as described in Claims 1 to 3,
with activation of the carboxylic acid function in (VII), or is reacted with the corresponding acid chloride of the formula (VIII) 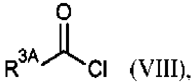
in which R^{3A} has the meaning given above to give a compound of the formula (I-B) 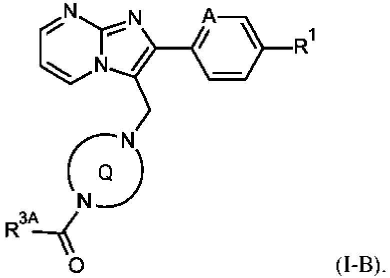
in which A, R¹, R^{3A} and the ring Q have the meanings given above
or
[B-2] with a chloroformate or carbamoyl chloride of the formula (IX) 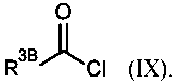 in which
R^{3B} represents the -OR¹⁰ or -NR^{11A}R¹² group in which
R¹⁰ and R¹² have the definitions specified in Claims 1 to 3
and
R^{11A} has the definition of R¹¹ specified in Claims 1 to 3, but is not hydrogen,
to give a compound of the formula (I-C) 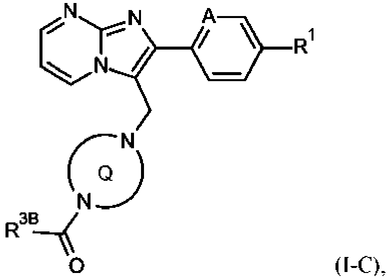
in which A, R¹, R^{3B} and the ring Q have the meanings given above
or
[B-3] with an isocyanate of the formula (X)
R¹²-N=C=O (X),
in which R¹² has the definition specified in Claims 1 to 3
to give a compound of the formula (I-D) 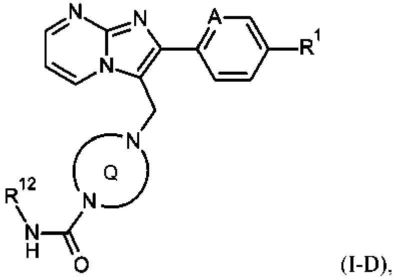
in which A, R¹, R¹² and the ring Q have the meanings given above
and the compounds of the formulae (I-A), (I-B), (I-C) and (I-D) thus obtained are optionally separated into their enantiomers and/or diastereomers and/or optionally converted with the appropriate (*i*) solvents and/or (*ii*) acids to the solvates, salts and/or solvates of the salts thereof.

10. Compound as defined in any of Claims 1 to 8 for treatment and/or prevention of diseases.

11. Compound as defined in any of Claims 1 to 8 for use in a method for the treatment and/or prevention of respiratory disorders, sleep-related respiratory disorders, obstructive sleep apnoeas, central sleep apnoeas, snoring, cardiac arrhythmias, neurodegenerative disorders, neuroinflammatory disorders and neuroimmunological disorders.

12. Use of a compound as defined in any of Claims 1 to 8 for preparing a medicament for the treatment and/or prevention of respiratory disorders, sleep-related respiratory disorders, obstructive sleep apnoeas, central sleep apnoeas, snoring, cardiac arrhythmias, neurodegenerative disorders, neuroinflammatory disorders and neuroimmunological disorders.

13. Medicament comprising a compound as defined in any of Claims 1 to 8 in combination with one or more inert, non-toxic, pharmaceutically suitable excipients.

14. Medicament comprising a compound as defined in any of Claims 1 to 8 in combination with one or more further active compounds selected from the group consisting of respiratory stimulants, psychostimulating compounds, serotonin re-uptake inhibitors, noradrenergic, serotonergic and tricyclic antidepressants, sGC stimulators, mineralocorticoid receptor antagonists, anti-inflammatory drugs, immunomodulators, immunosuppressives and cytotoxic drugs.

15. Medicament according to Claim 13 or 14 for treatment and/or prevention of respiratory disorders, sleep-related respiratory disorders, obstructive sleep apnoeas, central sleep apnoeas, snoring, cardiac arrhythmias, neurodegenerative disorders, neuroinflammatory disorders and neuroimmunological disorders.

## Revendications

1. Composé de formule (I) 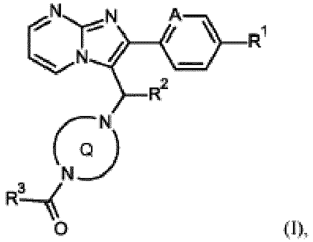 dans laquelle
le cycle Q représente un diaza-hétérobicycle de formule 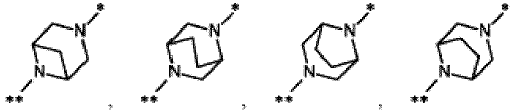 ou 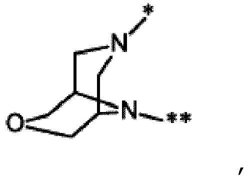
où * désigne la liaison au groupe CHR² adjacent et ** désigne la liaison au groupe carbonyle,
A représente CH ou N,
R¹ représente un atome d'halogène, un groupe cyano, alkyle en C₁-C₄, cyclopropyle ou cyclobutyle,
le groupe alkyle en C₁-C₄ pouvant être jusqu'à trois fois substitué par le fluor et les groupes cyclopropyle et cyclobutyle pouvant être jusqu'à deux fois substitués par le fluor,
R² représente un atome d'hydrogène ou un groupe méthyle, et
R³ représente un groupe cycloalkyle en C₄-C₆, dans lequel un groupe CH₂ formant le cycle peut être remplacé par -O-, ou
R³ représente un groupe phényle de formule (a), un groupe pyridyle de formule (b) ou (c) ou un groupe azole de formule (d), (e) ou (f), 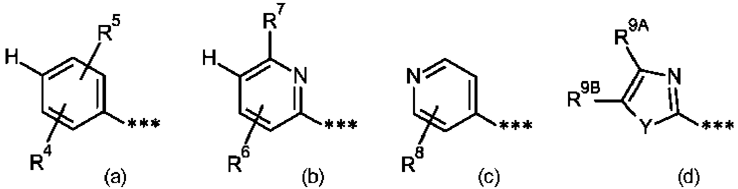 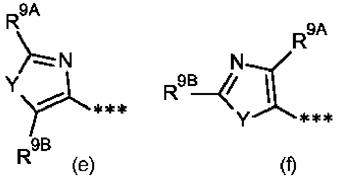
où *** désigne la liaison au groupe carbonyle adjacent et
R⁴ représente un atome d'hydrogène, de fluor, chlore, brome ou un groupe méthyle,
R⁵ représente un atome d'hydrogène, de fluor, chlore, brome, un groupe cyano, alkyle en C₁-C₃ ou alcoxy en C₁-C₃,
les groupes alkyle en C₁-C₃ et alcoxy en C₁-C₃ pouvant être substitués chacun jusqu'à trois fois par le fluor, R⁶ représente un atome d'hydrogène, de fluor, chlore, brome ou un groupe méthyle,
R⁷ représente un atome d'hydrogène, un groupe alcoxy en C₁-C₃, cyclobutyloxy, oxétan-3-yloxy, tétrahydrofuran-3-yloxy, tétrahydro-2*H*-pyran-4-yloxy, monoalkyl(C₁-C₃)-amino, dialkyl(C₁-C₃)-amino ou alkyl(C₁-C₃)-sulfanyle,
le groupe alcoxy en C₁-C₃ pouvant être jusqu'à trois fois substitué par le fluor,
R⁸ représente un atome d'hydrogène, de fluor, chlore, brome, un groupe alkyle en C₁-C₃ ou alcoxy en C₁-C₃,
R^{9A} et R^{9B} sont identiques ou différents et représentent indépendamment l'un de l'autre un atome d'hydrogène, de fluor, chlore, brome, un groupe alkyle en C₁-C₃, cyclopropyle ou alcoxy en C₁-C₃,
les groupes alkyle en C₁-C₃ et alcoxy en C₁-C₃ pouvant être substitués chacun jusqu'à trois fois par le fluor, et
Y représente O ou S,
ou
R³ représente un groupe -OR¹⁰ ou -NR¹¹R¹², où R¹⁰ représente un groupe alkyle en C₁-C₆, cycloalkyle en C₄-C₆ ou [cycloalkyl(C₃-C₆)]méthyle,
R¹¹ représente un atome d'hydrogène ou un groupe alkyle en C₁-C₃
et
R¹² représente un groupe alkyle en C₁-C₆, cycloalkyle en C₃-C₆, phényle ou benzyle,
le groupe alkyle en C₁-C₆ pouvant être jusqu'à trois fois substitué par le fluor
et
le groupe phényle ainsi que le groupe phényle dans le radical benzyle pouvant être jusqu'à trois fois substitués, de façon identique ou différente, par un radical choisi dans la série constituée par fluoro, chloro, méthyl, éthyle, trifluorméthyle, méthoxy, éthoxy, trifluorométhoxy et (trifluorométhyl)sulfanyle, ou
R¹¹ et R¹² sont liés l'un à l'autre et forment conjointement avec l'atome d'azote, auquel ils sont liés, un cycle pyrrolidine, pipéridine, morpholine ou thiomorpholine,
ainsi que ses sels, produits de solvatation et produits de solvatation des sels.

2. Composé de formule (I) selon la revendication 1, dans lequel
le cycle Q représente un diaza-hétérobicycle de formule 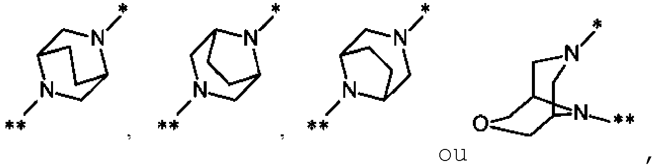
où * désigne la liaison au groupe CHR² adjacent et ** désigne la liaison au groupe carbonyle,
A représente CH,
R¹ représente un atome de fluor, chlore, brome, un groupe méthyle, isopropyle, *tert*.-butyle, cyclopropyle ou cyclobutyle,
R² représente un atome d'hydrogène,
et
R³ représente un groupe cyclobutyle, cyclopentyle ou cyclohexyle
ou
R³ représente un groupe phényle de formule (a), un groupe pyridyle de formule (b) ou un groupe azole de formule (d), (e) ou (f), 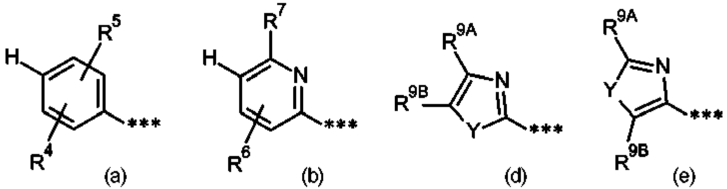 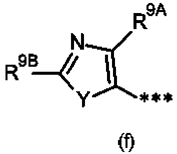
où *** désigne la liaison au groupe carbonyle adjacent et
R⁴ représente un atome d'hydrogène, de fluor ou de chlore, R⁵ représente un atome de fluor, de chlore, un groupe cyano, alkyle en C₁-C₃, alcoxy en C₁-C₃ ou trifluorométhoxy,
R⁶ représente un atome d'hydrogène, de fluor, chlore, brome ou un groupe méthyle,
R⁷ représente un groupe alcoxy en C₁-C₃, cyclobutyloxy ou alkyl(C₁-C₃)-sulfanyle, le groupe alcoxy en C₁-C₃ pouvant être jusqu'à trois fois substitué par le fluor,
R^{9A} et R^{9B} sont identiques ou différents et représentent indépendamment l'un de l'autre un atome d'hydrogène, de chlore, de brome, un groupe alkyle en C₁-C₃ ou cyclopropyle,
le groupe alkyle en C₁-C₃ pouvant être jusqu'à trois fois substitué par le fluor,
et
Y représente O ou S,
ainsi que ses sels, produits de solvatation et produits de solvatation des sels.

3. Composé de formule (I) selon la revendication 1 ou 2, dans laquelle
le cycle Q représente un diaza-hétérobicycle de formule 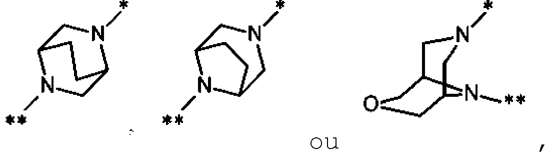
où * désigne la liaison au groupe CHR² adjacent et ** désigne la liaison au groupe carbonyle,
A représente CH,
R¹ représente un atome de chlore, de brome, un groupe isopropyle ou cyclopropyle,
R² représente un atome d'hydrogène,
et
R³ représente un groupe cyclopentyle ou cyclohexyle
ou
R³ représente un groupe phényle de formule (a), un groupe pyridyle de formule (b) ou un groupe azole de formule (d), (e) ou (f), 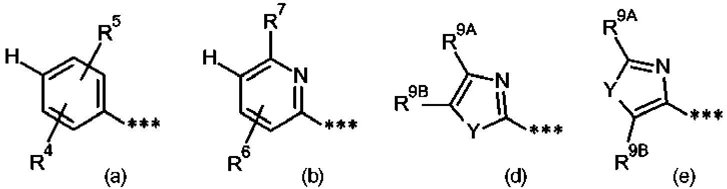 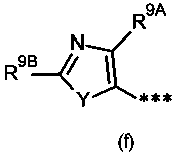
où *** désigne la liaison au groupe carbonyle adjacent et
R⁴ représente un atome d'hydrogène, de fluor ou de chlore, R⁵ représente un atome de fluor, de chlore, un groupe méthyle, isopropyle, méthoxy ou éthoxy,
R⁶ représente un atome d'hydrogène, de fluor, chlore, brome ou un groupe méthyle,
R⁷ représente un groupe méthoxy, difluorométhoxy, trifluorométhoxy, isopropoxy, cyclobutyloxy ou méthylsulfanyle,
R^{9A} et R^{9B} sont identiques ou différents et représentent indépendamment l'un de l'autre un atome d'hydrogène, un groupe méthyle, trifluorométhyle, éthyle, isopropyle ou cyclopropyle
et
Y représente O ou S,
ainsi que ses sels, produits de solvatation et produits de solvatation des sels.

4. Composé de formule (I) selon la revendication 1, ayant le nom systématique (5{[2-(4-chlorophényl)imidazo[1,2-a]pyrimidin-3-yl]méthyl}-2,5-diazabicyclo[2.2.2]-oct-2-yl)(3-fluoro-6-méthoxypyridin-2-yl)méthanone (énantiomère 2) et la formule développée

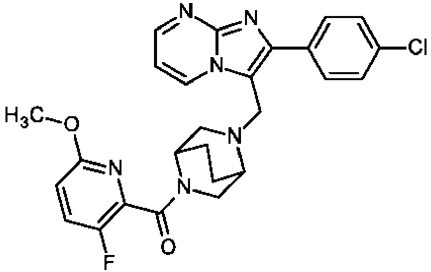

5. Composé de formule (I) selon la revendication 1, ayant le nom systématique (3-fluoro-6-méthoxypyridin-2-yl)(3-{[2-(4-isopropylphényl)imidazo[1,2-a]pyrimidin-3-yl]méthyl}-3,8-diazabicyclo[3.2.1]oct-8-yl)méthanone et la formule développée

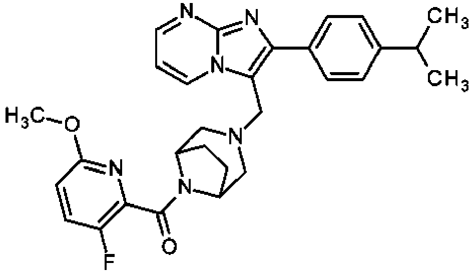

6. Composé de formule (I) selon la revendication 1, ayant le nom systématique ((3-chloro-6-méthoxypyridin-2-yl)(3-{[2-(4-isopropylphényl)imidazo[1,2-a]pyrimidin-3-yl]méthyl}-3,8-diazabicyclo[3.2.1]oct-8-yl)méthanone et la formule développée

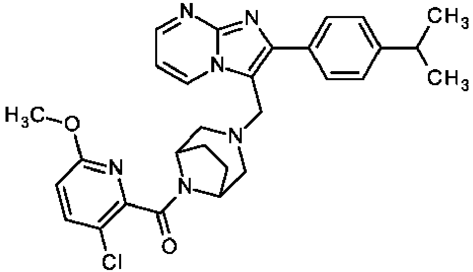

7. composé de formule (I) selon la revendication 1, ayant le nom systématique (3-chloro-6-méthoxypyridin-2-yl)(5-{[2-(4-isopropylphényl)imidazo[1,2-a]pyrimidin-3-yl]méthyl}-2,5-diazabicyclo[2.2.2]oct-2-yl)méthanone (énantiomère 2) et la formule développée

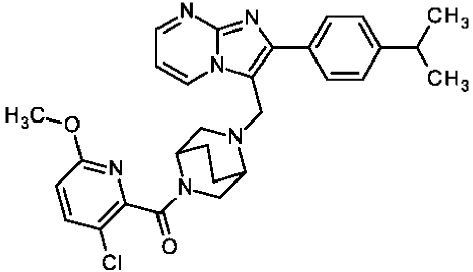

8. Composé de formule (I) selon la revendication 1, ayant le nom systématique (3-chloro-6-méthoxypyridin-2-yl)(3-{[2-(4-chlorophényl)imidazo[1,2-a]pyrimidin-3-yl]méthyl}-3,8-diazabicyclo[3.2.1]oct-8-yl)méthanone et la formule développée

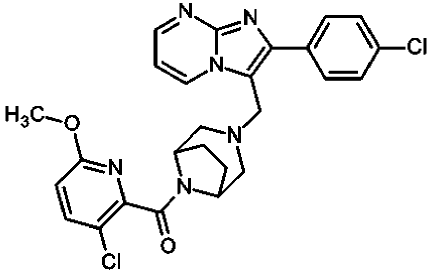

9. Procédé pour la préparation d'un composé de formule (I), telle que définie dans les revendications 1 à 3, dans laquelle le radical R² représente un atome d'hydrogène, **caractérisé en ce qu'**on fait réagir un composé de formule (II) 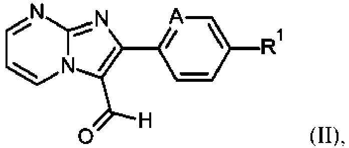 dans laquelle A et R¹ ont les significations indiquées dans les revendications 1 à 3, en présence d'un réducteur approprié, soit
[A] avec un composé de formule (III) 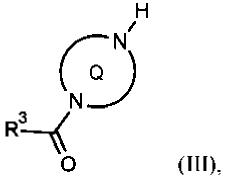
dans laquelle R³ et le cycle Q ont les significations indiquées dans les revendications 1 à 3,
pour aboutir à un composé de formule (I-A) 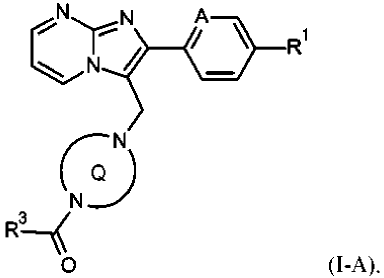
dans laquelle A, R¹, R³ et le cycle Q ont les significations indiquées plus haut
ou
[B] avec un diaza-hétérobicycle protégé de formule (IV) 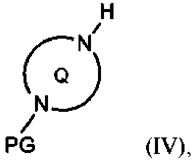
dans laquelle le cycle Q a la signification indiquée dans les revendications 1 à 3
et
PG représente un groupe protecteur de fonction amino approprié, comme par exemple le groupe tert.-butoxycarbonyle, benzyloxycarbonyle ou (9H-fluorén-9-ylméthoxy)carbonyle,
d'abord pour aboutir à un composé de formule (V) 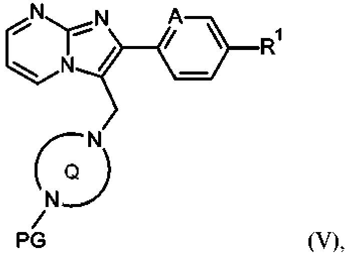
dans laquelle A, PG, R¹ et le cycle Q ont les significations indiquées plus haut,
ensuite on élimine le groupe protecteur PG et on fait alors réagir le composé résultant de formule (VI) 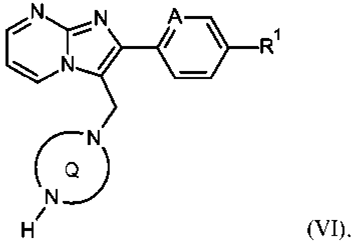
dans laquelle A, R¹ et le cycle Q ont les significations indiquées plus haut, en fonction de la signification spécifique du radical R³
[B-1] avec un acide carboxylique de formule (VII) 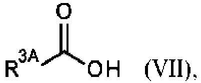 dans laquelle
R^{3A} représente un groupe cycloalkyle en C₄-C₆, dans lequel un groupe CH₂ formant le cycle peut être remplacé par -O-, ou représente un groupe phényle de formule (a), un groupe pyridyle de formule (b) ou (c) ou un groupe azole de formule (d), (e) ou (f), comme décrit dans les revendications 1 à 3,
sous activation de la fonction acide carboxylique dans (VII) ou avec le chlorure d'acide correspondant de formule (VIII) 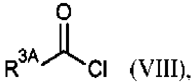
dans laquelle R^{3A} a la signification indiquée plus haut, pour aboutir à un composé de formule (I-B) 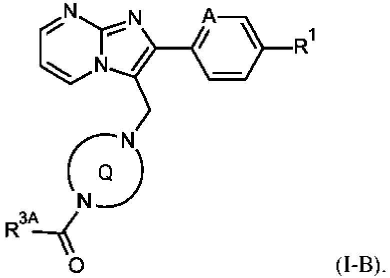
dans laquelle A, R¹, R^{3A} et le cycle Q ont les significations indiquées plus haut,
ou
[B-2] avec un chloroformiate ou chlorure de carbamoyle de formule (IX) 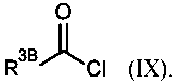 dans laquelle
R^{3B} représente le groupe -OR¹⁰ ou -NR^{11A}R¹², où R¹⁰ et R¹² ont les significations indiquées dans les revendications 1 à 3
et
R^{11A} a la signification de R¹¹ indiquée dans les revendications 1 à 3, mais est différent d'un atome d'hydrogène,
pour aboutir à un composé de formule (I-C) 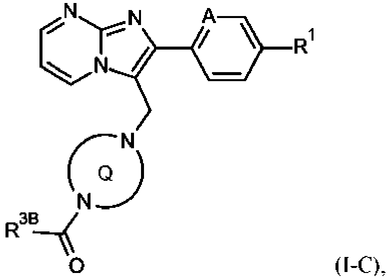
dans laquelle A, R¹, R^{3B} et le cycle Q ont les significations indiquées plus haut
ou
[B-3] avec un isocyanate de formule (X)
R¹²-N=C=O (X),
dans laquelle R¹² a la signification indiquée dans les revendications 1 à 3,
pour aboutir à un composé de formule (I-D) 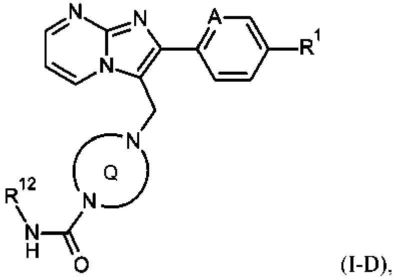
dans laquelle A, R¹, R¹² et le cycle Q ont les significations indiquées plus haut,
et éventuellement on fractionne les composés de formules (I-A), (I-B), (I-C) ou (I-D) ainsi obtenus en leurs énantiomères et/ou diastéréoisomères et/ou éventuellement on les convertit, à l'aide des (*i*) solvants et/ou *(ii)* acides correspondants, en leurs produits de solvatation, sels et/ou produits de solvatation des sels.

10. Composé, tel que défini dans l'une quelconque des revendications 1 à 8, destiné au traitement et/ou à la prévention de maladies.

11. Composé, tel que défini dans l'une quelconque des revendications 1 à 8, destiné à l'utilisation dans un procédé pour le traitement et/ou la prévention de troubles respiratoires, de troubles respiratoires liés au sommeil, d'apnées obstructives du sommeil, d'apnées centrales du sommeil, de ronflements, de troubles du rythme cardiaque, d'arythmies, de maladies neurodégénératives, maladies neuro-inflammatoires et maladies neuro-immunologiques.

12. Utilisation d'un composé, tel que défini dans l'une quelconque des revendications 1 à 8, pour la fabrication d'un médicament destiné au traitement et/ou à la prévention de troubles respiratoires, de troubles respiratoires liés au sommeil, d'apnées obstructives du sommeil, d'apnées centrales du sommeil, de ronflements, de troubles du rythme cardiaque, d'arythmies, de maladies neurodégénératives, maladies neuro-inflammatoires et maladies neuro-immunologiques.

13. Médicament contenant un composé tel que défini danss l'une quelconque des revendications 1 à 8, en association avec un ou plusieurs excipients inertes, non toxiques, pharmaceutiquement appropriés.

14. Médicament contenant un composé tel que défini dans l'une quelconque des revendications 1 à 8, en association avec un ou plusieurs autres principes actifs choisis dans le groupe constitué par les stimulants respiratoires, les composés psychostimulants, les inhibiteurs de la recapture de la sérotonine, les noradrénergiques, serotoninergiques et antidépresseurs tricycliques, les stimulants de la sGC, les antagonistes des récepteurs de minéralocorticoïdes, les agents à action anti-inflammatoire, les agents à action immunomodulatrice, les agents à action immunosuppressive et les agents à action cytotoxique.

15. Médicament selon la revendication 13 ou 14, destiné au traitement et/ou à la prévention de troubles respiratoires, de troubles respiratoires liés au sommeil, d'apnées obstructives du sommeil, d'apnées centrales du sommeil, de ronflements, de troubles du rythme cardiaque, d'arythmies, de maladies neurodégénératives, maladies neuro-inflammatoires et maladies neuro-immunologiques.
